# EUROPEAN PATENT SPECIFICATION

(11) **EP 0 743 938 B1**
(45) Date of publication and mention of the grant of the patent: **21.04.1999**
(21) Application number: 95909447.5
(22) Date of filing: 07.02.1995
(51) Int. Cl.: C07C 317/14, C07C 317/22, C07C 311/16, C07C 311/29, C07C 323/65, C07D 213/56, C07D 213/52, C07D 213/61, C07D 213/70, A61K 31/18, A61K 31/10

(54) **SUBSTITUTED SPIRO COMPOUNDS FOR THE TREATMENT OF INFLAMMATION**
SUBSTITUIERTE SPIROVERBINDUNGEN ZUR BEHANDLUNG VON ENTZÜNDUNGEN
COMPOSES SPIRANNIQUES SUBSTITUES UTILISES DANS LE TRAITEMENT DES INFLAMMATIONS

(30) Priority: 10.02.1994 US 194762; 18.11.1994 US 341734
(43) Date of publication of application: 27.11.1996
(73) Proprietor: G.D. SEARLE & CO., Chicago IL 60680-5110 (US)
(72) Inventor: REITZ, David, B., Chesterfield, MO 63017 (US); MANNING, Robert, E., St. Louis, MO 63019 (US); HUANG, Horng-Chi, Chesterfield, MO 63017 (US); LI, Jinglin, Chesterfield, MO 63017 (US)
(74) Representative: Beil, Hans Christoph, Dr,
(86) International application number: US9501385
(87) International publication number: WO9521817

(56) References cited:
- US-A- 5 344 991
- GENERAL PHARMACOLOGY, vol. 24,no. 1, January 1993 OXFORD, GB, pages 105-110, N. FUTAKI, ET AL.: 'NS-398, a novel non-steroidal anti-inflammatory drug with potent analgesic and antipyretic effects, which causes minimal stomach lesions'
- JOURNAL OF BIOLOGICAL CHEMISTRY, vol. 268,no. 9, 25 March 1993 BALTIMORE, MD, US, pages 6610-6614, E.A. MEADE, ET AL.: 'Differential inhibition of prostoglandin endoperoxide synthetase (cyclooxygenase) isoenzymes by aspirin and other non-steroidal anti-inflammatory drugs'

## Description

### FIELD OF THE INVENTION

This invention is in the field of antiinflammatory pharmaceutical agents and specifically relates to compounds, compositions and methods for treating inflammation and inflammation-associated disorders, such as arthritis.

### BACKGROUND OF THE INVENTION

Prostaglandins play a major role in the inflammation process and the inhibition of prostaglandin production, especially production of PGG₂, PGH₂ and PGE_{2,} has been a common target of antiinflammatory drug discovery. However, common non-steroidal antiinflammatory drugs (NSAIDs) that are active in reducing the prostaglandin-induced pain and swelling associated with the inflammation process are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved.

Previous NSAIDs have been found to prevent the production of prostaglandins by inhibiting enzymes in the human arachidonic acid/prostaglandin pathway, including the enzyme cyclooxygenase (COX). The recent discovery of an inducible enzyme associated with inflammation (named "cyclooxygenase-2 (COX-2)" or "prostaglandin G/H synthase II") provides a viable target of inhibition which more effectively reduces inflammation and produces fewer and less drastic side effects.

The substituted spiro compounds disclosed herein preferably selectively inhibit cyclooxygenase-2 over cyclooxygenase-1 and relieve the effects of inflammation. These compounds, in addition, do not display substantial inhibition of cyclooxygenase-1 and produce a reduced amount of side effects.

Diarylcycloalkenes have been made and used for a variety of utilities. For example, Offenlegungsschrift 4,212,628, published Oct. 21, 1993, describes 1,2-bis(4-alkylphenyl)cyclohex-1-ene compounds as having anti-tumor activity. 2,3-Bis-(4-hydroxyphenyl)-2-cyclopenten-1-one has been identified from the knot resin powder of Arqaucaria angustifolia [H. Ohash, et al., Phytochemistry, 31, 1371-73 (1992)].

Substituted 1,2-diphenylcyclopentenes have been synthesized for use in studies of their rotational behavior, and specifically, 1-(2,4-dimethylphenyl)-2-phenylcyclopentene [D. Y. Curtin, et al., J. Org. Chem., 36, 565-72 (1971)]. 1,2-Di-(2'-methoxyphenyl)-Δ¹-cyclopentene has been identified as an impurity in the synthesis of cannabinoids [O.P. Malik, et al., Ind. J. Chem., 14B, 975-78 (1976)].

1-(Substitutedphenyl)-2-phenylcyclopentenes have been synthesized to study their photochemical reactions into phenanthrene derivatives. Compounds with meta substituents, such as 1-(3-chlorophenyl)-2-phenylcyclopentene, are described in Somers, et al., J. Photochem. Photobiol., 48A, 353-74 (1989). Para substituents, including specifically 1-(4-fluorophenyl)-2-phenylcyclopentene, are described in Laarhoven, Pure & Appl. Chem., 56, 1225-40 (1984).

U.S. Patent No. 3,214,470 to Grogan describes aminospiroalkanes as having anesthetic properties.

The synthesis of 7,8-diphenyl-1,4-dioxaspiro[4.4]non-7-ene is described as an intermediate for forming 1,5-diphenylbicyclo[3.1.0]hexan-3-ol [E. J. Corey, et al., J. Amer. Chem. Soc., 85, 1788-1792 (1963)]. U.S. Patent No. 3,728,404 to Kubicek describes a method to make spiro compounds, and specifically 1,1-dichloro-2,2,5-triphenylspiro[2.4]hept-5-ene.

The invention's spiro compounds are found to show usefulness in vivo as antiinflammatory agents with minimal side effects.

### DESCRIPTION OF THE INVENTION

A class of substituted spiro compounds useful in treating inflammation-related disorders is defined by Formula I: 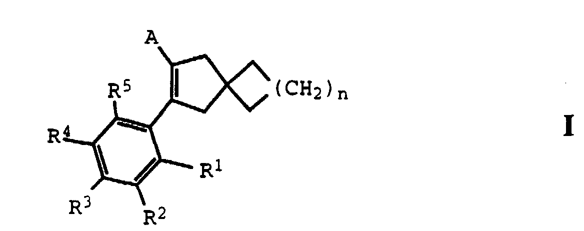 wherein A is selected from 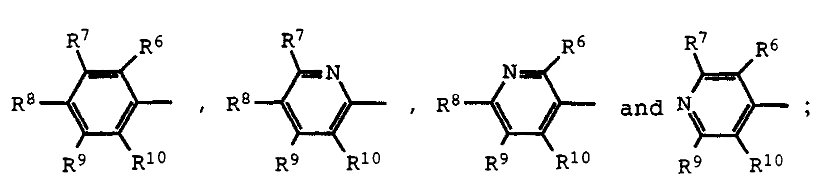 wherein each of R¹ through R¹⁰, if present, is independently selected from hydrido, halo, alkyl, alkoxy, alkylthio, cyano, haloalkyl, haloalkoxy, hydroxyalkyl, alkoxyalkyl, hydroxyl, mercapto, alkylamino, alkylsulfonyl, haloalkylsulfonyl and aminosulfonyl; and wherein n is a number selected from 0, 1, 2 and 3; or a pharmaceutically-acceptable salt thereof.

Compounds of Formula I would be useful for, but not limited to, the treatment of inflammation in a subject, and for treatment of other inflammation-associated disorders, such as, as an analgesic in the treatment of pain and headaches, or as an antipyretic for the treatment of fever. For example, compounds of Formula I would be useful to treat arthritis, including but not limited to rheumatoid arthritis, spondyloarthopathies, gouty arthritis, osteoarthritis, systemic lupus erythematosus and juvenile arthritis. Such compounds of Formula I would be useful in the treatment of asthma, bronchitis, menstrual cramps, tendinitis, bursitis, and skin related conditions such as psoriasis, eczema, burns and dermatitis. Compounds of Formula I also would be useful to treat gastrointestinal conditions such as inflammatory bowel disease, Crohn's disease, gastritis, irritable bowel syndrome and ulcerative colitis and for the prevention of colorectal cancer. Compounds of Formula I would be useful in treating inflammation in such diseases as vascular diseases, migraine headaches, periarteritis nodosa, thyroiditis, aplastic anemia, Hodgkin's disease, sclerodoma, rheumatic fever, type I diabetes, myasthenia gravis, sarcoidosis, nephrotic syndrome, Behcet's syndrome, polymyositis, gingivitis, hypersensitivity, conjunctivitis, swelling occurring after injury, myocardial ischemia, and the like. The compounds are useful as antiinflammatory agents, such as for the treatment of arthritis, with the additional benefit of having significantly less harmful side effects.

The present invention also includes compounds which selectively inhibit cyclooxygenase-2 over cyclooxygenase-1 and do not significantly inhibit one or more other arachidonic pathway steps, such as thromboxane B₂ (TXB2) production.

The present compounds may also be used in co-therapies, partially or completely, in place of other conventional antiinflammatories, such as together with steroids, NSAIDs, 5-lipoxygenase inhibitors, LTB₄ inhibitors and LTA₄ hydrolase inhibitors.

More preferably, the compounds also have a selectivity ratio of cyclooxygenase-2 inhibition over cyclooxygenase-1 inhibition of at least 50, and preferably of at least 100. Even more preferably, the compounds have a cyclooxygenase-1 IC₅₀ of greater than about 0.5 µM, and more preferably of greater than 5 µM. Such preferred selectivity may indicate an ability to reduce the incidence of common NSAID-induced side effects, such as ulcers.

A preferred class of compounds consists of those compounds of Formula I wherein, if present, each of R¹, R², R⁴ through R⁷, R⁹ and R¹⁰ is independently selected from hydrido, halo, lower alkyl, lower alkoxy, lower alkylthio, cyano, lower haloalkyl, lower haloalkoxy, lower hydroxyalkyl, lower alkoxyalkyl, hydroxyl and mercapto; and wherein R³ is selected from lower alkylsulfonyl, lower haloalkylsulfonyl and aminosulfonyl, and R⁸, if present, is selected from hydrido, halo, lower alkyl, lower alkoxy, lower alkylthio, cyano, lower haloalkyl, lower haloalkoxy, lower hydroxyalkyl, lower alkoxyalkyl, lower alkylamino, hydroxyl and mercapto; or wherein further R⁸ and R⁹, if present, together form methylenedioxy; or wherein further, R³ is selected from hydrido, halo, lower alkyl, lower alkoxy, lower alkylthio, cyano, lower haloalkyl, lower haloalkoxy, lower hydroxyalkyl, lower alkoxyalkyl, lower alkylamino, hydroxyl and mercapto, and R⁸ is selected from lower alkylsulfonyl, lower haloalkylsulfonyl and aminosulfonyl; or wherein further R³ and R⁴, if present, together form methylenedioxy; or a pharmaceutically-acceptable salt thereof.

A class of compounds of particular interest consists of those compounds of Formula I wherein, if present, each of R¹, R², R⁴ through R⁷, R⁹ and R¹⁰ is independently selected from hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl; and wherein R³ is selected from methylsulfonyl, fluoromethylsulfonyl, difluoromethylsulfonyl, trifluoromethylsulfonyl and aminosulfonyl, and R⁸, if present, is selected from hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methylamino, N,N-dimethylamino, trifluoromethoxy, hydroxymethyl, methoxymethyl and ethoxymethyl; or wherein further R⁸ and R⁹, if present, together form methylenedioxy; or wherein further R³ is selected from hydrido, fluoro, chloro; bromo, iodo, methyl, ethyl, n-propyl, isopropyl, butyl, *tert*-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl, and R⁸ is selected from methylsulfonyl, fluoromethylsulfonyl, difluoromethylsulfonyl, trifluoromethylsulfonyl and aminosulfonyl; or wherein further R³ and R⁴, if present, together form methylenedioxy; or a pharmaceutically-acceptable salt thereof.

Within Formula I there is a subclass of compounds of high interest wherein A is 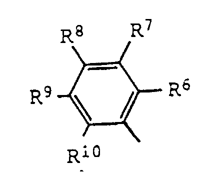 wherein each of R¹ through R¹⁰ is independently selected from hydrido, halo, alkyl, alkoxy, alkylthio, cyano, haloalkyl, haloalkoxy, hydroxyalkyl, alkoxyalkyl, hydroxyl, mercapto, alkylamino, alkylsulfonyl, haloalkylsulfonyl and aminosulfonyl; and wherein n is a number selected from 0, 1, 2 and 3; or a pharmaceutically-acceptable salt thereof.

A preferred class of compounds consists of those above compounds with A as indicated wherein n is a number selected from 0, 1 and 2; wherein each of R¹, R² and R⁴ through R¹⁰ is independently selected from hydrido, halo, lower alkyl, lower alkylamino, lower alkylthio, cyano, lower haloalkyl, lower haloalkoxy, lower alkoxy, hydroxyl, mercapto, lower hydroxyalkyl and lower alkoxyalkyl; and wherein R³ is selected from lower alkylsulfonyl, lower haloalkylsulfonyl and aminosulfonyl; or wherein R⁸ and R⁹ together form methylenedioxy; or a pharmaceutically-acceptable salt thereof.

A class of compounds of particular interest consists of those above compounds with A as indicated wherein each of R¹, R² and R⁴ through R¹⁰ is independently selected from hydrido, fluoro, chloro, bromo, iodo, methyl, ethyl, n-propyl, isopropyl, butyl, *tert*-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methylamino, N,N-dimethylamino, trifluoromethoxy, hydroxymethyl, methoxymethyl and ethoxymethyl; and wherein R³ is selected from methylsulfonyl, fluoromethylsulfonyl, difluoromethylsulfonyl, trifluoromethylsulfonyl and aminosulfonyl; or wherein R⁸ and R⁹ together form methylenedioxy; or a pharmaceutically-acceptable salt thereof.

A family of specific compounds of particular interest of the above compounds with A as indicated consists of compounds and pharmaceutically-acceptable salts thereof as follows:
5-[6-[4-(methylsulfonyl)phenyl]spiro[2.4]hept-5-en-5-yl]-1,3-benzodioxole;
2,6-dichloro-4-[6-[4-(methylsulfonyl)phenyl]spiro[2.4]hept-5-en-5-yl]phenol
5-(4-trifluoromethoxyphenyl)-6-[4-(methylsulfonyl)phenyl]spiro[2.4]hept-5-ene;
4-[6-(4-trifluoromethoxyphenyl)spiro[2.4]hept-5-en-5-yl]benzenesulfonamide;
5-(2,4-difluorophenyl)-6-[4-(methylsulfonyl)phenyl]spiro[2.4]hept-5-ene;
5-(2,4-dichlorophenyl)-6-[4-(methylsulfonyl)phenyl]spiro[2.4]hept-5-ene;
5-(3-chloro-4-methylphenyl)-6-[4-(methylsulfonyl)phenyl]spiro[2.4]hept-5-ene;
5-(3-methyl-4-trifluoromethoxyphenyl)-6-[4-(methylsulfonyl)phenyl]spiro[2.4]hept-5-ene;
5-(3-chloro-4-trifluoromethoxyphenyl)-6-[4-(methylsulfonyl)phenyl]spiro[2.4]hept-5-ene;
4-[6-(3-methyl-4-trifluoromethoxyphenyl)spiro[2.4]hept-5-en-5-yl]benzenesulfonamide;
4-[6-(3-chloro-4-trifluoromethoxyphenyl)spiro[2.4]hept-5-en-5-yl]benzenesulfonamide;
5-phenyl-6-[4-(methylsulfonyl)phenyl]spiro[2.4]hept-5-ene;
5-(4-fluorophenyl)-6-[4-(methylsulfonyl)phenyl] spiro[2.4]hept-5-ene;
5-(4-chlorophenyl)-6-[4-(methylsulfonyl)phenyl]spiro [2.4]hept-5-ene;
5-(4-bromophenyl)-6-[4-(methylsulfonyl)phenyl]spiro [2.4]hept-5-ene;
5-(4-iodophenyl)-6-[4-(methylsulfonyl)phenyl]spiro [2.4]hept-5-ene;
5-(4-methylphenyl)-6-[4-(methylsulfonyl)phenyl]spiro [2.4]hept-5-ene;
5-(4-ethylphenyl)-6-[4-(methylsulfonyl)phenyl]spiro [2.4]hept-5-ene;
5-(4-methoxyphenyl)-6-[4-(methylsulfonyl) phenyl]spiro[2.4]hept-5-ene;
5-(4-methylthiophenyl)-6-[4-(methylsulfonyl)phenyl] spiro[2.4]hept-5-ene;
5-(4-cyanophenyl)-6-[4-(methylsulfonyl)phenyl]spiro [2.4]hept-5-ene;
5-(4-trifluoromethylphenyl)-6-[4-(methylsulfonyl) phenyl]spiro[2.4]hept-5-ene;
5-(4-hydroxymethylphenyl)-6-[4-(methylsulfonyl) phenyl]spiro[2.4]hept-5-ene;
5-(4-methoxymethylphenyl)-6-[4-(methylsulfonyl) phenyl]spiro[2.4]hept-5-ene;
5-(4-hydroxyphenyl)-6-[4-(methylsulfonyl) phenyl]spiro[2.4]hept-5-ene;
5-(4-mercaptophenyl)-6-[4-(methylsulfonyl) phenyl]spiro[2.4]hept-5-ene;
5-[4-(N-methylamino)phenyl]-6-[4-(methylsulfonyl) phenyl]spiro[2.4]hept-5-ene;
5-[4-(N,N-dimethylamino)phenyl]-6-[4-(methylsulfonyl) phenyl]spiro[2.4]hept-5-ene;
4-(6-phenylspiro[2.] hept-5-en-5-yl) benzenesulfonamide;
4-[6-(4-fluorophenyl)spiro[2.4]hept-5-en-5-yl] benzenesulfonamide;
4-[6-(4-chlorophenyl)spiro[2.4]hept-5-en-5-yl] benzenesulfonamide;
4-[6-(4-bromophenyl)spiro[2.4]hept-5-en-5-yl] benzenesulfonamide;
4-[6-(4-iodophenyl)spiro[2.4]hept-5-en-5-yl] benzenesulfonamide;
4-[6-(4-methylphenyl)spiro[2.4]hept-5-en-5-yl] benzenesulfonamide;
4-[6-(4-ethylphenyl)spiro[2.4]hept-5-en-5-yl] benzenesulfonamide;
4-[6-(4-methoxyphenyl)spiro[2.4]hept-5-en-5-yl] benzenesulfonamide;
4-[6-(4-methylthiophenyl)spiro[2.4]hept-5-en-5-yl] benzenesulfonamide;
4-[6-(4-cyanophenyl)spiro[2.4]hept-5-en-5-yl] benzenesulfonamide;
4-[6-(4-trifluoromethylphenyl)spiro[2.4]hept-5-en-5-yl]benzenesulfonamide;
4-[6-(4-hydroxymethylphenyl)spiro[2.4]hept-5-en-5-yl]benzenesulfonamide;
4-[6-(4-methoxymethylphenyl)spiro[2.4]hept-5-en-5-yl]benzenesulfonamide;
4-[6-(4-hydroxyphenyl)spiro[2.4]hept-5-en-5-yl] benzenesulfonamide;
4-[6-(4-mercaptophenyl)spiro[2.4]hept-5-en-5-yl] benzenesulfonamide;
4-[6-[4-(N-methylamino)phenyl]spiro[2.4]hept-5-en-5-yl] benzenesulfonamide;
4-[6-[4-(N,N-dimethylamino)phenyl]spiro[2.4]hept-5-en-5-yl]benzenesulfonamide;
6-phenyl-7-[4-(methylsulfonyl)phenyl]spiro[3.4]oct-6-ene;
6-(4-fluorophenyl)-7-[4-(methylsulfonyl)phenyl] spiro[3.4]oct-6-ene;
6-(4-chlorophenyl)-7-[4-(methylsulfonyl)phenyl] spiro[3.4]oct-6-ene;
6-(4-bromophenyl)-7-[4-(methylsulfonyl)phenyl] spiro[3.4]oct-6-ene;
6-(4-iodophenyl)-7-[4-(methylsulfonyl)phenyl] spiro[3.4]oct-6-ene;
6-(4-methylphenyl)-7-[4-(methylsulfonyl)phenyl] spiro[3.4]oct-6-ene;
6-(4-ethylphenyl)-7-[4-(methylsulfonyl)phenyl] spiro[3.4]oct-6-ene;
6-(4-methoxyphenyl)-7-[4-(methylsulfonyl)phenyl] spiro[3.4]oct-6-ene;
6-(4-methylthiophenyl)-7-[4-(methylsulfonyl)phenyl] spiro[3.4]oct-6-ene;
6-(4-cyanophenyl)-7-[4-(methylsulfonyl)phenyl] spiro[3.4]oct-6-ene;
6-(4-trifluoromethylphenyl)-7-[4-(methylsulfonyl) phenyl]spiro[3.4]oct-6-ene;
6-(4-hydroxymethylphenyl)-7-[4-(methylsulfonyl) phenyl]spiro[3.4]oct-6-ene;
6-(4-methoxymethylphenyl)-7-[4-(methylsulfonyl) phenyl]spiro[3.4]oct-6-ene;
6-(4-hydroxyphenyl)-7-[4-(methylsulfonyl)phenyl] spiro[3.4]oct-6-ene;
6-(4-mercaptophenyl)-7-[4-(methylsulfonyl)phenyl] spiro[3.4]oct-6-ene;
4-(7-phenylspiro[3.4]oct-6-en-6-yl) benzenesulfonamide;
4-[7-(4-fluorophenyl)spiro[3.4]oct-6-en-6-yl] benzenesulfonamide;
4-[7-(4-chlorophenyl)spiro[3.4]oct-6-en-6-yl] benzenesulfonamide;
4-[7-(4-bromophenyl)spiro[3.4]oct-6-en-6-yl] benzenesulfonamide;
4-[7-(4-iodophenyl)spiro[3.4]oct-6-en-6-yl] benzenesulfonamide;
4-[7-(4-methylphenyl)spiro[3.4]oct-6-en-6-yl] benzenesulfonamide;
4-[7-(4-ethylphenyl)spiro[3.4]oct-6-en-6-yl] benzenesulfonamide;
4-[7-(4-methoxyphenyl)spiro[3.4]oct-6-en-6-yl] benzenesulfonamide;
4-[7-(4-methylthiophenyl)spiro[3.4]oct-6-en-6-yl] benzenesulfonamide;
4-[7-(4-cyanophenyl)spiro[3.4]oct-6-en-6-yl] benzenesulfonamide;
4-[7-(4-trifluoromethylphenyl)spiro[3.4]oct-6-en-6-yl]benzenesulfonamide;
4-[7-(4-hydroxymethylphenyl)spiro[3.4]oct-6-en-6-yl] benzenesulfonamide;
4-[7-(4-methoxymethylphenyl)spiro[3.4]oct-6-en-6-yl] benzenesulfonamide;
4-[7-(4-hydroxyphenyl)spiro[3.4]oct-6-en-6-yl] benzenesulfonamide;
4-[7-(4-mercaptophenyl)spiro[3.4]oct-6-en-6-yl] benzenesulfonamide;
2-phenyl-3-[4-(methylsulfonyl)phenyl]spiro[4.4]non-2-ene;
2-(4-fluorophenyl)-3-[4-(methylsulfonyl)phenyl] spiro[4.4]non-2-ene;
2-(4-chlorophenyl)-3-[4-(methylsulfonyl)phenyl] spiro[4.4]non-2-ene;
2-(4-bromophenyl)-3-[4-(methylsulfonyl)phenyl] spiro[4.4]non-2-ene;
2-(4-iodophenyl)-3-[4-(methylsulfonyl)phenyl] spiro[4.4]non-2-ene;
2-(4-methylphenyl)-3-[4-(methylsulfonyl)phenyl] spiro[4.4]non-2-ene;
2-(4-ethylphenyl)-3-[4-(methylsulfonyl)phenyl] spiro[4.4]non-2-ene;
2-(4-methoxyphenyl)-3-[4-(methylsulfonyl)phenyl] spiro[4.4]non-2-ene;
2-(4-methylthiophenyl)-3-[4-(methylsulfonyl)phenyl] spiro[4.4]non-2-ene;
2-(4-cyanophenyl)-3-[4-(methylsulfonyl)phenyl] spiro[4.4]non-2-ene;
2-(4-trifluoromethylphenyl)-3-[4-(methylsulfonyl) phenyl]spiro[4.4]non-2-ene;
2-(4-hydroxymethylphenyl)-3-[4-(methylsulfonyl) phenyl]spiro[4.4]non-2-ene;
2-(4-methoxymethylphenyl)-3-[4-(methylsulfonyl) phenyl]spiro[4.4]non-2-ene;
2-(4-hydroxyphenyl)-3-[4-(methylsulfonyl)phenyl] spiro[4.4]non-2-ene;
2-(4-mercaptophenyl)-3-[4-(methylsulfonyl)phenyl] spiro[4.4]non-2-ene;
4-(3-phenylspiro[4.4]non-2-en-2-yl) benzenesulfonamide;
4-[3-(4-fluorophenyl)spiro[4.4]non-2-en-2-yl] benzenesulfonamide;
4-[3-(4-chlorophenyl)spiro[4.4]non-2-en-2-yl] benzenesulfonamide;
4-[3-(4-bromophenyl)spiro[4.4]non-2-en-2-yl] benzenesulfonamide;
4-[3-(4-iodophenyl)spiro[4.4]non-2-en-2-yl] benzenesulfonamide;
4-[3-(4-methylphenyl)spiro[4.4]non-2-en-2-yl] benzenesulfonamide;
4-[3-(4-ethylphenyl)spiro[4.4]non-2-en-2-yl] benzenesulfonamide;
4-[3-(4-methoxyphenyl)spiro[4.4]non-2-en-2-yl] benzenesulfonamide;
4-[3-(4-methylthiophenyl)spiro[4.4]non-2-en-2-yl] benzenesulfonamide;
4-[3-(4-cyanophenyl)spiro[4.4]non-2-en-2-yl] benzenesulfonamide;
4-[3-(4-trifluoromethylphenyl)spiro[4.4]non-2-en-2-yl]benzenesulfonamide;
4-[3-(4-hydroxymethylphenyl)spiro[4.4]non-2-en-2-yl] benzenesulfonamide;
4-[3-(4-methoxymethylphenyl)spiro[4.4]non-2-en-2-yl] benzenesulfonamide;
4-[3-(4-hydroxyphenyl)spiro[4.4]non-2-en-2-yl] benzenesulfonamide;
4-[3-(4-mercaptophenyl)spiro[4.4]non-2-en-2-yl] benzenesulfonamide;
5-(3-trifluoromethyl-4-methylphenyl)-6-[4-(methylsulfonyl)phenyl]spiro[2.4]hept-5-ene;
5-(3-trifluoromethyl-4-fluorophenyl)-6-[4-(methylsulfonyl)phenyl]spiro[2.4]hept-5-ene;
5-(3-trifluoromethyl-4-chlorophenyl)-6-[4-(methylsulfonyl)phenyl]spiro[2.4]hept-5-ene;
5-(3-trifluoromethyl-4-bromophenyl)-6-[4-(methylsulfonyl)phenyl]spiro[2.4]hept-5-ene;
5-(3-methyl-4-fluorophenyl)-6-[4-(methylsulfonyl)phenyl]spiro[2.4]hept-5-ene;
5-(3-methyl-4-chlorophenyl)-6-[4-(methylsulfonyl)phenyl]spiro[2.4]hept-5-ene;
5-(3-methyl-4-bromophenyl)-6-[4-(methylsulfonyl)phenyl]spiro[2.4]hept-5-ene;
5-(3-methyl-4-trifluorophenyl)-6-[4-(methylsulfonyl)phenyl]spiro[2.4]hept-5-ene;
5-(3-trifluoromethyl-4-methoxyphenyl)-6-[4-(methylsulfonyl)phenyl]spiro[2.4]hept-5-ene;
5-(3-methyl-4-methoxyphenyl)-6-[4-(methylsulfonyl)phenyl]spiro[2.4]hept-5-ene;
5-(3-fluoro-4-methoxyphenyl)-6-[4-(methylsulfonyl)phenyl]spiro[2.4]hept-5-ene;
5-(3-chloro-4-methoxyphenyl)-6-[4-(methylsulfonyl)phenyl]spiro[2.4]hept-5-ene;
5-(3-bromo-4-methoxyphenyl)-6-[4-(methylsulfonyl)phenyl]spiro[2.4]hept-5-ene;
5-(2,3,4,5,6-pentafluorophenyl)-6-[4-(methylsulfonyl)phenyl]spiro[2.4]hept-5-ene;
5-(4-methoxy-2,3,5,6-tetrafluorophenyl)-6-[4-(methylsulfonyl)phenyl]spiro[2.4]hept-5-ene;
5-(2,3,5,6-tetrafluoro-4-trifluoromethylphenyl)-6-[4-(methylsulfonyl)phenyl]spiro[2.4]hept-5-ene;
5-(3,5-difluoro-4-methoxyphenyl)-6-[4-(methylsulfonyl)phenyl]spiro[2.4]hept-5-ene;
5-(3,5-dichloro-4-methoxyphenyl)-6-[4-(methylsulfonyl)phenyl]spiro[2.4]hept-5-ene;
5-(3,5-dibromo-4-methoxyphenyl)-6-[4-(methylsulfonyl)phenyl]spiro[2.4]hept-5-ene;
5-(2,3,4-trifluoro-4-methoxyphenyl)-6-[4-(methylsulfonyl)phenyl]spiro[2.4]hept-5-ene;
5-(2,3,4-trichloro-4-methoxyphenyl)-6-[4-(methylsulfonyl)phenyl]spiro[2.4]hept-5-ene;
5-(2,3,4-tribromo-4-methoxyphenyl)-6-[4-(methylsulfonyl)phenyl]spiro[2.4]hept-5-ene;
5-(2,4,5-trifluoro-4-methoxyphenyl)-6-[4-(methylsulfonyl)phenyl]spiro[2.4]hept-5-ene;
5-(2,4,5-trichloro-4-methoxyphenyl)-6-[4-(methylsulfonyl)phenyl]spiro[2.4]hept-5-ene;
5-(2,4,5-tribromo-4-methoxyphenyl)-6-[4-(methylsulfonyl)phenyl]spiro[2.4]hept-5-ene;
5-(3,4-dimethoxyphenyl)-6-[4-(methylsulfonyl)phenyl]spiro[2.4]hept-5-ene;
5-(3,4,5-trimethoxyphenyl)-6-[4-(methylsulfonyl)phenyl]spiro[2.4]hept-5-ene;
5-[3,4-bis(trifluoromethyl)phenyl]-6-[4-(methylsulfonyl)phenyl]spiro[2.4]hept-5-ene;
5-(3,4-dimethylphenyl)-6-[4-(methylsulfonyl)phenyl]spiro[2.4]hept-5-ene;
5-(3,4-difluorophenyl)-6-[4-(methylsulfonyl)phenyl]spiro[2.4]hept-5-ene;
5-(3,4-dichlorophenyl)-6-[4-(methylsulfonyl)phenyl]spiro[2.4]hept-5-ene;
5-(3,4-dibromophenyl)-6-[4-(methylsulfonyl)phenyl]spiro[2.4]hept-5-ene;
5-(3-chloro-4-fluorophenyl)-6-[4-(methylsulfonyl)phenyl]spiro[2.4]hept-5-ene;
5-(3-chloro-4-bromophenyl)-6-[4-(methylsulfonyl)phenyl]spiro[2.4]hept-5-ene;
5-(4-chloro-3-fluorophenyl)-6-[4-(methylsulfonyl)phenyl]spiro[2.4]hept-5-ene;
5-(4-chloro-3-bromophenyl)-6-[4-(methylsulfonyl)phenyl]spiro[2.4]hept-5-ene;
5-(3-trifluoromethyl-4-methylphenyl)-6-[4-(fluoromethylsulfonyl)phenyl]spiro[2.4]hept-5-ene;
5-(3-trifluoromethyl-4-fluorophenyl)-6-[4-(fluoromethylsulfonyl)phenyl]spiro[2.4]hept-5-ene;
5-(3-trifluoromethyl-4-chlorophenyl)-6-[4-(fluoromethylsulfonyl)phenyl]spiro[2.4]hept-5-ene;
5-(3-trifluoromethyl-4-bromophenyl)-6-[4-(fluoromethylsulfonyl)phenyl]spiro[2.4]hept-5-ene;
5-(3-methyl-4-fluorophenyl)-6-[4-(fluoromethylsulfonyl)phenyl]spiro[2.4]hept-5-ene;
5-(3-methyl-4-chlorophenyl)-6-[4-(fluoromethylsulfonyl)phenyl]spiro[2.4]hept-5-ene;
5-(3-methyl-4-bromophenyl)-6-[4-(fluoromethylsulfonyl)phenyl]spiro[2.4]hept-5-ene;
5-(3-methyl-4-trifluorophenyl)-6-[4-(fluoromethylsulfonyl)phenyl]spiro[2.4]hept-5-ene;
5-(3-trifluoromethyl-4-methoxyphenyl)-6-[4-(fluoromethylsulfonyl)phenyl]spiro[2.4]hept-5-ene;
5-(3-methyl-4-methoxyphenyl)-6-[4-(fluoromethylsulfonyl)phenyl]spiro[2.4]hept-5-ene;
5-(3-fluoro-4-methoxyphenyl)-6-[4-(fluoromethylsulfonyl)phenyl]spiro[2.4]hept-5-ene;
5-(3-chloro-4-methoxyphenyl)-6-[4-(fluoromethylsulfonyl)phenyl]spiro[2.4]hept-5-ene;
5-(3-bromo-4-methoxyphenyl)-6-[4-(fluoromethylsulfonyl)phenyl]spiro[2.4]hept-5-ene;
5-(2,3,4,5,6-pentafluorophenyl)-6-[4-(fluoromethylsulfonyl)phenyl]spiro[2.4]hept-5-ene;
5-(4-methoxy-2,3,5,6-tetrafluorophenyl)-6-[4-(fluoromethylsulfonyl)phenyl]spiro[2.4]hept-5-ene;
5-(2,3,5,6-tetrafluoro-4-trifluoromethylphenyl)-6-[4-(fluoromethylsulfonyl)phenyl]spiro[2.4]hept-5-ene;
5-(3,5-difluoro-4-methoxyphenyl)-6-[4-(fluoromethylsulfonyl)phenyl]spiro[2.4]hept-5-ene;
5-(3,5-dichloro-4-methoxyphenyl)-6-[4-(fluoromethylsulfonyl)phenyl]spiro[2.4]hept-5-ene;
5-(3,5-dibromo-4-methoxyphenyl)-6-[4-(fluoromethylsulfonyl)phenyl]spiro[2.4]hept-5-ene;
5-(2,3,4-trifluoro-4-methoxyphenyl)-6-[4-(fluoromethylsulfonyl)phenyl]spiro[2.4]hept-5-ene;
5-(2,3,4-trichloro-4-methoxyphenyl)-6-[4-(fluoromethylsulfonyl)phenyl]spiro[2.4]hept-5-ene;
5-(2,3,4-tribromo-4-methoxyphenyl)-6-[4-(fluoromethylsulfonyl)phenyl]spiro[2.4]hept-5-ene;
5-(2,4,5-trifluoro-4-methoxyphenyl)-6-[4-(fluoromethylsulfonyl)phenyl]spiro[2.4]hept-5-ene;
5-(2,4,5-trichloro-4-methoxyphenyl)-6-[4-(fluoromethylsulfonyl)phenyl]spiro[2.4]hept-5-ene;
5-(2,4,5-tribromo-4-methoxyphenyl)-6-[4-(fluoromethylsulfonyl)phenyl]spiro[2.4]hept-5-ene;
5-(3,4-dimethoxyphenyl)-6-[4-(fluoromethylsulfonyl) phenyl]spiro[2.4]hept-5-ene;
5-(3,4,5-trimethoxyphenyl)-6-[4-(fluoromethylsulfonyl) phenyl]spiro[2.4]hept-5-ene;
5-[3,4-bis(trifluoromethyl)phenyl]-6-[4-(fluoromethylsulfonyl)phenyl]spiro[2.4]hept-5-ene;
5-(3,4-dimethylphenyl)-6-[4-(fluoromethylsulfonyl) phenyl]spiro[2.4]hept-5-ene;
5-(3,4-difluorophenyl)-6-[4-(fluoromethylsulfonyl) phenyl]spiro[2.4]hept-5-ene;
5-(3,4-dichlorophenyl)-6-[4-(fluoromethylsulfonyl) phenyl]spiro[2.4]hept-5-ene;
5-(3,4-dibromophenyl)-6-[4-(fluoromethylsulfonyl) phenyl]spiro[2.4]hept-5-ene;
5-(3-chloro-4-fluorophenyl)-6-[4-(fluoromethylsulfonyl) phenyl]spiro[2.4]hept-5-ene;
5-(3-chloro-4-bromophenyl)-6-[4-(fluoromethylsulfonyl) phenyl]spiro[2.4]hept-5-ene;
5-(4-chloro-3-fluorophenyl)-6-[4-(fluoromethylsulfonyl) phenyl]spiro[2.4]hept-5-ene;
5-(4-chloro-3-bromophenyl)-6-[4-(fluoromethylsulfonyl) phenyl]spiro[2.4]hept-5-ene;
5-(3-trifluoromethyl-4-methylphenyl)-6-[4-(difluoromethylsulfonyl)phenyl]spiro[2.4]hept-5-ene;
5-(3-trifluoromethyl-4-fluorophenyl)-6-[4-(difluoromethylsulfonyl)phenyl]spiro[2.4]hept-5-ene;
5-(3-trifluoromethyl-4-chlorophenyl)-6-[4-(difluoromethylsulfonyl)phenyl]spiro[2.4]hept-5-ene;
5-(3-trifluoromethyl-4-bromophenyl)-6-[4-(difluoromethylsulfonyl)phenyl]spiro[2.4]hept-5-ene;
5-(3-methyl-4-fluorophenyl)-6-[4-(difluoromethylsulfonyl)phenyl]spiro[2.4]hept-5-ene;
5-(3-methyl-4-chlorophenyl)-6-[4-(difluoromethylsulfonyl)phenyl]spiro[2.4]hept-5-ene;
5-(3-methyl-4-bromophenyl)-6-[4-(difluoromethylsulfonyl)phenyl]spiro[2.4]hept-5-ene;
5-(3-methyl-4-trifluorophenyl)-6-[4-(difluoromethylsulfonyl)phenyl]spiro[2.4]hept-5-ene;
5-(3-trifluoromethyl-4-methoxyphenyl)-6-[4-(difluoromethylsulfonyl)phenyl]spiro[2.4]hept-5-ene;
5-(3-methyl-4-methoxyphenyl)-6-[4-(difluoromethylsulfonyl)phenyl]spiro[2.4]hept-5-ene;
5-(3-fluoro-4-methoxyphenyl)-6-[4-(difluoromethylsulfonyl)phenyl]spiro[2.4]hept-5-ene;
5-(3-chloro-4-methoxyphenyl)-6-[4-(difluoromethylsulfonyl)phenyl]spiro[2.4]hept-5-ene;
5-(3-bromo-4-methoxyphenyl)-6-[4-(difluoromethylsulfonyl)phenyl]spiro[2.4]hept-5-ene;
5-(2,3,4,5,6-pentafluorophenyl)-6-[4-(difluoromethylsulfonyl)phenyl]spiro[2.4]hept-5-ene;
5-(4-methoxy-2,3,5,6-tetrafluorophenyl)-6-[4-(difluoromethylsulfonyl)phenyl]spiro[2.4]hept-5-ene;
5-(2,3,5,6-tetrafluoro-4-trifluoromethylphenyl)-6-[4-(difluoromethylsulfonyl)phenyl]spiro[2.4]hept-5-ene;
5-(3,5-difluoro-4-methoxyphenyl)-6-[4-(difluoromethylsulfonyl)phenyl]spiro[2.4]hept-5-ene;
5-(3,5-dichloro-4-methoxyphenyl)-6-[4-(difluoromethylsulfonyl)phenyl]spiro[2.4]hept-5-ene;
5-(3,5-dibromo-4-methoxyphenyl)-6-[4-(difluoromethylsulfonyl)phenyl]spiro[2.4]hept-5-ene;
5-(2,3,4-trifluoro-4-methoxyphenyl)-6-[4-(difluoromethylsulfonyl)phenyl]spiro[2.4]hept-5-ene;
5-(2,3,4-trichloro-4-methoxyphenyl)-6-[4-(difluoromethylsulfonyl)phenyl]spiro[2.4]hept-5-ene;
5-(2,3,4-tribromo-4-methoxyphenyl)-6-[4-(difluoromethylsulfonyl)phenyl]spiro[2.4]hept-5-ene;
5-(2,4,5-trifluoro-4-methoxyphenyl)-6-[4-(difluoromethylsulfonyl)phenyl]spiro[2.4]hept-5-ene;
5-(2,4,5-trichloro-4-methoxyphenyl)-6-[4-(difluoromethylsulfonyl)phenyl]spiro[2.4]hept-5-ene;
5-(2,4,5-tribromo-4-methoxyphenyl)-6-[4-(difluoromethylsulfonyl)phenyl]spiro[2.4]hept-5-ene;
5-(3,4-dimethoxyphenyl)-6-[4-(difluoromethylsulfonyl) phenyl]spiro[2.4]hept-5-ene;
5-(3,4,5-trimethoxyphenyl)-6-[4-(difluoromethylsulfonyl) phenyl]spiro[2.4]hept-5-ene;
5-[3,4-bis(trifluoromethyl)phenyl]-6-[4-(difluoromethylsulfonyl)phenyl]spiro [2.4]hept-5-ene;
5-(3,4-dimethylphenyl)-6-[4-(difluoromethylsulfonyl) phenyl]spiro[2.4]hept-5-ene;
5-(3,4-difluorophenyl)-6-[4-(difluoromethylsulfonyl) phenyl]spiro[2.4]hept-5-ene;
5-(3,4-dichlorophenyl)-6-[4-(difluoromethylsulfonyl)phenyl]spiro[2.4]hept-5-ene;
5-(3,4-dibromophenyl)-6-[4-(difluoromethylsulfonyl)phenyl]spiro [2.4]hept-5-ene;
5-(3-chloro-4-fluorophenyl)-6-[4-(difluoromethylsulfonyl)phenyl]spiro[2.4]hept-5-ene;
5-(3-chloro-4-bromophenyl)-6-[4-(difluoromethylsulfonyl)phenyl]spiro[2.4]hept-5-ene;
5-(4-chloro-3-fluorophenyl)-6-[4-(difluoromethylsulfonyl)phenyl]spiro[2.4]hept-5-ene;
5-(4-chloro-3-bromophenyl)-6-[4-(difluoromethylsulfonyl)phenyl]spiro[2.4]hept-5-ene;
5-(3-trifluoromethyl-4-methylphenyl)-6-[4-(trifluoromethylsulfonyl)phenyl]spiro[2.4]hept-5-ene;
5-(3-trifluoromethyl-4-fluorophenyl)-6-[4-(trifluoromethylsulfonyl)phenyl]spiro[2.4]hept-5-ene;
5-(3-trifluoromethyl-4-chlorophenyl)-6-[4-(trifluoromethylsulfonyl)phenyl]spiro[2.4]hept-5-ene;
5-(3-trifluoromethyl-4-bromophenyl)-6-[4-(trifluoromethylsulfonyl)phenyl]spiro[2.4]hept-5-ene;
5-(3-methyl-4-fluorophenyl)-6-[4-(trifluoromethylsulfonyl)phenyl]spiro[2.4]hept-5-ene;
5-(3-methyl-4-chlorophenyl)-6-[4-(trifluoromethylsulfonyl)phenyl]spiro[2.4]hept-5-ene;
5-(3-methyl-4-bromophenyl)-6-[4-(trifluoromethylsulfonyl)phenyl]spiro[2.4]hept-5-ene;
5-(3-methyl-4-trifluorophenyl)-6-[4-(trifluoromethylsulfonyl)phenyl]spiro[2.4]hept-5-ene;
5-(3-trifluoromethyl-4-methoxyphenyl)-6-[4-(trifluoromethylsulfonyl)phenyl]spiro[2.4]hept-5-ene;
5-(3-methyl-4-methoxyphenyl)-6-[4-(trifluoromethylsulfonyl)phenyl]spiro[2.4]hept-5-ene;
5-(3-fluoro-4-methoxyphenyl)-6-[4-(trifluoromethylsulfonyl)phenyl]spiro[2.4]hept-5-ene;
5-(3-chloro-4-methoxyphenyl)-6-[4-(trifluoromethylsulfonyl)phenyl]spiro[2.4]hept-5-ene;
5-(3-bromo-4-methoxyphenyl)-6-[4-(trifluoromethylsulfonyl)phenyl]spiro[2.4]hept-5-ene;
5-(2,3,4,5,6-pentafluorophenyl)-6-[4-(trifluoromethylsulfonyl)phenyl]spiro[2.4]hept-5-ene;
5-(4-methoxy-2,3,5,6-tetrafluorophenyl)-6-[4-(trifluoromethylsulfonyl)phenyl]spiro[2.4]hept-5-ene;
5-(2,3,5,6-tetrafluoro-4-trifluoromethylphenyl)-6-[4-(trifluoromethylsulfonyl)phenyl]spiro[2.4]hept-5-ene;
5-(3,5-difluoro-4-methoxyphenyl)-6-[4-(trifluoromethylsulfonyl)phenyl]spiro[2.4]hept-5-ene;
5-(3,5-dichloro-4-methoxyphenyl)-6-[4-(trifluoromethylsulfonyl)phenyl]spiro[2.4]hept-5-ene;
5-(3,5-dibromo-4-methoxyphenyl)-6-[4-(trifluoromethylsulfonyl)phenyl]spiro[2.4]hept-5-ene;
5-(2,3,4-trifluoro-4-methoxyphenyl)-6-[4-(trifluoromethylsulfonyl)phenyl]spiro[2.4]hept-5-ene;
5-(2,3,4-trichloro-4-methoxyphenyl)-6-[4-(trifluoromethylsulfonyl)phenyl]spiro[2.4]hept-5-ene;
5-(2,3,4-tribromo-4-methoxyphenyl)-6-[4-(trifluoromethylsulfonyl)phenyl]spiro[2.4]hept-5-ene;
5-(2,4,5-trifluoro-4-methoxyphenyl)-6-[4-(trifluoromethylsulfonyl)phenyl]spiro[2.4]hept-5-ene;
5-(2,4,5-trichloro-4-methoxyphenyl)-6-[4-(trifluoromethylsulfonyl)phenyl]spiro[2.4]hept-5-ene;
5-(2,4,5-tribromo-4-methoxyphenyl)-6-[4-(trifluoromethylsulfonyl)phenyl]spiro[2.4]hept-5-ene;
5-(3,4-dimethoxyphenyl)-6-[4-(trifluoromethylsulfonyl)phenyl]spiro[2.4]hept-5-ene;
5-(3,4,5-trimethoxyphenyl)-6-[4-(trifluoromethylsulfonyl)phenyl]spiro[2.4]hept-5-ene;
5-[3,4-bis(trifluoromethyl)phenyl]-6-[4-(trifluoromethylsulfonyl)phenyl]spiro[2.4]hept-5-ene;
5-(3,4-dimethylphenyl)-6-[4-(trifluoromethylsulfonyl)phenyl]spiro[2.4]hept-5-ene;
5-(3,4-difluorophenyl)-6-[4-(trifluoromethylsulfonyl)pheny]spiro[2.4]hept-5-ene;
5-(3,4-dichlorophenyl)-6-[4-(trifluoromethylsulfonyl)phenyl]spiro[2.4]hept-5-ene;
5-(3,4-dibromophenyl)-6-[4-(trifluoromethylsulfonyl)phenyl]spiro[2.4]hept-5-ene;
5-(3-chloro-4-fluorophenyl)-6-[4-(trifluoromethylsulfonyl)phenyl]spiro[2.4]hept-5-ene;
5-(3-chloro-4-bromophenyl)-6-[4-(trifluoromethylsulfonyl)phenyl]spiro[2.4]hept-5-ene;
5-(4-chloro-3-fluorophenyl)-6-[4-(trifluoromethylsulfonyl)phenyl]spiro[2.4]hept-5-ene;
5-(4-chloro-3-bromophenyl)-6-[4-(trifluoromethylsulfonyl)phenyl]spiro[2.4] hept-5-ene;
4-[6-(3-methyl-4-fluorophenyl)spiro[2.4]hept-5-en-5-yl]benzenesulfonamide;
4-[6-(3-methyl-4-chlorophenyl)spiro[2.4]hept-5-en-5-yl]benzenesulfonamide;
4-[6-(3-methyl-4-bromophenyl)spiro[2.4]hept-5-en-5-yl]benzenesulfonamide;
4-[6-(3-methyl-4-trifluoromethylphenyl)spiro[2.4]hept-5-en-5-yl]benzenesulfonamide;
4-[6-(3-methyl-4-methoxyphenyl)spiro[2.4]hept-5-en-5-yl]benzenesulfonamide;
4-[6-(3-trifluoromethyl-4-fluorophenyl)spiro[2.4]hept-5-en-5-yl]benzenesulfonamide;
4-[6-(3-trifluoromethyl-4-chlorophenyl)spiro[2.4]hept-5-en-5-yl]benzenesulfonamide;
4-[6-(3-trifluoromethyl-4-bromophenyl)spiro[2.4]hept-5-en-5-yl]benzenesulfonamide;
4-[6-(3-trifluoromethyl-4-methylphenyl)spiro[2.4]hept-5-en-5-yl]benzenesulfonamide;
4-[6-(3-trifluoromethyl-4-methoxyphenyl)spiro[2.4]hept-5-en-5-yl]benzenesulfonamide;
4-[6-(3-fluoro-4-methoxyphenyl)spiro[2.4]hept-5-en-5-yl]benzenesulfonamide;
4-[6-(3-chloro-4-methoxyphenyl)spiro[2.4]hept-5-en-5-yl]benzenesulfonamide;
4-[6-(3-bromo-4-methoxyphenyl)spiro[2.4]hept-5-en-5-yl]benzenesulfonamide;
4-[6-(2,3,4,5,6-pentafluorophenyl)spiro[2.4]hept-5-en-5-yl]benzenesulfonamide;
4-[6-(4-methoxy-2,3,5,6-tetrafluorophenyl)spiro[2.4]hept-5-en-5-yl]benzenesulfonamide;
4-[6-(2,3,5,6-tetrafluoro-4-trifluoromethylphenyl)spiro[2.4]hept-5-en-5-yl]benzenesulfonamide;
4-[6-(3,5-difluoro-4-methoxyphenyl)spiro[2.4]hept-5-en-5-yl]benzenesulfonamide;
4-[6-(3,5-dichloro-4-methoxyphenyl)spiro[2.4]hept-5-en-5-yl]benzenesulfonamide;
4-[6-(3,5-dibromo-4-methoxyphenyl)spiro[2.4]hept-5-en-5-yl]benzenesulfonamide;
4-[6-(2,3,4-trifluoro-4-methoxyphenyl)spiro[2.4]hept-5-en-5-yl]benzenesulfonamide;
4-[6-(2,3,4-trichloro-4-methoxyphenyl)spiro[2.4]hept-5-en-5-yl]benzenesulfonamide;
4-[6-(2,3,4-tribromo-4-methoxyphenyl)spiro[2.4]hept-5-en-5-yl]benzenesulfonamide;
4-[6-(2,4,5-trifluoro-4-methoxyphenyl)spiro[2.4]hept-5-en-5-yl]benzenesulfonamide;
4-[6-(2,4,5-trichloro-4-methoxyphenyl)spiro[2.4]hept-5-en-5-yl]benzenesulfonamide;
4-[6-(2,4,5-tribromo-4-methoxyphenyl)spiro[2.4]hept-5-en-5-yl]benzenesulfonamide;
4-[6-(3,4-dimethoxyphenyl)spiro[2.4]hept-5-en-5-yl]benzenesulfonamide;
4-[6-(3,4,5-trimethoxyphenyl)spiro[2.4]hept-5-en-5-yl]benzenesulfonamide;
4-[6-[3,4-bis(trifluoromethyl)phenyl]spiro[2.4]hept-5-en-5-yl]benzenesulfonamide;
4-[6-(3,4-dimethylphenyl)spiro[2.4]hept-5-en-5-yl]benzenesulfonamide;
4-[6-(3,4-difluorophenyl)spiro[2.4]hept-5-en-5-yl]benzenesulfonamide;
4-[6-(3,4-dibromophenyl)spiro[2.4]hept-5-en-5-yl]benzenesulfonamide;
4-[6-(3-chloro-4-fluorophenyl)spiro[2.4]hept-5-en-5-yl]benzenesulfonamide;
4-[6-(3-chloro-4-bromophenyl)spiro[2.4]hept-5-en-5-yl]benzenesulfonamide;
4-[6-(4-chloro-3-fluorophenyl)spiro[2.4]hept-5-en-5-yl]benzenesulfonamide; and
4-[6-(4-chloro-3-bromophenyl)spiro[2.4]hept-5-en-5-yl]benzenesulfonamide.

Within Formula I there is a second subclass of compounds of high interest
wherein A is as defined above, and each of R¹, R², R⁴, R⁵, R¹⁰ is hydrogen;
wherein n is a number selected from 0, 1 and 2;
wherein R⁶ is selected from hydrido and halo;
wherein R⁷ is selected from hydrido and halo;
wherein R³ is selected from lower alkylsulfonyl and aminosulfonyl;
wherein R⁸ is selected from hydrido, halo, lower alkyl, lower alkoxy, lower haloalkyl, lower haloalkoxy, and hydroxyl;
wherein R⁹ is selected from hydrido, halo, and lower alkyl; or wherein R⁸ and R⁹ together form methylenedioxy;
or a pharmaceutically-acceptable salt thereof.

A class of compounds of particular interest consists of those above compounds wherein A is as indicated wherein R³ is methylsulfonyl or aminosulfonyl; wherein R⁶ is selected from hydrido, fluoro, chloro, bromo, and iodo; wherein R⁷ is selected from hydrido, fluoro, chloro, bromo, and iodo; wherein R⁸ is selected from hydrido, fluoro, chloro, bromo, iodo, methyl, ethyl, n-propyl, isopropyl, butyl, *tert*-butyl, isobutyl, methoxy, ethoxy, propoxy, butoxy, hydroxyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, and trifluoromethoxy; wherein R⁹ is selected from hydrido, fluoro, chloro, bromo, iodo, methyl, ethyl, n-propyl, isopropyl, butyl, *tert*-butyl, and isobutyl; or where R⁸ and R⁹ together form methylenedioxy; and wherein R¹¹ is methyl or amino; and or a pharmaceutically-acceptable salt thereof.

A family of specific compounds of particular interest consists of above compounds with A as indicated and pharmaceutically-acceptable salts thereof as follows:
5-(3-chloro-4-methoxyphenyl)-6-[4-(methylsulfonyl)phenyl]spiro[2.4]hept-5-ene;
4-[6-(3-chloro-4-methoxyphenyl)spiro[2.4]hept-5-en-5-yl]benzenesulfonamide;
5-(3-fluoro-4-methoxyphenyl)-6-[4-(methylsulfonyl)phenyl]spiro[2.4]hept-5-ene;
4-[6-(3-fluoro-4-methoxyphenyl)spiro[2.4]hept-5-en-5-yl]benzenesulfonamide;
5-(3,4-difluorophenyl)-6-[4-(methylsulfonyl)phenyl]spiro[2.4]hept-5-ene;
5-[6-[4-(methylsulfonyl)phenyl]spiro[2.4]hept-5-en-5-yl]-1,3-benzodioxole;
4-[6-(3,4-difluorophenyl)spiro[2.4]hept-5-en-5-yl]benzenesulfonamide;
2,6-dichloro-4-[6-[4-(methylsulfonyl)phenyl]spiro[2.4]hept-5-en-5-yl]phenol
5-(4-trifluoromethoxyphenyl)-6-[4-(methylsulfonyl)phenyl]spiro[2.4]hept-5-ene;
5-(4-methoxyphenyl)-6-[4-(methylsulfonyl)phenyl]spiro[2.4]hept-5-ene;
5-(3-bromo-4-methoxyphenyl)-6-[4-(methylsulfonyl)phenyl]spiro[2.4]hept-5-ene;
4-[6-(4-methoxyphenyl)spiro[2.4]hept-5-en-5-yl]benzenesulfonamide;
4-[6-(3-bromo-4-methoxyphenyl)spiro[2.4]hept-5-en-5-yl]benzenesulfonamide;
5-(4-trifluoromethylphenyl)-6-[4-(methylsulfonyl)phenyl]spiro[2.4]hept-5-ene;
5-(3,5-dichloro-4-methoxy-phenyl)-6-[4-(methylsulfonyl)phenyl]spiro[2.4]hept-5-ene;
4-[6-(4-trifluoromethoxyphenyl)spiro[2.4]hept-5-en-5-yl]benzenesulfonamide;
5-(3-chloro-4-fluorophenyl)-6-[4-(methylsulfonyl)phenyl]spiro[2.4]hept-5-ene;
5-(2,4-difluorophenyl)-6-[4-(methylsulfonyl)phenyl]spiro[2.4]hept-5-ene;
5-(2,4-dichlorophenyl)-6-[4-(methylsulfonyl)phenyl]spiro[2.4]hept-5-ene;
4-[6-(4-trifluoromethylphenyl)spiro[2.4]hept-5-en-5-yl]benzenesulfonamide;
4-[6-(3-chloro-4-fluorophenyl)spiro[2.4]hept-5-en-5-yl]benzenesulfonamide;
5-(3,4-dichlorophenyl)-6-[4-(methylsulfonyl)phenyl]spiro[2.4]hept-5-ene;
5-(4-chlorophenyl)-6-[4-(methylsulfonyl)phenyl]spiro[2.4]hept-5-ene;
4-[6-(3,4-dichlorophenyl)spiro[2.4]hept-5-en-5-yl]benzenesulfonamide;
4-[6-(4-chlorophenyl)spiro[2.4]hept-5-en-5-yl]benzenesulfonamide;
5-(3-chloro-4-methylphenyl)-6-[4-(methylsulfonyl)phenyl]spiro[2.4]hept-5-ene;
5-(3,4-dimethylphenyl)-6-[4-(methylsulfonyl)phenyl]spiro[2.4]hept-5-ene;
5-(4-methylphenyl)-6-[4-(methylsulfonyl)phenyl]spiro[2.4]hept-5-ene;
5-(3-methyl-4-trifluoromethoxyphenyl)-6-[4-(methylsulfonyl)phenyl]spiro[2.4]hept-5-ene;
5-(3-chloro-4-trifluoromethoxyphenyl)-6-[4-(methylsulfonyl)phenyl]spiro[2.4]hept-5-ene;
4-[6-(3,5-dichloro-4-methoxyphenyl)spiro[2.4]hept-5-en-5-yl]benzenesulfonamide;
4-[6-(3-methyl-4-trifluoromethoxyphenyl)spiro[2.4]hept-5-en-5-yl]benzenesulfonamide;
4-[6-(3-chloro-4-trifluoromethoxyphenyl)spiro[2.4]hept-5-en-5-yl]benzenesulfonamide;
5-(4-fluorophenyl)-6-[4-(methylsulfonyl)phenyl]spiro[2.4]hept-5-ene;
4-[6-(4-fluorophenyl)spiro[2.4]hept-5-en-5-yl]benzenesulfonamide;
6-(4-fluorophenyl)-7-[4-(methylsulfonyl)phenyl] spiro[3.4]oct-5-ene;
4-[7-(4-fluorophenyl)spiro[3.4]oct-6-en-6-yl] benzenesulfonamide; and
2-(4-fluorophenyl)-3-[4-(methylsulfonyl)phenyl] spiro[4.4]non-5-ene.

Within Formula I there is a third subclass of compounds of high interest wherein A is 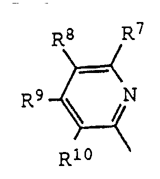
wherein n is a number selected from 0, 1, 2 and 3; and
wherein each of R¹ through R⁵ and R⁷ through R¹⁰ is independently selected from hydrido, halo, alkyl, alkoxy, alkylthio, cyano, haloalkyl, haloalkoxy, alkylamino, hydroxyalkyl, alkoxyalkyl, hydroxyi, mercapto, alkylsulfonyl, haloalkylsulfonyl and aminosulfonyl; or a pharmaceutically-acceptable salt thereof.

A preferred class of compounds consists of those compounds as shown above with A as indicated wherein n is a number selected from 0, 1 and 2; wherein each of R¹, R², R⁴, R⁵, R⁷, R⁹ and R¹⁰ is independently selected from hydrido, halo, lower alkyl, lower alkoxy, lower alkylthio, cyano, lower haloalkyl, lower haloalkoxy, lower hydroxyalkyl, lower alkoxyalkyl, hydroxyl and mercapto; and wherein R³ is selected from lower alkylsulfonyl and aminosulfonyl and R⁸ is selected from hydrido, halo, lower alkyl, lower alkoxy, lower alkylthio, lower haloalkoxy, lower hydroxyalkyl, lower alkylamino, mercapto, hydroxyl, lower alkoxyalkyl, cyano and lower haloalkyl; or wherein further R⁸ and R⁹ together form methylenedioxy; or wherein further, R³ is selected from hydrido, halo, lower alkyl, lower alkoxy, lower alkylthio, lower haloalkoxy, lower alkylamino, lower hydroxyalkyl, hydroxyl, mercapto, lower alkoxyalkyl, cyano and lower haloalkyl, and R⁸ is selected from lower alkylsulfonyl and aminosulfonyl; or wherein further R³ and R⁴ together form methylenedioxy; or a pharmaceutically-acceptable salt thereof.

A class of compounds of particular interest consists of those above compounds with A as indicated wherein each of R¹, R², R⁴, R⁵, R⁷, R⁹ and R¹⁰ is hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methylamino, N,N-dimethylamino, trifluoromethoxy, hydroxymethyl, methoxymethyl and ethoxymethyl; and wherein R³ is methylsulfonyl or aminosulfonyl, and R⁸ is selected from hydrido, fluoro, chloro, bromo, iodo, methyl, ethyl, n-propyl, isopropyl, butyl, tert-butyl, isobutyl, methoxy, ethoxy, propoxy, butoxy, trifluoromethoxy, methylamino, N,N-dimethylamino, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl; or wherein further R⁸ and R⁹ together form methylenedioxy; or wherein further, R³ is selected from hydrido, fluoro, chloro, bromo, iodo, methyl, ethyl, n-propyl, isopropyl, butyl, tert-butyl, isobutyl, methoxy, ethoxy, propoxy, butoxy, trifluoromethoxy, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl, and R⁸ is methylsulfonyl or aminosulfonyl; or wherein further R³ and R⁴ together form methylenedioxy; or a pharmaceutically-acceptable salt thereof.

A family of specific compounds of particular interest consists of above compounds with A as indicated and pharmaceutically-acceptable salts thereof as follows:
2-[6-[4-(methylsulfonyl)phenyl]spiro[2.4]hept-5-en-5-yl]pyridine;
5-fluoro-2-[6-[4-(methylsulfonyl)phenyl]spiro [2.4]hept-5-en-5-yl]pyridine;
5-chloro-2-[6-[4-(methylsulfonyl)phenyl]spiro [2.4]hept-5-en-5-yl]pyridine;
5-methyl-2-[6-[4-(methylsulfonyl)phenyl]spiro [2.4]hept-5-en-5-yl]pyridine;
5-methoxy-2-[6-[4-(methylsulfonyl)phenyl]spiro [2.4]hept-5-en-5-yl]pyridine;
5-methylthio-2-[6-[4-(methylsulfonyl)phenyl]spiro [2.4]hept-5-en-5-yl]pyridine;
5-cyano-2-[6-[4-(methylsulfonyl)phenyl]spiro [2.4]hept-5-en-5-yl]pyridine;
5-trifluoromethyl-2-[6-[4-(methylsulfonyl) phenyl]spiro[2.4]hept-5-en-5-yl]pyridine;
4-[6-(pyridin-2-yl)spiro[2.4]hept-5-en-5-yl] benzenesulfonamide;
4-[6-(5-fluoropyridin-2-yl)spiro[2.4]hept-5-en-5-yl] benzenesulfonamide;
4-[6-(5-chloropyridin-2-yl)spiro[2.4]hept-5-en-5-yl] benzenesulfonamide;
4-[6-(5-methylpyridin-2-yl)spiro[2.4]hept-5-en-5-yl] benzenesulfonamide;
4-[6-(5-methoxypyridin-2-yl)spiro[2.4]hept-5-en-5-yl]benzenesulfonamide;
4-[6-(5-methylthiopyridin-2-yl)spiro[2.4]hept-5-en-5-yl]benzenesulfonamide;
4-[6-(5-cyanopyridin-2-yl)spiro[2.4]hept-5-en-5-yl] benzenesulfonamide;
4-[6-(5-trifluoromethylpyridin-2-yl)spiro[2.4]hept-5-en-5-yl]benzenesulfonamide;
2-[7-[4-(methylsulfonyl)phenyl]spiro[3.4]oct-6-en-6-yl]pyridine;
5-fluoro-2-[7-[4-(methylsulfonyl) phenyl]spiro[3.4]oct-6-en-6-yl]pyridine;
5-chloro-2-[7-[4-(methylsulfonyl)phenyl] spiro[3.4]oct-6-en-6-yl]pyridine;
5-methyl-2-[7-[4-(methylsulfonyl)phenyl] spiro[3.4]oct-6-en-6-yl]pyridine;
5-methoxy-2-[7-[4-(methylsulfonyl)phenyl]spiro[3.4] oct-6-en-6-yl]pyridine;
5-methylthio-2-[7-[4-(methylsulfonyl)phenyl] spiro[3.4]oct-6-en-6-yl]pyridine;
5-cyano-2-[7-[4-(methylsulfonyl)phenyl] spiro[3.4]oct-6-en-6-yl]pyridine;
5-trifluoromethyl-2-[7-[4-(methylsulfonyl)phenyl] spiro[3.4]oct-6-en-6-yl]pyridine;
4-[7-(pyridin-2-yl)spiro[3.4]oct-6-en-6-yl] benzenesulfonamide;
4-[7-(5-fluoropyridin-2-yl)spiro[3.4]oct-6-en-6-yl] benzenesulfonamide;
4-[7-(5-chloropyridin-2-yl)spiro[3.4]oct-6-en-6-yl] benzenesulfonamide;
4-[7-(5-methylpyridin-2-yl)spiro[3.4]oct-6-en-6-yl] benzenesulfonamide;
4-[7-(5-methoxypyridin-2-yl)spiro[3.4]oct-6-en-6-yl] benzenesulfonamide;
4-[7-(5-methylthiopyridin-2-yl)spiro[3.4]oct-6-en-6-yl]benzenesulfonamide;
4-[7-(5-cyanopyridin-2-yl)spiro[3.4]oct-6-en-6-yl] benzenesulfonamide;
4-[7-(5-trifluoromethylpyridin-2-yl)spiro[3.4]oct-6-en-6-yl]benzenesulfonamide;
2-[3-[4-(methylsulfonyl)phenyl]spiro[4.4]non-2-en-2-yl]pyridine;
5-fluoro-2-[3-[4-(methylsulfonyl)phenyl]spiro[4.4] non-2-en-2-yl]pyridine;
5-chloro-2-[3-[4-(methylsulfonyl)phenyl]spiro[4.4] non-2-en-2-yl]pyridine;
5-methyl-2-[3-[4-(methylsulfonyl)phenyl]spiro[4.4] non-2-en-2-yl]pyridine;
5-methoxy-2-[3-[4-(methylsulfonyl)phenyl]spiro[4.4] non-2-en-2-yl]pyridine;
5-methylthio-2-[3-[4-(methylsulfonyl)phenyl] spiro[4.4]non-2-en-2-yl]pyridine;
5-cyano-2-[3-[4-(methylsulfonyl)phenyl]spiro[4.4] non-2-en-2-yl]pyridine;
5-trifluoromethyl-2-[3-[4-(methylsulfonyl)phenyl] spiro[4.4]non-2-en-2-yl]pyridine;
4-[3-(pyridin-2-yl)spiro[4.4]non-2-en-2-yl] benzenesulfonamide;
4-[3-(5-fluoropyridin-2-yl)spiro[4.4]non-2-en-2-yl] benzenesulfonamide;
4-[3-(5-chloropyridin-2-yl)spiro[4.4]non-2-en-2-yl] benzenesulfonamide;
4-[3-(5-methylpyridin-2-yl)spiro[4.4]non-2-en-2-yl] benzenesulfonamide;
4-[3-(5-methoxypyridin-2-yl)spiro[4.4]non-2-en-2-yl] benzenesulfonamide;
4-[3-(5-methylthiopyridin-2-yl)spiro[4.4]non-2-en-2-yl]benzenesulfonamide;
4-[3-(5-cyanopyridin-2-yl)spiro[4.4]non-2-en-2-yl] benzenesulfonamide;
4-[3-(5-trifluoromethylpyridin-2-yl)spiro[4.4]non-2-en-2-yl]benzenesulfonamide;
2-(6-phenylspiro[2.4]hept-5-en-5-yl)-5-(methylsulfonyl)pyridine;
2-[6-(4-fluorophenyl)spiro[2.4]hept-5-en-5-yl]-5-(methylsulfonyl)pyridine;
2-[6-(4-chlorophenyl)spiro[2.4]hept-5-en-5-yl]-5-(methylsulfonyl)pyridine;
2-[6-(4-methylphenyl)spiro[2.4]hept-5-en-5-yl]-5-(methylsulfonyl)pyridine;
2-[6-(4-methoxyphenyl)spiro[2.4]hept-5-en-5-yl]-5-(methylsulfonyl)pyridine;
2-[6-(4-methylthiophenyl)spiro[2.4]hept-5-en-5-yl]-5-(methylsulfonyl)pyridine;
2-[6-(4-cyanophenyl)spiro[2.4]hept-5-en-5-yl]-5-(methylsulfonyl)pyridine;
2-[6-(4-trifluoromethylphenyl)spiro[2.4]hept-5-en-5-yl]-5-(methylsulfonyl)pyridine;
2-(6-phenylspiro[2.4]hept-5-en-5-yl]-5-pyridinesulfonamide;
2-[6-(4-fluorophenyl)spiro[2.4]hept-5-en-5-yl]-5-pyridinesulfonamide;
2-[6-(4-chlorophenyl)spiro[2.4]hept-5-en-5-yl]-5-pyridinesulfonamide;
2-[6-(4-methylphenyl)spiro[2.4]hept-5-en-5-yl]-5-pyridinesulfonamide;
2-[6-(4-methoxyphenyl)spiro[2.4]hept-5-en-5-yl]-5-pyridinesulfonamide;
2-[6-(4-methylthiophenyl)spiro[2.4]hept-5-en-5-yl]-5-pyridinesulfonamide;
2-[6-(4-cyanophenyl)spiro[2.4]hept-5-en-5-yl]-5-pyridinesulfonamide;
2-[6-(4-trifluoromethylphenyl)spiro[2.4]hept-5-en-5-yl]-5-pyridinesulfonamide;
2-(7-phenylspiro[3.4]oct-6-en-6-yl)-5-(methylsulfonyl)pyridine;
2-[7-(4-fluorophenyl)spiro[3.4]oct-6-en-6-yl]-5-(methylsulfonyl)pyridine;
2-[7-(4-chlorophenyl)spiro[3.4]oct-6-en-6-yl]-5-(methylsulfonyl)pyridine;
2-[7-(4-methylphenyl)spiro[3.4]oct-6-en-6-yl]-5-(methylsulfonyl)pyridine;
2-[7-(4-methoxyphenyl)spiro[3.4]oct-6-en-6-yl]-5-(methylsulfonyl)pyridine;
2-[7-(4-methylthiophenyl)spiro[3.4]oct-6-en-6-yl]-5-(methylsulfonyl)pyridine;
2-[7-(4-cyanophenyl)spiro[3.4]oct-6-en-6-yl]-5-(methylsulfonyl)pyridine;
2-[7-(4-trifluoromethylphenyl)spiro[3.4]oct-6-en-6-yl]-5-(methylsulfonyl)pyridine;
2-(7-phenylspiro[3.4]oct-6-en-6-yl)-5-pyridinesulfonamide;
2-[7-(4-fluorophenyl)spiro[3.4]oct-6-en-6-yl]-5-pyridinesulfonamide;
2-[7-(4-chlorophenyl)spiro[3.4]oct-6-en-6-yl]-5-pyridinesulfonamide;
2-[7-(4-methylphenyl)spiro[3.4]oct-6-en-6-yl]-5-pyridinesulfonamide;
2-[7-(4-methoxyphenyl)spiro[3.4]oct-6-en-6-yl]-5-pyridinesulfonamide;
2-[7-(4-methylthiophenyl)spiro[3.4]oct-6-en-6-yl]-5-pyridinesulfonamide;
2-[7-(4-cyanophenyl)spiro[3.4]oct-6-en-6-yl]-5-pyridinesulfonamide;
2-[7-(4-trifluoromethylphenyl)spiro[3.4]oct-6-en-6-yl]-5-pyridinesulfonamide;
2-(3-phenylspiro[4.4]non-2-en-2-yl)-5-(methylsulfonyl)pyridine;
2-[3-(4-fluorophenyl)spiro[4.4]non-2-en-2-yl]-5-(methylsulfonyl)pyridine;
2-[3-(4-chlorophenyl)spiro[4.4]non-2-en-2-yl]-5-(methylsulfonyl)pyridine;
2-[3-(4-methylphenyl)spiro[4.4]non-2-en-2-yl]-5-(methylsulfonyl)pyridine;
2-[3-(4-methoxyphenyl)spiro[4.4]non-2-en-2-yl]-5-(methylsulfonyl)pyridine;
2-[3-(4-methylthiophenyl)spiro[4.4]non-2-en-2-yl]-5-(methylsulfonyl)pyridine;
2-[3-(4-cyanophenyl)spiro[4.4]non-2-en-2-yl]-5-(methylsulfonyl)pyridine;
2-[3-(4-trifluoromethylphenyl)spiro[4.4]non-2-en-2-yl]-5-(methylsulfonyl)pyridine;
2-(3-phenylspiro[4.4]non-2-en-2-yl)-5-pyridinesulfonamide;
2-[3-(4-fluorophenyl)spiro[4.4]non-2-en-2-yl]-5-pyridinesulfonamide;
2-[3-(4-chlorophenyl)spiro[4.4]non-2-en-2-yl]-5-pyridinesulfonamide;
2-[3-(4-methylphenyl)spiro[4.4]non-2-en-2-yl]-5-pyridinesulfonamide;
2-[3-(4-methoxyphenyl)spiro[4.4]non-2-en-2-yl]-5-pyridinesulfonamide;
2-[3-(4-cyanophenyl)spiro[4.4]non-2-en-2-yl]-5-pyridinesulfonamide; and
2-[3-(4-trifluoromethylphenyl)spiro[4.4]non-2-en-2-yl]-5-pyridinesulfonamide.

Within Formula I there is a fourth subclass of compounds of high interest wherein A is 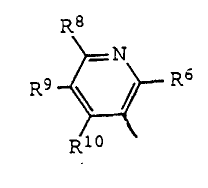
wherein n is a number selected from 0, 1, 2 and 3; and
wherein each of R¹ through R⁶ and R⁸ through R¹⁰ is independently selected from hydrido, halo, alkyl, alkoxy, alkylthio, cyano, haloalkyl, haloalkoxy, hydroxyalkyl, alkoxyalkyl, hydroxyl, mercapto, alkylamino, alkylsulfonyl and aminosulfonyl; or a pharmaceutically-acceptable salt thereof.

A preferred class of compounds consists of those above compounds with A as indicated wherein n is a number selected from 0, 1 and 2; wherein each of R¹, R², R⁴, R⁵, R⁶, R⁹ and R¹⁰ is independently selected from hydrido, halo, lower alkyl, lower alkoxy, lower alkylthio, cyano, lower haloalkyl, lower haloalkoxy, lower hydroxyalkyl, lower alkoxyalkyl, hydroxyl and mercapto; and wherein R³ is selected from lower alkylsulfonyl and aminosulfonyl and R⁸ is selected from hydrido, halo, lower alkyl, lower alkoxy, lower alkylthio, lower alkylamino, lower haloalkoxy, lower hydroxyalkyl, mercapto, hydroxyl, lower alkoxyalkyl, cyano and lower haloalkyl; or wherein further R⁸ and R⁹ together form methylenedioxy; or wherein further, R³ is selected from hydrido, halo, lower alkyl, lower alkoxy, lower alkylthio, lower alkylamino, lower haloalkoxy, lower hydroxyalkyl, hydroxyl, lower alkoxyalkyl, cyano and lower haloalkyl, and R⁸ is selected from lower alkylsulfonyl and aminosulfonyl; or wherein further R³ and R⁴ together form methylenedioxy; or a pharmaceutically-acceptable salt thereof.

A class of compounds of particular interest consists of those compounds with A as indicated wherein each of R¹, R², R⁴, R⁵, R⁶, R⁹ and R¹⁰ is hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl; and wherein R³ is methylsulfonyl or aminosulfonyl, and R⁸ is selected from hydrido, fluoro, chloro, bromo, iodo, methyl, ethyl, n-propyl, isopropyl, butyl, *tert*-butyl, isobutyl, methoxy, ethoxy, propoxy, butoxy, trifluoromethoxy, methylamino, N,N-dimethylamino, hydroxyl, methylthio, ethylthio, cyano, mercapto, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl; or wherein further R⁸ and R⁹ together form methylenedioxy; or wherein further, R³ is selected from hydrido, fluoro, chloro, bromo, iodo, methyl, ethyl, n-propyl, isopropyl, butyl, *tert*-butyl, isobutyl, methoxy, ethoxy, propoxy, butoxy, trifluoromethoxy, hydroxyl, methylamino, N,N-dimethylamino, methylthio, ethylthio, cyano, mercapto, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl, and R⁸ is methylsulfonyl or aminosulfonyl; or wherein further R³ and R⁴ together form methylenedioxy; or a pharmaceutically-acceptable salt thereof.

A family of specific compounds of particular interest consists of above compounds and pharmaceutically-acceptable salts thereof with A as indicated as follows:
5-[6-[4-(methylsulfonyl)phenyl]spiro[2.4]hept-5-en-5-yl]pyridine;
2-fluoro-5-[6-[4-(methylsulfonyl)phenyl]spiro[2.4] hept-5-en-5-yl]pyridine;
2-chloro-5-[6-[4-(methylsulfonyl)phenyl]spiro[2.4] hept-5-en-5-yl]pyridine;
2-methyl-5-[6-[4-(methylsulfonyl)phenyl]spiro[2.4] hept-5-en-5-yl]pyridine;
2-methoxy-5-[6-[4-(methylsulfonyl)phenyl]spiro[2.4] hept-5-en-5-yl]pyridine;
2-methylthio-5-[6-[4-(methylsulfonyl)phenyl] spiro[2.4]hept-5-en-5-yl]pyridine;
2-cyano-5-[6-[4-(methylsulfonyl)phenyl]spiro[2.4] hept-5-en-5-yl]pyridine;
2-trifluoromethyl-5-[6-[4-(methylsulfonyl)phenyl] spiro[2.4]hept-5-en-5-yl]pyridine;
4-[6-(pyridin-5-yl)spiro[2.4]hept-5-en-5-yl] benzenesulfonamide;
4-[6-(2-fluoropyridin-5-yl)spiro[2.4]hept-5-en-5-yl] benzenesulfonamide;
4-[6-(2-chloropyridin-5-yl)spiro[2.4]hept-5-en-5-yl] benzenesulfonamide;
4-[6-(2-methylpyridin-5-yl)spiro[2.4]hept-5-en-5-yl] benzenesulfonamide;
4-[6-(2-methoxypyridin-5-yl)spiro[2.4]hept-5-en-5-yl]benzenesulfonamide;
4-[6-(2-methylthiopyridin-5-yl)spiro[2.4]hept-5-en-5-yl]benzenesulfonamide;
4-[6-(2-cyanopyridin-5-yl)spiro[2.4]hept-5-en-5-yl] benzenesulfonamide;
4-[6-(2-trifluoromethylpyridin-5-yl)spiro[2.4]hept-5-en-5-yl]benzenesulfonamide;
5-[7-[4-(methylsulfonyl)phenyl]spiro[3.4]oct-6-en-6-yl]pyridine;
2-fluoro-5-[7-[4-(methylsulfonyl)phenyl] spiro[3.4]oct-6-en-6-yl]pyridine;
2-chloro-5-[7-[4-(methylsulfonyl)phenyl] spiro[3.4]oct-6-en-6-yl]pyridine;
2-methyl-5-[7-[4-(methylsulfonyl)phenyl] spiro[3.4]oct-6-en-6-yl]pyridine;
2-methoxy-5-[7-[4-(methylsulfonyl)phenyl]spiro [3.4]oct-6-en-6-yl]pyridine;
2-methylthio-5-[7-[4-(methylsulfonyl)phenyl]spiro [3.4]oct-6-en-6-yl]pyridine;
2-cyano-5-[7-[4-(methylsulfonyl)phenyl] spiro[3.4]oct-6-en-6-yl]pyridine;
2-trifluoromethyl-5-[7-[4-(methylsulfonyl)phenyl] spiro[3.4]oct-6-en-6-yl]pyridine;
4-[7-(pyridin-5-yl)spiro[3.4]oct-6-en-6-yl] benzenesulfonamide;
4-[7-12-fluoropyridin-5-yl)spiro[3.4]oct-6-en-6-yl] benzenesulfonamide;
4-[7-(2-chloropyridin-5-yl)spiro[3.4]oct-6-en-6-yl] benzenesulfonamide;
4-[7-(2-methylpyridin-5-yl)spiro[3.4]oct-6-en-6-yl] benzenesulfonamide;
4-[7-(2-methoxypyridin-5-yl)spiro[3.4]oct-6-en-6-yl] benzenesulfonamide;
4-[7-(2-methylthiopyridin-5-yl)spiro[3.4]oct-6-en-6-yl]benzenesulfonamide;
4-[7-(2-cyanopyridin-5-yl)spiro[3.4]oct-6-en-6-yl] benzenesulfonamide;
4-[7-(2-trifluoromethylpyridin-5-yl)spiro[3.4]oct-6-en-6-yl]benzenesulfonamide;
5-[3-[4-(methylsulfonyl)phenyl]spiro[4.4]non-2-en-2-yl]pyridine;
2-fluoro-5-[3-[4-(methylsulfonyl)phenyl] spiro[4.4]non-2-en-2-yl]pyridine;
2-chloro-5-[3-[4-(methylsulfonyl)phenyl] spiro[4.4]non-2-en-2-yl]pyridine;
2-methyl-5-[3-[4-(methylsulfonyl)phenyl] spiro[4.4]non-2-en-2-yl]pyridine;
2-methoxy-5-[3-[4-(methylsulfonyl)phenyl] spiro[4.4]non-2-en-2-yl]pyridine;
2-methylthio-5-[3-[4-(methylsulfonyl)phenyl]spiro [4.4]non-2-en-2-yl]pyridine;
2-trifluoromethyl-5-[3-[4-(methylsulfonyl)phenyl] spiro[4.4]non-2-en-2-yl]pyridine;
4-[3-(pyridin-5-yl)spiro[4.4]non-2-en-2-yl] benzenesulfonamide;
4-[3-(2-fluoropyridin-5-yl)spiro[4.4]non-2-en-2-yl] benzenesulfonamide;
4-[3-(2-chloropyridin-5-yl)spiro[4.4]non-2-en-2-yl] benzenesulfonamide;
4-[3-(2-methylpyridin-5-yl)spiro[4.4]non-2-en-2-yl] benzenesulfonamide;
4-[3-(2-methoxypyridin-5-yl)spiro[4.4]non-2-en-2-yl] benzenesulfonamide;
4-[3-(2-methylthiopyridin-5-yl)spiro[4.4]non-2-en-2-yl]benzenesulfonamide;
4-[3-(2-cyanopyridin-5-yl)spiro[4.4]non-2-en-2-yl] benzenesulfonamide;
4-[3-(2-trifluoromethylpyridin-5-yl)spiro[4.4]non-2-en-2-yl]benzenesulfonamide;
5-(6-phenylspiro[2.4]hept-5-en-5-yl)-2-(methylsulfonyl)pyridine;
5-[6-(4-fluorophenyl)spiro[2.4]hept-5-en-5-yl]-2-(methylsulfonyl)pyridine;
5-[6-(4-chlorophenyl)spiro[2.4]hept-5-en-5-yl]-2-(methylsulfonyl)pyridine;
5-[6-(4-methylphenyl)spiro[2.4]hept-5-en-5-yl]-2-(methylsulfonyl)pyridine;
5-[6-(4-methoxyphenyl)spiro[2.4]hept-5-en-5-yl]-2-(methylsulfonyl)pyridine;
5-[6-(4-methylthiophenyl)spiro[2.4]hept-5-en-5-yl]-2-(methylsulfonyl)pyridine;
5-[6-(4-cyanophenyl)spiro[2.4]hept-5-en-5-yl]-2-(methylsulfonyl)pyridine;
5-[6-(4-trifluoromethylphenyl)spiro[2.4]hept-5-en-5-yl]-2-(methylsulfonyl)pyridine;
5-(6-phenylspiro[2.4]hept-5-en-5-yl)-2-pyridinesulfonamide;
5-[6-(4-fluorophenyl)spiro[2.4]hept-5-en-5-yl]-2-pyridinesulfonamide;
5-[6-(4-chlorophenyl)spiro[2.4]hept-5-en-5-yl]-2-pyridinesulfonamide;
5-[6-(4-methylphenyl)spiro[2.4]hept-5-en-5-yl]-2-pyridinesulfonamide;
5-[6-(4-methoxyphenyl)spiro[2.4]hept-5-en-5-yl]-2-pyridinesulfonamide;
5-[6-(4-methylthiophenyl)spiro[2.4]hept-5-en-5-yl]-2-pyridinesulfonamide;
5-[6-(4-cyanophenyl)spiro[2.4]hept-5-en-5-yl]-2-pyridinesulfonamide;
5-[6-(4-trifluoromethylphenyl)spiro[2.4]hept-5-en-5-yl]-2-pyridinesulfonamide;
5-(7-phenylspiro[3.4]oct-6-en-6-yl)-2-(methylsulfonyl)pyridine;
5-[7-(4-fluorophenyl)spiro[3.4]oct-6-en-6-yl]-2-(methylsulfonyl)pyridine;
5-[7-(4-chlorophenyl)spiro[3.4]oct-6-en-6-yl]-2-(methylsulfonyl)pyridine;
5-[7-(4-methylphenyl)spiro[3.4]oct-6-en-6-yl]-2-(methylsulfonyl)pyridine;
5-[7-(4-methoxyphenyl)spiro[3.4]oct-6-en-6-yl]-2-(methylsulfonyl)pyridine;
5-[7-(4-methylthiophenyl)spiro[3.4]oct-6-en-6-yl]-2-(methylsulfonyl)pyridine;
5-[7-(4-cyanophenyl)spiro[3.4]oct-6-en-6-yl]-2-(methylsulfonyl)pyridine;
5-[7-(4-trifluoromethylphenyl)spiro[3.4]oct-6-en-6-yl]-2-(methylsulfonyl)pyridine;
5-(7-phenylspiro[3.4]oct-6-en-6-yl)-2-pyridinesulfonamide;
5-[7-(4-fluorophenyl)spiro[3.4]oct-6-en-6-yl]-2-pyridinesulfonamide;
5-[7-(4-chlorophenyl)spiro[3.4]oct-6-en-6-yl]-2-pyridinesulfonamide;
5-[7-(4-methylphenyl)spiro[3.4]oct-6-en-6-yl]-2-pyridinesulfonamide;
5-[7-(4-methoxyphenyl)spiro[3.4]oct-6-en-6-yl]-2-pyridinesulfonamide;
5-[7-(4-cyanophenyl)spiro[3.4]oct-6-en-6-yl]-2-pyridinesulfonamide;
5-[7-(4-trifluoromethylphenyl)spiro[3.4]oct-6-en-6-yl]-2-pyridinesulfonamide;
5-(3-phenylspiro[4.4]non-2-en-2-yl)-2-(methylsulfonyl)pyridine;
5-[3-(4-fluorophenyl)spiro[4.4]non-2-en-2-yl]-2-(methylsulfonyl)pyridine;
5-[3-(4-chlorophenyl)spiro[4.4]non-2-en-2-yl]-2-(methylsulfonyl)pyridine;
5-[3-(4-methylphenyl)spiro[4.4]non-2-en-2-yl]-2-(methylsulfonyl)pyridine;
5-[3-(4-methoxyphenyl)spiro[4.4]non-2-en-2-yl]-2-(methylsulfonyl)pyridine;
5-[3-(4-methylthiophenyl)spiro[4.4]non-2-en-2-yl]-2-(methylsulfonyl)pyridine;
5-[3-(4-cyanophenyl)spiro[4.4]non-2-en-2-yl]-2-(methylsulfonyl)pyridine;
5-[3-(4-trifluoromethylphenyl)spiro[4.4]non-2-en-2-yl]-2-(methylsulfonyl)pyridine;
5-(3-phenylspiro[4.4]non-2-en-2-yl)-2-pyridinesulfonamide;
5-[3-(4-fluorophenyl)spiro[4.4]non-2-en-2-yl]-2-pyridinesulfonamide;
5-[3-(4-chlorophenyl)spiro[4.4]non-2-en-2-yl]-2-pyridinesulfonamide;
5-[3-(4-methylphenyl)spiro[4.4]non-2-en-2-yl]-2-pyridinesulfonamide;
5-[3-(4-methoxyphenyl)spiro[4.4]non-2-en-2-yl]-2-pyridinesulfonamide;
5-[3-(4-methylthiophenyl)spiro[4.4]non-2-en-2-yl]-2-pyridinesulfonamide;
5-[3-(4-cyanophenyl)spiro[4.4]non-2-en-2-yl]-2-pyridinesulfonamide; and
5-[3-(4-trifluoromethylphenyl)spiro[4.4]non-2-en-2-yl]-2-pyridinesulfonamide.

Within Formula I there is a fifth subclass of compounds of high interest wherein A is 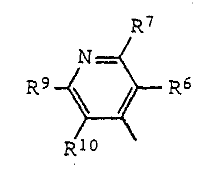
wherein n is a number selected from 0, 1, 2 and 3; and
wherein each of R¹ through R⁷, R⁹ and R¹⁰ is independently selected from hydrido, halo, alkyl, alkoxy, alkylthio, cyano, haloalkyl, haloalkoxy, hydroxyalkyl, alkoxyalkyl, hydroxyl, mercapto, alkylamino, alkylsulfonyl and aminosulfonyl; or a pharmaceutically-acceptable salt thereof.

A preferred class of compounds consists of those compounds as shown above wherein n is a number selected from 0, 1 and 2; wherein each of R¹, R², R⁴ through R⁷, R⁹ and R¹⁰ is independently selected from hydrido, halo, lower alkyl, lower alkoxy, lower alkylthio, lower haloalkoxy, lower hydroxyalkyl, hydroxyl, lower alkoxyalkyl, mercapto, cyano and lower haloalkyl; and wherein R³ is selected from lower alkylsulfonyl and aminosulfonyl; or a pharmaceutically-acceptable salt thereof.

A class of compounds of particular interest consists of those compounds as shown above wherein each of R¹, R², R⁴ through R⁷, R⁹ and R¹⁰ is independently selected from hydrido, fluoro, chloro, bromo, iodo, methyl, ethyl, n-propyl, isopropyl, butyl, tert-butyl, isobutyl, methoxy, ethoxy, propoxy, butoxy, trifluoromethoxy, methylthio, ethylthio, cyano, hydroxyl, mercapto, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxymethyl, methoxymethyl and ethoxymethyl; and wherein R³ is methylsulfonyl or aminosulfonyl; or a pharmaceutically-acceptable salt thereof.

A family of specific compounds of particular interest consists of above compounds and pharmaceutically-acceptable salts thereof with A as indicated as follows:
4-[6-[4-(methylsulfonyl)phenyl]spiro[2.4]hept-5-en-5-yl]pyridine;
4-[6-(4-pyridinyl)spiro[2.4]hept-5-en-5-yl] benzenesulfonamide;
4-[7-[4-(methylsulfonyl)phenyl]spiro[3.4]oct-6-en-6-yl]pyridine;
4-[7-(4-pyridinyl)spiro[3.4]oct-6-en-6-yl] benzenesulfonamide;
4-[3-[4-(methylsulfonyl)phenyl]spiro[4.4]non-2-en-2-yl]pyridine; and
4-[3-(4-pyridinyl)spiro[4.4]non-2-en-2-yl] benzenesulfonamide.

Where the term "alkyl" is used, either alone or within other terms such as "haloalkyl", "alkylsulfonyl", "alkoxyalkyl" and "hydroxyalkyl", embraces linear or branched radicals having one to about twenty carbon atoms or, preferably, one to about twelve carbon atoms. More preferred alkyl radicals are "lower alkyl" radicals having one to about ten carbon atoms. Most preferred are lower alkyl radicals having one to about six carbon atoms. Examples of such radicals include methyl, ethyl, n-propyl, isopropyl, n-butyl, isobutyl, sec-butyl, tert-butyl, pentyl, iso-amyl, hexyl and the like. The term "hydrido" denotes a single hydrogen atom (H). This hydrido radical may be attached, for example, to an oxygen atom to form a "hydroxyl" radical, to a sulfur atom to form a "mercapto" radical, or two hydrido radicals may be attached to a carbon atom to form a methylene (-CH₂-) radical. The term "halo" means halogens such as fluorine, chlorine, bromine or iodine. The term "haloalkyl" embraces radicals wherein any one or more of the alkyl carbon atoms is substituted with halo as defined above. Specifically embraced are monohaloalkyl, dihaloalkyl and polyhaloalkyl radicals. A monohaloalkyl radical, for one example, may have either an iodo, bromo, chloro or fluoro atom within the radical. Dihalo and polyhaloalkyl radicals may have two or more of the same halo atoms or a combination of different halo radicals. The term "hydroxyalkyl" embraces linear or branched alkyl radicals having one to about ten carbon atoms any one of which may be substituted with one or more hydroxyl radicals. The terms "alkoxy" and "alkoxyalkyl" embrace linear or branched oxy-containing radicals each having alkyl portions of one to about ten carbon atoms. More preferred alkoxy radicals are "lower alkoxy" radicals having one to six carbon atoms. Examples of such radicals include methoxy, ethoxy, propoxy, butoxy and tert-butoxy. The term "alkoxyalkyl" also embraces alkyl radicals having two or more alkoxy radicals attached to the alkyl radical, that is, to form monoalkoxyalkyl and dialkoxyalkyl radicals. The "alkoxy" or "alkoxyalkyl" radicals may be further substituted with one or more halo atoms, such as fluoro, chloro or bromo, to provide "haloalkoxy" or "haloalkoxyalkyl" radicals. The term "alkylamino" embraces amino radicals having one or more alkyl radicals attached to the nitrogen atom, that is, to form N-alkylamino and N,N-dialkylamino radicals. The term "alkylthio" embraces radicals containing a linear or branched alkyl radical, of one to about ten carbon atoms attached to a divalent sulfur atom, such as a methythio radical, (CH₃-S-). The term "sulfonyl", whether used alone or linked to other terms such as alkylsulfonyl, denotes respectively divalent radicals -SO₂-. "Alkylsulfonyl" embraces alkyl radicals attached to a sulfonyl radical, where alkyl is defined as above. The "alkylsulfonyl" radicals may be further substituted with one or more halo atoms, such as fluoro, chloro or bromo, to provide "haloalkylsulfonyl" radicals. The terms "aminosulfonyl" "sulfamyl" and "sulfonamidyl" denotes a sulfonyl radical substituted with an amine radical, forming a sulfonamide (-SO₂NH₂).

The present invention comprises a pharmaceutical composition comprising a therapeutically-effective amount of a compound of Formula I in association with at least one pharmaceutically-acceptable carrier, adjuvant or diluent.

The present invention also comprises a method of treating inflammation or inflammation-associated disorders in a subject, the method comprising administering to the subject having such inflammation or disorder a therapeutically-effective amount of a compound of Formula I.

Also included in the family of compounds of Formula I are the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicyclic, salicyclic, *p*-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, stearic, cyclohexylaminosulfonic, algenic, β-hydroxybutyric, salicyclic, galactaric and galacturonic acid. Suitable pharmaceutically-acceptable base addition salts of compounds of Formula I include metallic salts made from aluminum, calcium, lithium, magnesium, potassium, sodium and zinc or organic salts made from N,N'-dibenzylethylenediamine, chloroprocaine, choline, diethanolamine, ethylenediamine, meglumine (N-methylglucamine) and procaine. All of these salts may be prepared by conventional means from the corresponding compound of Formula I by reacting, for example, the appropriate acid or base with the compound of Formula I.

### GENERAL SYNTHETIC PROCEDURES

The compounds of the invention can be synthesized according to the following procedures of Schemes I-XIX, wherein the R¹-R¹¹ substituents are as defined for Formula I, above, except where further noted.

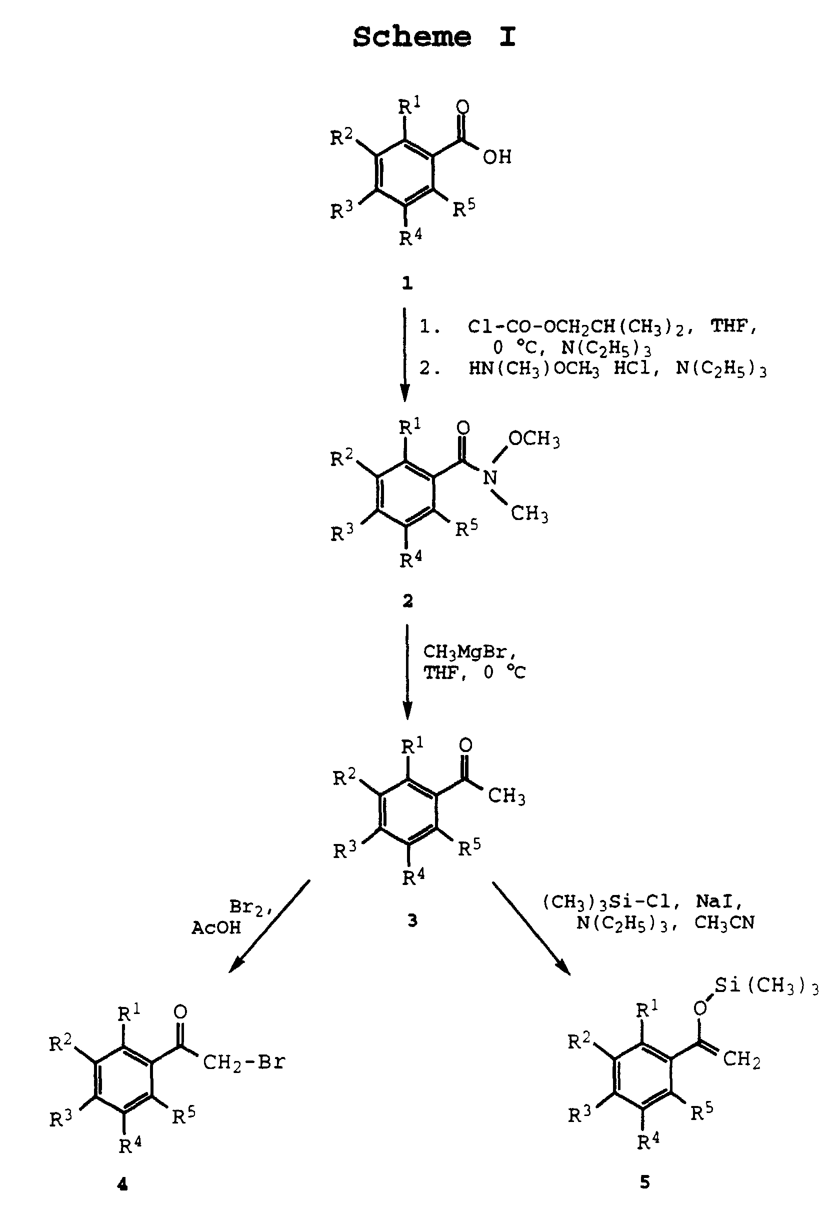

Synthetic Scheme I shows the three step procedures used to prepare the bromoacetophenones **4** and the phenyl silyl enol ethers **5** from commercially available benzoic acids **1**. In step one, a THF solution at 0°C of the benzoic acids **1** and two equivalents of triethylamine are sequentially treated with isobutyl chloroformate and N-hydroxymethyl-N-methylamine hydrochloride to give the Weinreb amides **2** [see: S. Nahm and S. M. Weinreb, Tetrahedron Lett., 21, 3815 (1981)]. In step two, the amides **2** are reacted with methylmagnesium bromide to give the corresponding acetophenones **3**. In step three, the acetophenones **3** are either treated with bromine in acetic acid to give the corresponding bromoacetophenones **4** or chlorotrimethylsilane in acetonitrile in the presence of triethylamine and sodium iodide to give the corresponding phenyl silyl enol ethers **5**.

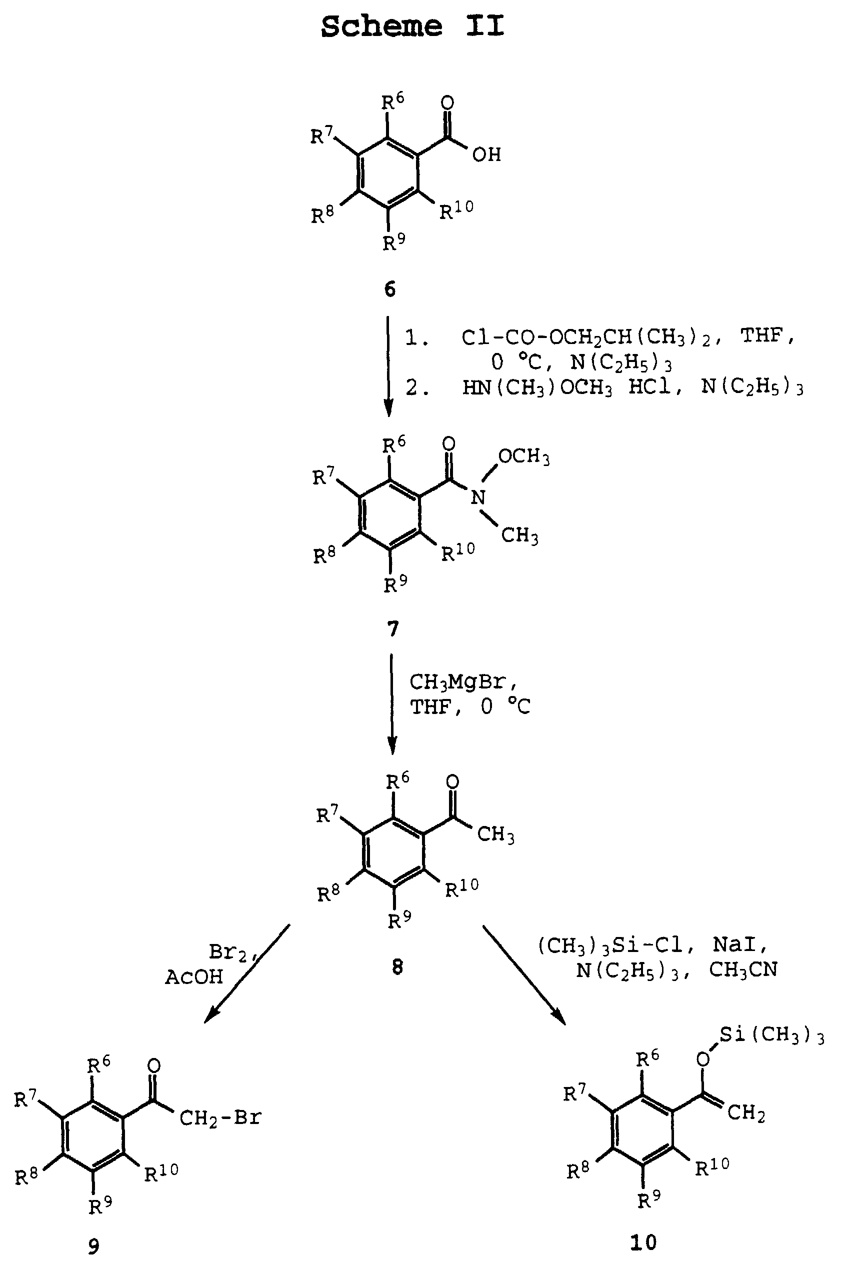

Synthetic Scheme II shows the three step procedures used to prepare the bromoacetophenones **9** and the phenyl silyl enol ethers **10** from commercially available benzoic acids **6**. In step one, a THF solution at 0°C of the benzoic acids **6** and two equivalents of triethylamine are sequentially treated with isobutyl chloroformate and N-hydroxymethyl-N-methylamine hydrochloride to give the Weinreb amides **7**. In step two, the amides **7** are reacted with methylmagnesium bromide to give the corresponding acetophenones **8**. In step three, the acetophenones **8** are either treated with bromine in acetic acid to give the corresponding bromoacetophenones **9** or chlorotrimethylsilane in acetonitrile in the presence of triethylamine and sodium iodide to give the corresponding phenyl silyl enol ethers **10**.

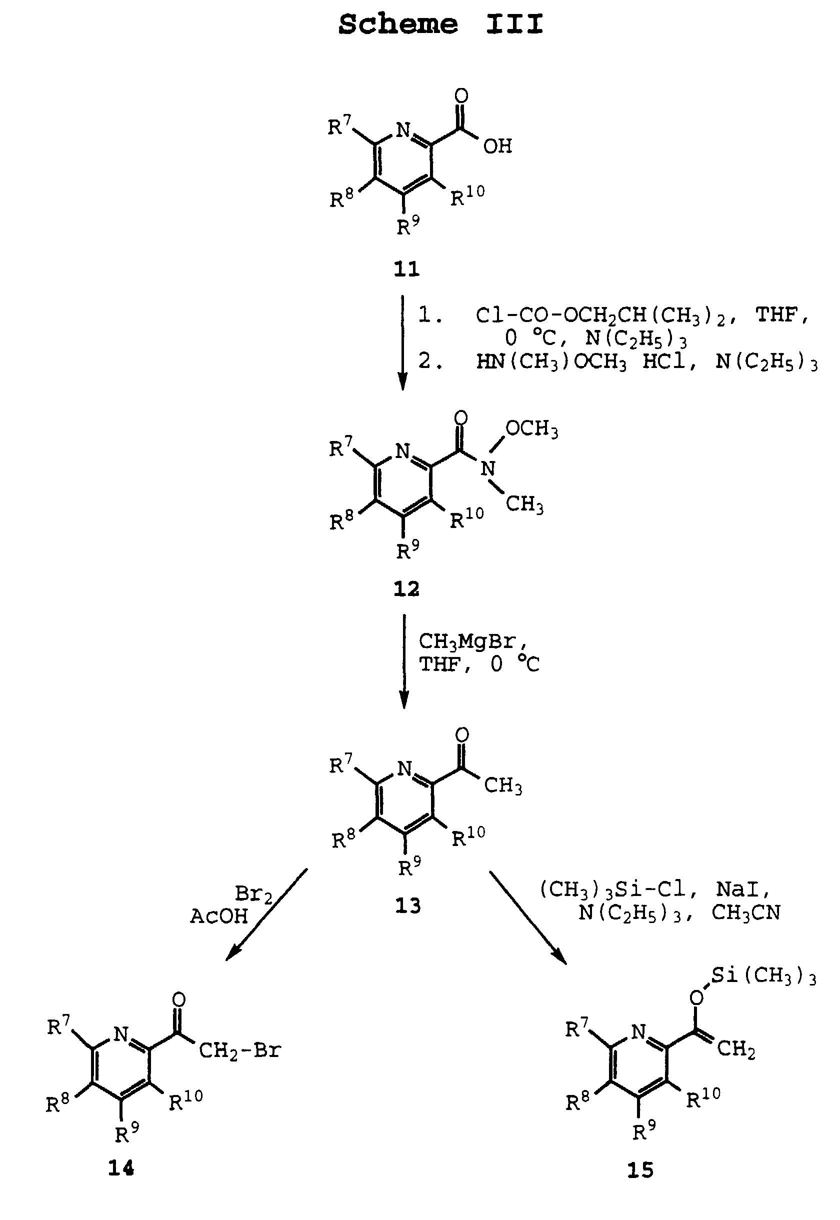

Synthetic Scheme III shows the three step procedures used to prepare the 2-(bromoacetyl)pyridines **14** and the 2-pyridinyl silyl enol ethers **15** from commercially available picolinic acids **11**. In step one, a THF solution at 0°C of the picolinic acids **11** and two equivalents of triethylamine are sequentially treated with isobutyl chloroformate and N-hydroxymethyl-N-methylamine hydrochloride to give the Weinreb amides **12**. In step two, the amides **12** are reacted with methylmagnesium bromide to give the corresponding 2-acetylpyridines **13**. In step three, the 2-acetylpyridines **13** are either treated with bromine in acetic acid to give the corresponding 2-(bromoacetyl)pyridines **14** or chlorotrimethylsilane in acetonitrile in the presence of triethylamine and sodium iodide to give the corresponding 2-pyridinyl silyl enol ethers **15**.

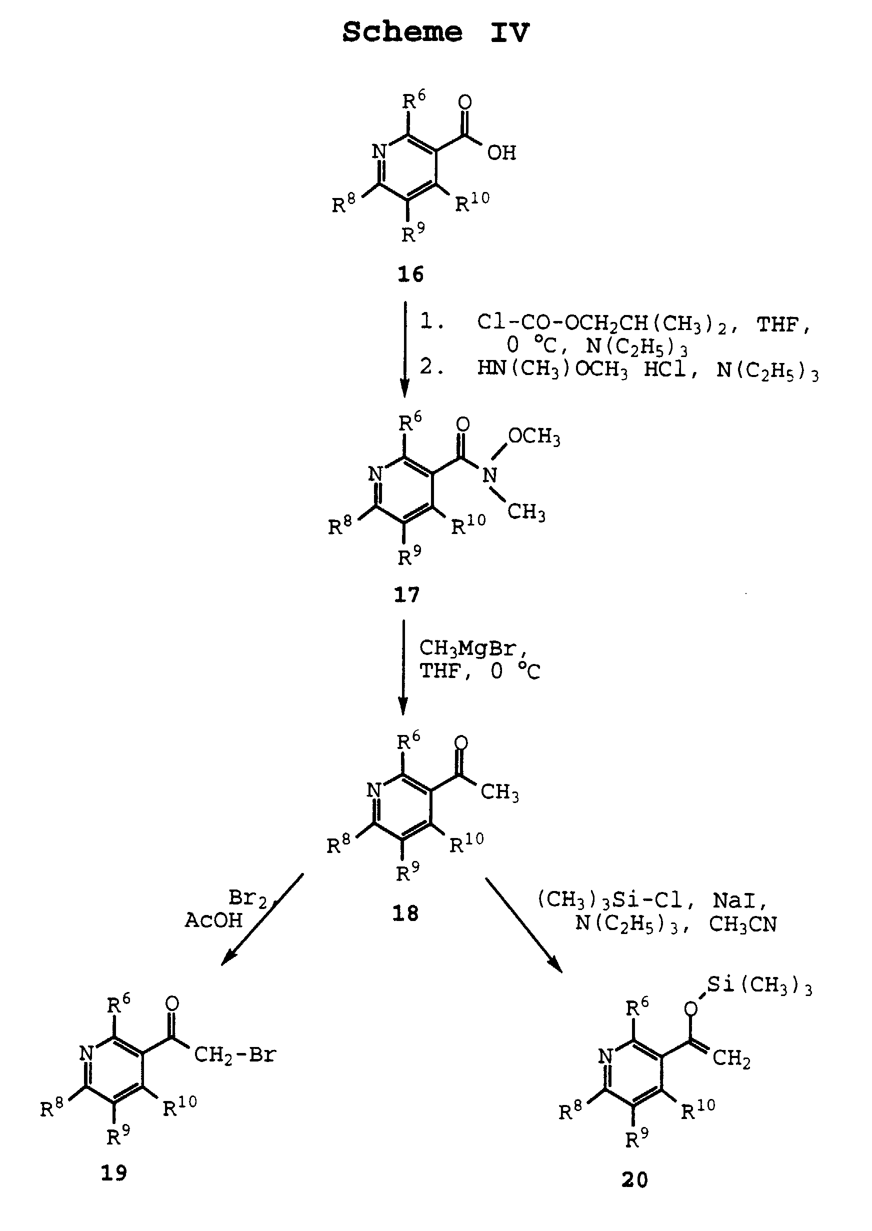

Synthetic Scheme IV shows the three step procedures used to prepare the 3-(bromoacetyl)pyridines **19** and the 3-pyridinyl silyl enol ethers **20** from commercially available nicotinic acids **16**. In step one, a THF solution at 0°C of the nicotinic acids **16** and two equivalents of triethylamine are sequentially treated with isobutyl chloroformate and N-hydroxymethyl-N-methylamine hydrochloride to give the Weinreb amides **17**. In step two, the amides **17** are reacted with methylmagnesium bromide to give the corresponding 3-acetylpyridines **18**. In step three, the 3-acetylpyridines **18** are either treated with bromine in acetic acid to give the corresponding 3-(bromoacetyl)pyridines **19** or chlorotrimethylsilane in acetonitrile in the presence of triethylamine and sodium iodide to give the corresponding 3-pyridinyl silyl enol ethers **20**.

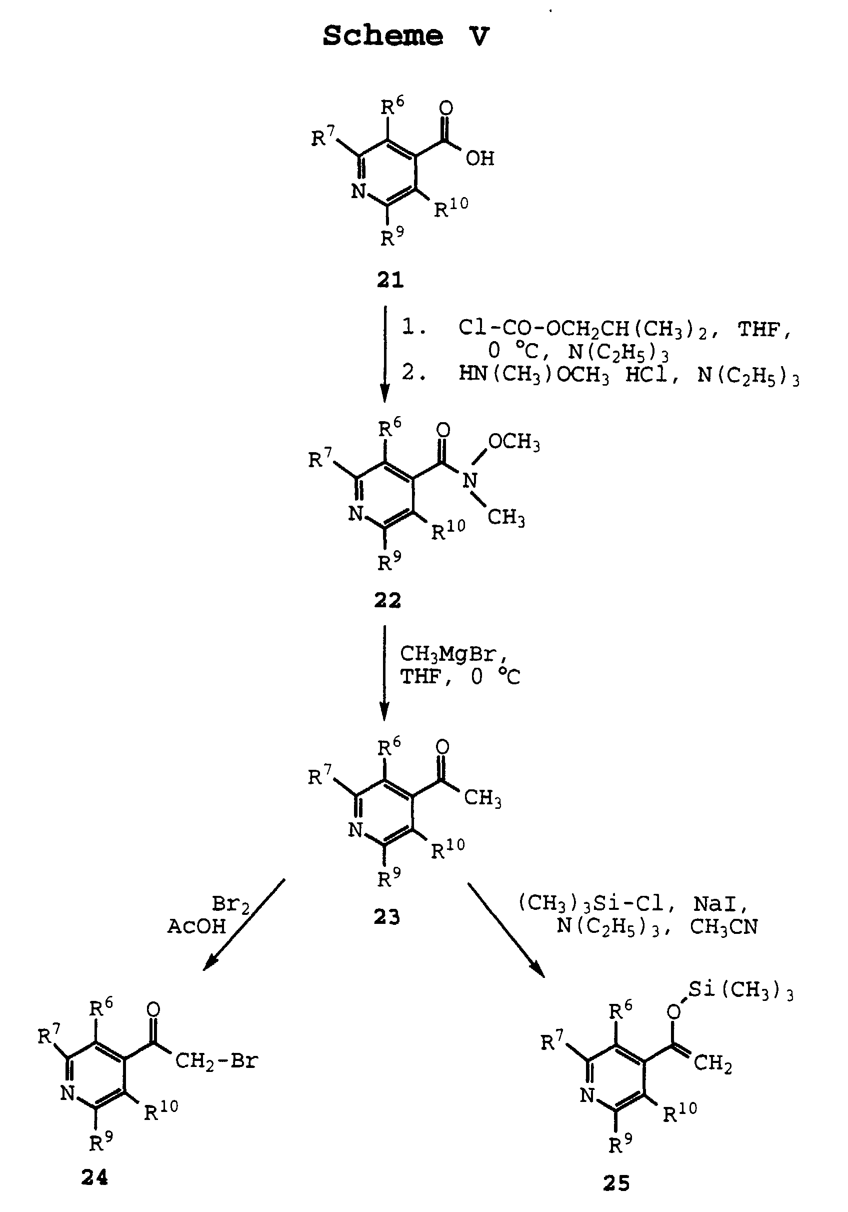

Synthetic Scheme V shows the three step procedures used to prepare the 4-(bromoacetyl)pyridines **24** and the 4-pyridinyl silyl enol ethers **25** from commercially available isonicotinic acids **21**. In step one, a THF solution at 0°C of the isonicotinic acids **21** and two equivalents of triethylamine are sequentially treated with isobutyl chloroformate and N-hydroxymethyl-N-methylamine hydrochloride to give the Weinreb amides **22**. In step two, the amides **22** are reacted with methylmagnesium bromide to give the corresponding 4-acetylpyridines **23**. In step three, the 4-acetylpyridines **23** are either treated with bromine in acetic acid to give the corresponding 4-(bromoacetyl)pyridines **24** or chlorotrimethylsilane in acetonitrile in the presence of triethylamine and sodium iodide to give the corresponding 4-pyridinyl silyl enol ethers **25**.

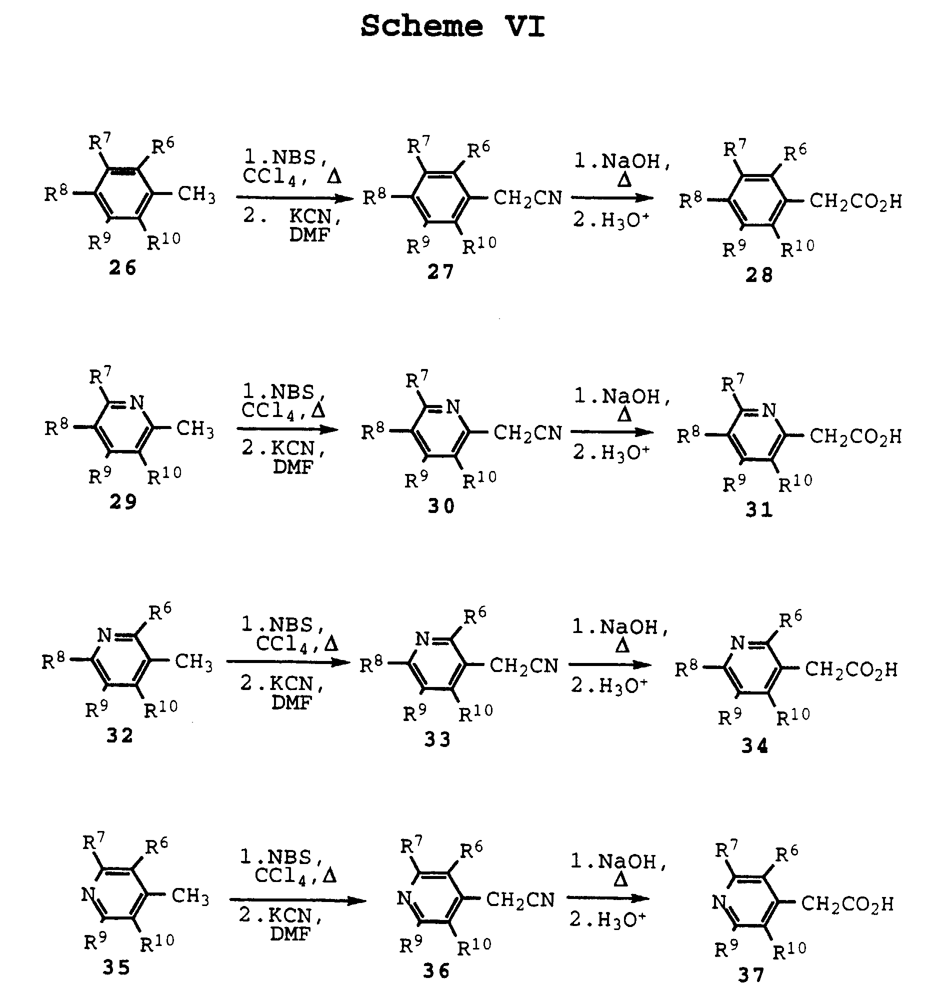

Synthetic Scheme VI shows the two step procedures which can be used to prepare the phenylacetic acids **28**, 2-pyridinylacetic acids **31**, 3-pyridinylacetic acids **34**, and 4-pyridinylacetic acids **37** from commercially available toluenes **26**, 2-picolines **29**, 3-picolines **32**, and 4-picolines **35**, respectively. In step one, toluenes **26**, 2-picolines **29**, 3-picolines **32**, and 4-picolines **35** are sequentially treated with N-bromosuccinimide (NBS) in carbon tetrachloride at reflux in the presence of a free radical initiater, e.g., 2,2'-azobis(2-methylpropionitrile) (AIBN), and potassium cyanide in DMF to give the corresponding phenylacetonitriles **27**, 2-pyridinylacetonitriles **30**, 3-pyridinylacetonitriles **33**, and 4-pyridinylacetonitriles **36**, respectively. In step two, phenylacetonitriles **27**, 2-pyridinylacetonitriles **30**, 3-pyridinylacetonitriles **33,** and 4-pyridinylacetonitriles **36** are hydrolyzed with aqueous sodium hydroxide; acidification provides the phenylacetic acids **28**, 2-pyridinylacetic acids **31**, 3-pyridinylacetic acids **34**, and 4-pyridinylacetic acids **37**, respectively.

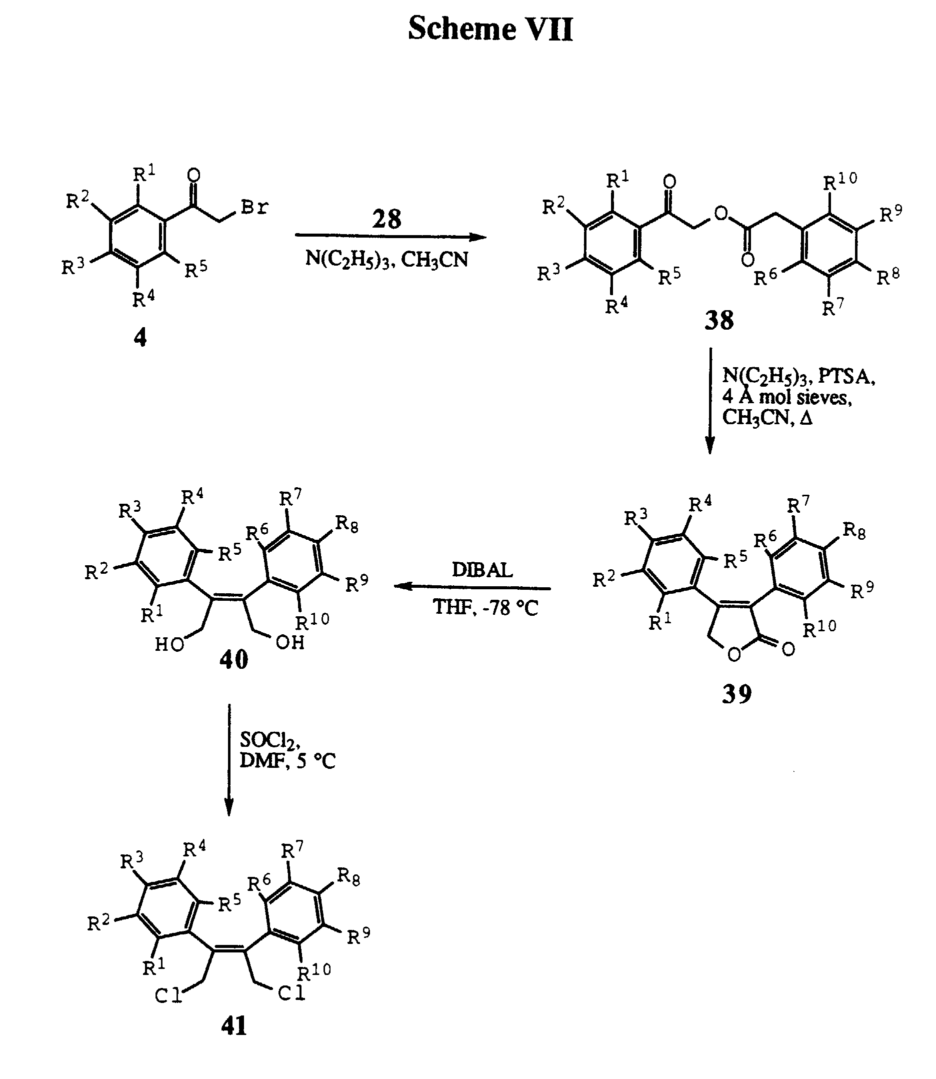

Synthetic Scheme VII shows the four step procedures used to prepare the cis-2,3-diaryl-1,4-dichloro-2-butenes **41** from the bromoacetophenones **4** (prepared in Synthetic Scheme I) and the phenylacetic acids **28** (prepared in Synthetic Scheme VI). In step one, bromoacetophenones **4** are reacted with phenylacetic acids **28** in acetonitrile in the presence of triethylamine to give the corresponding esters **38**. In step two, the esters **38** are cyclized to the corresponding furanones **39** on treatment with *p*-toluenesulfonic acid (PTSA) and triethylamine in the presence of 4 Å molecular sieves in acetonitrile at reflux. In step three, the furanones **39** are reacted with diisobutylaluminum hydride (DIBAL) to give the corresponding cis-diols **40**. In step four, the cis-diols **40** are reacted with thionyl chloride in DMF at 5°C to give the corresponding cis-2,3-diaryl-1,4-dichloro-2-butenes **41**.

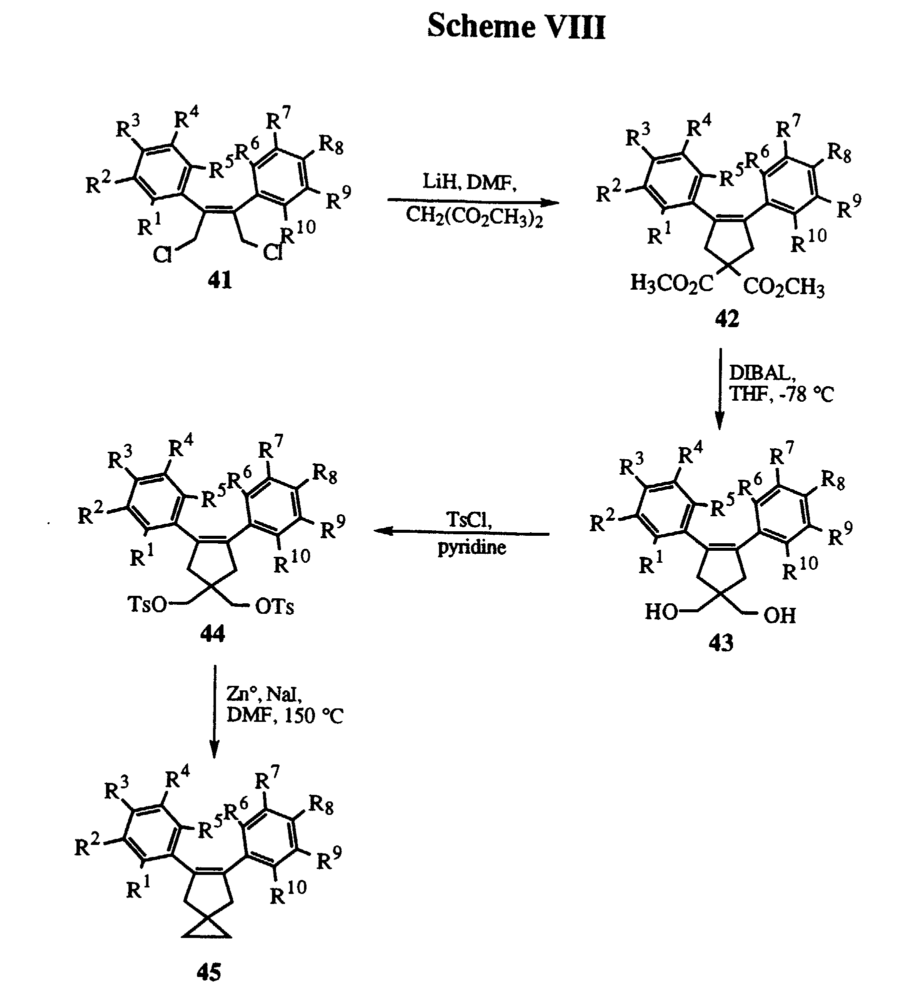

Synthetic Scheme VIII shows the four step procedure used to prepare the 5,6-diarylspiro[2.4]hept-5-enes **45** from the cis-2,3-diaryl-1,4-dichloro-2-butenes **41** (prepared in Synthetic Scheme VII). In step one, the cis-2,3-diaryl-1,4-dichloro-2-butenes **41** are reacted with dimethyl malonate in DMF in the presence of two equivalents of lithium hydride to give the corresponding 4,4-dicarbomethoxycyclopentenes **42**. In step two, the 4,4-dicarbomethoxycyclopentenes **42** are reacted with DIBAL in THF to give the corresponding 4,4-di(hydroxymethyl)cyclopentenes **43**. In step three, the 4,4-di(hydroxymethyl)cyclopentenes **43** are reacted with p-toluenesulfonyl chloride (TsCl) in pyridine to give the corresponding 4,4-ditosylates **44**. In step four, the 4,4-ditosylates **44** are reacted with metallic zinc and sodium iodide in DMF at 150°C to give the 5,6-diarylspiro[2.4]hept-5-ene antiinflammatory agents **45** of this invention.

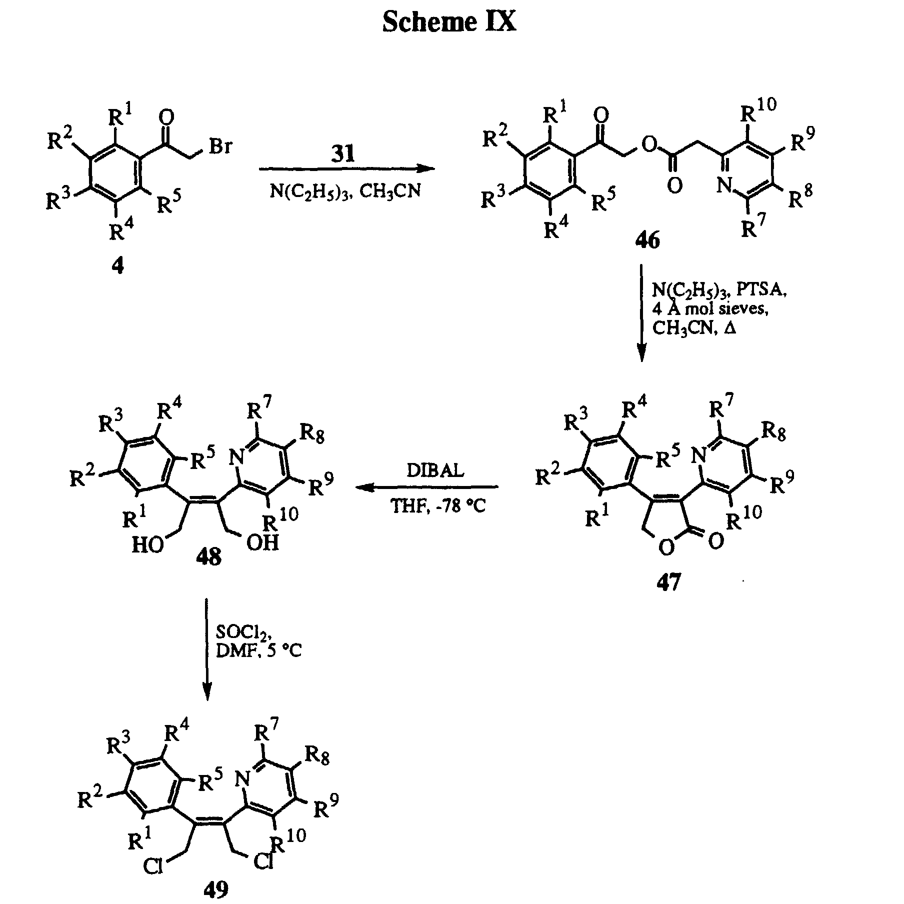

Synthetic Scheme IX shows the four step procedure used to prepare the cis-2-(2-pyridinyl)-3-aryl-1,4-dichloro-2-butenes **49** from the bromoacetophenones **4** (prepared in Synthetic Scheme I) and the 2-pyridinylacetic acids **31** (prepared in Synthetic Scheme VI). In step one, bromoacetophenones **4** are reacted with 2-pyridinylacetic acids **31** in acetonitrile in the presence of triethylamine to give the corresponding esters **46**. In step two, the esters **46** are cyclized to the corresponding furanones **47** on treatment with *p*-toluenesulfonic acid (PTSA) and triethylamine in the presence of 4 Å molecular sieves in acetonitrile at reflux. In step three, the furanones **47** are reacted with diisobutylaluminum hydride (DIBAL) to give the corresponding cis-diols **48**. In step four, the cis-diols **48** are reacted with thionyl chloride in DMF at 5°C to give the corresponding cis-2-(2-pyridinyl)-3-aryl-1,4-dichloro-2-butenes **49**.

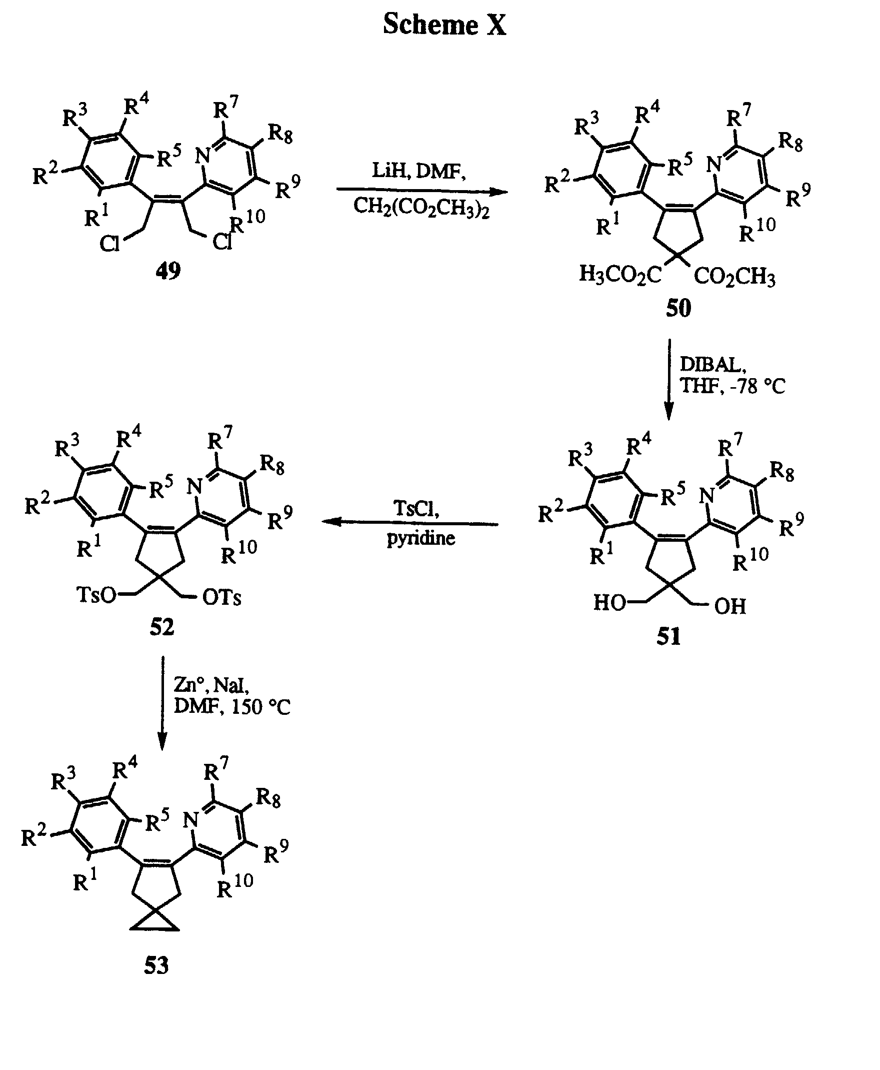

Synthetic Scheme X shows the four step procedure used to prepare the 5-(2-pyridinyl)-6-arylspiro[2.4]hept-5-enes **53** from the cis-2-(2-pyridinyl)-3-aryl-1,4-dichloro-2-butenes **49** (prepared in Synthetic Scheme IX). In step one, the cis-2-(2-pyridinyl)-3-aryl-1,4-dichloro-2-butenes **49** are reacted with dimethyl malonate in DMF in the presence of two equivalents of lithium hydride to give the corresponding 4,4-dicarbomethoxycyclopentenes **50**. In step two, the 4,4-dicarbomethoxycyclopentenes **50** are reacted with DIBAL in THF to give the corresponding 4,4-di(hydroxymethyl)cyclopentenes **51**. In step three, the 4,4-di(hydroxymethyl)cyclopentenes 51 are reacted with *p*-toluenesulfonyl chloride (TsCl) in pyridine to give the corresponding 4,4-ditosylates **52**. In step four, the 4,4-ditosylates **52** are reacted with metallic zinc and sodium iodide in DMF at 150°C to give the 5-(2-pyridinyl)-6-arylspiro[2.4]hept-5-ene antiinflammatory agents **53** of this invention.

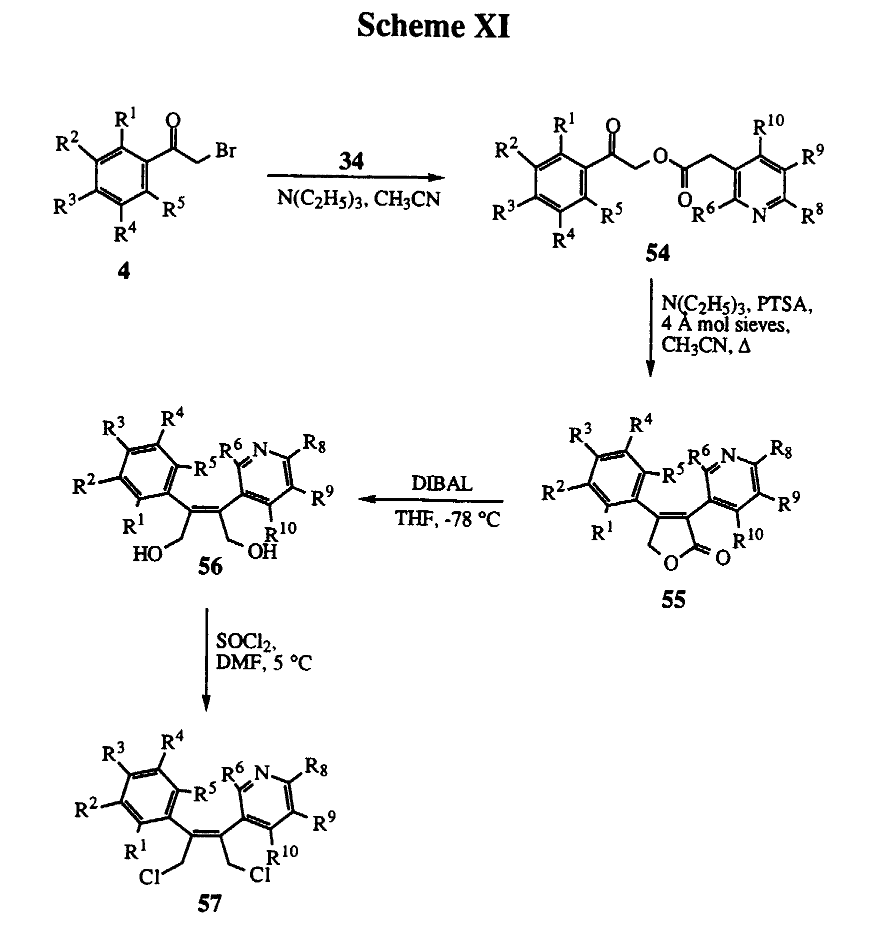

Synthetic Scheme XI shows the four step procedure used to prepare the cis-2-(3-pyridinyl)-3-aryl-1,4-dichloro-2-butenes **57** from the bromoacetophenones **4** (prepared in Synthetic Scheme I) and the 3-pyridinylacetic acids **34** (prepared in Synthetic Scheme VI). In step one, bromoacetophenones **4** are reacted with 3-pyridinylacetic acids **34** in acetonitrile in the presence of triethylamine to give the corresponding esters **54**. In step two, the esters **54** are cyclized to the corresponding furanones **55** on treatment with *p*-toluenesulfonic acid (PTSA) and triethylamine in the presence of 4 Å molecular sieves in acetonitrile at reflux. In step three, the furanones **55** are reacted with diisobutylaluminum hydride (DIBAL) to give the corresponding cis-diols **56**. In step four, the cis-diols **56** are reacted with thionyl chloride in DMF at 5°C to give the corresponding cis-2-(3-pyridinyl)-3-aryl-1,4-dichloro-2-butenes **57**.

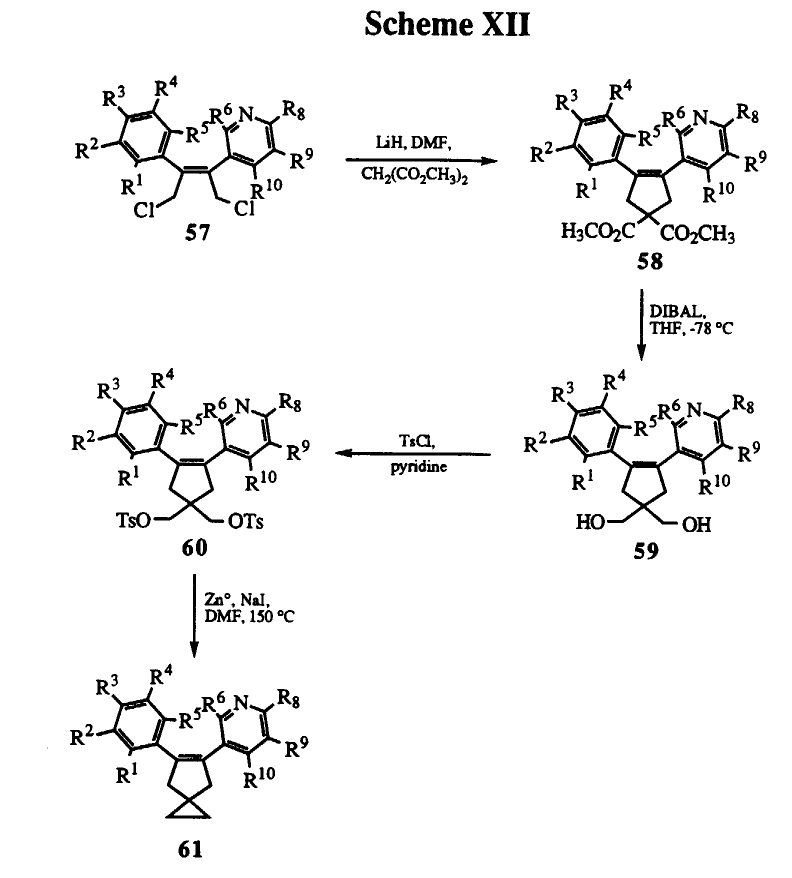

Synthetic Scheme XII shows the four step procedure used to prepare the 5-(3-pyridinyl)-6-arylspiro[2.4]hept-5-enes **61** from the cis-2-(3-pyridinyl)-3-aryl-1,4-dichloro-2-butenes **57** (prepared in Synthetic Scheme XI). In step one, the cis-2-(3-pyridinyl)-3-aryl-1,4-dichloro-2-butenes **57** are reacted with dimethyl malonate in DMF in the presence of two equivalents of lithium hydride to give the corresponding 4,4-dicarbomethoxycyclopentenes **58**. In step two, the 4,4-dicarbomethoxycyclopentenes **58** are reacted with DIBAL in THF to give the corresponding 4,4-di(hydroxymethyl)cyclopentenes **59**. In step three, the 4,4-di(hydroxymethyl)cyclopentenes **59** are reacted with *p*-toluenesulfonyl chloride (TsCl) in pyridine to give the corresponding 4,4-ditosylates **60**. In step four, the 4,4-ditosylates **60** are reacted with metallic zinc and sodium iodide in DMF at 150°C to give the 5-(3-pyridinyl)-6-arylspiro[2.4]hept-5-ene antiinflammatory agents **61** of this invention.

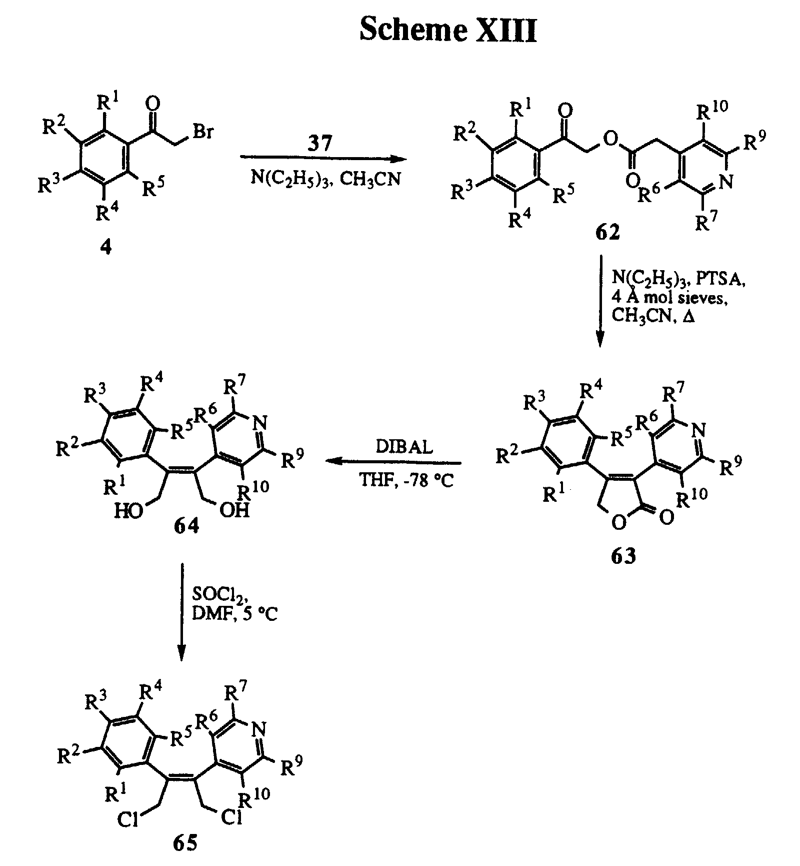

Synthetic Scheme XIII shows the four step procedure used to prepare the cis-2-(4-pyridinyl)-3-aryl-1,4-dichloro-2-butenes **65** from the bromoacetophenones **4** (prepared in Synthetic Scheme I) and the 4-pyridinylacetic acids **37** (prepared in Synthetic Scheme VI). In step one, bromoacetophenones **4** are reacted with 4-pyridinylacetic acids **37** in acetonitrile in the presence of triethylamine to give the corresponding esters **62**. In step two, the esters **62** are cyclized to the corresponding furanones **63** on treatment with *p*-toluenesulfonic acid (PTSA) and triethylamine in the presence of 4 Å molecular sieves in acetonitrile at reflux. In step three, the furanones **63** are reacted with diisobutylaluminum hydride (DIBAL) to give the corresponding cis-diols **64**. In step four, the cis-diols **64** are reacted with thionyl chloride in DMF at 5°C to give the corresponding cis-2-(4-pyridinyl)-3-aryl-1,4-dichloro-2-butenes **65**.

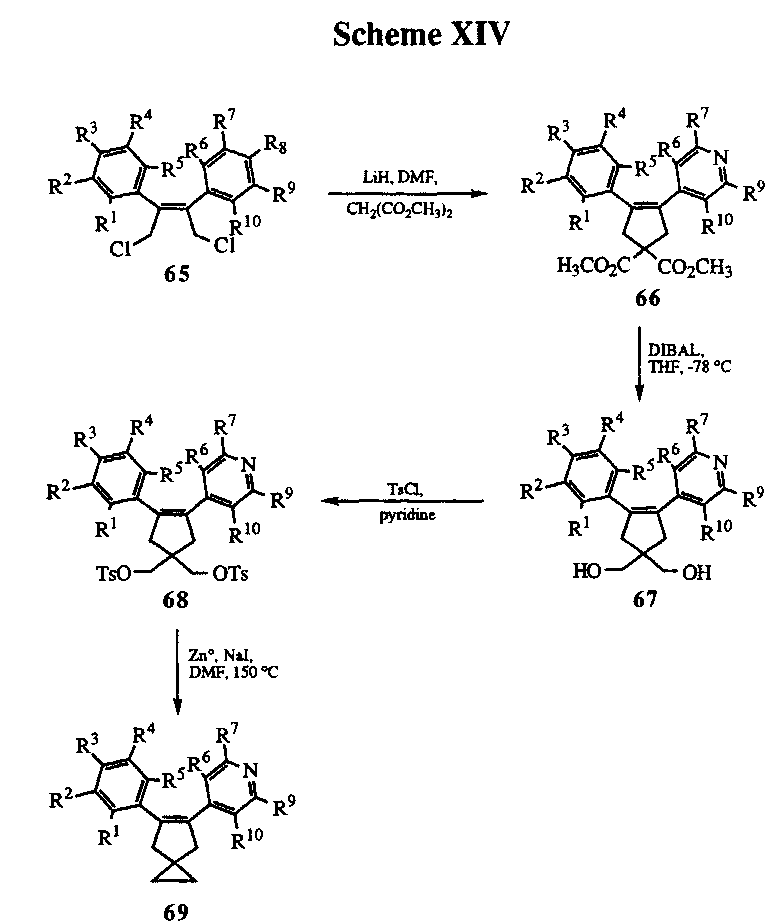

Synthetic Scheme XIV shows the four step procedure used to prepare the 5-(4-pyridinyl)-6-arylspiro[2.4]hept-5-enes **69** from the cis-2-(4-pyridinyl)-3-aryl-1,4-dichloro-2-butenes **65** (prepared in Synthetic Scheme XIII). In step one, the cis-2-(4-pyridinyl)-3-aryl-1,4-dichloro-2-butenes **65** are reacted with dimethyl malonate in DMF in the presence of two equivalents of lithium hydride to give the corresponding 4,4-dicarbomethoxycyclopentenes **66**. In step two, the 4,4-dicarbomethoxycyclopentenes **66** are reacted with DIBAL in THF to give the corresponding 4,4-di(hydroxymethyl)cyclopentenes **67**. In step three, the 4,4-di(hydroxymethyl)cyclopentenes **67** are reacted with *p*-toluenesulfonyl chloride (TsCl) in pyridine to give the corresponding 4,4-ditosylates **68**. In step four, the 4,4-ditosylates **68** are reacted with metallic zinc and sodium iodide in DMF at 150°C to give the 5-(4-pyridinyl)-6-arylspiro[2.4]hept-5-ene antiinflammatory agents **69** of this invention.

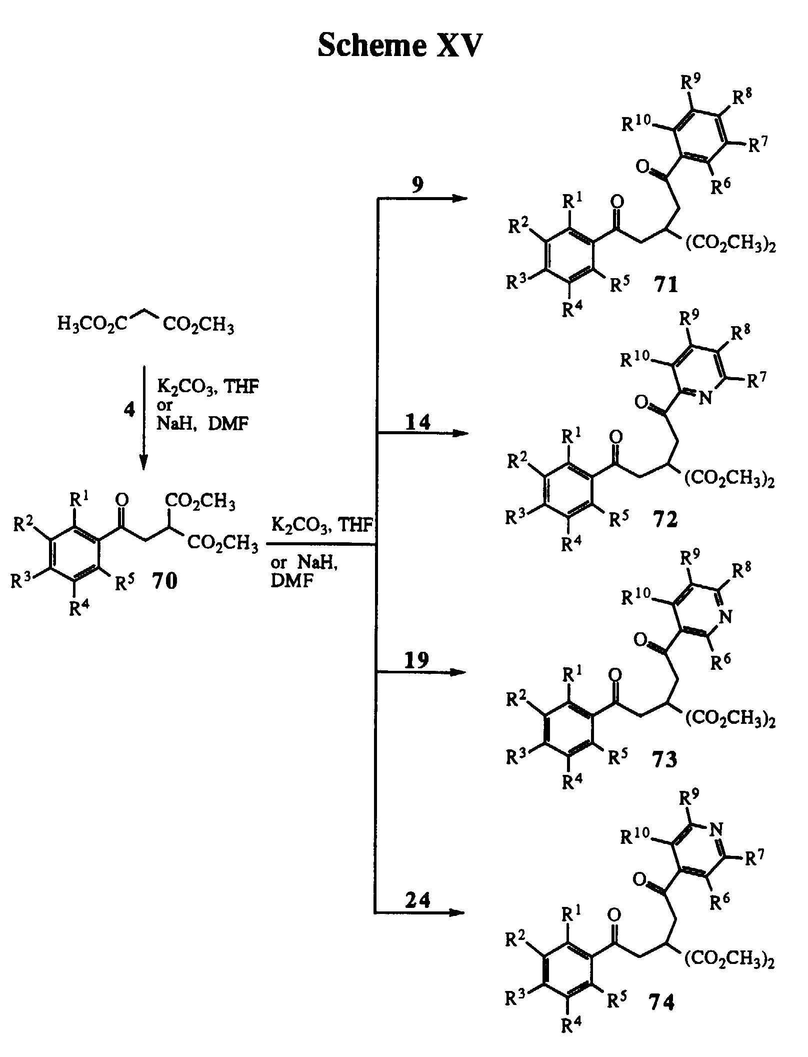

Synthetic Scheme XV shows the two step procedures which can be used to prepare the dialkylated compounds **71, 72, 73**, and **74**. In step one, dimethyl malonate and potassium carbonate in THF (or sodium hydride in DMF) is reacted with the bromoacetophenones **4** (prepared in Synthetic Scheme I) to give the monoalkylated compounds **70**. In step two, the monoalkylated compounds **70** are reacted with the bromoacetophenones **9** (prepared in Synthetic Scheme II), the 2-(bromoacetyl)pyridines **14** (prepared in Synthetic Scheme III), the 3-(bromoacetyl)pyridines **19** (prepared in Synthetic Scheme IV), and the 4-(bromoacetyl)pyridines **24** (prepared in Synthetic Scheme V) in THF in the presence of potassium carbonate (or sodium hydride in DMF) to give the dialkylated compounds **71, 72, 73,** and **74**, respectively.

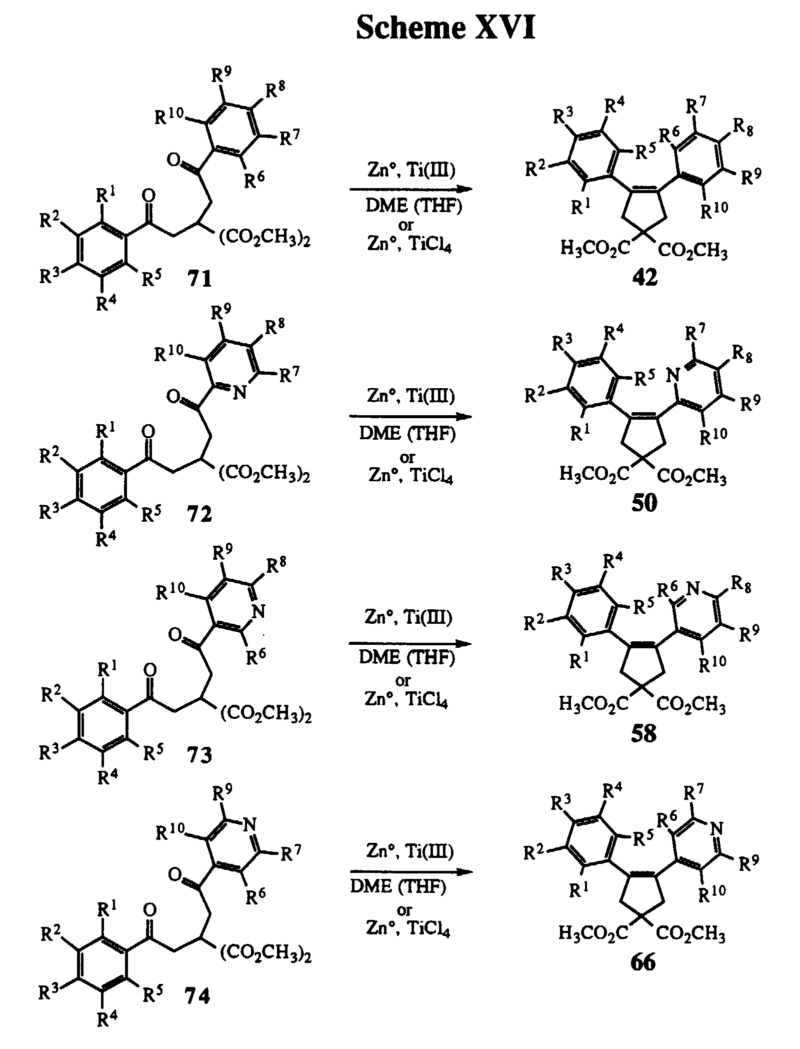

Synthetic Scheme XVI shows alternative procedures which can be used to prepare the 4,4-dicarbomethoxycyclopentenes **42, 50, 58**, and **66** from the dialkylated compounds **71, 72, 73**, and **74**, respectively (prepared in Synthetic Scheme XV). The dialkylated compounds **71, 72, 73**, and **74** are reacted with metallic zinc and titanium(III) chloride [or titanium(IV) chloride] in DME (or THF) to give the 4,4-dicarbomethoxycyclopentenes **42, 50, 58**, and **66,** respectively. By procedures outlined in Schemes VIII, X, XII, and XIV, **42, 50, 58**, and **66** can be converted to the 5,6-diarylspiro[2.4]hept-5-ene antiinflammatory agents **45**, 5-(2-pyridinyl)-6-arylspiro[2.4]hept-5-ene antiinflammatory agents **53**, 5-(3-pyridinyl)-6-arylspiro[2.4]hept-5-ene antiinflammatory agents **61**, and 5-(4-pyridinyl)-6-arylspiro[2.4]hept-5-ene antiinflammatory agents **69**, respectively, of this invention.

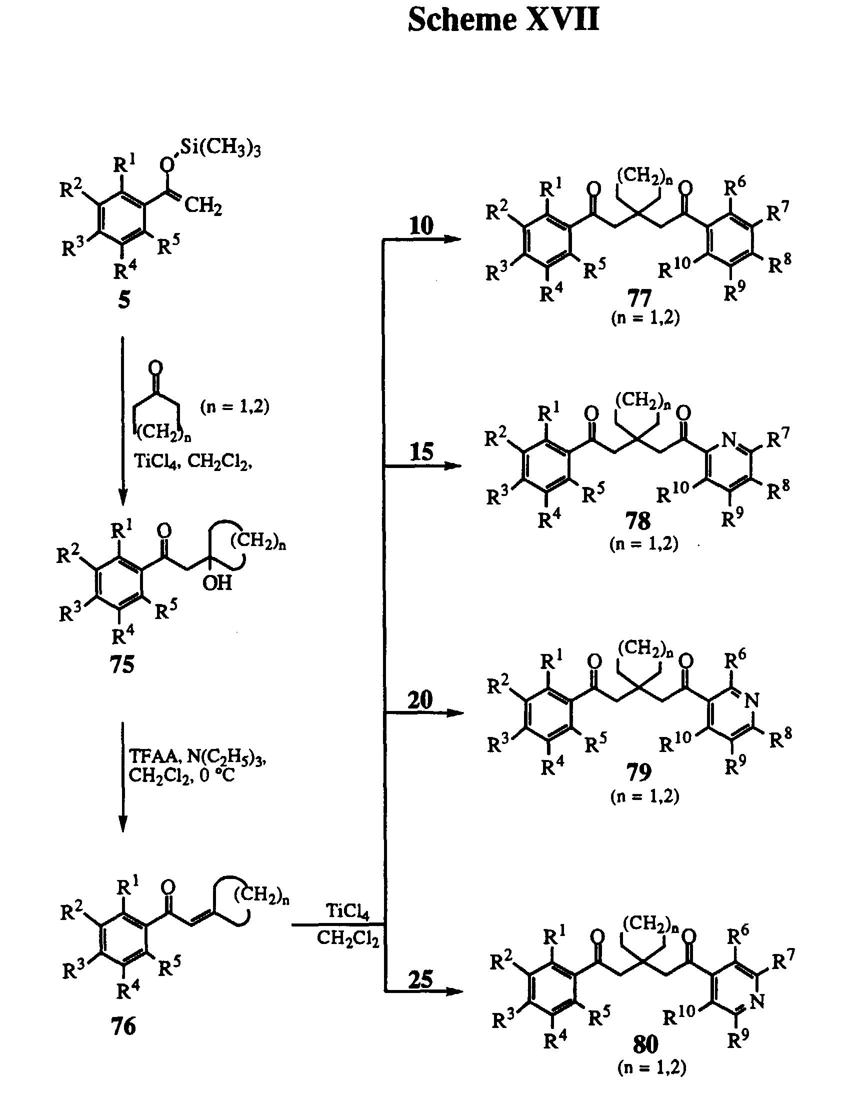

Synthetic Scheme XVII shows the three step procedures used to prepare the cycloalkyldiketones **77**, **78, 79** and **80** from the phenyl silyl enol ethers 5 (prepared in Synthetic Scheme I) and cycloalkanones (n = 1,2). In step one, the silyl enol ethers **5** are reacted with cycloalkanones (n = 1,2) in methylene chloride in the presence of titanium(IV) chloride to give the corresponding cycloalkanols **75**. In step two, the cycloalkanols **75** are dehydrated with trifluoroacetic anhydride and triethylamine in methylene chloride at 0°C to give the corresponding conjugated exocyclic olefins **76**. In step three, the olefins **76** are reacted with the phenyl silyl enol ethers **10** (prepared in Synthetic Scheme II), 2-pyridinyl silyl enol ethers **15** (prepared in Synthetic Scheme III), 3-pyridinyl silyl enol ethers **20** (prepared in Synthetic Scheme IV), and 4-pyridinyl silyl enol ethers **25** (prepared in Synthetic Scheme V) to give the cycloalkyldiketones (n = 1,2) **77, 78, 79** and **80**, respectively.

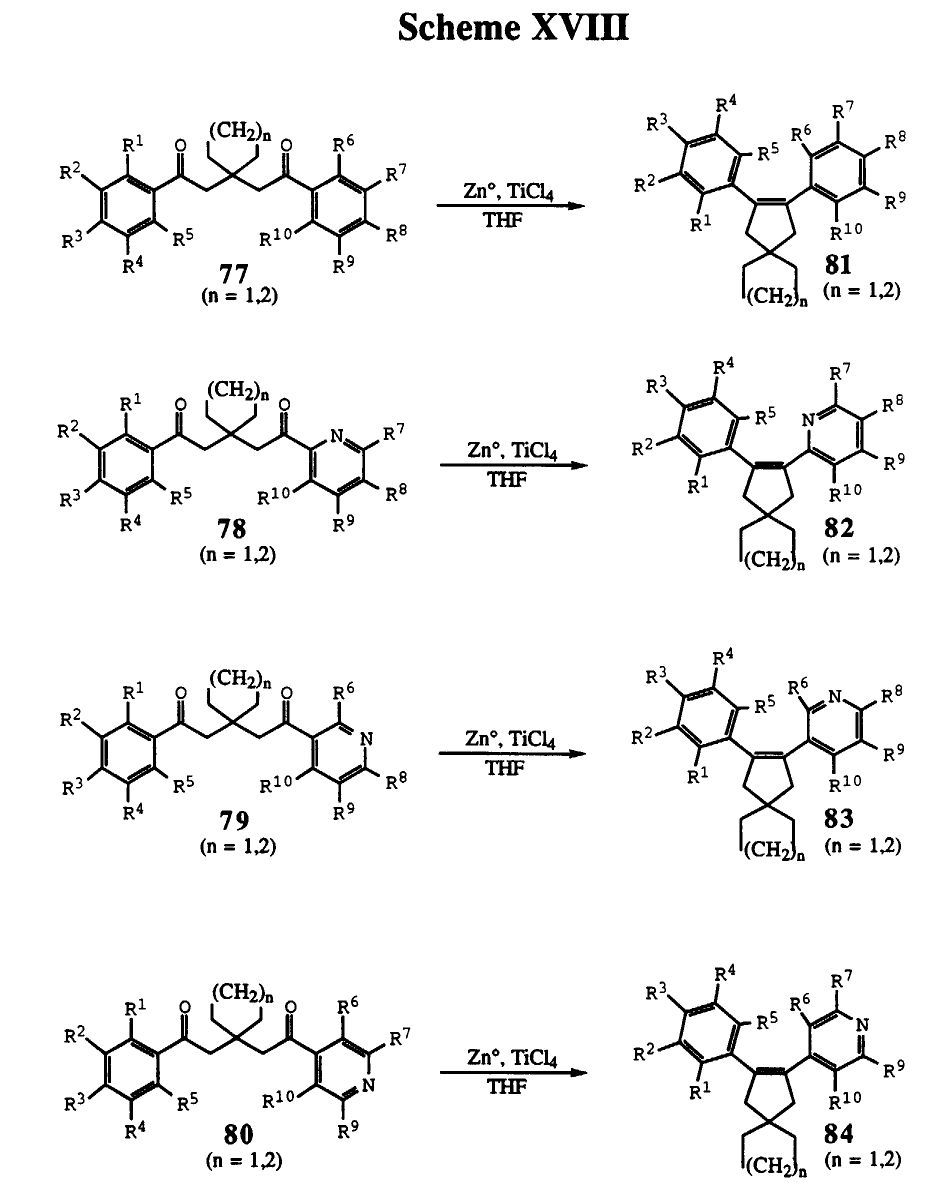

Synthetic Scheme XVIII shows the procedures used to prepare 6,7-diarylspiro[3.4]oct-6-enes **81** (n = 1) and 2,3-diarylspiro[4.4]non-2-enes **81** (n = 2), 6-(2-pyridinyl)-7-arylspiro[3.4]oct-6-enes **82** (n = 1) and 2-(2-pyridinyl)-3-arylspiro[4.4]non-2-enes **82** (n = 2), 6-(3-pyridinyl)-7-arylspiro[3.4]oct-6-enes **83** (n = 1) and 2-(3-pyridinyl)-3-arylspiro[4.4]non-2-enes **83** (n = 2), and 6-(4-pyridinyl)-7-arylspiro[3.4]oct-6-enes **84** (n = 1) and 2-(4-pyridinyl)-3-arylspiro[4.4]non-2-enes **84** (n = 2) from cycloalkyldiketones (n = 1,2) **77, 78, 79** and **80**, respectively (prepared in Synthetic Scheme XVII). The cycloalkyldiketones (n = 1,2) **77, 78, 79** and **80** are reacted metallic zinc and titanium(IV) chloride in THF to give the 6,7-diarylspiro[3.4]oct-6-ene antiinflammatory agents **81** (n = 1) and 2,3-diarylspiro[4.4]non-2-ene antiinflammatory agents **81** (n = 2), 6-(2-pyridinyl)-7-arylspiro[3.4]oct-6-ene antiinflammatory agents **82** (n = 1) and 2-(2-pyridinyl)-3-arylspiro[4.4]non-2-ene antiinflammatory agents **82** (n = 2), the 6-(3-pyridinyl)-7-arylspiro[3.4]oct-6-ene antiinflammatory agents **83** (n = 1) and 2-(3-pyridinyl)-3-arylspiro[4.4]non-2-ene antiinflammatory agents **83** (n = 2), and the 6-(4-pyridinyl)-7-arylspiro[3.4]oct-6-ene antiinflammatory agents **84** (n = 1) and 2-(4-pyridinyl)-3-arylspiro[4.4]non-2-ene antiinflammatory agents **84** (n = 2), respectively, of this invention.

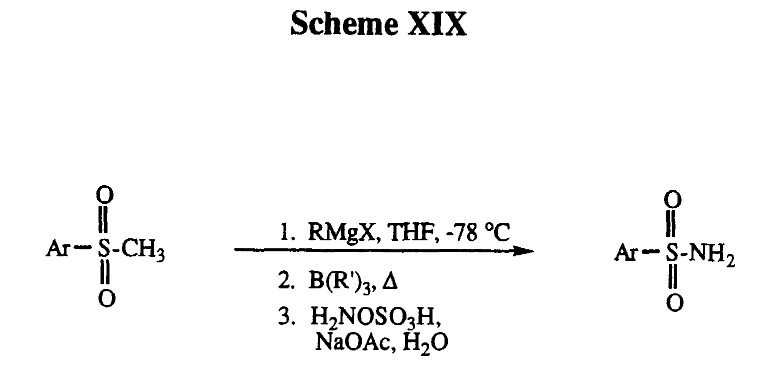

Synthetic Scheme XIX shows the three step procedure used to prepare sulfonamide antiinflammatory agents from their corresponding methyl sulfones. In step one, a THF solution of the methyl sulfones at -78°C is treated with a grignard reagent (RMgX), e.g. methylmagnesium bromide, propylmagnesium chloride, etc., or an alkyllithium reagent, e.g., methyllithium, n-butyllithium, etc. In step two, the anions generated in step one is treated with an organoborane, e.g., triethylborane, tributylborane, etc., at -78°C then allowed to warm to ambient temperature prior to stirring at reflux. In step three, an aqueous solution of sodium acetate and hydroxyamine-O-sulfonic acid is added to provide the corresponding sulfonamide antiinflammatory agents of this invention.

The following examples contain detailed descriptions of the methods of preparation of compounds of Formula I-VI. These detailed descriptions fall within the scope, and serve to exemplify, the above described General Synthetic Procedures which form part of the invention. These detailed descriptions are presented for illustrative purposes only and are not intended as a restriction on the scope of the invention. All parts are by weight and temperatures are in Degrees centigrade unless otherwise indicated.

### Example 1

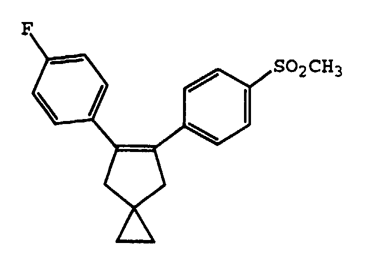

### 5-(4-Fluorophenyl)-6-[4-(methylsulfonyl)phenyl]spiro[2.4]hept-5-ene

### Step 1: Preparation of 4-(methylthio)acetophenone

To a stirred solution of 50 g (340 mmol) of 4-(methylthio)benzonitrile in 2 L of THF at -78°C under an atmosphere of nitrogen was added 282 mL (390 mmol) of methyllithium (1.4 M in diethyl ether) over a period of ten minutes. The solution was stirred at -78°C for one hour, and then the dry ice bath was removed. After five hours, 100 mL of water followed by 200 mL of 3N hydrochloric acid were added to the reaction mixture and it was stirred overnight. Concentration in vacuo gave a residue which was partitioned between ethyl acetate and water. The water layer was extracted with three portions of ethyl acetate and the combined ethyl acetate layers were dried (MgSO₄). Concentration in vacuo gave 58 g of crude (4-methylthio)acetophenone as a solid: NMR (CDCl₃) δ 2.52 (s, 3H), 2.57 (s, 3H), 7.26 (d, J = 9 Hz, 2H), 7.87 (d, J = 9 Hz, 2H).

### Step 2: Preparation of 4-(methylsulfonyl)acetophenone

To a solution of 11.73 g (71.1 mmol) of 4-(methylthio)acetophenone (prepared in Step 1) in 500 mL of methylene chloride at ambient temperature was added 61.14 g (177 mmol) of *m*-chloroperoxybenzoic acid (50%) (MCPBA) in portions over 20 minutes. The reaction was stirred for two hours, quenched slowly with aqueous sodium bisulfite, washed with three 100 mL portions of saturated sodium bicarbonate, dried (MgSO₄), and concentrated in vacuo to give 11.91 g (91%) of (4-methylsulfonyl)acetophenone as a colorless solid: NMR (CDCl₃) δ 2.67 (s, 3H), 3.08 (s, 3H), 8.06 (d, J = 9 Hz, 2H), 8.14 (d, J = 9 Hz, 2H).

### Step 3: Preparation of 2-bromo-4'-(methylsulfonyl) acetophenone

To a stirred solution of 11.91 g (60.5 mmol) of 4-(methylsulfonyl)acetophenone (prepared in Step 2) in 133 mL of glacial acetic acid and 0.11 mL of hydrochloric acid at ambient temperature was added a solution of 8.22 g (51.4 mmol) of bromine in 9.3 mL of glacial acetic acid over a period of three hours. The reaction mixture was diluted with 500 mL of water and extracted with chloroform. The combined extracts were dried (MgSO₄) and concentrated in vacuo to give 15.7 g of crude 2-bromo-(4'-methylsulfonyl)acetophenone as a solid: NMR (CDCl₃) δ 3.10 (s, 3H), 4.45 (s, 2H), 8.08 (d, J = 9 Hz, 2H), 8.17 (d, J = 9 Hz, 2H).

### Step 4: Preparation of 2-(4-fluorophenyl)-1-[2-[4-(methylsulfonyl)phenyl]-2-oxoethoxy]ethanone

To a stirred solution of 4.45 g (28.9 mmol) of 4-fluorophenylacetic acid in 3.26 g (31.8 mmol) of triethylamine and 275 mL of acetonitrile was added 8.9 g (28.9 mmol) of 2-bromo-4'-(methylsulfonyl)acetophenone (prepared in Step 3) at ambient temperature. The reaction mixture was stirred for 30 minutes, concentrated in vacuo, and partitioned between ethyl acetate and water. The organic phase was dried (MgSO₄) and concentrated in vacuo. Purification by silica gel chromatography with ethyl acetate/hexane (1:1) gave 6.87 g (68%) of 2-(4-fluorophenyl)-1-[2-[4-(methylsulfonyl)phenyl]-2-oxoethoxy]ethanone as a colorless solid: NMR (CDCl₃) δ 3.08 (s, 3H), 3.79 (s, 2H), 5.35 (s, 2H), 7.06 (s, t, J = 9 Hz, 2H), 7.32 (dd, J = 6 and 9 Hz, 2H), 8.06 (s, 4H).

### Step 5: Preparation of 3-(4-fluorophenyl)-4-[(4-methylsulfonyl)phenyl]-5H-furan-2-one

Under nitrogen, 4.10 g (11.7 mmol) of 2-(4-fluorophenyl)-1-[2-[4-(methylsulfonyl)phenyl]-2-oxoethoxy]ethanone (prepared in Step 4), 6.52 mL (46.8 mmol) of triethylamine, 4.89 g (25.7 mmol) of *p*-toluenesulfonic acid, and 12 g of 4Å molecular sieves were added to 117 mL of acetonitrile and stirred at reflux for 16 hours. The reaction mixture was concentrated in vacuo and the residue partitioned between methylene chloride and water. The methylene chloride layer was dried (MgSO₄) and reconcentrated in vacuo. Recrystallization from hexane/ethyl acetate (2:1) gave 3.65 g (94%) of 3-(4-fluorophenyl)-4-[(4-methylsulfonyl)phenyl]-5H-furan-2-one as a solid: mp 166-l67°C; NMR (CDCl₃) δ 3.08 (s, 3H), 5.19 (s, 2H), 7.10 (t, J = 9 Hz, 2H), 7.42 (dd, J = 6 and 9 Hz, 2H), 7.52 (d, J = 9 Hz, 2H), 7.97 (d, J = 9 Hz, 2H). HRMS Calc'd for C₁₇H₁₃FO₄S: 332.0519. Found: 332.0501. Anal. Calc'd for C₁₇H₁₃FO₄S: C, 61.44; H, 3.94; O, 19.26. Found: C, 61.11; H, 4.06; O, 19.32.

### Step 6: Preparation of 2-(4-fluorophenyl)-3-[(4-methylsulfonyl)phenyl]-1,4-dihydroxy-2-butene

To a solution of 3.08 g (9.28 mmol) of 3-(4-fluorophenyl)-4-[(4-methylsulfonyl)phenyl]-5H-furan-2-one (prepared in Step 5) in 93 mL of tetrahydrofuran (THF) at -78°C under an atmosphere of nitrogen was added 20 mL (30 mmol) of diisobutylaluminum hydride (DIBAL) (1.5 M in THF) over a 10 minute period. The solution was stirred at -78°C for 20 minutes, allowed to warm to ambient temperature, and stirred overnight. An additional 15 mL (22 mmol) aliquot of DIBAL was added and stirring was continued for 2 hours. The reaction was cooled to -78°C, treated dropwise with 25 mL of acetone, warmed to room temperature, and slowly treated with 25 mL of water. The mixture was stirred for 30 minutes prior to the careful addition of 35 mL of 1.2 N sodium hydroxide. The mixture was extracted with ethyl acetate, washed with 1 N hydrochloric acid followed by brine, dried (MgSO₄), and concentrated in vacuo to give 3.8 g of crude 2-(4-fluorophenyl)-3-[(4-methylsulfonyl)phenyl]-1,4-dihydroxy-2-butene as a colorless oil: NMR (CDCl₃) δ 2.98 (s, 3H), 4.60 (d, J = 6 Hz, 4H), 6.8 (t, J = 9 Hz, 2H), 6,94-7.02 (m, 2H), 7.22 (d, J = 9 Hz, 2H), 7.65 (d, J = 9 Hz, 2H).

### Step 7: Preparation of 2-(4-fluorophenyl)-3-[(4-methylsulfonyl)phenyl]-1,4-dichloro-2-butene

To a solution of 3.5 g (7.62 mmol) of crude 2-(4-fluorophenyl)-3-[(4-methylsulfonyl)phenyl]-1,4-dihydroxy-2-butene (prepared in Step 6) in 58 mL of N,N-dimethylformamide (DMF) at 5°C under an atmosphere of nitrogen was added dropwise 1.52 mL (20.84 mmol) of thionyl chloride. The reaction was stirred at 5°C for 22 hours, stirred at ambient temperature for an additional 8 hours, and concentrated in vacuo. The residue was partitioned between ethyl acetate and water; the ethyl acetate phase was dried (MgSO₄) and concentrated in vacuo to give crude 2-(4-fluorophenyl)-3-[(4-methylsulfonyl)phenyl]-1,4-dichloro-2-butene as a solid: NMR (CDCl₃) δ 3.0 (s, 3H), 4.55 (d, J = 3.4 Hz, 4H), 6.86 (t, J = 9 Hz, 2H), 6.75 (d, J = 8.3 Hz, 2H), 7.45 (d, J = 9 Hz, 2H).

### Step 8, A: Preparation of 1-[2-(4-fluorophenyl)-4,4-dicarbomethoxycyclopenten-1-yl]-4-(methylsulfonyl)benzene

To a solution of 1.2 mL (10.5 mmol) of dimethyl malonate in 10 mL of DMF under an atmosphere of nitrogen was added 215 mg (26.9 mmol) of lithium hydride in portions. The resulting suspension was stirred at ambient temperature for 20 minutes prior to the addition of a solution of crude 2-(4-fluorophenyl)-3-[(4-methylsulfonyl)phenyl]-1,4-dichloro-2-butene (prepared in Step 7) in 10 mL of DMF. The reaction was stirred at ambient temperature for 15 hours, treated with another 150 mg (18.8 mmol) of lithium hydride, and stirred for another 4 hours. The mixture was concentrated in vacuo and partitioned between ethyl acetate and water; the organic phase was dried (MgSO₄), and concentrated in vacuo. The residue was chromatographed on silica gel to give 1.1 g (34%) of 1-[2-(4-fluorophenyl)-4,4-dicarbomethoxycyclopenten-1-yl]-4-(methylsulfonyl)benzene as an oil: NMR (CDCl₃) δ 3.03 (s, 3H), 3.55 (s, 4H), 3.79 (s, 6H), 6.93 (t, J = 9 Hz, 2H), 7.11 (dd, J= 6 and 9 Hz, 2H), 7.32 (d, J = 9 Hz, 2H), 7.77 (d, J = 9 Hz, 2H).

### Step 8, B: Preparation of 1-[2-(4-fluorophenyl)-4,4-dicarbomethoxycyclopenten-1-yl]-4-(methylsulfonyl)benzene

To a solution of 7.18 mL (63 mmol) of dimethyl malonate in 160 mL of DMF at 0°C under an atmosphere of nitrogen was added 3.0 g (75 mmol) of sodium hydride (60% suspension in oil). The reaction was stirred at ambient temperature for 15 minutes (or until the gas evolution has ceased), cooled to -20°C, and treated with 15 g (69 mmol) of 2-bromo-4'-fluoroacetophenone (Aldrich) in one portion. The mixture was stirred at ambient temperature for 1 hour and then cooled to 0°C; another 75 mmol of sodium hydride was added and the resulting mixture stirred at ambient temperature for 15 minutes (or until the gas evolution has ceased). The reaction was recooled to -20°C and treated with 19.1 g (69 mmol) of 2-bromo-4'-(methylsulfonyl)acetophenone (prepared in Step 3). The reaction was stirred at room temperature for 2 hours and concentrated in vacuo. The residue was partitioned between water and ethyl acetate; the ethyl acetate phase was dried (MgSO₄) and reconcentrated in vacuo. The residue was chromatographed on silica gel to give 13.8 g (51%) of dimethyl 2-[2-(4-fluorophenyl)-2-oxoethyl]-2-[2-[4-(methylsulfonyl)phenyl]-2-oxoethyl]propanedioate as an oil: NMR (CDCl₃) δ 3.06 (s, 3H), 3.76 (s, 6H), 4.03 (s, 2H), 4.08 (s, 2H), 7.13 (t, J = 8.6 Hz, 2H), 7.97-8.05 [m with d at 8.03 (J= 8.7 Hz), 4H], 8.14 (d, J = 8.5 Hz, 2H).

To a vigorously stirred mixture of 50.4 g (771 mmol) of zinc dust in 640 mL of THF at -78°C under an atmosphere of nitrogen was added dropwise 60.4 mL (551 mmol) of titanium(IV) chloride. The reaction was warmed to ambient temperature with a water bath and then stirred at reflux for 1 hour. To the resulting dark mixture under reflux was added a solution of 15 g (32.3 mmol) of dimethyl 2-[2-(4-fluorophenyl)-2-oxoethyl]-2-[2-[4-(methylsulfonyl)phenyl]-2-oxoethyl]propanedioate (prepared above) in 20 mL of THF. The resulting mixture was stirred at ambient temperature for 16 hours, filtered through a pad of Celite®, rinsed with ethyl acetate, and concentrated in vacuo. The residue was partitioned between water and ethyl acetate; the organic phase was washed with brine, dried (MgSO₄), and concentrated in vacuo. The residue was chromatographed on silica gel to give 6.26 g (44%) of 1-[2-(4-fluorophenyl)-4,4-dicarbomethoxycyclopenten-l-yl]-4-(methylsulfonyl)benzene which was identical to the material prepared in Step 8, Method A.

### Step 9: Preparation of 1-[2-(4-fluorophenyl)-4,4-di(hydroxymethyl)cyclopenten-1-yl]-4-(methylsulfonyl)benzene

Under nitrogen, a solution of 1.01 g (2.34 mmol) of 1-[2-(4-fluorophenyl)-4,4-dicarbomethoxycyclopenten-l-yl]-4-(methylsulfonyl)benzene (prepared in Step 8) in 1.5 mL of THF at -78°C was treated with 11.6 mL (11.6 mmol) of DIBAL (1.0 M in THF). The reaction was stirred at ambient temperature for 1.5 hours, quenched with acetone and aqueous NaOH, extracted with ethyl acetate, dried (MgSO₄), and concentrated in vacuo to give 840 mg of crude 1-[2-(4-fluorophenyl)-4,4-di(hydroxymethyl)cyclopenten-1-yl]-4-(methylsulfonyl)benzene as a colorless oil: NMR (CDCl₃) δ 2.82 (d, J = 5 Hz, 4H), 3.04 (s, 3H), 3.86 (d, J= 5 Hz, 4H), 6.94 (t, J = 9 Hz, 2H), 7.11 (dd, J = 5 and 9 Hz, 2H), 7.33 (d, J = 9 Hz, 2H), 7.77 (d, J = 9 Hz, 2H).

### Step 10: Preparation of 1-[2-(4-fluorophenyl)-4,4-di(tosylmethyl)cyclopenten-1-yl]-4-(methylsulfonyl)benzene

Under nitrogen, a solution of 2.34 mmol of the crude l-[2-(4-fluorophenyl)-4,4-di(hydroxymethyl)cyclopenten-1-yl]-4-(methylsulfonyl)benzene (prepared in Step 9) in 8 mL of pyridine at ambient temperature was treated with 1.2 g (6.3 mmol) of *p*-toluenesulfonyl chloride (tosyl chloride). The resulting solution was stirred at room temperature for 17 hours, concentrated in vacuo, and chromatographed on silica gel to give 1.06 g (66% overall yield from Step 9) of l-[2-(4-fluorophenyl)-4,4-di(tosylmethyl)cyclopenten-l-yl]-4-(methylsulfonyl)benzene as a colorless solid: NMR (CDCl₃) δ 2.46 (s, 6H), 2.73 (s, 3H), 3.04 (s, 3H), 4.05 (s, 4H), 6.85-7.0 (m, 4H), 7.20 (d, J = 8 Hz, 2H), 7.34 (d, J = 8 Hz, 4H), 7.75 (d, J = 8 Hz, 6H).

### Step 11: Preparation of 5-(4-fluorophenyl)-6-[4-(methylsulfonylphenyl]spiro[2,4]hept-5-ene

Under nitrogen, a solution of 1.02 g (1.49 mmol) of 1-[2-(4-fluorophenyl)-4,4-di(tosylmethyl)cyclopenten-1-yl]-4-(methylsulfonyl)benzene (prepared in Step 10) in 24 mL of DMF was treated with 3.23 g (21.55 mmol) of sodium iodide and 1.61 g (24.63 mmol) of zinc dust. The reaction was stirred at 150°C for 1.5 hour, concentrated in vacuo, and partitioned between water and ethyl acetate. The organic phase was washed with sodium sulfite, water, brine, dried (MgSO₄), and concentrated in vacuo. The residue was chromatographed on silica gel to give 437 mg (86%) of 5-(4-fluorophenyl)-6-[4-(methylsulfonyl)phenyl]spiro[2.4]hept-5-ene as a colorless solid: mp 140.5-142.0°C; NMR (CDCl₃) δ 0.69 (s, 4H), 2.92 (s, 4H), 3.04 (s, 3H), 6.93 (t, J = 9 Hz, 2H), 7.10 (dd, J = 5 and 9 Hz, 2H), 7.32 (d, J = 8 Hz, 2H), 7.76 (d, J = 8 Hz, 2H). HRMS Calc'd for C₂₀H₁₉FO₂S: 342.1090. Found: 342.1126. Anal. Calc'd for C₂₀H₁₉FO₂S: C, 70.15; H, 5.59; F, 5.55; S, 9.36. Found: C, 70.10; H, 5.69; F, 5.50; S, 9.60.

### Example 2

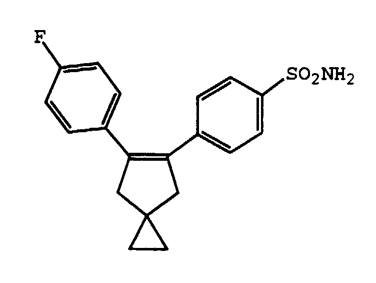

### 4-[6-(4-Fluorophenyl)spiro[2.4]hept-5-en-5-yl]benzenesulfonamide

Under nitrogen, a solution of 90 mg (0.248 mmol) of 5-(4-fluoro phenyl)-6-[4-(methylsulfonyl)phenyl]spiro[2.4]hept-5-ene (the title compound of Example 1) in 1 mL of THF at -78°C was treated with 0.21 mL (0.27 mmol) of methyllithium (1.3 M in ether) over a period of 2 minutes. The reaction was stirred at ambient temperature for 25 minutes, cooled to -78°C, and treated with 0.3 mL (0.3 mmol) of tributylborane (1.0 M in THF). The resulting dark brown solution was stirred at ambient temperature for 20 minutes and then at reflux for 16 hours prior to the addition of 350 mg (4.27 mmol) of sodium acetate, 2 mL of water, and 250 mg (2.21 mmol) of hydroxyamine-O-sulfonic acid. The resulting light orange mixture was stirred at ambient temperature for 3 hours and the aqueous phase extracted with ethyl acetate. The combined extracts were washed with water, brine, dried (MgSO₄), and concentrated in vacuo. The residue was chromatographed on silica gel to give 24 mg (27%) of 4-[6-(4-fluoro phenyl)spiro[2.4]hept-5-en-5-yl]benzenesulfonamide as a colorless solid: mp 131.0-133.0°C; NMR (CDCl₃) δ 0.68 (s, 4H), 2.90 (s, 3H), 4.81 (s, 2H), 6.92 (t, J = 9 Hz, 2H), 7.11 (dd, J = 6 and 9 Hz, 2H), 7.27 (d, J = 9 Hz, 2H), 7.74 (d, J = 9 Hz, 2H). HRMS Calc'd for C₁₉H₁₈FNO₂S: 344.1121. Found: 344.1122. Anal. Calc'd for [C₁₉H₁₈FNO₂S + 0.1 CH₃CO₂CH₂CH₃]: C, 66.16; H, 3.98; S, 9.11. Found: C, 65.86; H, 5.52; N, 3.92; S, 9.57.

### Example 3

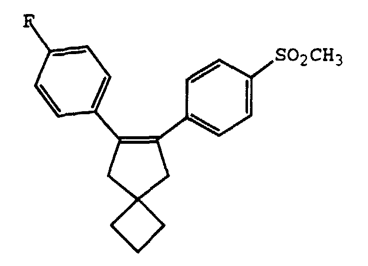

### 6-(4-Fluorophenyl)-7-[4-(methylsulfonyl) phenyl]spiro[3.4]oct-6-ene

### Step 1: Preparation of 1-methylthio-4-[1-[(trimethylsilyl)oxy]ethenyl]benzene

Under nitrogen, 11.0 g (66.2 mmol) of 4-(methylthio)acetophenone (prepared in Step 1 of Example 1) and 13.8 mL (99 mmol) of triethylamine in 50 mL of acetonitrile was treated with 12.6 mL (99.3 mmol) of chlorotrimethylsilane at ambient temperature and allowed to stir for 20 minutes prior to the slow addition of a suspension of 14.9 g (99.4 mmol) of sodium iodide in 60 mL of acetonitrile. The reaction was stirred for 3 hours, poured into ice/water, and extracted with hexane. The extracts were combined, dried (K₂CO₃), and concentrated in vacuo to give 16 g of crude 1-methylthio-4-[1[(trimethylsilyl)oxy]ethenyl]benzene as an oil: NMR (CDCl₃) δ 0.26 (s, 9H), 2.48 (s, 3H), 4.39 (d, J= 2 Hz, 1H), 4.87 (d, J= 2 Hz, 1H), 7.20 (d, J= 8 Hz, 2H), 7.50 (d, J= 8 Hz, 2H).

### Step 2: Preparation of 1-fluoro-4-[1-[(trimethylsilyl)oxy]ethenyl]benzene

Under nitrogen, 17.7 g (128 mmol) of 4-fluoroacetophenone (Aldrich) and 20.7 mL (192 mmol) of triethylamine at ambient temperature was treated with 24.4 mL (192.3 mmol) of chlorotrimethylsilane and allowed to stir of 20 minutes prior to the slow addition of a suspension of 30 g (200 mmol) of sodium iodide in 200 mL of acetonitrile. The extracts were combined, dried (K₂CO₃), and concentrated in vacuo to give 27 g of crude l-fluoro-4-[l[(trimethylsilyl)oxy]ethenyl]benzene as an oil: NMR (CDCl₃) δ 0.28 (s, 9H), 4.41 (d, J= 2 Hz, 1H), 4.84 (d, J= 2 Hz, 1H), 7.00 (d, J= 8 Hz, 2H), 7.53-7.60 (m, 2H).

### Step 3: Preparation of 1-(4-fluorophenyl)-2-(1-hydroxycyclobutan-1-yl)ethan-1-one

Under nitrogen, 11.0 g (100 mmol) of titanium(IV) chloride in 140 mL of methylene chloride at 0°C was slowly treated with a solution of 8.2 mL (110 mmol) of cyclobutanone in 30 mL of methylene chloride prior to the dropwise addition of a solution of 21.1 g (100 mmol) of 1-fluoro-4-[1[(trimethylsilyl)oxy]ethenyl]benzene (obtained from Step 2 ) in 15 mL of methylene chloride. The reaction was stirred for 15 minutes and then poured into 200 mL of ice/water; the phases were separated. The aqueous phase was extracted twice with 30 mL of methylene chloride and combined with the original methylene chloride phase. The combined extracts were washed 3 times with 120 mL of saturated sodium carbonate/water (1:1) and once with brine, dried (MgSO₄), and concentrated in vacuo to give 20.4 g (98%) of crude 1-(4-fluorophenyl)-2-(1-hydroxycyclobutan-1-yl)ethan-1-one as an oil: NMR (CDCl₃) δ 1.53-1.70 (m, 1H), 1.80-1.94 (m, 1H), 1.99-2.10 (m, 2H), 2.17-2.31 (m, 2H), 3.31 (s, 2H), 7.10-7.19 (m, 2H), 7.95-8.03 (m, 2H).

### Step 4: Preparation of 1-(4-fluorophenyl)-2-(cyclobutanyliden-1-yl)ethan-1-one

Under nitrogen, 20.3 g (98 mmol) of 1-(4-fluorophenyl)-2-(1-hydroxycyclobutan-1-yl)ethan-1-one (prepared in Step 3), 37 mL (260 mmol) of triethylamine, and 50 mg of 4-dimethylaminopyridine (DMAP) in 80 mL of methylene chloride at 0°C was slowly treated with a solution of 16.6 mL (118 mmol) of trifluoroacetic anhydride (TFAA) in 40 mL of methylene chloride. The reaction was allowed to stir for 3 hours at 0°C and warmed to ambient temperature to stir for an additional 3 hours prior to the addition of 200 mL of saturated sodium carbonate/water (1:1) and 300 mL of ether. The phases were separated and the aqueous phase was extracted twice with 100 mL of ether. The ether extracts were combined with the original ether/methylene chloride phase, washed with brine, dried (MgSO₄), and concentrated in vacuo. Purification by silica gel chromatography (Waters Prep-500A) with ethyl acetate/hexane (2:98) gave 12.1 g (65%) of 1-(4-fluorophenyl)-2-(cyclobutanyliden-1-yl)ethan-1-one as an oil: NMR (CDCl₃) δ 2.11-2.24 (m, 2H), 2.95 (t, J= 8 Hz, 2H), 3.19-3.29 (m, 2H), 2.68-2.74 (m, 1H), 7.05-7.16 (m, 2H), 7.84-7.97 (m, 2H).

### Step 5: Preparation of 1-(4-fluorophenyl)-2-[1-[2-[4-(methylthio)phenyl]-2-oxoethyl]cyclobutan-1-yl] ethan-1-one

Under nitrogen, 7.2 mL (70.2 mmol) of titanium(IV) chloride in 100 mL of methylene chloride at -78°C was slowly treated with a solution of 12.1 g (63.8 mmol) of l-(4-fluorophenyl)-2-(cyclobutanyliden-1-yl)ethan-1-one (prepared in Step 4) in 30 mL of methylene chloride. The mixture was stirred for 10 minutes, then 16.7 g (70.2 mmol) of the silyl enol ether (from Step 1) in 40 mL of methylene chloride was added dropwise. The reaction was stirred at -78°C for 1 hour, poured into a solution of 22 g of sodium carbonate in 160 mL of water, and filtered through Celite®. The phases were separated and the aqueous phase extracted twice with 40 mL of methylene chloride. The extracts were combined with the original methylene chloride phase and washed with brine, dried (MgSO₄), and concentrated in vacuo. Purification by silica gel chromatography (Waters Prep-500A) with ethyl acetate/hexane (10:90) gave 1-(4-fluorophenyl)-2-[1-[2-[4-(methylthio)phenyl]-2-oxoethyl]cyclobutan-1-yl]ethan-1-one as an oil: NMR (CDCl₃) δ 1.91-2.04 (m, 2H), 2.11 (t, J= 8 Hz, 4H), 2.49 (s, 3H), 3.48 (s, 2H), 3.49 (s, 2H), 7.08 (t, J= 8 Hz, 2H), 7.23 (t, J= 8 Hz, 2H), 7.84 (d, J= 9 Hz, 2H), 7.91-7.99 (m, 2H).

### Step 6: Preparation of 1-(4-fluorophenyl)-2-[1-[2-[4-(methylsulfonyl)phenyl]-2-oxoethyl]cyclobutan-1-yl]ethan-1-one

A solution of 18.3 g (51.4 mmol) of 1-(4-fluorophenyl)-2-[1-[2-[4-(methylthio)phenyl]-2-oxoethyl]cyclobutan-l-yl]ethan-1-one (prepared in Step 5) in 200 mL of chloroform at 10°C was slowly treated with 35.6 g (ca. 103 mmol) of solid m-chloroperbenzoic acid (50-60%). The reaction was allowed to stir for 30 minutes and treated with aqueous sodium bisulfite. The chloroform was removed in vacuo and the residue partitioned between ethyl acetate and water. The ethyl acetate extracts were washed 3 times with saturated sodium bicarbonate and once with brine, dried (MgSO₄), and concentrated in vacuo to give 19.27 g (97%) of 1-(4-fluorophenyl)-2-[1-[2-[4-(methylsulfonyl)phenyl]-2-oxoethyl]cyclobutan-l-yl]ethan-l-one as an oil: NMR (CDCl₃) δ 1.95-2.06 (M, 2H), 2.11 (t, J= 7 Hz, 4H), 3.05 (s, 3H), 3.52 (s, 2H), 3.59 (s, 2H), 7.09 (t, J= 9 Hz, 2H), 7.92-8.04 (m, 4H), 8.19 (d, J= 9 Hz, 2H).

### Step 7: Preparation of 6-(4-fluorophenyl)-7-[4-(methylsulfonyl)phenyl]spiro[3.4]oct-6-ene

Under nitrogen, 16.3 mL (149 mmol) of titanium(IV) chloride was slowly added to a suspension of 19.5 g (298 mmol) of zinc dust in 500 mL of anhydrous THF at -78°C. The resulting mixture was allowed to warm to ambient temperature and then to stir at reflux for 45 minutes. The reaction was cooled to ambient temperature prior to the addition of 19.27 g (49.6 mmol) of neat 1-(4-fluorophenyl)-2-[1-[2-[4-(methylsulfonyl)phenyl]-2-oxoethyl]cyclobutan-l-yl]ethan-l-one (prepared in Step 6) by syringe. The reaction was allowed to stir at ambient temperature overnight, filtered through Celite®, and concentrated in vacuo. The residue was partitioned between ethyl acetate and water; the ethyl acetate phase was washed with brine, dried (MgSO₄), and concentrated in vacuo. Purification by silica gel chromatography (Waters Prep-500A) with ethyl acetate/hexane (20:80) gave 13.5 g (76%) of 6-(4-fluorophenyl)-7-[4-(methylsulfonyl)phenyl] spiro[3.4]oct-6-ene as a colorless solid: mp 123-124°C; NMR (CDCl₃) δ 1.85-1.98 (m, 2H), 2.08 (t, J= 7 Hz, 4H), 2.98 (s, 4H), 3.04 (s, 3H), 6.92 (t, J= 9 Hz, 7.05-7.13 (m, 2H), 7.30 (t, J= 8 HZ, 2H), 7.75 (t, J= 8 Hz, 2H). MS (FAB) m/e 357 (M+H). Anal. Calc'd for C₂₁H₂₁FO₂S: C, 70.76; H, 5.94; F, 5.53; S, 8.99. Found: C, 70.76; H, 6.10; F, 5.20; S, 8.96.

### Example 4

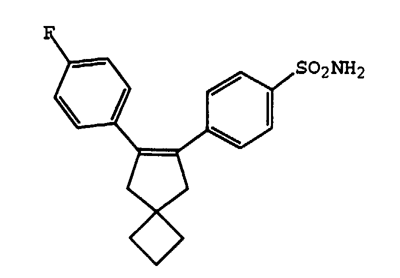

### 4-[7-(4-Fluorophenyl)spiro[3.4]oct-6-en-6-yl]benzenesulfonamide

Following a procedure similar to the one described in Example 2, 1.76 g (4.94 mmol) of 6-(4-fluorophenyl)-7-[4-(methylsulfonyl)phenyl]spiro[3.4]oct-6-ene (the title compound of Example 3) was converted to 1.61 g of crude sulfonamide. Purification by silica gel chromatography with ethyl acetate/hexane (20:80) and subsequent recrystallization from chloroform/hexane gave 970 mg (55%) of 4-[7-(4-fluorophenyl)spiro[3.4]oct-6-en-6-yl]benzenesulfonamide as a colorless solid: mp 118-119°C; NMR (CDCl₃) δ 1.92 (m, J= 8 Hz, 2H), 2.08 (t, J= 7 Hz, 4H), 2.97 (s, 3H), 4.74 (s, 2H), 6.92 (t, J= 9 Hz, 2H), 7.06-7.13 (m, 2H), 7.23-7.30 (m, 2H), 7.74 (t, J= 8 Hz, 2H). MS (EI) m/e (rel intensity) 357 (100), 329 (48), 248 (66), 233 (44), 109 (32). Anal. Calc'd for C₂₀H₂₀FNO₂S: C, 67.21; H, 5.64; N, 3.93; F, 5.32; S, 8.97. Found: C, 66.83; H, 5.89; N, 3.83; F, 4.96; S, 9.03.

### Example 5

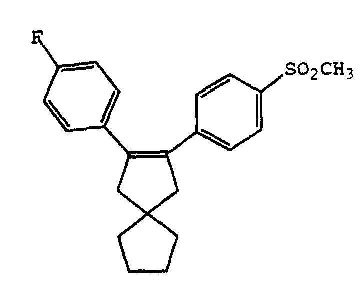

### 2-(4-Fluorophenyl)-3-[4-(methylsulfonyl) phenyl]spiro[4.4]non-2-ene

Following a procedure similar to the one described in Example 3 with the substitution of cyclopentanone for cyclobutanone, 23 mg of 2-(4-fluorophenyl)-3-[4-(methylsulfonyl)phenyl]spiro[4.4]non-2-ene was obtained as a colorless solid: mp 142-143°C; NMR (CDCl₃) δ 1.72 (s, 8H), 2.83 (s, 4H), 3.04 (s, 3H), 6.93 (t, J= 9 Hz, 2H), 7.10 (dd, J= 5 and 9 Hz, 2H), 7.31 (d, J= 9 Hz, 2H), 7.76 (d, J= 9 Hz, 2H). HRMS Calc'd for C₂₂H₂₃FO₂S: 370.1403. Found: 370.1411. Anal. Calc'd for C₂₂H₂₃FO₂S: C, 71.32; H, 6.26; F, 5.13; S, 8.65. Found: C, 71.66; H, 6.36; F, 4.91; S, 9.13.

### Example 6

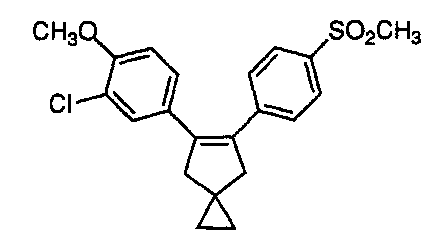

### 5-(3-Chloro-4-methoxyphenyl)-6-[4-(methylsulfonyl)phenyl)spiro[2.4]hept-5-ene

### Stet 1: Preparation of 4-(methylthio)acetophenone

To a stirred solution of 98.93 g (0.63 mol) of 4-(methylthio)benzonitrile in 1.2 L of THF under nitrogen at -78 °C was added 568 mL (0.795 mol) of methyllithium (1.4 M in ethyl ether). The resulting dark red solution was warmed to room temperature, and stirred for another 2.5 hours. The reaction was slowly quenched with 400 mL of 3 N HCl, and the resulting mixture was stirred overnight at room temperature. The mixture was concentrated in vacuo to about 500 mL, diluted with ethyl acetate and washed with saturated NaHCO₃, brine, dried (MgSO₄) and concentrated in vacuo to give 108.5 g (98.5%) of 4-(methylthio)acetophenone as a yellow solid: NMR (CDCl₃) δ 2.52 (s, 3H), 2.56 (s, 3H), 7.28 (d, J = 8.6 Hz, 2H), 7.86 (d, J = 8.6 Hz, 2H).

### Step 2: Preparation of 4-(methylsulfonyl)acetophenone

To a solution of 108.5 g (0.653 mol) of 4-(methylthio)acetophenone (prepared in Step 1) in 3 L of methylene chloride at 0 °C was added in portions 414 g (68%, 1.63 mol) of MCPBA over a period of one hour. The mixture was stirred at room temperature overnight. To the cooled white suspension was added slowly a solution of 124 g (0.653 mol) of meta bisulfite in 300 mL of water and the mixture was stirred at room temperature for one hour, then filtered. The filtrate was concentrated in vacuo to about 1 L, and repeatedly washed carefully with saturated NaHCO₃ to remove 3-chlorobenzoic acid. The extract was dried (MgSO₄) and concentrated in vacuo to give 117.33 g (91%) of 4-(methylsulfonyl)acetophenone as a bright yellow solid: NMR (CDCl₃) δ 2.67 (s, 3H), 3.08 (s, 3H), 8.05 (d, J = 8.5 Hz, 2H), 8.14 (d, J = 8.5 Hz, 2H).

### Step 3: Preparation of 2-bromo-4'-(methylsulfonyl) acetophenone

To a solution of 117 g (0.593 mol) of 4-(methylsulfonyl)acetophenone (prepared in Step 2) in 1 L of glacial acetic acid was added 1 mL of concentrated HCl. To the resulting solution was added dropwise a solution of 94.75 g (0.593 mol) of bromine in 100 mL of glacial acetic acid over a period of about 45 minutes, and the resulting light orange solution was poured onto about 2 L of ice. The resulting yellow precipitate was collected by filtration, washed with 2 L of water and dried (MgSO₄) to give 161 g (87% pure by ¹H NMR analysis, 86% calculated yield) of 2-bromo-4'-(methylsulfonyl)acetophenone as a yellow solid: NMR (CDCl₃) δ 3.10 (s, 3H), 4.45 (s, 2H), 8.08 (d, J = 8.7 Hz, 2H), 8.17 (d, J = 8.6 Hz, 2H).

### Step 4: Preparation of dimethyl keto malonate

Under nitrogen, 161.8 g (0.508 mol) of 2-bromo-4'-(methylsulfonyl)acetophenone (prepared in Step 3) was added to a suspension of 133.44 g (1.01 mol) of dimethyl malonate, 350.5 g (2.54 mol) of potassium carbonate (Aldrich), and 38.1 g (0.254 mol) of potassium iodide in 450 mL of THF, and the resulting suspension was stirred at room temperature for 6 hours (exothermic, temperature reached 40 °C in 30 minutes). The mixture was filtered, concentrated in vacuo, and the residue was recrystallized from ethyl acetate. The mother liquor was concentrated in vacuo and purified by silica gel chromatography (Prep-500, Waters), eluted with 15% of ethyl acetate in methylene chloride, to give a total of 100 g (63.3 %) of dimethyl keto malonate as a white solid: ¹H NMR (CDCl₃) δ 3.07 (s, 3H), 3.64 (d, J = 7.05 Hz, 2H), 3.78 (s, 6H), 4.09 (t, J = 7.04 Hz, 1H), 8.05 (d, J = 8.7 Hz, 2H), 8.15 (d, J = 8.7 Hz, 2H).

### Step 5: Preparation of 3-chloro-4-methoxybenzamide

To a solution of 132.5 g (0.71 mol) of 3-chloro-4-methoxybenzoic acid in 514 mL (7.05 mol) of thionyl chloride was added in portions 2.5 mL of DMF, and the resulting solution was stirred under reflux for 4 hours. The mixture was concentrated in vacuo and dissolved in 600 mL of methylene chloride. To the resulting solution was added 83.1 g (0.85 mol) of N,O-dimethylhydroxyamine (HCl salt) and cooled to 0 °C. To the suspension was added slowly 198 mL (1.4 mL) of triethylamine, and the mixture was stirred at room temperature overnight. The resulting solution was washed twice with 1 N KHSO₄, NaHCO₃, brine, dried (MgSO₄) and concentrated in vacuo to give 163.2 g (quantitative) of 3-chloro-4-methoxybenzamide as a light brown oil: ¹H NMR (CDCl₃) δ 3.35 (s, 3H), 3.56 (s, 3H), 3.94 (s, 3H), 6.93 (d, J = 8.7 Hz, 1H), 7.68 (dd, J = 2.2, 8.7 Hz, 1H), 7.82 (d, J = 2.1 Hz, 1H).

### Step 6: Preparation of 3-chloro-4-methoxyacetophenone

To a stirred solution of 64.7 g (0.28 mol) of 3-chloro-4-methoxybenzamide (prepared in Step 5) in 1 L of THF under nitrogen at -78 °C was added 110 mL (3 M in ethyl ether, 0.3 mol) of methylmagnesium bromide. The resulting solution was warmed to room temperature, and stirred for another 3 hours. The reaction was slowly quenched with 3 N HCl, diluted with ethyl acetate and washed with saturated NaHCO₃, brine, dried (MgSO₄) and concentrated in vacuo to give 51.7 g (99%) of 3-chloro-4-methoxyacetophenone as a off-white solid: ¹H NMR (CDCl₃) δ 2.55 (s, 3H), 3.97 (s, 3H), 6.96 (d, J= 8.5 Hz, 1H), 7.86 (dd, J= 2.2, 8.7 Hz, 1H), 7.98 (d, J = 2.2 Hz, 1H).

### Step 7: Preparation of 2-bromo-(3'-chloro-4'-methoxy)acetophenone

To a solution of 51.7 g (0.28 mol) of 3-chloro-4-methoxyacetophenone (prepared in Step 6) in 103 mL of glacial acetic acid was added 1 mL of concentrated HCl. To the resulting solution was added dropwise a solution of 14.5 mL (0.28 mol) of bromine in 20 mL of glacial acetic acid over a period of about 1.5 hours, and the resulting dark solution was stirred at room temperature for 2 hours. The precipitate was collected by filtration and washed with water. More solid was collected from the filtrate. The combined solid was dried to give 65 g (88%) of 2-bromo-(3'-chloro-4'-methoxy)acetophenone as a yellow solid: NMR (CDCl₃) 6 3.99 (s, 3H), 4.37 (s, 2H), 6.99 (d, J = 8.7 Hz, 1H), 7.91 (dd, J = 2.4, 8.7 Hz, 1H), 8.03 (d, J= 2.2 Hz, 1H).

### Step 8: Preparation of dimethyl diketo malonate

Under nitrogen, 30 g (0.114 mol) of 2-bromo-(3'-chloro-4'-methoxy)acetophenone (prepared in Step 7) was added in three portions over 24 hours to a stirred suspension of 24 g (0.077 mol) of dimethyl keto malonate (prepared in Step 4), 42 g (0.3 mol) of potassium carbonate (Aldrich), and 6 g (0.04 mol) of potassium iodide in 85 mL of THF. The mixture was filtered through a silica gel plug, eluted with ethyl acetate/hexane (1:1) and concentrated in vacuo. The residue was purified by silica gel chromatography (Prep-500, Waters), eluted with 33% of ethyl acetate in hexane, to give 27.8 g (73%) of dimethyl diketo malonate as a white solid.

### Step 9: Preparation of diaryl cyclopentenyl diester

To a vigorously stirred suspension of 38.4 g (0.249 mol) of titanium(III) chloride in 400 mL of DME under nitrogen was added 14 g (0.214 mol) of zinc dust (Aldrich), and the resulting mixture was stirred under reflux for one hour. To the dark solution at reflux was added 27.8 g (0.054 mol) of dimethyl diketo malonate (prepared in Step 8), and the resulting mixture was stirred under reflux for one hour. The mixture was filtered, concentrated in vacuo, diluted with ethyl acetate, washed with water, saturated NaHCO₃, brine, dried (MgSO₄) and concentrated in vacuo. The residue was purified by silica gel chromatography to give 13 g (50%) of diaryl cyclopentenyl diester as a pale yellow solid: ¹H NMR (CDCl₃) δ 3.04 (s, 3H), 3.5-3.6 (m, 4H), 3.80 (s, 6H), 3.88 (s, 3H), 6.77 (d, J = 8.7 Hz, 1H), 6.95 (dd, J = 2.2, 8.7 Hz, 1H), 7.19 (d, J = 2.0 Hz, 1H), 7.35 (dd, J = 1.8, 6.9 Hz, 2H), 7.79 (dd, J = 1.8, 6.8 Hz, 2H).

### Step 10: Preparation of diaryl cyclopentenyl diol

To a solution of 13 g (27.2 mmol) of cyclopentenyl diester (prepared in Step 9) in 200 mL of THF under nitrogen at -78 °C was added 100 mL (150 mmol) of DIBAL (1.5 M in toluene) over a period of 30 minutes. The resulting solution was stirred at -78 °C for 15 minutes, then at room temperature overnight. The reaction mixture was carefully quenched sequentially with 15 mL of acetone, 30 mL of water (caution: exothermic) and 90 mL of 10% NaOH. The aqueous layer was extracted with ethyl acetate, and the combined extracts were washed with saturated NaHCO₃, 1 N HCl, water, and brine. The extract was dried (MgSO₄) and concentrated in vacuo to give 11.8 g of colorless oil which was used directly in Step 11: ¹H NMR (CDCl₃) δ 2.35 (s, 2H), 2.78 (d, J = 10.5 Hz, 4H), 3.04 (s, 3H), 3.83 (s, 4H), 3.87 (s, 3H), 6.76 (d, J = 8.7 Hz, lH), 6.95 (dd, J= 2.2, 8.7 Hz, lH), 7.18 (d, J = 2.1 Hz, lH), 7.34 (d, J = 1.8, 6.8 Hz, 2H), 7.77 (dd, J = 1.8, 6.6 Hz, 2H).

### Step 11: Preparation of diaryl cyclopentenyl ditosylate

To a solution of 11.8 g (26.8 mmol) of crude diaryl cyclopentenyl diol (prepared in Step 10) in 92 mL of pyridine under nitrogen at 0 °C was added 23 g (120 mmol) of p-toluenesulfonyl chloride in portions (exothermic) and the resulting dark solution was stirred at room temperature overnight. The mixture was concentrated in vacuo to remove pyridine, and the residue was dissolved in ethyl acetate. The solution was washed with water, 1 N HCl, NaHCO₃, brine, dried (MgSO₄) and concentrated in vacuo. The residue was chromatographed to give 11.6 g (59% from diester) of diaryl cyclopentenyl ditosylate as a tan solid: ¹H NMR (CDCl₃) δ 2.46 (s, 6H), 2.70 (d, J = 15.1 Hz, 4H), 3.04 (s, 3H), 3.88 (s, 3H), 4.03 (s, 4H), 6.74 (d, J = 8.7 Hz, 1H), 6.85 (dd, J = 2.2, 8.7 Hz, 1H), 7.00 (d, J = 2.0 Hz, 1H), 7.19 -7.25 (m, 2H), 7.35 (d, J = 8.1 Hz, 4H), 7.7-7.82 (m, 6H).

### Step 12: Preparation of 5-(3-chloro-4-methoxyphenyl)-6-[4-(methylsulfonyl)phenyl]spiro[2.4]hept-5-ene

Under nitrogen, a solution of 11.6 g (15.9 mmol) of diaryl cyclopentenyl ditosylate (prepared in Step 11) in 260 mL of DMF was treated with 34.7 g (231 mmol) of sodium iodide and 17.2 g (263 mmol) of zinc dust. The resulting mixture was stirred at 150 °C for 3 hours, and concentrated in vacuo. The residue was dissolved in ethyl acetate, and the solid was filtered off. The filtrate was washed with sodium sulfite, water, brine, dried (MgSO₄), and concentrated in vacuo. The residue was chromatographed on silica gel to give 5.32 g (86%) of pale yellow solid The solid was recrystallized to give 2.32 g of 5-(3-chloro-4-methoxyphenyl)-6-[4-(methylsulfonyl)phenyl]spiro[2.4]hept-5-ene as a white solid: mp 140.0-140.5 °C; ¹H NMR (CDCl₃) δ 0.68 (s, 4H), 2.84-2.95 (m, 4H), 3.04 (s, 3H), 3.88 (s, 3H), 6.76 (d, J = 8.5 Hz, 1H), 6.95 (dd, J = 2,0, 8.5 Hz, 1H), 7.18 (d, J = 2.0 Hz, 1H), 7.35 (dd, J = 1.6, 6.7 Hz, 2H), 7.77 (dd, J = 1.8, 6.6 Hz, 2H). HRMS (EI) Calc'd for C₂₁H₂₁ClO₃S: 388.0900. Found: 388.0909. Anal. Calc'd for C₂₁H₂₁ClO₃S: C, 64.93; H, 5.45; S, 8.24. Found: C, 64.77; H, 5.65; S, 8.53.

The mother liquor from the recrystallization described above was concentrated in vacuo to give 3.0 g of pale yellow solid which was used directly in the preparation of the title compound of Example 7.

### Example 7

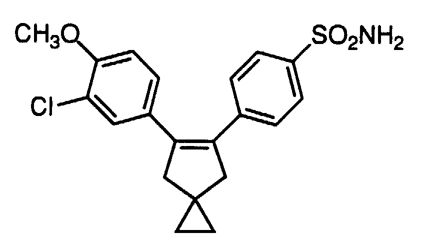

### 4-[6-(3-Chloro-4-methoxyphenyl)spiro[2.4]hept-5-en-5-yl]benzenesulfonamide

Under nitrogen, a solution of 3.6 g (9.26 mmol) of 5-(3-chloro-4-methoxyphenyl)-6-[4-(methylsulfonyl)phenyl]spiro[2.4]hept-5-ene (the title compound of Example 6) in 10 mL of THF at 0 °C was treated with 6.3 mL (10.08 mmol) of propylmagnesium chloride (1.6 M in ether). The reaction mixture was stirred at ambient temperature for 25 minutes, cooled to 0 °C, and treated with 16.5 mL (1.0 M in THF, 16.5 mmol) of tributylborane (or triethylborane). The resulting solution was stirred at ambient temperature for 15 minutes and then at reflux for 18 hours prior to the addition of 7 g (85 mmol) of sodium acetate, 18 mL of water, and 4 g (35 mmol) of hydroxyamine-O-sulfonic acid at 0 °C. The resulting light orange mixture was stirred at ambient temperature for 3.5 hours and the aqueous phase was extracted with ethyl acetate. The combined extracts were washed with water, brine, dried (MgSO₄), and concentrated in vacuo. The residue was chromatographed on silica gel to give 2.5 g (59%) of 4-[6-(3-chloro-4-methoxyphenyl)spiro[2.4]hept-5-en-5-yl]benzenesulfonamide as a white solid: mp 191.0-192.0 °C; ¹H NMR (CDCl₃) δ 0.63 (s, 4H), 2.84 (s, 4H), 3.83 (s, 3H), 5.74 (br s, 2H), 6.72 (d, J = 8.5 Hz, 1H), 6.92 (dd, J = 2.0, 8.5 Hz, 1H), 7.15 (d, J = 2.2 Hz, 1H), 7.24 (dd, J = 2.0, 6.9 Hz, 2H), 7.72 (dd, J = 1.8, 6.7 Hz, 2H). HRMS (EI) Calc'd for C₂₀H₂₀ClNO₃S: 389.0852. Found: 389.0869. Anal. Calc'd for [C₂₀H₂₀ClNO₃S + 0.05 CH₂Cl₂] : C, 61.61; H, 5.17; N, 3.59; S, 8.22. Found: C, 61.06; H, 5.25; N, 3.51; S, 8.24.

### Example 8

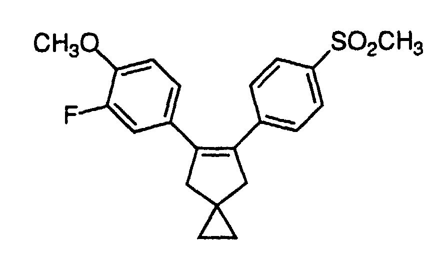

### 5-(3-Fluoro-4-methoxyphenyl)-6-[4-(methylsulfonyl)phenyl]spiro[2.4]hept-5-ene

Following a procedure similar to the one described in Example 6, with the substitution of 3'-fluoro-4'-methoxyacetophenone (Aldrich) for 3'-chloro-4'-methoxyacetophenone (Example 6, Step 7), 5-(3-fluoro-4-methoxyphenyl)-6-[4-(methylsulfonyl)pheny]spiro[2.4]hept-5-ene was prepared as a white solid: mp 110.5-111.5 °C; ¹H NMR (CDCl₃) δ 0.68 (s, 4H), 2.85-2.95 (m, 4H), 3.05 (s, 3H), 3.87 (s, 3H), 6.77-6.94 (m, 3H), 7.35 (d, J = 8.4 Hz, 2H), 7.78 (d, J = 8.7 Hz, 2H). HRMS (EI) Calc'd for C₂₁H₂₁FO₃S: 372.1192. Found: 372.1187. Anal. Calc'd for C₂₁H₂₁FO₃S: C, 67.72; H, 5.68; F, 5.10; S, 8.61. Found: C, 67.31; H, 5.68; F, 5.16; S, 8.62.

### Example 9

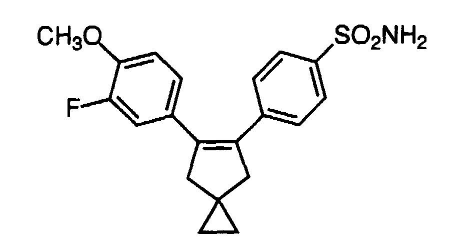

### 4-[6-(3-Fluoro-4-methoxyphenyl)spiro[2.4]hept-5-en-5-yl]benzenesulfonamide

Following a procedure similar to the one described in Example 7, 1.40 g (3.76 mmol) of 5-(3-fluoro-4-methoxyphenyl)-6-[4-(methylsulfonyl)phenyl]spiro[2.4]hept-5-ene (the title compound of Example 8) was converted to crude sulfonamide. Purification by silica gel chromatography gave 0.88 g (63%) of 4-[6-(3-fluoro-4-methoxyphenyl)spiro[2.4]hept-5-en-5-yl]benzenesulfonamide as a white solid: mp 153.0-154.0 °C; ¹H NMR (CDCl₃) δ 0.68 (s, 4H), 2.89 (s, 4H), 3.87 (s, 3H), 4.79 (s, 2H), 6.75-6.93 (m, 3H), 7.31 (d, J = 8.7 Hz, 2H), 7.77 (d, J = 8.4 Hz, 2H). HRMS (EI) Calc'd for C₂₀H₂₀FNO₃S: 373.1148. Found: 373.1172. Anal. Calc'd for C₂₀H₂₀FNO₃S: C, 64.33; H, 5.40; N, 3.75; F, 5.09. Found: C, 64.28; H, 5.49; N, 3.77; F, 5.23.

### Example 10

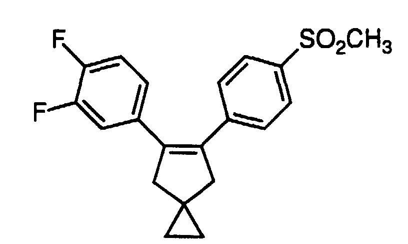

### 5-(3,4-Difluorophenyl)-6-[4-(methylsulfonyl)phenyl)spiro[2.4]hept-5-ene

Following a procedure similar to the one described in Example 6 with the substitution of 3',4'-difluroacetophenone (Aldrich) for 3'-chloro-4'-methoxyacetophenone (Example 6, Step 7), 5-(3,4-difluorophenyl)-6-[4-(methylsulfonyl)phenyl]spiro[2.4]hept-5-ene was obtained as a white solid: mp 113-114 °C. MS (FAB): m/z 367 (M+Li), HRMS Calc'd for C₂₀H₁₈F₂O₂S: 360.0996, found 360.1014. ¹H NMR (CDCl₃) δ 0.69 (s, 4H), 2.90 (s, 2H), 2.92 (s, 2H), 3.06 (s, 3H), 6.80-6.87 (m, 1H), 6.91-7.07 (m, 2H), 7.33 (d, J = 8 Hz, 2H), 7.79 (d, J = 8 Hz, 2H). Anal. Calc'd for C₂₀H₁₈F₂O₂S: C, 66.65; H, 5.03. Found: C, 66.50; H, 5.02.

### Example 11

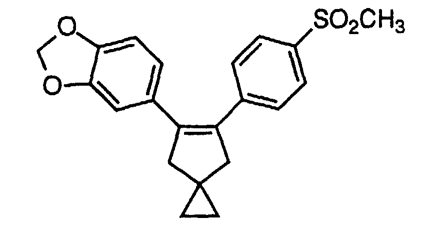

### 5-[6-[4-(Methylsulfonyl)phenyl]spiro[2.4]hept-5- en-5-yl]-1,3-benzodioxole

Following a procedure similar to that described in Example 6, with the substitution of 3',4'-(methylenedioxy)acetophenone [prepared by the addition of methyllithium to piperonylonitrile (Aldrich), see Example 6, Step 1] for 3'-chloro-4'-methoxyacetophenone (Example 6, Step 7), 5-[6-[4-(methylsulfonyl)phenyl]spiro[2.4]hept-5-en-5-yl]-1,3-benzodioxole was prepared as a white solid: mp 110.5-111.5 °C; ¹H NMR (CDCl₃) δ 0.67 (s, 4H), 2.89 (br d, J = 2.4 Hz, 4H), 3.05 (s, 3H), 5.94 (s, 2H), 6.55-6.67 (m, 2H), 6.70 (d, J = 8.1 Hz, 1H), 7.36 (d, J = 8.5 Hz, 2H), 7.76 (d, J = 8.5 Hz, 2H). HRMS (EI) Calc'd for C₂₁H₂₀O₄S: 368.1082. Found: 368.1077. Anal. Calc'd for C₂₁H₂₀O₄S: C, 68.46; H, 5.47; S, 8.70. Found: C, 68.13; H,5.65; S, 8.81.

### Example 12

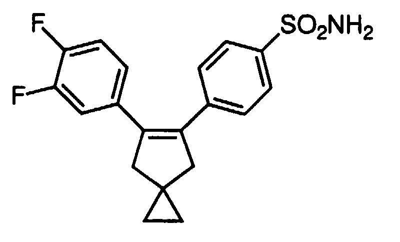

### 4-[6-(3,4-Difluorophenyl)spiro[2.4]hept-5-en-5-yl]benzenesulfonamide

Following a procedure similar to the one described in Example 7, 880 mg (2.44 mmol) of 5-(3,4-difluorophenyl)-6-[4-(methylsulfonyl)phenyl]spiro[2.4]hept-5-ene (the title compound of Example 10) was converted to crude sulfonamide. Purification by silica gel chromatography (MPLC) with ethyl acetate/hexane (1:5) as the eluent followed by recrystallization from methylene chloride/hexane gave 370 mg (42%) of 4-[6-(3,4-difluorophenyl)spiro[2.4]hept-5-en-5-yl]benzenesulfonamide as a white solid: mp 136-137 °C. MS (FAB): m/z 362 (M+H); HRMS Calc'd for C₁₉H₁₇F₂NO₂S: 361.0948, found: 361.0952. ¹H NMR (CDCl₃) δ 0.68 (s, 4H), 2.89 (s, 2H), 2.90 (s, 2H), 4.79 (s, 2H), 6.81-6.87 (m, 1H), 6.91-7.06 (m, 2H), 7.28 (d, J = 8 Hz, 2H), 7.78 (d, J = 8 Hz, 2H). Anal. Calc'd for [C₁₉H₁₇F₂NO₂S + 0.29 CH₂Cl₂] : C, 60.00; H, 4.59; N, 3.63; F, 9.84; S, 8.30. Found: C, 59.96; H, 4.57; N, 3.50; F, 10.05; S, 8.35.

### Example 13

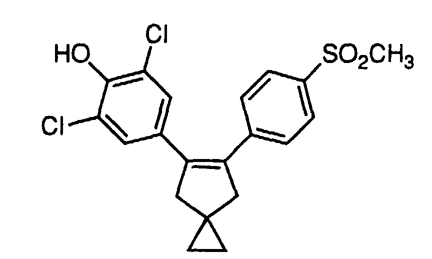

### 2,6-Dichloro-4-[6-[4-(methylsulfonyl)phenyl]spiro[2.4]hept-5-en-5-yl]phenol

### Step 1: Preparation of 3,5-dichloro-4-methoxybenzoic acid

Under nitrogen, a mixture of 41.4 g (0.200 mol) of 3-chloro-4-hydroxybenzoic acid, 75 mL (1.2 mol) of iodomethane, and 81.5 g (0.25 mol) of potassium carbonate in 250 mL of DMF was stirred at 55 °C for 18 hours. The reaction mixture was filtered and concentrated in vacuo. The residue was dissolved in ethyl acetate, washed with water, brine, dried (MgSO₄) and concentrated in vacuo. The residue was dissolved in 84 mL of methanol and 84 mL of 2.5 N NaOH, and the resulting mixture was stirred at reflux for 4 hours. The reaction was concentrated in vacuo. The residue was dissolved in 600 mL of water, and pH was adjusted to 2 with concentrated HCl. The solution was extracted with ethyl acetate, and the combined extracts were washed with brine, dried (MgSO₄) and concentrated in vacuo to give 38.12 g (89%) of 3,5-dichloro-4-methoxybenzoic acid as a white solid: ¹H NMR (CDCl₃) δ 3.95 (s, 3H), 7.98 (s, 2H)

### Step 2: Preparation of 2,6-dichloro-4-[6-[4-(methylsulfonyl)phenyl]spiro[2.4]hept-5-en-5-yl]phenol

Following a procedure similar to the one described in Example 6, with the substitution of 3,5-dichloro-4-methoxybenzoic acid (prepared in Step 1) for 3-chloro-4-methoxybenzoic acid (Example 6, Step 5), 2,6-dichloro-4-[6-[4-(methylsulfonyl)phenyl]spiro[2.4]hept-5-en-5-yl]phenol was isolated instead of the expected 5-(3,5-dichloro-4-methoxy-phenyl)-6-[4-(methylsulfonyl)phenyl]spiro[2.4]hept-5-ene. The title product compound was recrystallized as a white solid: mp 163.5-164.5 °C; ¹H NMR (CDCl₃) δ 0.68 (s, 4H), 2.88 (br d, J =12.5 Hz, 4H), 3.05 (s, 3H), 5.83 (s, 2H), 7.02 (s, 2H), 7.35 (d, J = 8.4 Hz, 2H), 7.81 (d, J = 8.4 Hz, 2H). HRMS (EI) Calc'd for C₂₀H₁₈Cl₂O₃S: 408.0354. Found: 408.0349. Anal. Calc'd for C₂₀H₁₈Cl₂O₃S: C, 58.69; H, 4.43; Cl, 17.32; S, 7.83. Found: C, 58.47; H, 4.55; Cl, 17.24; S, 7.65

### Example 14

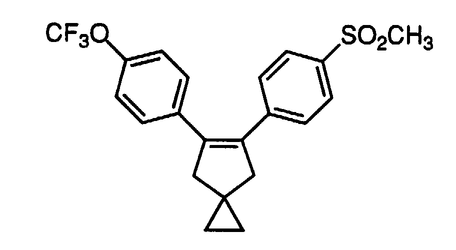

### 6-[4-(Methylsulfonyl)phenyl]-5-(4-trifluoromethoxyphenyl)spiro[2.4]hept-5-ene

Following a procedure similar to the one described in Example 6, with the substitution of 4'-trifluoromethoxyacetophenone (Aldrich) for 3'-chloro-4'-methoxyacetophenone (Example 6, Step 7), 6-[4-(methylsulfonyl)phenyl]-5-(4-trifluoromethoxyphenyl)spiro[2.4]hept-5-ene was prepared as a white solid: mp 126.0-127.0 °C; ¹H NMR (CDCl₃) δ 0.69 (s, 4H), 2.93 (s, 4H), 3.05 (s, 3H), 7.08 (d, J = 8.7 Hz, 2H), 7.16 (d, J = 8.7 Hz, 2H), 7.33 (d, J = 8.4 Hz, 2H), 7.78 (d, J = 8.1 Hz, 2H). HRMS (EI) Calc'd for C₂₁H₁₉F₃O₃S: 408.1007. Found: 408.1017. Anal. Calc'd for [C₂₁H₁₉F₃O₃S + 0.12 H₂O]: C, 61.43; H, 4.72; F, 13.88; S, 7.81. Found: C, 61.54; H, 4.76; F, 13.32; S, 7.98

### Example 15

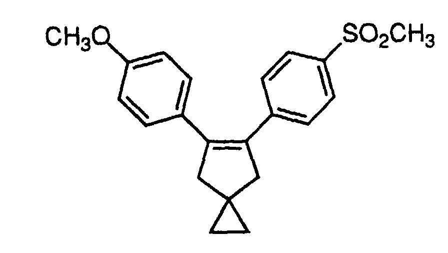

### 5-(4-Methoxyphenyl)-6-[4-(methylsulfonyl)phenyl]spiro[2.4]hept-5-ene

Following a procedure similar to the one described in Example 6, with the substitution of 2-bromo-4'-methoxyacetophenone (Aldrich) for 2-bromo-3'-chloro-4'-methoxyacetophenone (Example 6, Step 8), 5-(4-methoxyphenyl)-6-[4-(methylsulfonyl)phenyl]spiro[2.4]hept-5-ene was prepared as a white solid: mp 170.2-173.0 °C; ¹H NMR (CDCl₃) δ 0.67 (s, 4H), 2.91 (s, 4H), 3.04 (s, 3H), 3.79 (s, 3H), 6.77 (d, J = 8.9 Hz, 2H), 7.07 (d, J = 8.9 Hz, 2H), 7.35 (d, J = 8.5 Hz, 2H), 7.75 (d, J = 8.7 Hz, 2H). HRMS (EI) Calc'd for C₂₁H₂₂O₃S: 354.1290. Found: 354.1317. Anal. Calc'd for C₂₁H₂₂O₃S: C, 71.16; H, 6.26; S, 9.04. Found: C, 70.92; H, 6.20; S, 8.96.

### Example 16

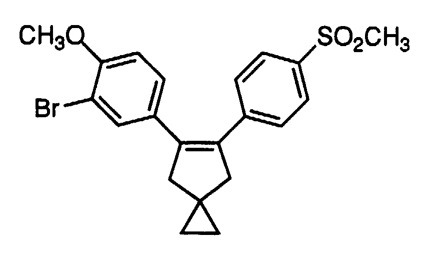

### 5-(3-Bromo-4-methoxyphenyl)-6-[4-(methylsulfonyl)phenyl]spiro[2.4]hept-5-ene

Following a procedure similar to the one described in Example 6, with the substitution of 3'-bromo-4'-methoxyacetophenone [prepared by the addition of methylmagnesium bromide to 3-bromo-4-methoxybenzaldehyde (Aldrich), followed by MnO₂ oxidation of the resulting alcohol] for 3'-chloro-4'-methoxyacetophenone (Example 6, Step 7), 5-(3-bromo-4-methoxyphenyl)-6-[4-(methylsulfonyl)phenyl]spiro[2.4]hept-5-ene was prepared as a white solid: mp 89.5-91.8 °C; ¹H NMR (CDCl₃) δ 0.68 (s, 4H), 2.85-2.93 (m, 4H), 3.04 (s, 3H), 3.87 (s, 3H), 6.73 (d, J = 8.7 Hz, 1H), 6.97 (dd, J = 2,5, 7.5 Hz, 1H), 7.3-7.4 (m, 3H), 7.78 (d, J = 8.5 Hz, 2H). HRMS (EI) Calc'd for C₂₁H₂₁BrO₃S: 432.0395. Found: 432.0375. Anal. Calc'd for [C₂₁H₂₁BrO₃S + 0.64 CH₂Cl₂]: C, 53.31; H, 4.61; Br, 16.39. Found: C, 53.12; H, 4.54; Br, 16.74.

### Example 17

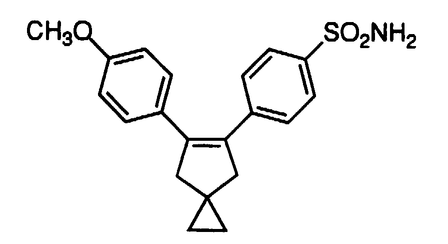

### 4-[6-(4-Methoxyphenyl)spiro[2.4]hept-5-en-5-yl]benzenesulfonamide

Following a procedure similar to the one described in Example 7, 200 mg (0.564 mmol) of 5-(4-methoxyphenyl)-6-[4-(methylsulfonyl)phenyl]spiro[2.4]hept-5-ene (the title compound of Example 15) was converted to crude sulfonamide. Purification by silica gel chromatography gave 96 mg (48%) of 4-[6-(4-methoxyphenyl)spiro[2.4]hept-5-en-5-yl]benzenesulfonamide as a white solid: ¹H NMR (CDCl₃) δ 0.67 (s, 4H), 2.90 (s, 4H), 3.78 (s, 3H), 4.86 (br s, 2H), 6.76 (d, J = 8.9 Hz, 2H), 7.08 (d, J = 8.9 Hz, 2H), 7.30 (d, J = 8.5 Hz, 2H), 7.73 (d, J = 8.5 Hz, 2H). HRMS (EI) Calc'd for C₂₀H₂₁NO₃S: 355.1242. Found: 355.1250. Anal. Calc'd for [C₂₀H₂₁NO₃S + 0.6 H₂O]: C, 65.60; H, 6.11; N, 3.82. Found: C, 65.59; H, 5.85; N, 3.66.

### Example 18

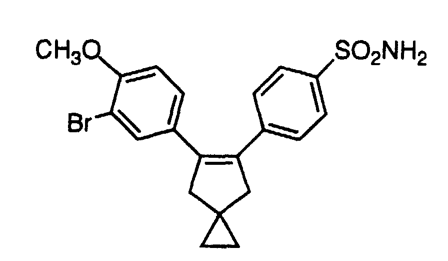

### 4-[6-(3-Bromo-4-methoxyphenyl)spiro[2.4]hept-5-en-5-yl]benzenesulfonamide

Following a procedure similar to the one described in Example 7, 3 g (6.92 mmol) of 5-(3-bromo-4-methoxyphenyl)-6-[4-(methylsulfonyl)phenyl]spiro[2.4]hept-5-ene (the title compound of Example 16) was converted to crude sulfonamide. Purification by silica gel chromatography gave 1.32 g (44%) of 4-[6-(3-bromo-4-methoxyphenyl)spiro[2.4]hept-5-en-5-yl]benzenesulfonamide as a write solid: mp 187.5-189.8 °C; ¹H NMR (CDCl₃) δ 0.67 (s, 4H), 2.89 (s, 4H), 3.87 (s, 3H), 4.76 (br s, 2H), 6.73 (d, J = 8.5 Hz, 1H), 7.0 (dd, J = 2.1, 8.5 Hz, 1H), 7.30 (d, J = 8.7 Hz, 2H), 7.37 (dd, J = 2.1 Hz, 1H), 7.76 (d, J = 8.7 Hz, 2H). HRMS (EI) Calc'd for C₂₀H₂₀BrNO₃S: 433.0347. Found: 433.0310. Anal. Calc'd for C₂₀H₂₀BrNO₃S: C, 55.31; H, 4.64; N, 3.22. Found: C, 55.31; H, 4.77; N, 2.93.

### Example 19

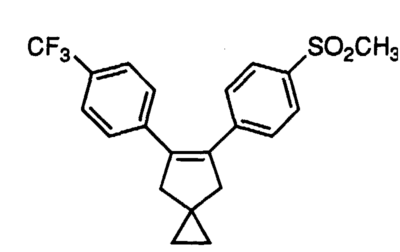

### 6-[4-(Methylsulfonyl)phenyl)-5-(4-trifluoromethylphenyl)spiro[2.4]hept-5-ene

Following a procedure similar to the one described in Example 6, with the substitution of 4-trifluoromethylacetophenone [prepared by the addition of methyllithium to α,α,α-trifluoro-p-tolunitrile (Aldrich), see Example 6, Step 1] for 3'-chloro-4'-methoxyacetophenone (Example 6, Step 7), 6-[4-(methylsulfonyl)phenyl]-5-(4-trifluoromethylphenyl)spiro[2.4]hept-5-ene was prepared as a white solid: mp 170.0-170.8 °C; ¹H NMR (CDCl₃) δ 0.70 (s, 4H), 2.95 (s, 4H), 3.05 (s, 3H), 7.24 (d, J = 8.4 Hz, 2H), 7.31 (d, J = 8.3 Hz, 2H), 7.49 (d, J = 8.1 Hz, 2H), 7.78 (d, J = 8.2 Hz, 2H). HRMS (EI) Calc'd for C₂₁H₁₉F₃O₂S: 392.1058. Found: 392.1080. Anal. Calc'd for C₂₁H₁₉F₃O₂5: C, 64.27; H, 4.88; F, 14.52; S, 8.17. Found: C, 63.98; H, 4.90; F, 14.65; S, 8.33.

### Example 20

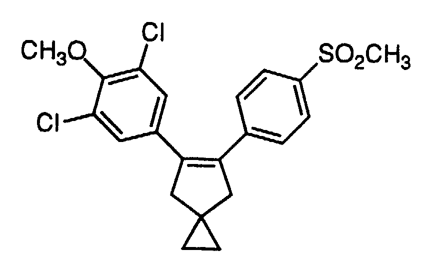

### 5-(3,5-Dichloro-4-methoxyphenyl)-6-[4-(methylsulfonyl)phenyl]spiro[2.4]hept-5-ene

Under nitrogen, a mixture of 0.2 g (0.49 mmol) of 2,6-dichloro-4-[6-[4-(methylsulfonyl)phenyl]spiro[2.4]hept-5-en-5-yl]phenol (the title compound of Example 13), 91 µL (1.5 mmol) of iodomethane and 0.32 g (1 mmol) of cesium carbonate in 6 mL of DMF was stirred at 25 °C for 16 hours. The reaction mixture was diluted in ethyl acetate, washed with water, brine, dried (MgSO₄) and concentrated in vacuo. The residue was recrystallized to give 0.18 g (90%) of 5-(3,5-dichloro-4-methoxyphenyl)-6-[4-(methylsulfonyl)phenyl]spiro[2.4]hept-5-ene as a white solid: mp 107.0-108.0 °C; ¹H NMR (CDCl₃) δ 0.69 (s, 4H), 2.89 (d, J = 14 Hz, 4H), 3.05 (s, 3H), 3.89 (s, 3H), 7.04 (s, 2H), 7.34 (d, J = 8.7 Hz, 2H), 7.82 (d, J = 8.4 Hz, 2H). HRMS (EI) Calc'd for C₂₁H₂₀Cl₂O₃S: 422.0510. Found: 422.0513. Anal. Calc'd for [C₂₁H₂₀Cl₂O₃S + 0.67 H₂O]: C, 57.92; H, 4.94; Cl, 16.28; S, 7.36. Found: C, 57.79; H, 4.73; Cl, 16.68; S, 7.31.

### Example 21

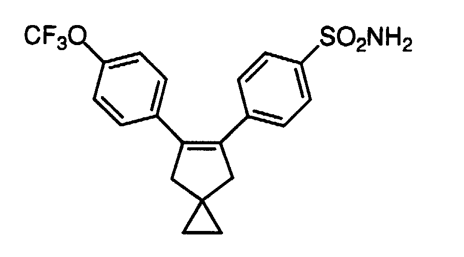

### 4-[6-(4-Trifluoromethoxyphenyl)spiro[2.4]hept-5-en-5-yl]benzenesulfonamide

Following a procedure similar to the one described in Example 7, 1.80 g (4.4 mmol) of 6-[4-(methylsulfonyl)phenyl]-5-(4-trifluoromethoxyphenyl)spiro[2.4]hept-5-ene (the title compound of Example 14) was converted to crude sulfonamide. Purification by silica gel chromatography gave 0.73 g (40%) of 4-[6-(4-trifluoromethoxyphenyl)spiro[2.4]hept-5-en-5-yl]benzenesulfonamide as a white solid: mp 144.0-145.0 °C; ¹H NMR (CDCl₃) δ 0.69 (s, 4H), 2.92 (s, 4H), 4.78 (br s, 2H), 7.08 (d, J = 8.9 Hz, 2H), 7.16 (d, J = 9.0 Hz, 2H), 7.28 (d, J = 8.7 Hz, 2H), 7.76 (d, J = 8.7 Hz, 2H). HRMS (EI) Calc'd for C₂₀H₁₈F₃NO₃S: 409.0960. Found: 409.0974. Anal. Calc'd for [C₂₀H₁₈F₃NO₃S + 0.29 C₃H₆O₂]: C, 58.17; H, 4.62; N, 3.25; F, 13.23; S, 7.44. Found: C, 58.57; H, 4.47; N, 3.29; F, 12.62; S, 7.92.

### Example 22

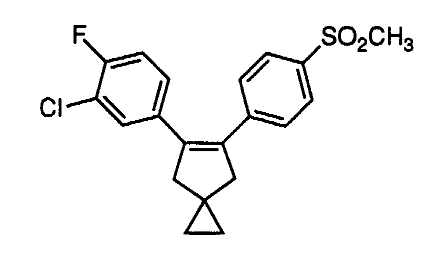

### 5-(3-Chloro-4-fluorophenyl)-6-[4-(methylsulfonyl)phenyl]spiro[2.4]hept-5-ene

Following a procedure similar to the one described in Example 6, with the substitution of 3'-chloro-4'-fluoroacetophenone (Lancaster) for 3'-chloro-4'-methoxyacetophenone (Example 6, Step 7), 5-(3-chloro-4-fluorophenyl)-6-[4-(methylsulfonyl)phenyl]spiro[2.4]hept-5-ene was prepared as a white solid: mp 128.0-130.0 °C; ¹H NMR (CDCl₃) δ 0.69 (s, 4H), 2.91 (d, J = 3.6 Hz, 4H), 3.05 (s, 3H), 6.9-7.02 (m, 2H), 7.19 (d, J = 7.5 Hz, 1H), 7.33 (d, J = 8.4 Hz, 2H), 7.79 (d, J = 8.7 Hz, 2H). HRMS (EI) Calc'd for C₂₀H₁₈ClFO₂S: 376.0700. Found: 376.0710. Anal. Calc'd for C₂₀H₁₈ClFO₂S: C, 63.74; H, 4.81; F, 5.04; Cl, 9.41; S, 8.51. Found: C, 63.61; H, 4.85; F, 4.70; Cl, 9.58; S, 8.66.

### Example 23

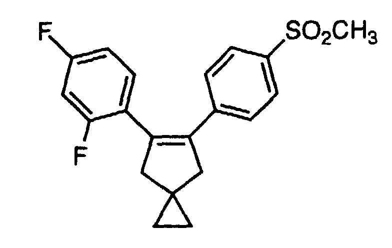

### 5-(2,4-Difluorophenyl)-6-[4-(methylsulfonyl)phenyl]spiro[2.4]hept-5-ene

Following a procedure similar to the one described in Example 6, with the substitution of 2-chloro-2',4'-difluoroacetophenone (Aldrich) for 2-bromo-(3'-chloro-4'-methoxy)acetophenone (Example 6, Step 8), 5-(2,4-difluorophenyl)-6-[4-(methylsulfonyl)phenyl]spiro[2.4]hept-5-ene was prepared as a white solid: mp 116.0-117.5 °C; ¹H NMR (CDCl₃) δ 0.69 (s, 4H), 2.89 (s, 2H), 2.94 (s, 2H), 3.03 (s, 3H), 6.78 (t, J = 8.4 Hz, 2H), 7.05 (q, J = 6.6 Hz, 2H), 7.28 (d, J = 8.1 Hz, 2H), 7.74 (dd, J = 1.8, 6.9 Hz, 2H). HRMS (EI) Calc'd for C₂₀H₁₈F₂O₂S: 360.0996. Found: 360.1010. Anal. Calc'd for [C₂₀H₁₈F₂O₂S+ 0.22 H₂O]: C, 65.93; H, 5.10; S, 8.80. Found: C, 66.18; H, 5.16; S, 8.97.

### Example 24

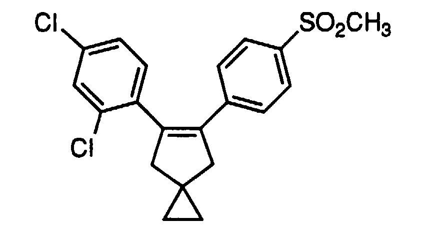

### 5-(2,4-Dichlorophenyl)-6-[4-(methylsulfonyl)phenyl]spiro[2.4]hept-5-ene

Following a procedure similar to the one described in Example 6, with the substitution of 2,2'4'-trichloroacetophenone (Aldrich) for 2-bromo-(3'-chloro-4'-methoxy)acetophenone (Example 6, Step 8), 5-(2,4-dichlorophenyl)-6-[4-(methylsulfonyl)phenyl]spiro[2.4]hept-5-ene was prepared as a white solid: mp 90.0-91.5 °C; ¹H NMR (CDCl₃) δ 0.69 (s, 4H), 2.85 (s, 2H), 2.95 (s, 2H), 3.01 (s, 3H), 7.02 (d, J = 8.1 Hz, 1H), 7.16 (d, J = 2.1 Hz, 1H), 7.20 (d, J = 8.4 Hz, 2H), 7.42 (d, J = 2.1 Hz, 1H), 7.72 (d, J = 8.7 Hz, 2H). HRMS (EI) Calc'd for C₂₀H₁₈Cl₂O₂S: 392.0405. Found: 392.0423. Anal. Calc'd for [C₂₀H₁₈Cl₂O₂S + 0.36 H₂O + 0.05 C₆H₁₄] : C, 60.31; H, 4.84; Cl, 17.56; S, 7.94. Found: C, 60.33; H, 4.53; Cl, 17.21; S, 8.32.

### Example 25

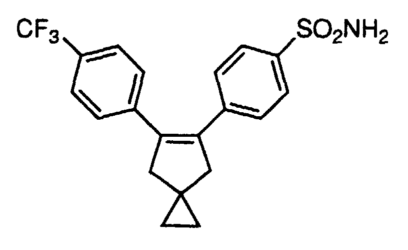

### 4-[6-(4-Trifluoromethylphenyl)spiro[2.4]hept-5-en-5-yl]benzenesulfonamide

Following a procedure similar to the one described in Example 7, 1.81 g (4.6 mmol) of 6-[4-(methylsulfonyl)phenyl]-5-(4-trifluoromethylphenyl)spiro[2.4]hept-5-ene (the title compound of Example 19) was converted to crude sulfonamide. Purification by silica gel chromatography gave 1.20 g (66%) of 4-[6-(4-trifluoromethylphenyl)spiro[2.4]hept-5-en-5-yl]benzenesulfonamide as a white solid: mp 157.2-188.8 °C; ¹H NMR (CDCl₃) δ 0.70 (s, 4H), 2.94 (s, 4H), 4.80 (s, 2H), 7.21-7.30 (m, 4H), 7.48 (d, J = 8.5 Hz, 2H), 7.77 (d, J = 8.3 Hz, 2H). HRMS (EI) Calc'd for C₂₀H₁₈F₃NO₂S: 393.1010. Found: 393.1045. Anal. Calc'd for [C₂₀H₁₈F₃NO₂S + 0.07 CH₂Cl₂]: C, 60.38; H, 4.58; N, 3.51; S, 8.03. Found: C, 60.30; H, 4.69; N, 3.50; S, 8.44.

### Example 26

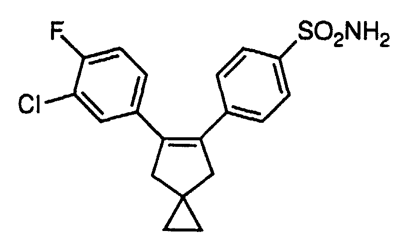

### 4-[6-(3-Chloro-4-fluorophenyl)spiro[2.4]hept-5-en-5-yl]benzenesulfonamide

Following a procedure similar to the one described in Example 7, 1.0 g (2.65 mmol) of 5-(3-chloro-4-fluorophenyl)-6-[4-(methylsulfonyl)phenyl]spiro[2.4]hept-5-ene (the title compound of Example 22) was converted to crude sulfonamide. Purification by silica gel chromatography gave 0.19 g (19%) of 4-[6-(3-chloro-4-fluorophenyl)spiro[2.4]hept-5-en-5-yl]benzenesulfonamide as a white solid: mp 163.0-165.0 °C; ¹H NMR (CDCl₃) δ 0.69 (s, 4H), 2.90 (d, J = 2.7 Hz, 4H), 4.75 (br s, 2H), 6.70-7.05 (m, 2H), 7.18-7.24 (m, 1H), 7.24-7.32 (m, 3H), 7.78 (d, J = 8.7 Hz, 2H). HRMS (EI) Calc'd for C₁₉H₁₇ClFNO₂S: 377.0652. Found: 377.0639. Anal. Calc'd for [C₁₉H₁₇ClFNO₂S + 0.018 CH₂Cl₂]: C, 60.21; H, 4.53; N, 3.69; F, 5.01; Cl, 9.68; S, 8.45. Found: C, 60.49; H, 4.63; N, 3.50; F, 4.91; Cl, 9.86; S, 8.61.

### Example 27

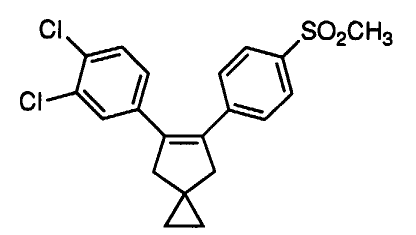

### 5-(3,4-Dichlorophenyl)-6-[4-(methylsulfonyl)phenyl]spiro[2.4]hept-5-ene

Following a procedure similar to the one described in Example 6, with the substitution of 2-bromo-(3',4'-dichloro)acetophenone (Lancaster) for 2-bromo-(3'-chloro-4'-methoxy)acetophenone (Example 6, Step 8), 5-(3,4-dichlorophenyl)-6-[4-(methylsulfonyl)phenyl]spiro[2.4]hept-5-ene was prepared as a white solid: mp 107.5-108.5 °C; ¹H NMR (CDCl₃) δ 0.69 (s, 4H), 2.91 (d, J = 3.0 Hz, 4H), 3.05 (s, 3H), 6.90 (dd, J = 1.9, 8.7 Hz, 1H), 7.24 (d, J = 2,1 Hz, 1H), 7.27 (d, J = 8.7 Hz, 1H), 7.33 (d, J = 8.4 Hz, 2H), 7.80 (d, J = 8.4 Hz, 2H). MS (FAB): m/z 399 (100, M+Li). Anal. Calc'd for [C₂₀H₁₈C1₂O₂S + 0.08 C₆H₁₄]: C, 61.45; H, 4.81; Cl, 17.72; S, 8.02. Found: C, 61.28; H, 4.73; Cl, 17.33; S, 8.30.

### Example 28

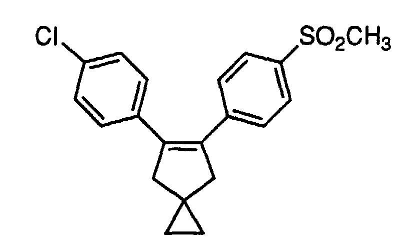

### 5-(4-Chlorophenyl)-6-[4-(methylsulfonyl)phenyl]spiro[2.4]hept-5-ene

Following a procedure similar to the one described in Example 6, with the substitution of 2-bromo-4'-chloroacetophenone (Aldrich) for 2-bromo-(3'-chloro-4'-methoxy)acetophenone (Example 6, Step 8), 5-(4-chlorophenyl)-6-[4-(methylsulfonyl)pheny]spiro[2.4]hept-5-ene was prepared as a white solid: mp 143.0-145.0 °C; ¹H NMR (CDCl₃) δ 0.69 (s, 4H), 2.92 (s, 4H), 3.05 (s, 3H), 7.07 (d, J = 8.7 Hz, 2H), 7.21 (d, J = 8.7 Hz, 2H), 7.33 (d, J = 8.4 Hz, 2H), 7.77 (dd, J = 8.7 Hz, 2H). MS (FAB): m/z 365 (100, M+Li) Anal. Calc'd for C₂₀H₁₉ClO₂S: C, 66.94; H, 5.36; Cl, 9.88; S, 8.93. Found: C, 66.34; H, 5.38; Cl, 9.96; S, 9.01.

### Example 29

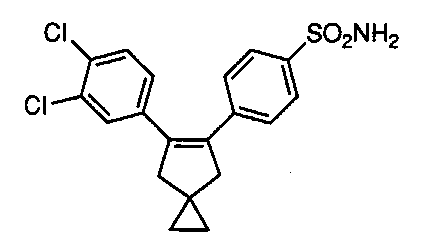

### 4-[6-(3,4-Dichlorophenyl)spiro[2.4]hept-5-en-5-yl]benzenesulfonamide

Following a procedure similar to the one described in Example 7, 5.20 g (13.2 mmol) of 5-(3,4-dichlorophenyl)-6-[4-(methylsulfonyl)phenyl]spiro[2.4]hept-5-ene (the title compound of Example 27) was converted to crude sulfonamide. Purification by silica gel chromatography gave 1.40 g (27%) of 4-[6-(3,4-dichlorophenyl)spiro[2.4]hept-5-en-5-yl]benzenesulfonamide as a white solid: mp 162.0-163.0 °C; ¹H NMR (CDCl₃) δ 0.69 (s, 4H), 2.90 (d, J =2.1 Hz, 4H), 4.77 (s, 2H), 6.92 (dd, J = 2.1, 8.4 Hz, 1H), 7.23-7.32 (m, 4H), 7.78 (d, J = 8.7 Hz, 2H). HRMS (EI) Calc'd for C₁₉H₁₇Cl₂NO₂S: 393.0357. Found: 393.0354. Anal. Calc'd for [C₁₉H₁₇Cl₂NO₂S+ 0.035 C₆H₁₄] : C, 58.07; H, 4.44; N, 3.52; Cl, 17.84; S, 8.07. Found: C, 58.15; H, 4.41; N, 3.43; Cl, 17.59; S, 8.88.

### Example 30

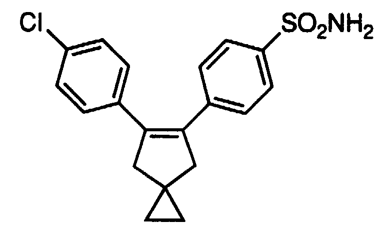

### 4-[6-(4-Chlorophenyl)spiro[2.4]hept-5-en-5-yl]benzenesulfonamide

Following a procedure similar to the one described in Example 7, 7.5 g (20.9 mmol) of 5-(4-chlorophenyl)-6-[4-(methylsulfonyl)phenyl]spiro[2.4]hept-5-ene (the title compound of Example 28) was converted to crude sulfonamide. Purification by silica gel chromatography gave 2.82 g (37%) of 4-[6-(4-chlorophenyl)spiro[2.4]hept-5-en-5-yl]benzenesulfonamide as a white solid: mp 152.5-153.5 °C; ¹H NMR (CDCl₃) δ 0.68 (s, 4H), 2.91 (s, 4H), 4.85-5.05 (br s, 2H), 7.07 (d, J = 8.4 Hz, 2H), 7.20 (d, J = 8.4 Hz, 2H), 7.27 (d, J = 8.1 Hz, 2H), 7.75 (d, J = 8.4 Hz, 2H). HRMS (EI) Calc'd for C₁₉H₁₈ClNO₂S: 359.0747. Found: 359.0747. Anal. Calc'd for C₁₉H₁₈ClNO₂S: C, 63.41; H, 5.04; Cl, 9.85; N, 3.89; S, 8.91. Found: C, 63.47; H, 5.12; Cl, 10.21; N, 3.78; S, 8.98.

### Example 31

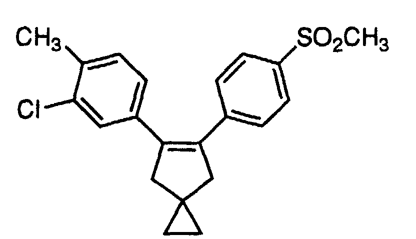

### 5-(3-Chloro-4-methylphenyl)-6-[4-(methylsulfonyl)phenyl]spiro[2.4]hept-5-ene

Following a procedure similar to the one described in Example 6, with the substitution of 3'-chloro-4'-methylacetophenone [prepared by the addition of methyllithium to 3-chloro-4-methylbenzonitrile (Aldrich), see Example 6, Step 1] for 3'-chloro-4'-methoxyacetophenone (Example 6, Step 7), 5-(3-chloro-4-methylphenyl)-6-[4-(methylsulfonyl)phenyl]spiro[2.4]hept-5-ene was prepared as a white solid: mp 142-144 °C; ¹H NMR (CDCl₃) δ 0.68 (s, 4H), 2.33 (s, 3H) 2.90 (d, J = 2.1 Hz, 4H), 3.04 (s, 3H), 6.88 (dd, J = 1.5, 7.5 Hz, 1H), 7.06 (d, J = 7.5 Hz, 1H), 7.14 (d, J = 1.5 Hz, 1H), 7.34 (d, J = 8.7 Hz, 2H), 7.78 (d, J = 8.7 Hz, 2H). HRMS (EI) Calc'd for C₂₁H₂₁ClO₂S: 372.0951. Found: 372.0922. Anal. Calc'd for C₂₁H₂₁ClO₂S: C, 67.58; H, 5.63; S, 8.58. Found: C, 67.47; H, 5.86; S, 8.52.

### Example 32

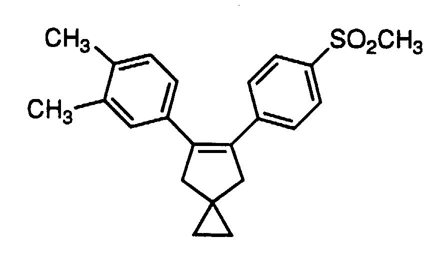

### 5-(3,4-Dimethylphenyl)-6-[4-(methylsulfonyl)phenyl]spiro[2.4]hept-5-ene

Following a procedure similar to the one described in Example 6, with the substitution of 3',4'-dimethylacetophenone (Aldrich) for 3'-chloro-4'-methoxyacetophenone (Example 6, Step 7), 5-(3,4-dimethylphenyl)-6-[4-(methylsulfonyl)phenyl]spiro[2.4]hept-5-ene was prepared as a white solid: mp 95-96.5 °C; ¹H NMR (CDCl₃) δ 0.67 (s, 4H), 2.17 (s, 3H), 2.23 (s, 3H), 2.91 (s, 4H), 3.03 (s, 3H), 6.82-6.87 (m, 1H), 6.91-7.01 (m, 2H), 7.35 (d, J = 8.7 Hz, 2H), 7.74 (d, J = 8.7 Hz, 2H). HRMS (EI) Calc'd for C₂₂H₂₄O₂S: 352.1497. Found: 352.1496. Anal. Calc'd for C₂₂H₂₄O₂S: C, 74.89; H, 6.81; S, 9.08. Found: C, 74.45; H, 6.96; S, 8.93.

### Example 33

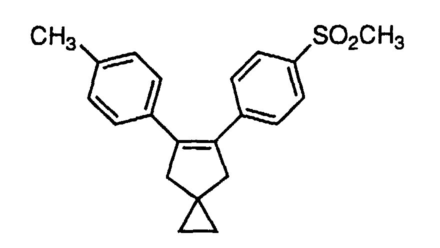

### 5-(4-Methylphenyl)-6-[4-(methylsulfonyl)phenyl]spiro[2.4]hept-5-ene

Following a procedure similar to the one described in Example 6, with the substitution of 2-bromo-4'-methylacetophenone (Aldrich) for 2-bromo-(3'-chloro-4'-methoxy)acetophenone (Example 6, Step 8), 5-(4-methylphenyl)-6-[4-(methylsulfonyl)phenyl]spiro[2.4]hept-5-ene was prepared as a white solid: mp 146-148 °C; ¹H NMR (CDCl₃) δ 0.67 (s, 4H), 2.32 (s, 3H), 2.91 (s, 4H), 3.03 (s, 3H), 7.04 (s, 4H), 7.34 (d, J = 8.7 Hz, 2H), 7.74 (d, J = 8.7 Hz, 2H). HRMS (EI) Calc'd for C₂₁H₂₂O₂S: 338.1341. Found: 338.1323. Anal. Calc'd for C₂₁H₂₂O₂S: C, 74.45; H, 6.50; S, 9.45. Found: C, 73.90; H, 6.64; S, 9.31.

### Example 34

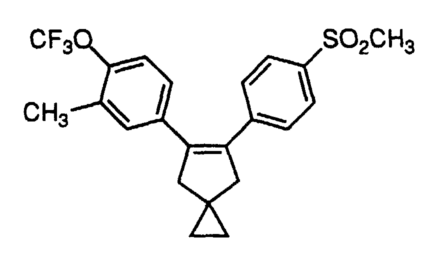

### 5-(3-Methyl-4-trifluoromethoxyphenyl)-6-[4-(methylsulfonyl)phenyl]spiro[2.4]hept-5-ene

### Step 1: Preparation of 4-(trifluoromethoxy)acetophenone

Under nitrogen, to a stirred solution of 66 g (353 mmol) of 4-(trifluoromethoxy)benzonitrile (Aldrich) in 600 mL of anhydrous THF at -78 °C was added 303 mL (424 mmol) of methyllithium (1.4 M in diethyl ether, Aldrich). After stirred for three hours, the solution was warmed to room temperature. A 300 mL of 3 N HCl was added, and stirring was continued overnight. The THF was removed in vacuo, and the resulting solution was extracted twice with ethyl acetate. The ethyl acetate extracts were combined, washed with water, brine, and dried over MgSO₄. Concentration of solvent in vacuo gave 63.9 g (89%) of the 4-(trifluoromethoxy)acetophenone as a dark brown oil: ¹H NMR (CDCl₃) δ 2.61 (s, 3H), 7.29 (d, J = 9 Hz, 2H), 8.01 (d, J = 9 Hz, 2H).

### Step 2: Preparation of ketal

Under nitrogen, to a stirred solution of 63.9 g (313 mmol) of 4-(trifluoromethoxy)acetophenone (prepared in Step 1) in 200 mL of toluene in a 500 mL round bottom flask fitted with a Dean-Stark trap was added 65.2 g (626 mmol) of neopentyl glycol (Aldrich) and 1.3 g of p-toluenesulfonic acid monohydrate (Aldrich). After stirring overnight at reflux, the solution was cooled to room temperature and concentrated in vacuo. The residue was dissolved in diethyl ether, washed with 2 M NaHCO₃, and dried over MgSO₄. Purification by silica gel plug with triethylamine/hexane (1:99) as the eluent gave 79.7 g (88%) of the ketal as a brown oil: ¹H NMR (CDCl₃) δ 0.59 (s, 3H), 1.25 (s, 3H), 1.51 (s, 3H), 3.38 (s, 4H), 7.22 (d, J = 9 Hz, 2H), 7.45 (d, J = 9 Hz, 2H).

### Step 3: Metallation of the ketal

Under nitrogen, to a stirred solution of 79.7 g (275 mmol) of the ketal (prepared in Step 2) in 511 mL of dry hexane was added 114 mL (545 mmol) of TMEDA (N, N, N', N'-tetramethylethylenediamine). After cooling to -78 °C, the solution was treated with 582 mL (757 mmol) of sec-butyllithium (1.3 M in cyclohexane, Aldrich), and stirred vigorously for five hours at -78 °C. To this solution was added 51.4 mL (826 mmol) of iodomethane and stirred for one hour. After warming to room temperature, the reaction was quenched with 2 N HCl, and extracted with diethyl ether. The diethyl ether extracts were combined, washed with 2 N HCl, and dried over MgSO₄. Concentration of the solvent in vacuo gave 74 g of the ketal of 3'-methyl-4'-(trifluoromethoxy)acetophenone as a brown oil, and was used for next step without further purification.

### Step 4: Preparation of 3-methyl-4-(trifluoromethoxy)acetophenone

Under nitrogen, to a stirred solution of 74 g of the crude oil (prepared in Step 3) in 200 mL of acetone and 10 mL of water was added 130 g (683 mmol) of *p*-toluenesulfonic acid monohydrate (Aldrich). After stirring overnight, the solution was concentrated in vacuo, and the residue was dissolved in ethyl acetate. The ethyl acetate layer was washed with 2 N NaHCO₃, and dried over MgSO₄. Purification by silica gel plug with ethyl acetate/ hexane (10:90) as the eluent gave 31.9 g (53% for both Steps 3 and 4) of 3-methyl-4-(trifluoromethoxy)acetophenone as a yellow oil: ¹H NMR (CDCl₃) δ 2.37 (s, 3H), 2.59 (s, 3H), 7.27 (d, J = 8 Hz, 1H), 7.81 (d, J = 8 Hz, 1H), 7.86 (s, 1H).

### Step 5: Preparation of 5-(3-methyl-4-trifluoromethoxyphenyl)-6-[4-(methylsulfonyl)phenyl]spiro[2.4]hept-5-ene

Following a procedure similar to the one described in Example 6 with the substitution of 3'-methyl-4'-(trifluoromethoxy)acetophenone (prepared in Step 4) for 3'-chloro-4'-methoxyacetophenone (Example 6, Step 7), 5-(3-methyl-4-trifluoromethoxyphenyl)-6-[4-(methylsulfonyl)phenyl]spiro [2.4]hept-5-ene was prepared as a white solid: mp 79.1-80.3 °C. MS (FAB): m/z 429 (M+Li). ¹H NMR (CDCl₃) δ 0.69 (s, 4H), 2.22 (s, 3H), 2.92 (s, 4H), 3.04 (s, 3H), 6.91-6.97 (m, 1H), 7.03-7.08 (m, 2H), 7.34 (d, J = 8 Hz, 2H), 7.77 (d, J = 8 Hz, 2H). Anal. Calc'd for C₂₂H₂₁F₃O₃S: C, 62.55; H, 5.01; F, 13.49. Found: C, 62.29; H, 4.93; F, 13.49.

### Example 35

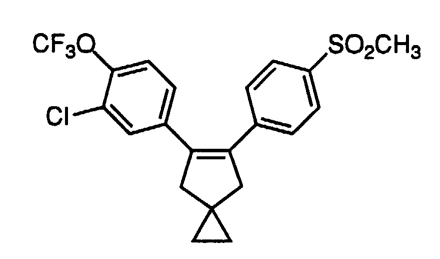

### 5-(3-Chloro-4-trifluoromethoxyphenyl)-6-[4-(methylsulfonyl)phenyl]spiro[2.4]hept-5-ene

### Step 1: Preparation of 3-chloro-4-(trifluoromethoxy)benzoic acid

A dried, 750-mL Parr reactor (steel) equipped with a sealed mechanical stirrer and an internal thermocouple was charged with 54.2 g (0.314 mol) of 3-chloro-4-hydroxybenzoic acid, 125.5 mL (1.3 mol) of carbon tetrachloride, 172 g (0.965 mol) of anhydrous antimony trifluoride, and 7.55 g (0.025 mol) of antimony pentachloride. The reactor was sealed and heated at 150 °C with stirring for 4 hours. After cooling, the reactor was opened and the contents were neutralized with saturated NaHCO3 and 50% NaOH, then adjusted down to pH 1 with concentrated HCl. The mixture was filtered, and the aqueous filtrate was separated and extracted with 500 mL of ethyl acetate. The combined extracts were extracted with dilute NaOH solution (pH = 11). To the aqueous extract was added 200 mL of brine, and the mixture was extracted with ethyl acetate. The extract was concentrated in vacuo, and the residue was treated with 250 mL of methanol and 290 mL (720 mmol) of 2.5 N NaOH and stirred at ambient temperature for 22 hours. The reaction mixture was treated with conc. HCl to pH 1, extracted with ethyl acetate, dried (MgSO₄), and concentrated in vacuo to give 35.5 g (47%) of 3-chloro-4-(trifluoromethoxy)benzoic acid as a white solid: ¹H NMR (CDCl₃) δ 7.69 (d, J = 8.7 Hz, 1H), 8.03 (dd, J = 1.8, 8.7 Hz, 1H), 8.13 (d, J = 1.5 Hz, 1H).

### Step 2: Preparation of 5-(3-chloro-4-trifluoromethoxyphenyl)-6-[4-(methylsulfonyl)phenyl]spiro[2.4]hept-5-ene

Following a procedure similar to the one described in Example 6, with the substitution of 3-chloro-4-(trifluoromethoxy)benzoic acid (prepared in Step 1) for 3-chloro-4-methoxybenzoic acid (Example 6, Step 5), 5-(3-chloro-4-trifluoromethoxyphenyl)-6-[4-(methylsulfonyl)phenyl]spiro[2.4]hept-5-ene was prepared as a white solid: mp 104-105.5 °C; ¹H NMR (CDCl₃) δ 0.69 (s, 4H), 2.92 (br d, J = 2.7 Hz, 4H), 3.05 (s, 3H), 7.00 (dd, J = 2.1, 8.4 Hz, 1H), 7.16 (dd, J = 1.2, 8.7 Hz, 1H), 7.25 (s, 1H), 7.33 (d, J = 8.4 Hz, 2H), 7.81 (d, J = 8.7 Hz, 2H). HRMS (EI) Calc'd for C₂₁H₁₈ClF₃O₃S: 442.0617. Found: 442.0617. Anal. Calc'd for C₂₁H₁₈ClF₃O₃S: C, 56.95; H, 4.10; Cl, 8.01; F, 12.87; S, 7.24. Found: C, 57.10; H, 4.15; Cl, 7.74; F, 12.55; S, 7.48.

### Example 36

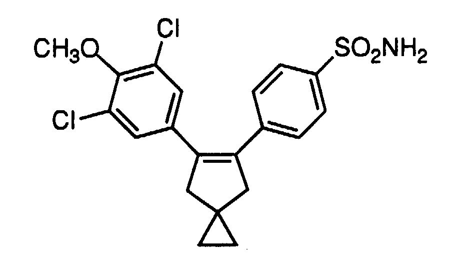

### 4-[6-(3,5-Dichloro-4-methoxyphenyl)spiro[2.4]hept-5-en-5-yl]benzenesulfonamide

Following a procedure similar to the one described in Example 7, with the substitution of butyllithium instead of propylmagnesium chloride, 423 mg (1 mmol) of 5-(3,5-dichloro-4-methoxy-phenyl)-6-[4-(methylsulfonyl)phenyl]spiro[2.4]hept-5-ene (the title compound of Example 20) was converted to crude sulfonamide. Purification by silica gel chromatography gave 87 mg (15%) of 4-[6-(3,5-dichloro-4-methoxyphenyl)spiro[2.4]hept-5-en-5-yl]benzenesulfonamide as a white solid: mp 94.5-97.0 °C (decomp); ¹H NMR (CDCl₃) 6 0.68 (s, 4H), 2.83-2.95 (m, 4H), 3.89 (s, 3H), 4.81 (br s, 2H), 7.05 (s, 2H), 7.29 (d, J = 8.5 Hz, 2H), 7.80 (d, J = 8.6 Hz, 2H). HRMS (EI) Calc'd for C₂₀H₁₉Cl₂NO₃S: 423.0463. Found: 423.0455.

### Example 37

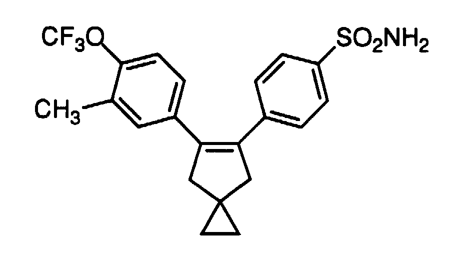

### 4-[6-(3-Methyl-4-trifluoromethoxyphenyl)spiro[2.4]hept-5-en-5-yl]benzenesulfonamide

Following a procedure similar to the one described in Example 7, 470 mg (1.16 mmol) of 5-(3-methyl-4-trifluoromethoxyphenyl)-6-[4-(methylsulfonyl)phenyl]spiro[2.4]hept-5-ene (the title compound of Example 34) was converted to crude sulfonamide. Purification by silica gel chromatography (MPLC) gave 70 mg (14%) of 4-[6-(3-methyl-4-trifluoromethoxyphenyl)spiro[2.4]hept-5-en-5-yl]benzenesulfonamide as a white solid: mp 136-137 °C. MS (FAB): m/z 430 (M+Li). ¹H NMR (CDCl₃) δ 0.68 (s, 4H), 2.22 (s, 3H), 2.91 (s, 4H), 4.71 (s, 2H), 6.92-6.97 (m, 1H), 7.02-7.07 (m, 2H), 7.29 (d, J = 8 Hz, 2H), 7.75 (d, J = 8 Hz, 2H). Anal. Calc'd for C₂₁H₂₀F₃NO₃S: C, 59.57; H, 4.76; N, 3.31. Found: C, 59.75; H, 4.88; N, 3.17.

### Example 38

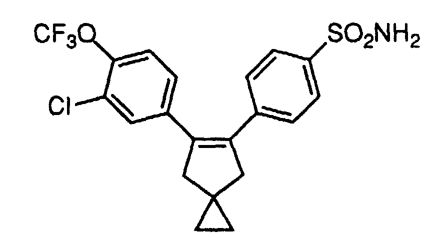

### 4-[6-(3-Chloro-4-trifluoromethoxyphenyl)spiro[2.4]hept-5-en-5-yl]benzenesulfonamide

Following a procedure similar to the one described in Example 7, 0.50 g (1.13 mmol) of 5-(3-chloro-4-trifluoromethoxyphenyl)-6-[4-(methylsulfonyl)phenyl]spiro[2.4]hept-5-ene (the title compound of Example 35) was converted to crude sulfonamide. Purification by silica gel chromatography gave 0.39 g (78%) of 4-[6-(3-chloro-4-trifluoromethoxyphenyl)spiro[2.4]hept-5-en-5-yl]benzenesulfonamide as a white solid: mp 125.0-128.0 °C; ¹H NMR (CDCl₃) δ 0.62 (s, 4H), 2.84 (s, 4H), 4.71 (br s, 2H), 6.94 (dd, J = 2.1, 8.4 Hz, 1H), 7.08 (dd, J = 1.2, 8.7 Hz, 1H), 7.14-7.26 (m, 3H), 7.72 (d, J = 8.7 Hz, 2H). HRMS (EI) Calc'd for C₂₀H₁₇ClF₃NO₃S: 443.0570. Found: 443.0603.

### BIOLOGICAL EVALUATION

### Rat Carrageenan Foot Pad Edema Test

The carrageenan foot edema test was performed with materials, reagents and procedures essentially as described by Winter, et al., (Proc. Soc. Exp. Biol. Med., 111, 544 (1962)). Male Sprague-Dawley rats were selected in each group so that the average body weight was as close as possible. Rats were fasted with free access to water for over sixteen hours prior to the test. The rats were dosed orally (1 mL) with compounds suspended in vehicle containing 0.5% methylcellulose and .025% surfactant, or with vehicle alone. One hour later a subplantar injection of 0.1 mL of 1% solution of carrageenan/sterile 0.9% saline was administered and the volume of the injected foot was measured with a displacement plethysmometer connected to a pressure transducer with a digital indicator. Three hours after the injection of the carrageenan, the volume of the foot was again measured. The average foot swelling in a group of drug-treated animals was compared with that of a group of placebo-treated animals and the percentage inhibition of edema was determined (Otterness and Bliven, Laboratory Models for Testing NSAIDs, in Non-steroidal Anti-Inflammatory Drugs, (J. Lombardino, ed. 1985)). Results are shown in Table I.

### Rat Carrageenan-induced Analgesia Test

The rat carrageenan analgesia test was performed with materials, reagents and procedures essentially as described by Hargreaves, et al., (Pain, 32, 77 (1988)). Male Sprague-Dawley rats were treated as previously described for the Carrageenan Foot Pad Edema test. Three hours after the injection of the carrageenan, the rats were placed in a special plexiglass container with a transparent floor having a high intensity lamp as a radiant heat source, positionable under the floor. After an initial twenty minute period, thermal stimulation was begun on either the injected foot or on the contralateral uninjected foot. A photoelectric cell turned off the lamp and timer when light was interrupted by paw withdrawal. The time until the rat withdraws its foot was then measured. The withdrawal latency in seconds was determined for the control and drug-treated groups, and percent inhibition of the hyperalgesic foot withdrawal determined. Results are shown in Table I.

**TABLE I.**

| | **RAT PAW EDEMA** | **ANALGESIA** |
|---|---|---|
| | % Inhibition @ 10mg/kg body weight | % Inhibition @ 10mg/kg body weight |
| Examples | | |
| 1 | 32 | 15 |
| 2 | 57 | 34 |
| 3 | 24 | - |
| 4 | 17 | - |
| 6 | 21 | 7 |
| 7 | 20 | 18 |
| 8 | 15 | 8 |
| 9 | 15 | 17 |
| 14 | 18 | - |
| 16 | 6 | - |
| 19 | 11 | 9 |
| 20 | 24 | 28 |
| 22 | 21 | 10 |
| 25 | 34 | 22 |
| 27 | 15 | - |
| 29 | 24 | 29 |

### Evaluation of COX-1 and COX-2 activity in vitro

### a. Preparation of recombinant COX baculoviruses

Recombinant COX-1 and COX-2 were prepared as described by Gierse et al, [J. Biochem, 305, 479-84 (1995)]. A 2.0 kb fragment containing the coding region of either human or murine COX-1 or human or murine COX-2 was cloned into a BamH1 site of the baculovirus transfer vector pVL1393 to generate the baculovirus transfer vector. Recombinant baculoviruses were isolated by transfecting 4 µg of baculovirus transfer vector DNA into SF9 cells (2x10⁸) along with 200 ng of linearized bacium phosphate method. Recombinant viruses were purified by three rounds of plaque purification and high titer (10⁷ - 10⁸ pfu/ml) stocks of virus were prepared. For large scale production, SF9 insect cells were infected in 10 liter fermentors (0.5 x 10⁶/ml) with the recombinant baculovirus stock such that the multiplicity of infection was 0.1. After 72 hours the cells were centrifuged and the cell pellet homogenized in Tris/Sucrose (50 mM: 25%, pH 8.0) containing 1% CHAPS. The homogenate was centrifuged at 10,000xG for 30 minutes, and the resultant supernatant was stored at -80°C before being assayed for COX activity.

### b. Assay for COX I and COX II activity:

COX activity was assayed as PGE₂ formed/µg protein/time using an ELISA to detect the prostaglandin released. CHAPS-solubilized insect cell membranes containing the appropriate COX enzyme were incubated in a potassium phosphate buffer (50 mM, pH 8.0) containing epinephrine, phenol, and heme with the addition of arachidonic acid (10 µM). Compounds were pre-incubated with the enzyme for 10-20 minutes prior to the addition of arachidonic acid. Any reaction between the arachidonic acid and the enzyme was stopped after ten minutes at 37°C/room temperature by transferring 40 µl of reaction mix into 160 µl ELISA buffer and 25 µM indomethacin. The PGE₂ formed was measured by standard ELISA technology (Cayman Chemical). Results are shown in Table II.

**TABLE II.**

| | **COX I** **ID**_{**50**} **µM** | **COX II** **ID** _{**50**} **µM** |
|---|---|---|
| Examples | | |
| 1 | 14 | <0.1 |
| 2 | 0.2 | <0.1 |
| 3 | >100 | <0.1 |
| 4 | 0.9 | <0.1 |
| 5 | >100 | <0.1 |
| 6 | >100 | <0.1 |
| 7 | 1.8 | <0.1 |
| 8 | >100 | <0.1 |
| 9 | .5 | <0.1 |
| 10 | >100 | <0.1 |
| 11 | 1.4 | <0.1 |
| 12 | 0.9 | <0.1 |
| 13 | >100 | 4.8 |
| 14 | >100 | 0.1 |
| 15 | 0.6 | <0.1 |
| 16 | 59 | <0.1 |
| 17 | <0.1 | <0.1 |
| 18 | 0.4 | <0.1 |
| 19 | >100 | <0.1 |
| 20 | >100 | <.1 |
| 21 | 1.8 | 0.1 |
| 22 | 6.0 | <0.1 |
| 23 | 0.7 | <0.1 |
| 24 | >100 | <0.1 |
| 25 | 0.4 | <0.1 |
| 26 | 0.3 | <0.1 |
| 27 | >100 | <0.1 |
| 28 | 3.1 | <0.1 |
| 29 | 0.4 | <0.1 |
| 30 | 0.1 | <0.1 |
| 31 | >100 | <0.1 |
| 32 | >100 | <0.1 |
| 33 | 4.0 | <0.1 |
| 34 | >100 | <0.1 |
| 36 | 16.4 | <0.1 |
| 37 | 20.6 | <0.1 |
| 38 | 25.2 | 0.3 |

Also embraced within this invention is a class of pharmaceutical compositions comprising one or more compounds of Formula I in association with one or more non-toxic, pharmaceutically-acceptable carriers and/or diluents and/or adjuvants (collectively referred to herein as "carrier" materials) and, if desired, other active ingredients. The compounds of the present invention may be administered by any suitable route, preferably in the form of a pharmaceutical composition adapted to such a route, and in a dose effective for the treatment intended. The compounds and composition may, for example, be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The active ingredient may also be administered by injection as a composition wherein, for example, saline, dextrose or water may be used as a suitable carrier.

The amount of therapeutically active compound that is administered and the dosage regimen for treating a disease condition with the compounds and/or compositions of this invention depends on a variety of factors, including the age, weight, sex and medical condition of the subject, the severity of the disease, the route and frequency of administration, and the particular compound employed, and thus may vary widely. The pharmaceutical compositions may contain active ingredient in the range of about 0.1 to 2000 mg, preferably in the range of about 0.5 to 500 mg and most preferably between about 1 and 100 mg. A daily dose of about 0.01 to 100 mg/kg body weight, preferably between about 0.1 and about 50 mg/kg body weight and most preferably between about 1 to 20 mg/kg body weight, may be appropriate. The daily dose can be administered in one to four doses per day.

For therapeutic purposes, the compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Although this invention has been described with respect to specific embodiments, the details of these embodiments are not to be construed as limitations.

## Claims

1. A compound of Formula I 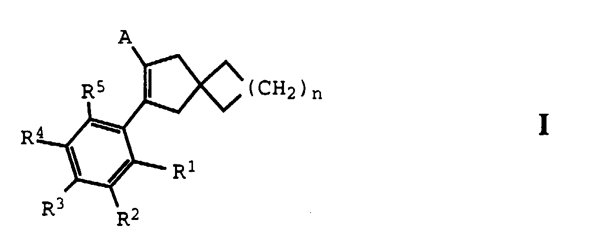
wherein A is selected from 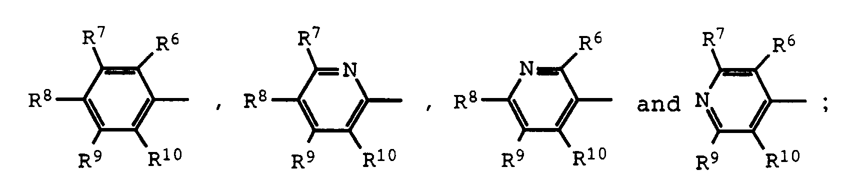
wherein each of R¹ through R¹⁰, if present, is independently selected from hydrido, halo, alkyl, alkoxy, alkylthio, alkylamino, cyano, haloalkyl, haloalkoxy, hydroxyalkyl, alkoxyalkyl, hydroxyl, mercapto, alkylsulfonyl, haloalkylsulfonyl and aminosulfonyl; and wherein n is a number selected from 0, 1, 2 and 3; or a pharmaceutically-acceptable salt thereof.

2. Compound of Claim 1 wherein, if present, each of R¹, R², R⁴ through R⁷, R⁹ and R¹⁰ is independently selected from hydrido, halo, lower alkyl, lower alkoxy, lower alkylthio, cyano, lower haloalkyl, lower haloalkoxy, lower hydroxyalkyl, lower alkoxyalkyl, hydroxyl and mercapto; and wherein R³ is selected from lower alkylsulfonyl, lower haloalkylsulfonyl and aminosulfonyl, and R⁸, if present, is selected from hydrido, halo, lower alkyl, lower alkoxy, lower alkylthio, lower alkylamino, cyano, lower haloalkyl, lower haloalkoxy, lower hydroxyalkyl, lower alkoxyalkyl, hydroxyl and mercapto; or wherein further R⁸ and R⁹, if present, together form methylenedioxy; or wherein further, R³ is selected from hydrido, halo, lower alkyl, lower alkoxy, lower alkylthio, lower alkylamino, cyano, lower haloalkyl, lower haloalkoxy, lower hydroxyalkyl, lower alkoxyalkyl, hydroxyl and mercapto, and R⁸ is selected . from lower alkylsulfonyl, lower haloalkylsulfonyl and aminosulfonyl; or wherein further R³ and R⁴, if present, together form methylenedioxy; or a pharmaceutically-acceptable salt thereof.

3. Compound of Claim 2 wherein, if present, each of R¹, R², R⁴ through R⁷, R⁹ and R¹⁰ is independently selected from hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl; and wherein R³ is selected from methylsulfonyl, fluoromethylsulfonyl, difluoromethylsulfonyl, trifluoromethylsulfonyl and aminosulfonyl, and R⁸, if present, is selected from hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, methylamino, N,N-dimethylamino, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl; or wherein further R⁸ and R⁹, if present, together form methylenedioxy; or wherein further R³ is selected from hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, methylamino, N,N-dimethylamino, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl, and R⁸ is selected from methylsulfonyl, fluoromethylsulfonyl, difluoromethylsulfonyl, trifluoromethylsulfonyl and aminosulfonyl; or wherein further R³ and R⁴, if present, together form methylenedioxy; or a pharmaceutically-acceptable salt thereof.

4. Compound of Claim 1 wherein A is 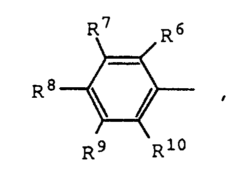 wherein each of R¹ through R¹⁰ is independently selected from hydrido, halo, alkyl, alkoxy, alkylthio, alkylamino, cyano, haloalkyl, haloalkoxy, hydroxyalkyl, alkoxyalkyl, hydroxyl, mercapto, alkylsulfonyl, haloalkylsulfonyl and aminosulfonyl; and wherein n is a number selected from 0, 1, 2 and 3; or a pharmaceutically-acceptable salt thereof.

5. Compound of Claim 4 wherein n is a number selected from 0, 1 and 2; wherein each of R¹, R² and R⁴ through R¹⁰ is independently selected from hydrido, halo, lower alkyl, lower alkylthio, lower alkylamino, cyano, lower haloalkyl, lower haloalkoxy, lower alkoxy, hydroxyl, mercapto, lower hydroxyalkyl and lower alkoxyalkyl; and wherein R³ is selected from lower alkylsulfonyl, lower haloalkylsulfonyl and aminosulfonyl; or wherein R⁸ and R⁹ together form methylenedioxy; or a pharmaceutically-acceptable salt thereof.

6. Compound of Claim 5 wherein each of R¹, R² and R⁴ through R¹⁰ is independently selected from hydrido, fluoro, chloro, bromo, iodo, methyl, ethyl, n-propyl, isopropyl, butyl, *tert*-butyl, isobutyl, methoxy, ethoxy, propoxy, butoxy, hydroxyl, mercapto, methylthio, ethylthio, methylamino, N,N-dimethylamino, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl; and wherein R³ is selected from methylsulfonyl, fluoromethylsulfonyl, difluoromethylsulfonyl, trifluoromethylsulfonyl and aminosulfonyl; or wherein R⁸ and R⁹ together form methylenedioxy; or a pharmaceutically-acceptable salt thereof.

7. Compound of Claim 6 selected from compounds, and their pharmaceutically-acceptable salts, of the group consisting of
5-[6-[4-(methylsulfonyl)phenyl]spiro[2.4]hept-5-en-5-yl]-1,3-benzodioxole;
2,6-dichloro-4-[6-[4-(methylsulfonyl)phenyl]spiro[2.4]hept-5-en-5-yl]phenol;
5-(4-trifluoromethoxyphenyl)-6-[4-(methylsulfonyl)phenyl]spiro[2.4]hept-5-ene;
5-(3-bromo-4-methoxyphenyl)-6-[4-(methylsulfonyl)phenyl]spiro[2.4]hept-5-ene;
4-[6-(3-bromo-4-methoxyphenyl)spiro[2.4]hept-5-en-5-yl]benzenesulfonamide;
5-(3,5-dichloro-4-methoxy-phenyl)-6-[4-(methylsulfonyl)phenyl]spiro[2.4]hept-5-ene;
4-[6-(4-trifluoromethoxyphenyl)spiro[2.4]hept-5-en-5-yl]benzenesulfonamide;
5-(2,4-difluorophenyl)-6-[4-(methylsulfonyl)phenyl]spiro[2.4]hept-5-ene;
5-(2,4-dichlorophenyl)-6-[4-(methylsulfonyl)phenyl]spiro[2.4]hept-5-ene;
5-(3-chloro-4-methylphenyl)-6-[4-(methylsulfonyl)phenyl]spiro[2.4]hept-5-ene;
5-(3,4-dimethylphenyl)-6-[4-( methylsulfonyl)phenyl]spiro[2.4]hept-5-ene;
5-(3-methyl-4-trifluoromethoxyphenyl)-6-[4-(methylsulfonyl)phenyl]spiro[2.4]hept-5-ene;
5-(3-chloro-4-trifluoromethoxyphenyl)-6-[4-(methylsulfonyl)phenyl]spiro[2.4]hept-5-ene;
4-[6-(3,5-dichloro-4-methoxyphenyl)spiro[2.4]hept-5-en-5-yl]benzenesulfonamide;
4-[6-(3-methyl-4-trifluoromethoxyphenyl)spiro[2.4]hept-5- en-5-yl]benzenesulfonamide;
4-[6-(3-chloro-4-trifluoromethoxyphenyl)spiro[2.4]hept-5- en-5-yl]benzenesulfonamide;
5-phenyl-6-[4-(methylsulfonyl)phenyl]spiro[2.4]hept-5-ene;
5-(4-fluorophenyl)-6-[4-(methylsulfonyl)phenyl] spiro[2.4]hept-5-ene;
5-(4-chlorophenyl)-6-[4-(methylsulfonyl)phenyl] spiro[2.4]hept-5-ene;
5-(4-methylphenyl)-6-[4-(methylsulfonyl)phenyl] spiro[2.4]hept-5-ene;
5-(4-methoxyphenyl)-6-[4-(methylsulfonyl)phenyl] spiro[2.4]hept-5-ene;
5-(4-methylthiophenyl)-6-[4-(methylsulfonyl)phenyl] spiro[2.4]hept-5-ene;
5-(4-cyanophenyl)-6-[4-(methylsulfonyl)phenyl] spiro[2.4]hept-5-ene;
5-(4-trifluoromethylphenyl)-6-[4-(methylsulfonyl) phenyl]spiro[2.4]hept-5-ene;
4-(6-phenylspiro[2.4]hept-5-en-5-yl) benzenesulfonamide;
4-[6-(4-fluorophenyl)spiro[2.4]hept-5-en-5-yl] benzenesulfonamide;
4-[6-(4-chlorophenyl)spiro[2.4]hept-5-en-5-yl] benzenesulfonamide;
4-[6-(4-methylphenyl)spiro[2.4]hept-5-en-5-yl] benzenesulfonamide;
4-[6-(4-methoxyphenyl)spiro[2.4]hept-5-en-5-yl] benzenesulfonamide;
4-[6-(4-methylthiophenyl)spiro[2.4]hept-5-en-5-yl] benzenesulfonamide;
4-[6-(4-cyanophenyl)spiro[2.4]hept-5-en-5-yl] benzenesulfonamide;
4-[6-(4-trifluoromethylphenyl)spiro[2.4]hept-5-en-5-yl)benzenesulfonamide;
6-phenyl-7-[4-(methylsulfonyl)phenyl]spiro[3.4]oct-6-ene;
6-(4-fluorophenyl)-7-[4-(methylsulfonyl)phenyl] spiro[3.4]oct-6-ene;
6-(4-chlorophenyl)-7-[4-(methylsulfonyl)phenyl] spiro[3.4]oct-6-ene;
6-(4-methylphenyl)-7-[4-(methylsulfonyl)phenyl] spiro[3.4]oct-6-ene;
6-(4-methoxyphenyl)-7-[4-(methylsulfonyl)phenyl] spiro[3.4]oct-6-ene;
6-(4-methylthiophenyl)-7-[4-(methylsulfonyl)phenyl] spiro[3.4]oct-6-ene;
6-(4-cyanophenyl)-7-[4-(methylsulfonyl)phenyl] spiro[3.4]oct-6-ene;
6-(4-trifluoromethylphenyl)-7-[4-(methylsulfonyl) phenyl]spiro[3.4]oct-6-ene;
4-(7-phenylspiro[3.4]oct-6-en-6-yl)benzenesulfonamide;
4-[7-(4-fluorophenyl)spiro[3.4]oct-6-en-6-yl] benzenesulfonamide;
4-[7-(4-chlorophenyl)spiro[3.4]oct-6-en-6-yl] benzenesulfonamide;
4-[7-(4-methylphenyl)spiro[3.4]oct-6-en-6-yl] benzenesulfonamide;
4-[7-(4-methoxyphenyl)spiro[3.4]oct-6-en-6-yl] benzenesulfonamide;
4-[7-(4-methylthiophenyl)spiro[3.4]oct-6-en-6-yl] benzenesulfonamide;
4-[7-(4-cyanophenyl)spiro[3.4]oct-6-en-6-yl] benzenesulfonamide;
4-[7-(4-trifluoromethylphenyl)spiro[3.4]oct-6-en-6-yl] benzenesulfonamide;
2-phenyl-3-[4-(methylsulfonyl)phenyl]spiro[4.4]non-2-ene;
2-(4-fluorophenyl)-3-[4-(methylsulfonyl)phenyl] spiro[4.4]non-2-ene;
2-(4-chlorophenyl)-3-[4-(methylsulfonyl)phenyl] spiro[4.4]non-2-ene;
2-(4-methylphenyl)-3-[4-(methylsulfonyl)phenyl] spiro[4.4]non-2-ene;
2-(4-methoxyphenyl)-3-[4-(methylsulfonyl)phenyl] spiro[4.4]non-2-ene;
2-(4-methylthiophenyl)-3-[4-(methylsulfonyl) phenyl]spiro[4.4]non-2-ene;
2-(4-cyanophenyl)-3-[4-(methylsulfonyl)phenyl] spiro[4.4]non-2-ene;
2-(4-trifluoromethylphenyl)-3-[4-(methylsulfonyl) phenyl]spiro[4.4]non-2-ene;
4-(3-phenylspiro[4.4]non-2-en-2-yl)benzenesulfonamide;
4-[3-(4-fluorophenyl)spiro[4.4]non-2-en-2-yl] benzenesulfonamide;
4-[3-(4-chlorophenyl)spiro[4.4]non-2-en-2-yl] benzenesulfonamide;
4-[3-(4-methylphenyl)spiro[4.4]non-2-en-2-yl] benzenesulfonamide;
4-[3-(4-methoxyphenyl)spiro[4.4]non-2-en-2-yl] benzenesulfonamide;
4-[3-(4-methylthiophenyl)spiro[4.4]non-2-en-2-yl] benzenesulfonamide;
4-[3-(4-cyanophenyl)spiro[4.4]non-2-en-2-yl] benzenesulfonamide;
4-[3-(4-trifluoromethylphenyl)spiro[4.4]non-2-en-2-yl] benzenesulfonamide;
5-(3-methyl-4-fluorophenyl)-6-[4-(methylsulfonyl)phenyl]spiro[2.4]hept-5-ene;
5-(3-trifluoromethyl-4-fluorophenyl)-6-[4-(methylsulfonyl)phenyl]spiro[2.4]hept-5-ene;
5-(3-methyl-4-chlorophenyl)-6-[4-(methylsulfonyl)phenyl]spiro[2.4]hept-5-ene;
5-(3-trifluoromethyl-4-chlorophenyl)-6-[4-(methylsulfonyl)phenyl]spiro[2.4]hept-5-ene;
5-(3-methyl-4-methoxyphenyl)-6-[4-(methylsulfonyl)phenyl]spiro[2.4]hept-5-ene;
5-(3-trifluoromethyl-4-methoxyphenyl)-6-[4-(methylsulfonyl)phenyl]spiro[2.4]hept-5-ene;
5-(3-fluoro-4-methoxyphenyl)-6-[4-(methylsulfonyl)phenyl]spiro[2.4]hept-5-ene;
5-(3-chloro-4-methoxyphenyl)-6-[4-(methylsulfonyl)phenyl]spiro[2.4]hept-5-ene;
5-(4-methoxy-2,3,5,6-tetrafluorophenyl)-6-[4-(methylsulfonyl)phenyl]spiro[2.4]hept-5-ene;
5-(3,4-dimethoxyphenyl)-6-[4-(methylsulfonyl)phenyl]spiro[2.4]hept-5-ene;
5-(3-chloro-4-fluorophenyl)-6-[4-(methylsulfonyl)phenyl]spiro[2.4]hept-5-ene;
5-(4-chloro-3-fluorophenyl)-6-[4-(methylsulfonyl)phenyl]spiro[2.4]hept-5-ene;
5-(3,4-difluorophenyl)-6-[4-(methylsulfonyl)phenyl]spiro[2.4]hept-5-ene;
5-(3,4-dichlorophenyl)-6-[4-(methylsulfonyl)phenyl]spiro[2.4]hept-5-ene;
4-[6-(3-trifluoromethyl-4-fluorophenyl)spiro[2.4]hept-5-en-5-yl]benzenesulfonamide;
4-[6-(3-trifluoromethyl-4-chlorophenyl)spiro[2.4]hept-5-en-5-yl]benzenesulfonamide;
4-[6-(3-methyl-4-fluorophenyl)spiro[2.4]hept-5-en-5-yl]benzenesulfonamide;
4-[6-(3-methyl-4-chlorophenyl)spiro[2.4]hept-5-en-5-yl]benzenesulfonamide;
4-[6-(4-methoxy-2,3,5,6-tetrafluorophenyl)spiro[2.4]hept-5-en-5-yl]benzenesulfonamide;
4-[6-(3-fluoro-4-methoxyphenyl)spiro[2.4]hept-5-en-5-yl]benzenesulfonamide;
4-[6-(3-trifluoromethyl-4-methoxyphenyl)spiro[2.4]hept-5-en-5-yl]benzenesulfonamide;
4-[6-(3-methyl-4-methoxyphenyl)spiro[2.4]hept-5-en-5-yl]benzenesulfonamide;
4-[6-(3-chloro-4-methoxyphenyl)spiro[2.4]hept-5-en-5-yl]benzenesulfonamide;
4-[6-(4-methoxy-2,3,5,6-tetrafluorophenyl)spiro[2.4]hept-5-en-5-yl]benzenesulfonamide;
4-[6-(3,4-dimethoxyphenyl)spiro[2.4]hept-5-en-5-yl]benzenesulfonamide;
4-[6-(3-chloro-4-fluorophenyl)spiro[2.4]hept-5-en-5-yl]benzenesulfonamide;
4-[6-(4-chloro-3-fluorophenyl)spiro[2.4]hept-5-en-5-yl]benzenesulfonamide;
4-[6-(3,4-difluorophenyl)spiro[2.4]hept-5-en-5-yl]benzenesulfonamide; and
4-[6-(3,4-dichlorophenyl)spiro[2.4]hept-5-en-5-yl]benzenesulfonamide.

8. Compound of Claim 7 which is 5-(3,5-dichloro-4-methoxyphenyl)-6-[4-(methylsulfonyl)phenyl]spiro[2.4]hept-5-ene, or a pharmaceutically-acceptable salt thereof.

9. Compound of Claim 7 which is 4-[6-(3-chloro-4-methoxyphenyl)spiro[2.4]hept-5-en-5-yl]benzenesulfonamide, or a pharmaceutically-acceptable salt thereof.

10. Compound of Claim 4
wherein n is a number selected from 0, 1 and 2;
wherein each of R¹, R², R⁴, R⁵, and R¹⁰, is hydrido;
wherein R³ is selected from lower alkylsulfonyl and aminosulfonyl;
wherein R⁶ is selected from hydrido and halo;
wherein R⁷ is selected from hydrido and halo;
wherein R⁸ is selected from hydrido, halo, lower alkyl, lower alkoxy, lower haloalkyl, lower haloalkoxy, and hydroxyl; and
wherein R⁹ is selected from hydrido, halo, and lower alkyl; or wherein R⁸ and R⁹ together form methylenedioxy; or a pharmaceutically-acceptable salt thereof.

11. The compound of Claim 10 wherein R³ is methylsulfonyl or aminosulfonyl; wherein R⁶ is selected from hydrido, fluoro, chloro, bromo, and iodo; wherein R⁷ is selected from hydrido, fluoro, chloro, bromo, and iodo; wherein R⁸ is selected from hydrido, fluoro, chloro, bromo, iodo, methyl, ethyl, n-propyl, isopropyl, butyl, tert-butyl, isobutyl, methoxy, ethoxy, propoxy, butoxy, hydroxyl, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, and trifluoromethoxy; wherein R⁹ is selected from hydrido, fluoro, chloro, bromo, iodo, methyl, ethyl, n-propyl, isopropyl, butyl, tert-butyl, and isobutyl; or where R⁸ and R⁹ together form methylenedioxy; and wherein R¹¹ is methyl or amino; and or a pharmaceutically-acceptable salt thereof.

12. Compound of Claim 11 selected from compounds, and their pharmaceutically-acceptable salts, of the group consisting of
5-(3-chloro-4-methoxyphenyl)-6-[4-(methylsulfonyl)phenyl]spiro[2,4]hept-5-ene;
4-[6-(3-chloro-4-methoxyphenyl)spiro[2,4]hept-5-en-5-yl]benzenesulfonamide;
5-(3-fluoro-4-methoxyphenyl)-6-[4-(methylsulfonyl)phenyl]spiro[2,4]hept-5-ene;
4-[6-(3-fluoro-4-methoxyphenyl)spiro[2,4]hept-5-en-5-yl]benzenesulfonamide;
5-(3,4-difluorophenyl)-6-[4-(methylsulfonyl)phenyl]spiro[2,4]hept-5-ene;
5-[6-[4-(methylsulfonyl)phenyl]spiro[2,4]hept-5-en-5-yl]-1,3-benzodioxole;
4-[6-(3,4-difluorophenyl)spiro[2,4]hept-5-en-5-yl]benzenesulfonamide;
2,6-dichloro-4-[6-[4-(methylsulfonyl)phenyl]spiro[2,4]hept-5-en-5-yl]phenol
5-(4-trifluoromethoxyphenyl)-6-[4-(methylsulfonyl)phenyl]spiro[2,4]hept-5-ene;
5-(4-methoxyphenyl)-6-[4-(methylsulfonyl)phenyl]spiro[2,4]hept-5-ene;
5-(3-bromo-4-methoxyphenyl)-6-[4-(methylsulfonyl)phenyl]spiro[2,4]hept-5-ene;
4-[6-(4-methoxyphenyl)spiro[2,4]hept-5-en-5-yl]benzenesulfonamide;
4-[6-(3-bromo-4-methoxyphenyl)spiro[2,4]hept-5-en-5-yl]benzenesulfonamide;
5-(4-trifluoromethylphenyl)-6-[4-(methylsulfonyl)phenyl]spiro[2,4]hept-5-ene;
5-(3,5-dichloro-4-methoxy-phenyl)-6-[4-(methylsulfonyl)phenyl]spiro[2,4]hept-5-ene;
4-[6-(4-trifluoromethoxyphenyl)spiro[2,4]hept-5-en-5-yl]benzenesulfonamide;
5-(3-chloro-4-fluorophenyl)-6-[4-(methylsulfonyl)phenyl]spiro[2,4]hept-5-ene;
5-(2,4-difluorophenyl)-6-[4-(methylsulfonyl)phenyl]spiro[2,4]hept-5-ene;
5-(2,4-dichlorophenyl)-6-[4-(methylsulfonyl)phenyl]spiro[2,4]hept-5-ene;
4-[6-(4-trifluoromethylphenyl)spiro[2,4]hept-5-en-5-yl]benzenesulfonamide;
4-[6-(3-chloro-4-fluorophenyl)spiro[2,4]hept-5-en-5-yl]benzenesulfonamide;
5-(3,4-dichlorophenyl)-6-[4-(methylsulfonyl)phenyl]spiro[2,4]hept-5-ene;
5-(4-chlorophenyl)-6-[4-(methylsulfonyl)phenyl]spiro[2,4]hept-5-ene;
4-[6-(3,4-dichlorophenyl)spiro[2,4]hept-5-en-5-yl]benzenesulfonamide;
4-[6-(4-chlorophenyl)spiro[2,4]hept-5-en-5-yl]benzenesulfonamide;
5-(3-chloro-4-methylphenyl)-6-[4-(methylsulfonyl)phenyl]spiro[2,4]hept-5-ene;
5-(3,4-dimethylphenyl)-6-[4-(methylsulfonyl)phenyl]spiro[2,4]hept-5-ene;
5-(4-methylphenyl)-6-[4-(methylsulfonyl)phenyl]spiro[2,4]hept-5-ene;
5-(3-methyl-4-trifluoromethoxyphenyl)-6-[4-(methylsulfonyl)phenyl]spiro[2,4]hept-5-ene;
5-(3-chloro-4-trifluoromethoxyphenyl)-6-[4-(methylsulfonyl)phenyl]spiro[2,4]hept-5-ene;
4-[6-(3,5-dichloro-4-methoxyphenyl)spiro[2,4]hept-5-en-5-yl]benzenesulfonamide;
4-[6-(3-methyl-4-trifluoromethoxyphenyl)spiro[2,4]hept-5-en-5-yl]benzenesulfonamide;
4-[6-(3-chloro-4-trifluoromethoxyphenyl)spiro[2,4]hept-5-en-5-yl]benzenesulfonamide;
5-(4-fluorophenyl)-6-[4-(methylsulfonyl)phenyl] spiro[2.4]hept-5-ene;
4-[6-(4-fluorophenyl)spiro[2.4]hept-5-en-5-yl]benzenesulfonamide;
6-(4-fluorophenyl)-7-[4-(methylsulfonyl)phenyl] spiro[3.4]oct-5-ene;
4-[7-(4-fluorophenyl)spiro[3.4]oct-6-en-6-yl] benzenesulfonamide; and
2-(4-fluorophenyl)-3-[4-(methylsulfonyl)phenyl] spiro[4.4]non-5-ene.

13. Compound of Claim 1 wherein A is 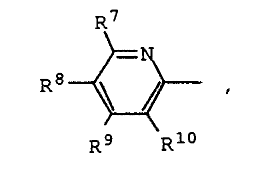
wherein n is a number selected from 0, 1, 2 and 3; and
wherein each of R¹ through R⁵ and R⁷ through R¹⁰ is independently selected from hydrido, halo, alkyl, alkoxy, alkylthio, alkylamino, cyano, haloalkyl, haloalkoxy, hydroxyalkyl, alkoxyalkyl, hydroxyl, mercapto, alkylsulfonyl, haloalkylsulfonyl and aminosulfonyl; or a pharmaceutically-acceptable salt thereof.

14. Compound of Claim 13 wherein n is a number selected from 0, 1 and 2; wherein each of R¹, R², R⁴, R⁵, R⁷, R⁹ and R¹⁰ is independently selected from hydrido, halo, lower alkyl, lower alkoxy, lower alkylthio, cyano, lower haloalkyl, lower haloalkoxy, lower hydroxyalkyl, lower alkoxyalkyl, hydroxyl and mercapto; and wherein R³ is selected from lower alkylsulfonyl and aminosulfonyl and R⁸ is selected from hydrido, halo, lower alkyl, lower alkoxy, lower alkylthio, lower alkylamino, lower haloalkoxy, lower hydroxyalkyl, mercapto, hydroxyl, lower alkoxyalkyl, cyano and lower haloalkyl; or wherein further R⁸ and R⁹ together form methylenedioxy; or wherein further, R³ is selected from hydrido, halo, lower alkyl, lower alkoxy, lower alkylthio, lower alkylamino, lower haloalkoxy, lower hydroxyalkyl, hydroxyl, mercapto, lower alkoxyalkyl, cyano and lower haloalkyl, and R⁸ is selected from lower alkylsulfonyl and aminosulfonyl; or wherein further R³ and R⁴ together form methylenedioxy; or a pharmaceutically-acceptable salt thereof.

15. Compound of Claim 14 wherein each of R¹, R², R⁴, R⁵, R⁷, R⁹ and R¹⁰ is hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, methylamino, N,N-dimethylamino, trifluoromethoxy, hydroxymethyl, methoxymethyl and ethoxymethyl; and wherein R³ is methylsulfonyl or aminosulfonyl, and R⁸ is selected from hydrido, fluoro, chloro, bromo, iodo, methyl, ethyl, n-propyl, isopropyl, butyl, *tert*-butyl, isobutyl, methoxy, ethoxy, propoxy, butoxy, trifluoromethoxy, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl; or wherein further R⁸ and R⁹ together form methylenedioxy; or wherein further, R³ is selected from hydrido, fluoro, chloro, bromo, iodo, methyl, ethyl, n-propyl, isopropyl, butyl, tert-butyl, isobutyl, methoxy, ethoxy, propoxy, butoxy, trifluoromethoxy, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl, and R⁸ is methylsulfonyl or aminosulfonyl; or wherein further R³ and R⁴ together form methylenedioxy; or a pharmaceutically-acceptable salt thereof.

16. Compound of Claim 15 selected from compounds, and their pharmaceutically-acceptable salts, of the group consisting of
2-[6-[4-(methylsulfonyl)phenyl]spiro[2.4]hept-5-en-5-yl]pyridine;
5-fluoro-2-[6-[4-(methylsulfonyl)phenyl] spiro[2.4]hept-5-en-5-yl]pyridine;
5-chloro-2-[6-[4-(methylsulfonyl)phenyl]spiro [2.4]hept-5-en-5-yl]pyridine;
5-methyl-2-[6-[4-(methylsulfonyl)phenyl]spiro [2.4]hept-5-en-5-yl]pyridine;
4-[6-(pyridin-2-yl)spiro[2.4]hept-5-en-5-yl] benzenesulfonamide;
4-[6-(5-fluoropyridin-2-yl)spiro[2.4]hept-5-en-5-yl] benzenesulfonamide;
4-[6-(5-chloropyridin-2-yl)spiro[2.4]hept-5-en-5-yl] benzenesulfonamide;
4-[6-(5-methylpyridin-2-yl)spiro[2.4]hept-5-en-5-yl] benzenesulfonamide;
2-[7-[4-(methylsulfonyl)phenyl]spiro[3.4]oct-6-en-6-yl]pyridine;
5-fluoro-2-[7-[4-(methylsulfonyl)phenyl]spiro[3.4]oct-6-en-6-yl]pyridine;
5-chloro-2-[7-[4-(methylsulfonyl)phenyl]spiro[3.4]oct-6-en-6-yl]pyridine;
5-methyl-2-[7-[4-(methylsulfonyl)phenyl]spiro[3.4]oct-6-en-6-yl]pyridine;
4-[7-(pyridin-2-yl)spiro[3.4]oct-6-en-6-yl] benzenesulfonamide;
4-[7-(5-fluoropyridin-2-yl)spiro[3.4]oct-6-en-6-yl] benzenesulfonamide;
4-[7-(5-chloropyridin-2-yl)spiro[3.4]oct-6-en-6-yl] benzenesulfonamide;
4-[7-(5-methylpyridin-2-yl)spiro[3.4]oct-6-en-6-yl] benzenesulfonamide;
2-[3-[4-(methylsulfonyl)phenyl]spiro[4.4]non-2-en-2-yl]pyridine;
5-fluoro-2-[3-[4-(methylsulfonyl)phenyl]spiro[4.4]non-2-en-2-yl]pyridine;
5-chloro-2-[3-[4-(methylsulfonyl)phenyl]spiro[4.4]non-2-en-2-yl]pyridine;
5-methyl-2-[3-[4-(methylsulfonyl)phenyl]spiro[4.4]non-2-en-2-yl]pyridine;
4-[3-(pyridin-2-yl)spiro[4.4]non-2-en-2-yl] benzenesulfonamide;
4-[3-(5-fluoropyridin-2-yl)spiro[4.4]non-2-en-2-yl] benzenesulfonamide;
4-[3-(5-chloropyridin-2-yl)spiro[4.4]non-2-en-2-yl] benzenesulfonamide;
4-[3-(5-methylpyridin-2-yl)spiro[4.4]non-2-en-2-yl] benzenesulfonamide;
2-(6-phenylspiro[2.4]hept-5-en-yl)-5-(methylsulfonyl) pyridine;
2-[6-(4-fluorophenyl)spiro[2.4]hept-5-en-yl]-5-(methylsulfonyl)pyridine;
2-[6-(4-chlorophenyl)spiro[2.4]hept-5-en-yl]-5-(methylsulfonyl)pyridine;
2-[6-(4-methylphenyl)spiro[2.4]hept-5-en-yl]-5-(methylsulfonyl)pyridine;
2-(6-phenylspiro[2.4]hept-5-en-5-yl)-5-pyridinesulfonamide;
2-[6-(4-fluorophenyl)spiro[2.4]hept-5-en-5-yl)-5-pyridinesulfonamide;
2-[6-(4-chlorophenyl)spiro[2.4]hept-5-en-5-yl)-5-pyridinesulfonamide;
2-[6-(4-methylphenyl)spiro[2.4]hept-5-en-5-yl)-5-pyridinesulfonamide;
2-(7-phenylspiro[3.4]oct-6-en-6-yl]-5-(methylsulfonyl) pyridine;
2-[7-(4-fluorophenyl)spiro[3.4]oct-6-en-6-yl]-5-(methylsulfonyl)pyridine;
2-[7-(4-chlorophenyl)spiro[3.4]oct-6-en-6-yl]-5-(methylsulfonyl)pyridine;
2-[7-(4-methylphenyl)spiro[3.4]oct-6-en-6-yl]-5-(methylsulfonyl)pyridine;
2-(7-phenylspiro[3.4]oct-6-en-6-yl)-5-pyridinesulfonamide;
2-[7-(4-fluorophenyl)spiro[3.4]oct-6-en-6-yl]-5-pyridinesulfonamide;
2-[7-(4-chlorophenyl)spiro[3.4]oct-6-en-6-yl]-5-pyridinesulfonamide;
2-[7-(4-methylphenyl)spiro[3.4]oct-6-en-6-yl]-5-pyridinesulfonamide;
2-(3-phenylspiro[4.4]non-2-en-2-yl)-5-(methylsulfonyl) pyridine;
2-[3-(4-fluorophenyl)spiro[4.4]non-2-en-2-yl]-5-(methylsulfonyl)pyridine;
2-[3-(4-chlorophenyl)spiro[4.4]non-2-en-2-yl]-5-(methylsulfonyl)pyridine;
2-[3-(4-methylphenyl)spiro[4.4]non-2-en-2-yl]-5-(methylsulfonyl)pyridine;
2-(3-phenylspiro[4.4]non-2-en-2-yl)-5-pyridinesulfonamide;
2-[3-(4-fluorophenyl)spiro[4.4]non-2-en-2-yl]-5-pyridinesulfonamide;
2-[3-(4-chlorophenyl)spiro[4.4]non-2-en-2-yl]-5-pyridinesulfonamide; and
2-[3-(4-methylphenyl)spiro[4.4]non-2-en-2-yl]-5-pyridinesulfonamide.

17. Compound of Claim 1 wherein A is 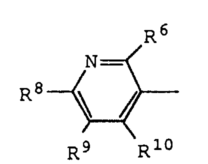
wherein n is a number selected from 0, 1, 2 and 3; and
wherein each of R¹ through R⁶ and R⁸ through R¹⁰ is independently selected from hydrido, halo, alkyl, alkoxy, alkylthio, alkylamino, cyano, haloalkyl, haloalkoxy, hydroxyalkyl, alkoxyalkyl, hydroxyl, mercapto, alkylsulfonyl and aminosulfonyl; or a pharmaceutically-acceptable salt thereof.

18. Compound of Claim 17 wherein n is a number selected from 0, 1 and 2; wherein each of R¹, R², R⁴, R⁵, R⁶, R⁹ and R¹⁰ is independently selected from hydrido, halo, lower alkyl, lower alkoxy, lower alkylthio, cyano, lower haloalkyl, lower haloalkoxy, lower hydroxyalkyl, lower alkoxyalkyl, hydroxyl and mercapto; and wherein R³ is selected from lower alkylsulfonyl and aminosulfonyl and R⁸ is selected from hydrido, halo, lower alkyl, lower alkoxy, lower alkylthio, lower alkylamino, lower haloalkoxy, lower hydroxyalkyl, mercapto, hydroxyl, lower alkoxyalkyl, cyano and lower haloalkyl; or wherein further R⁸ and R⁹ together form methylenedioxy; or wherein further, R³ is selected from hydrido, halo, lower alkyl, lower alkoxy, lower alkylthio, lower alkylamino, lower haloalkoxy, lower hydroxyalkyl, hydroxyl, lower alkoxyalkyl, cyano and lower haloalkyl, and R⁸ is selected from lower alkylsulfonyl and aminosulfonyl; or wherein further R³ and R⁴ together form methylenedioxy; or a pharmaceutically-acceptable salt thereof.

19. Compound of Claim 18 wherein each of R¹, R², R⁴, R⁵, R⁶, R⁹ and R¹⁰ is hydrido, fluoro, chloro, bromo, iodo, methyl, ethyl, n-propyl, isopropyl, butyl, tert-butyl, isobutyl, methoxy, ethoxy, propoxy, butoxy, hydroxyl, mercapto, methylthio, ethylthio, cyano, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, trifluoromethoxy, hydroxymethyl, methoxymethyl and ethoxymethyl; and wherein R³ is methylsulfonyl or aminosulfonyl, and R⁸ is selected from hydrido, fluoro, chloro, bromo, iodo, methyl, ethyl, n-propyl, isopropyl, butyl, tert-butyl, isobutyl, methoxy, ethoxy, propoxy, butoxy, trifluoromethoxy, hydroxyl, methylthio, ethylthio, methylamino, N,N-dimethylamino, cyano, mercapto, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl; or wherein further R⁸ and R⁹ together form methylenedioxy; or wherein further, R³ is selected from hydrido, fluoro, chloro, bromo, iodo, methyl, ethyl, n-propyl, isopropyl, butyl, *tert*-butyl, isobutyl, methoxy, ethoxy, propoxy, butoxy, trifluoromethoxy, hydroxyl, methylthio, ethylthio, cyano, mercapto, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl, and R⁸ is methylsulfonyl or aminosulfonyl; or wherein further R³ and R⁴ together form methylenedioxy; or a pharmaceutically-acceptable salt thereof.

20. Compound of Claim 19 selected from compounds, and their pharmaceutically-acceptable salts, of the group consisting of
5-[6-[4-(methylsulfonyl)phenyl]spiro[2.4]hept-5-en-5-yl]pyridine;
2-fluoro-5-[6-[4-(methylsulfonyl)phenyl]spiro [2.4]hept-5-en-5-yl]pyridine;
2-chloro-5-[6-[4-(methylsulfonyl)phenyl]spiro [2.4]hept-5-en-5-yl]pyridine;
2-methyl-5-[6-[4-(methylsulfonyl)phenyl] spiro[2.4]hept-5-en-5-yl]pyridine;
4-[6-(pyridin-5-yl)spiro[2.4]hept-5-en-5-yl] benzenesulfonamide;
4-[6-(2-fluoropyridin-5-yl)spiro[2.4]hept-5-en-5-yl] benzenesulfonamide;
4-[6-(2-chloropyridin-5-yl)spiro[2.4]hept-5-en-5-yl] benzenesulfonamide;
4-[6-(2-methylpyridin-5-yl)spiro[2.4]hept-5-en-5-yl] benzenesulfonamide;
5-[7-[4-(methylsulfonyl)phenyl]spiro[3.4]oct-6-en-6-yl]pyridine;
2-fluoro-5-[7-[4-(methylsulfonyl)phenyl]spiro[3.4]oct-6-en-6-yl]pyridine;
2-chloro-5-[7-[4-(methylsulfonyl)phenyl]spiro[3.4]oct-6-en-6-yl]pyridine;
2-methyl-5-[7-[4-(methylsulfonyl)phenyl]spiro[3.4]oct-6-en-6-yl]pyridine;
4-[7-(pyridin-5-yl)spiro[3.4]oct-6-en-6-yl] benzenesulfonamide;
4-[7-(2-fluoropyridin-5-yl)spiro[3.4]oct-6-en-6-yl] benzenesulfonamide;
4-[7-(2-chloropyridin-5-yl)spiro[3.4]oct-6-en-6-yl] benzenesulfonamide;
4-[7-(2-methylpyridin-5-yl)spiro[3.4]oct-6-en-6-yl] benzenesulfonamide;
5-[3-[4-(methylsulfonyl)phenyl]spiro[4.4]non-2-en-2-yl]pyridine;
2-fluoro-5-[3-[4-(methylsulfonyl)phenyl]spiro[4.4]non-2-en-2-yl]pyridine;
2-chloro-5-[3-[4-(methylsulfonyl)phenyl]spiro[4.4]non-2-en-2-yl]pyridine;
2-methyl-5-[3-[4-(methylsulfonyl)phenyl]spiro[4.4]non-2-en-2-yl]pyridine;
4-[3-(pyridin-5-yl)spiro[4.4]non-2-en-2-yl] benzenesulfonamide;
4-[3-(2-fluoropyridin-5-yl)spiro[4.4]non-2-en-2-yl] benzenesulfonamide;
4-[3-(2-chloropyridin-5-yl)spiro[4.4]non-2-en-2-yl] benzenesulfonamide;
4-[3-(2-methylpyridin-5-yl)spiro[4.4]non-2-en-2-yl] benzenesulfonamide;
5-(6-phenylspiro[2.4]hept-5-en-5-yl)-2-(methylsulfonyl) pyridine;
5-[6-(4-fluorophenyl)spiro[2.4]hept-5-en-5-yl]-2-(methylsulfonyl)pyridine;
5-[6-(4-chlorophenyl)spiro[2.4]hept-5-en-5-yl]-2-(methylsulfonyl)pyridine;
5-[6-(4-methylphenyl)spiro[2.4]hept-5-en-5-yl]-2-(methylsulfonyl)pyridine;
5-(6-phenylspiro[2.4]hept-5-en-5-yl)-2-pyridinesulfonamide;
5-[6-(4-fluorophenyl)spiro[2.4]hept-5-en-5-yl]-2-pyridinesulfonamide;
5-[6-(4-chlorophenyl)spiro[2.4]hept-5-en-5-yl]-2-pyridinesulfonamide;
5-[6-(4-methylphenyl)spiro[2.4]hept-5-en-5-yl]-2-pyridinesulfonamide;
5-(7-phenylspiro[3.4]oct-6-en-6-yl)-2-(methylsulfonyl) pyridine;
5-[7-(4-fluorophenyl)spiro[3.4]oct-6-en-6-yl]-2-(methylsulfonyl)pyridine;
5-[7-(4-chlorophenyl)spiro[3.4]oct-6-en-6-yl]-2-(methylsulfonyl)pyridine;
5-[7-(4-methylphenyl)spiro[3.4]oct-6-en-6-yl]-2-(methylsulfonyl)pyridine;
5-(7-phenylspiro[3.4]oct-6-en-6-yl)-2-pyridinesulfonamide;
5-[7-(4-fluorophenyl)spiro[3.4]oct-6-en-6-yl]-2-pyridinesulfonamide;
5-[7-(4-chlorophenyl)spiro[3.4]oct-6-en-6-yl]-2-pyridinesulfonamide;
5-[7-(4-methylphenyl)spiro[3.4]oct-6-en-6-yl]-2-pyridinesulfonamide;
5-(3-phenylspiro[4.4]non-2-en-2-yl)-2-(methylsulfonyl) pyridine;
5-[3-(4-fluorophenyl)spiro[4.4]non-2-en-2-yl]-2-(methylsulfonyl)pyridine;
5-[3-(4-chlorophenyl)spiro[4.4]non-2-en-2-yl]-2-(methylsulfonyl)pyridine;
5-[3-(4-methylphenyl)spiro[4.4]non-2-en-2-yl]-2-(methylsulfonyl)pyridine;
5-(3-phenylspiro[4.4]non-2-en-2-yl)-2-pyridinesulfonamide;
5-[3-(4-fluorophenyl)spiro[4.4]non-2-en-2-yl]-2-pyridinesulfonamide;
5-[3-(4-chlorophenyl)spiro[4.4]non-2-en-2-yl]-2-pyridinesulfonamide; and
5-[3-(4-methylphenyl)spiro[4.4]non-2-en-2-yl]-2-pyridinesulfonamide.

21. Compound of Claim 1 wherein A is 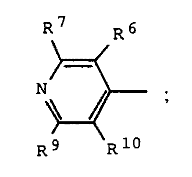
wherein n is a number selected from 0, 1, 2 and 3; and
wherein each of R¹ through R⁷, R⁹ and R¹⁰ is independently selected from hydrido, halo, alkyl, alkoxy, alkylthio, cyano, haloalkyl, haloalkoxy, hydroxyalkyl, alkoxyalkyl, hydroxyl, mercapto, alkylsulfonyl and aminosulfonyl; or a pharmaceutically-acceptable salt thereof.

22. Compound of Claim 21 wherein n is a number selected from 0, 1 and 2; wherein each of R¹, R², R⁴ through R⁷, R⁹ and R¹⁰ is independently selected from hydrido, halo, lower alkyl, lower alkoxy, lower alkylthio, lower haloalkoxy, lower hydroxyalkyl, hydroxyl, lower alkoxyalkyl, mercapto, cyano and lower haloalkyl; and wherein R³ is selected from lower alkylsulfonyl and aminosulfonyl; or a pharmaceutically-acceptable salt thereof.

23. Compound of Claim 22 wherein each of R¹, R², R⁴ through R⁷, R⁹ and R¹⁰ is independently selected from hydrido, fluoro, chloro, bromo, iodo, methyl, ethyl, n-propyl, isopropyl, butyl, *tert*-butyl, isobutyl, methoxy, ethoxy, propoxy, butoxy, trifluoromethoxy, methylthio, ethylthio, cyano, hydroxyl, mercapto, fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, dichloropropyl, hydroxymethyl, methoxymethyl and ethoxymethyl; and wherein R³ is methylsulfonyl or aminosulfonyl; or a pharmaceutically-acceptable salt thereof.

24. Compound of Claim 23 selected from compounds, and their pharmaceutically-acceptable salts, of the group consisting of
4-[6-[4-(methylsulfonyl)phenyl]spiro[2.4]hept-5-en-5-yl]pyridine;
4-[6-(4-pyridinyl)spiro[2.4]hept-5-en-5-yl] benzenesulfonamide;
4-[7-[4-(methylsulfonyl)phenyl]spiro[3.4]oct-6-en-6-yl]pyridine;
4-[7-(4-pyridinyl)spiro[3.4]oct-6-en-6-yl] benzenesulfonamide;
4-[3-[4-(methylsulfonyl)phenyl]spiro[4.4]non-2-en-2-yl] pyridine; and
4-[3-(4-pyridinyl)spiro[4.4]non-2-en-2-yl] benzenesulfonamide.

25. A pharmaceutical composition comprising a therapeutically-effective amount of a compound, said compound selected from a family of compounds of one of claims 1 or 4 to 10, 13, 17 or 21 or a pharmaceutically-acceptable salt thereof.

26. Use of a compound according to any of claims 1, 4 to 10, 13, 17 or 21 for preparing a medicament for treating inflammation or an inflammation-associated disorder in a subject.

27. Use of Claim 26 for use in treatment of inflammation.

28. Use of Claim 26 for use in treatment of an inflammation-associated disorder.

29. Use of Claim 28 wherein the inflammation-associated disorder is arthritis.

30. Use of Claim 28 wherein the inflammation-associated disorder is pain.

31. Use of Claim 28 wherein the inflammation-associated disorder is fever.

## Patentansprüche

1. Verbindung der Formel I 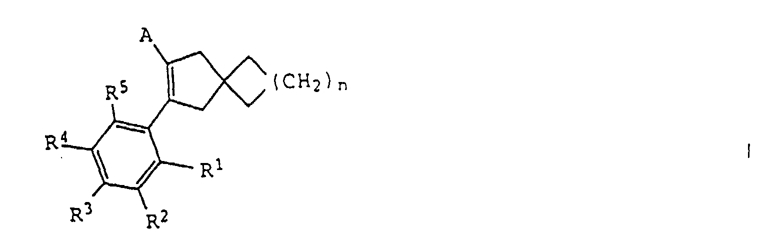
wobei A ausgewählt ist aus 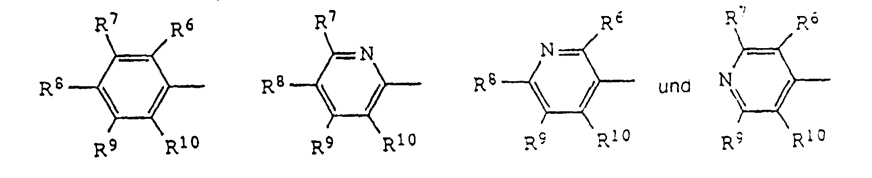
wobei R¹ bis R¹⁰ - wenn vorhanden - je unabhängig ausgewählt sind aus Hydrido, Halo, Alkyl. Alkoxy, Alkylthio, Alkylamino, Cyan, Haloalkyl, Haloalkoxy, Hydroxyalkyl, Alkoxyalkyl, Hydroxyl, Mercapto, Alkylsulfonyl, Haloalkylsulfonyl und Aminosulfonyl; und wobei n eine Zahl ist, ausgewählt aus 0, 1, 2, und 3; oder ein pharmazeutisch akzeptables Salz davon.

2. Verbindung gemäß Anspruch 1, wobei- wenn vorhanden - R¹, R², R⁴ bis R⁷, R⁹ und R¹⁰ jeweils unabhängig voneinander ausgewählt sind aus Hydrido, Halo, Niedrigalkyl, Niedrigalkoxy, Niedrigalkylthio, Cyan, Niedrighaloalkyl, Niedrighaloalkoxy, Niedrighydroxyalkyl, NiedrigalkoxyilKyl, Hydroxyl und Mercapto; und wobei R³ ausgewählt ist aus Niedrigalkylsulfonyl, Niedrighaloalkylsulfonyl und Aminosulfonyl, und R⁸, wenn vorhanden, ausgewählt ist aus Hydrido, Halo, Niedrigalkyl, Niedrigalkoxy, Niedrigalkylthio, Niedrigalkylamino, Cyan, Niedrighaloalkyl, Niedrighaloalkoxy, Niedrighydroxyalkyl, Niedrigalkoxyalkyl, Hydroxyl und Mercapto; oder wobei des weiteren R⁸ und R⁹-wenn vorhanden - gemeinsam Methylendioxy bilden; oder wobei des weiteren R³ ausgewählt ist aus Hydrido, Halo, Niedrigalkyl, Niedrigalkoxy, Niedrigalkylthio, Niedrigalkylamino, Cyan, Niedrighaloalkyl, Niedrighaloalkoxy, Niedrighydroxyalkyl, Niedrigalkoxyalkyl, Hydroxyl und Mercapto, und R⁸ ausgewählt ist aus Niedrigalkylsulfonyl, Niedrighaloalkylsulfonyl und Aminosulfonyl; oder wobei des weiteren R³ und R⁴ - wenn vorhanden - gemeinsam Methylendioxy bilden; oder ein pharmazeutisch akzeptables Salz davon.

3. Verbindung gemäß Anspruch 2, wobei - wenn vorhanden - R¹, R², R⁴ bis R⁷, R⁹ und R¹⁰ jeweils unabhängig voneinander ausgewählt sind aus Hydrido, Fluoro, Chloro, Bromo, Iodo, Methyl, Ethyl, n-Propyl, Isopropyl, Butyl, tert-Butyl, Isobutyl, Methoxy, Ethoxy, Propoxy, Butoxy, Hydroxyl, Mercapto, Methylthio, Ethylthio, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Trifluoromethoxy, Hydroxymethyl, Methoxymethyl und Ethoxymethyl; und wobei R³ ausgewählt ist aus Methylsulfonyl, Fluoromethylsulfonyl, Difluoromethylsulfonyl, Trifluoromethylsulfonyl und Aminosulfonyl, und R⁸ - wenn vorhanden - ausgewählt ist aus Hydrido, Fluoro, Chloro, Bromo, Iodo, Methyl, Ethyl, n-Propyl, Isopropyl, Butyl, tert-Butyl, Isobutyl, Methoxy, Ethoxy, Propoxy, Butoxy, Hydroxyl, Mercapto, Methylthio, Ethylthio, Methylamino, N,N-Dimethylamino, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Trifluoromethoxy, Hydroxymethyl, Methoxymethyl und Ethoxymethyl; oder wobei des weiteren R⁸ und R⁹ - wenn vorhanden - gemeinsam Methylendioxy bilden; oder wobei des weiteren R³ ausgewählt ist aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Hydroxyl, Mercapto, Methylthio, Ethylthio, Methylamino, N,N-Dimethylamino, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Trifluoromethoxy, Hydroxymethyl, Methoxymethyl und Ethoxymethyl; und R⁸ ist ausgewählt aus Methylsulfonyl, Fluoromethylsulfonyl, Difluoromethylsulfonyl, Trifluoromethylsulfonyl und Aminosulfonyl; oder wobei des weiteren R³ und R⁴ - wenn vorhanden - gemeinsam Methylendioxy bilden; oder ein pharmazeutisch akzeptables Salz davon.

4. Verbindung gemäß Anspruch 1, wobei A ist 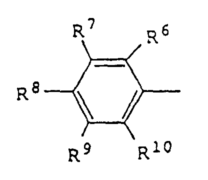 wobei R¹ bis R¹⁰ unabhängig voneinander ausgewählt sind aus Hydrido, Halo, Alkyl, Alkoxy, Alkylthio, Alkylamino, Cyan, Haloalkyl, Haloalkoxy, Hydroxyalkyl, Alkoxyalkyl, Hydroxyl, Mercapto, Alkylsulfonyl, Haloalkylsulfonyl und Aminosulfonyl; und wobei n eine Zahl ist, ausgewählt aus 0, 1, 2, und 3; oder ein pharmazeutisch akzeptables Salz davon.

5. Verbindung gemäß Anspruch 4, wobei n eine Zahl ist, ausgewählt aus 0, 1 und 2; wobei R¹, R² und R⁴ bis R¹⁰ jeweils unabhängig voneinander ausgewählt sind aus Hydrido, Halo, Niedrigalkyl, Niedrigalkylthio, Niedrigalkylamino, Cyan, Niedrighaloalkyl, Niedrighaloalkoxy, Niedrigalkoxy, Hydroxyl, Mercapto, Niedrighydroxyalkyl und Niedrigalkoxyalkyl; und wobei R³ ausgewählt ist aus Niedrigalkylsulfonyl, Niedrighaloalkylsulfonyl und Aminosulfonyl, und wobei R⁸ und R⁹ gemeinsam Methylendioxy bilden; oder ein pharmazeutisch akzeptables Salz davon.

6. Verbindung gemäß Anspruch 5, wobei R¹, R² und R⁴ bis R¹⁰ unabhängig voneinander ausgewählt sind aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Hydroxyl, Mercapto, Methylthio, Ethylthio, Methylamino, N,N-Dimethylamino, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Trifluoromethoxy, Hydroxymethyl, Methoxymethyl und Ethoxymethyl; und wobei R³ ausgewählt ist aus Methylsulfonyl, Fluoromethylsulfonyl, Difluoromethylsulfonyl, Trifluoromethylsulfonyl und Aminosulfonyl, oder wobei R⁸ und R⁹ gemeinsam Methylendioxy bilden; oder ein pharmazeutisch akzeptables Salz davon.

7. Verbindung von Anspruch 6, ausgewählt aus Verbindungen und deren pharmazeutisch akzeptablen Salzen der Gruppe bestehend aus
5-[6-[4-(Methylsulfonyl)phenyl]spiro [2.4] hept-5-en-5-yl]-1,3-Benzodioxol;
2,6-Dichlor-4-[6-[4-(Methylsulfonyl)phenyl]spiro[2.4] hept-5-en-5-yl]phenol;
5-(4-Trifluoromethoxyphenyl)-6-[4-(Methylsulfonyl)phenyl]spiro[2.4]hept-5-en;
5-(3-Brom-4-Methoxyphenyl)-6-[4-(Methylsulfonyl)phenyl]spiro[2.4]hept-5-en;
4-(6-(3-Brom4-Methoxyphenyl) spiro[2.4]hept-5-en-5-yl]benzensulfonamid;
5-(3,5-Dichlor4-Methoxy-phenyl)-6-[4-(Methylsulfonyl)phenyl] spiro[2.4]hept-5-en;
4-[6-(4-Trifluoromethoxyphenyl) spiro[2.4]hept-5-en-5-yl]benzensulfonamid;
5-(2,4-Difluorophenyl)-6-[4-(Methylsulfonyl)phenyl] spiro[2.4]hept-5-en;
5-(2,4-Dichlorphenyl)-6-[4-(Methylsulfonyl)phenyl]spiro[2.4]hept-5-en;
5-(3-Chlor-4-Methylphenyl)-6-[4-(Methylsulfonyl)phenyl] spiro[2.4]hept-5-en;
5-(3,4-Dimethylphenyl)-6-[4-(Methylsulfonyl)phenyl]spiro[2.4]hept-5-en;
5-(3-Methyl-4-Trifluoromethoxyphenyl)-6-[4-(Methylsulfonyl)phenyl]spiro[2.4]hept-5-en;
5-(3-Chlor-4-Trifluoromethoxyphenyl)-6-[4-(Methylsulfonyl)phenyl] spiro[2.4]hept-5-en;
4-[6-(3,5-Dichlor-4-Methoxyphenyl) spiro[2.4]hept-5-en-5-yl]benzensulfonamid;
4-[6-(3-Methyl4-Trifluoromethoxyphenyl) spiro[2.4]hept-5-en-5-yl]benzensulfonamid;
4-[6-(3-Chlor4-Trifluoromethoxyphenyl) spiro[2.4]hept-5-en-5-yl]benzensulfonamid;
5-Phenyl-6-[4-(Methylsulfonyl)phenyl] spiro[2.4]hept-5-en;
5-(4-Fluorophenyl)-6-[4-(Methylsulfonyl)phenyl]spiro[2.4]hept-5-en;
5-(4-Chlorphenyl)-6-[4-(Methylsulfonyl)phenyl]spiro[2.4]hept-5-en;
5-(4-Methylphenyl)-6-[4-(Methylsulfonyl)phenyl]spiro[2.4]hept-5-en;
5-(4-Methoxyphenyl)-6-[4-(Methylsulfonyl)phenyl]spiro[2.4]hept-5-en;
5-(4-Methylthiophenyl)-6-[4-(Methylsulfonyl)phenyl]spiro[2.4]hept-5-en;
5-(4-Cyanophenyl)4-[4-(Methylsulfonyl)phenyl]spiro[2.4]hept-5-en;
5-(4-Trifluoromethylphenyl)-6-[4-(Methylsulfonyl)phenyl]spiro[2.4]hept-5-en;
4-(6-Phenylspiro[2.4]hept-5-en-5-yl)benzensulfonamid;
4-[6-(4-Fluorophenyl)spiro[2.4]hept-5-en-5-yl]benzensulfonamid;
4-[6-(4-Chlorphenyl)spiro[2.4]hept-5-en-5-yl]benzensulfonamid;
4-[6-(4-Methylphenyl)spiro[2.4]hept-5-en-5-yl]benzensulfonamid;
4-[6-(4-Methoxyphenyl)spiro[2.4]hept-5-en-5-yl]benzensulfonamid;
4-[6-(4-Methylthiophenyl)spiro[2.4]hept-5-en-5-yl]benzensulfonamid;
4-[6-(4-Cyanophenyl)spiro[2.4]hept-5-en-5-yl]benzensulfonamid;
4-[6-(4-Trifluoromethylphenyl)spiro[2.4]hept-5-en-5-yl]benzensulfonamid;
6-Phenyl-7-[4-(Methylsulfonyl)phenyl)spiro[3.4]oct-6-en;
6-(4-Fluorophenyl)-7-[4-(Methylsulfonyl)phenyl]spiro[3.4]oct-6-en;
6-(4-Chlorphenyl)-7-[4-(Methylsulfonyl)phenyl]spiro[3.4]oct-6-en;
6-(4-Methylphenyl)-7-[4-(Methylsulfonyl)phenyl]spiro[3.4]oct-6-en;
6-(4-Methoxyphenyl)-7-[4-(Methylsulfonyl)phenyl]spiro[3.4]oct-6-en;
6-(4-Methylthiophenyl)-7-[4-(Methylsulfonyl)phenyl]spiro[3.4]oct-6-en;
6-(4-Cyanophenyl)-7-[4-(Methylsulfonyl)phenyl]spiro[3.4]oct-6-en;
6-(4-Trifluoromethylphenyl)-7-[4-(Methylsulfonyl)phenyl]spiro[3.4]oct-6-en;
4-(7-Phenylspiro[3.4]oct-6-en-6-yl)benzensulfonamid;
4-[7-(4-Fluorophenyl)spiro[3.4]oct-6-en-6-yl]benzensulfonamid;
4-[7-(4-Chlorphenyl)spiro[3.4]oct-6-en-6-yl]benzensulfonamid;
4-[7-(4-Methylphenyl)spiro[3.4]oct-6-en-6-yl]benzensulfonamid;
4-[7-(4-Methoxyphenyl)spiro[3.4]oct-6-en-6-yl]benzensulfonamid;
4-[7-(4-Methylthiophenyl)spiro[3.4]oct-6-en-6-yl]benzensulfonamid;
4-[7-(4-Cyanophenyl)spiro[3.4]oct-6-en-6-yl]benzensulfonamid;
4-[7-(4-Trifluoromethylphenyl)spiro[3.4]oct-6-en-6-yl]benzensulfonamid;
2-Phenyl-3-[4-(Methylsulfonyl)phenyl]spiro[4.4]non-2-en;
2-(4-Fluorphenyl)-3-[4-(Methylsulfonyl)phenyl]spiro[4.4]non-2-en;
2-(4-Chlorphenyl)-3-[4-(Methylsulfonyl)phenyl]spiro[4.4]non-2-en;
2-(4-Methylphenyl)-3-[4-(Methylsulfonyl)phenyl]spiro[4.4]non-2-en;
2-(4-Methoxyphenyl)-3-[4-(Methylsulfonyl)phenyl]spiro[4.4]non-2-en;
2-(4-Methylthiophenyl)-3-[4-(Methylsulfonyl)phenyl]spiro[4.4]non-2-en;
2-(4-Cyanophenyl)-3-[4-(Methylsulfonyl)phenyl]spiro[4.4]non-2-en;
2-(4-Trfluoromethylphenyl)-3-[4-(Methylsulfonyl)phenyl]spiro[4.4]non-2-en;
4-(3-Phenylspiro[4.4]non-2-en-2-yl)benzensulfonamid;
4-[3-(4-Fluorophenyl)spiro[4.4]non-2-en-2-yl]benzensulfonamid;
4-[3-(4-Chlorphenyl)spiro[4.4]non-2-en-2-yl]benzensulfonamid;
4-[3-(4-Methylphenyl)spiro[4.4]non-2-en-2-yl]benzensulfonamid;
4-[3-(4-Methoxyphenyl)spiro[4.4]non-2-en-2-yl]benzensulfonamid;
4-[3-(4-Methylthiophenyl)spiro[4.4]non-2-en-2-yl]benzensulfonamid;
4-[3-(4-Cyanophenyl)spiro[4.4]non-2-en-2-yl]benzensulfonamid;
4-[3-(4-Trifluoromethylphenyl)spiro[4.4]non-2-en-2-yl]benzensulfonamid;
5-(3-Methyl-4-Fluorophenyl)-6-[4-(Methylsulfonyl)phenyl]spiro[2.4]hept-5-en;
5-(3-Trifluoromethyl-4-Fluorophenyl)-6-[4-(Methylsulfonyl)phenyl]spiro[2.4]hept-5-en;
5-(3-Methyl-4-Chlorphenyl)-6-[4-(Methylsulfonyl)phenyl]spiro[2.4]hept-5-en;
5-(3-Trifluoromethyl-4-Chlorphenyl)-6-[4-(Methylsulfonyl)phenyl]spiro[2.4]hept-5-en;
5-(3-Methyl-4-Methoxyphenyl)-6-[4-(Methylsulfonyl)phenyl]spiro[2.4]hept-5-en;
5-(3-Trifluoromethyl-4-Methoxyphenyl)-6-[4-(Methylsulfonyl)phenyl]spiro[2.4]hept-5-en;
5-(3-Fluoro-4-Methoxyphenyl)-6-[4-(Methylsulfonyl)phenyl]spiro[2.4]hept-5-en;
5-(3-Chlor-4-Methoxyphenyl)-6-[4-(Methylsulfonyl)phenyl]spiro[2.4]hept-5-en;
5-(4-Methoxy-2,3,5,6-Tetrafluorophenyl)-6-[4-(Methylsulfonyl)phenyl]spiro[2.4]hept-5-en;
5-(3,4-Dimethoxyphenyl)-6-[4-(Methylsulfonyl)phenyl]spiro[2.4]hept-5-en;
5-(3-Chlor-4-Fluorophenyl)-6-[4-(Methylsulfonyl)phenyl]spiro[2.4]hept-5-en;
5-(4-Chlor-3-Fluorophenyl)-6-[4-(Methylsulfonyl)phenyl]spiro[2.4]hept-5-en;
5-(3,4-Difluorophenyl)-6-[4-(Methylsulfonyl)phenyl]spiro[2.4]hept-5-en;
5-(3,4-Dichlorphenyl)-6-[4-(Methylsulfonyl)phenyl]spiro[2.4]hept-5-en;
4-[6-(3-Trifluoromethyl-4-Fluorophenyl)spiro[2.4]hept-5-en-5-yl]benzensulfonamid;
4-[6-(3-Trifluoromethyl-4-Chlorphenyl)spiro[2.4]hept-5-en-5-yl]benzensulfonamid;
4-[6-(3-Methyl-4-Fluorophenyl)spiro[2.4]hept-5-en-5-yl]benzensulfonamid;
4-[6-(3-Methyl-4-Chlorphenyl)spiro[2.4]hept-5-en-5-yl]benzensulfonamid;
4-[6-(4-Methoxy-2,3,5,6-Tetrafluorophenyl)spiro[2.4]hept-5-en-5-yl]benzensulfonamid;
4-[6-(3-Fluoro-4-Methoxyphenyl)spiro[2.4]hept-5-en-5-yl]benzensulfonamid;
4-[6-(3-Trifluoromethyl-4-Methoxyphenyl)spiro[2.4]hept-5-en-5-yl]benzensulfonamid;
4-[6-(3-Methyl-4-Methoxyphenyl)spiro[2.4]hept-5-en-5-yl]benzensulfonamid;
4-[6-(3-Chlor-4-Methoxyphenyl)spiro[2.4]hept-5-en-5-yl]benzensulfonamid;
4-[6-(4-Methoxy-2,3,5,6-Tetrafluorophenyl)spiro[2.4]hept-5-en-5-yl]benzensulfonamid;
4-[6-(3,4-Dimethoxyphenyl)spiro[2.4]hept-5-en-5-yl]benzensulfonamid;
4-[6-(3-Chlor-4-Fluorophenyl)spiro[2.4]hept-5-en-5-yl]benzensulfonamid;
4-[6-(4-Chlor-3-Fluorophenyl)spiro[2,4]hept-5-en-5-yl]benzensulfonamid;
4-[6-(3,4-Difluorophenyl)spiro[2.4]hept-5-en-5-yl]benzensulfonamid; und
4-[6-(3,4-Dichlorphenyl)spiro[2.4]hept-5-en-5-yl]benzensulfonamid.

8. Verbindung gemäß Anspruch 7, welche 5-(3,5-Dichlor-4-Methoxyphenyl)-6-[4-(Methylsulfonyl)phenyl]spiro[2.4]hept-5-en oder ein pharmazeutisch akzeptables Salz davon ist.

9. Verbindung gemäß Anspruch 7, welche 4-[6-(3-Chlor-4-Methoxyphenyl)spiro[2.4]hept-5-en-5-yl]benzensulfonamid oder ein pharmazeutisch akzeptables Salz davon ist.

10. Verbindung gemäß Anspruch 4, wobei n eine Zahl ist, ausgewählt aus 0, 1 und 2;
wobei R¹, R², R⁴, R⁵ und R¹⁰ jeweils Hydrido ist;
wobei R³ ausgewählt ist aus Niedrigalkylsulfonyl und Aminosulfonyl;
wobei R⁶ ausgewählt ist aus Hydrido und Halo;
wobei R⁷ ausgewählt ist aus Hydrido und Halo;
wobei R⁸ ausgewählt ist aus Hydrido, Halo; Niedrigalkyl, Niedrigalkoxy, Niedrighaloalkyl, Niedrighaloalkoxy und Hydroxyl; und
wobei R⁹ ausgewählt ist aus Hydrido, Halo und Niedrigalkyl; oder wobei R⁸ und R⁹ gemeinsam Methylendioxy bilden; oder ein pharmazeutisch akzeptables Salz davon.

11. Verbindung von Anspruch 10, wobei R³ Methylsulfonyl oder Aminosulfonyl ist; wobei R⁶ ausgewählt ist aus Hydrido, Fluoro, Chloro, Bromo und lodo; wobei R⁷ ausgewählt ist aus Hydrido, Fluoro, Chloro, Bromo und lodo; wobei R⁸ ausgewählt ist aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Hydroxyl, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl und Trifluoromethoxy; wobei R⁹ ausgewählt ist aus Hydrido, Fluoro, Chloro, Bromo, lodo, Methyl, Ethyl, n-Propyl, Isopropyl, Butyl, tert-Butyl und Isobutyl; oder wobei R⁸ und R⁹ gemeinsam Methylendioxy bilden; und wobei R¹¹ Methyl oder Amino ist; und oder ein pharmazeutisch akzeptables Salz davon.

12. Verbindung gemäß Anspruch 11, ausgewählt aus Verbindungen und deren pharmazeutisch akzeptablen Salzen der Gruppe bestehend aus
5-(3-Chlor-4-Methoxyphenyl)-6-[4-(Methylsulfonyl)phenyl]spiro[2,4]hept-5-en;
4-[6-(3-Chlor-4-Methoxyphenyl)spiro[2,4]hept-5-en-5-yl]benzensulfonamid;
5-(3-Fluoro-4-Methoxyphenyl)-6-[4-(Methylsulfonyl)phenyl]spiro[2,4]hept-5-en;
4-[6-(3-Fluoro-4-Methoxyphenyl)spiro[2,4]hept-5-en-5-yl]benzensulfonamid;
5-(3,4-Difluorophenyl)-6-[4-(Methylsulfonyl)phenyl]spiro[2,4]hept-5-en;
5-[6-[4(Methylsulfonyl)phenyl]spiro[2,4]hept-5-en-5-yl]-1,3-Benzodioxol;
4-[6-(3,4-Difluorophenyl)spiro[2,4]hept-5-en-5-yl]benzensulfonamid;
2.6-Dichlor-4-[6-[4-(Methylsulfonyl)phenyl]spiro[2,4]hept-5-en-5-yl]phenol;
5-(4-Trifluoromethoxyphenyl)-6-[4-(Methylsulfonyl)phenyl]spiro[2,4]hept-5-en;
5-(4-Methoxyphenyl)-6-[4-(Methylsulfonyl)phenyl]spiro[2,4]hept-5-en;
5-(3-Brom-4-Methoxyphenyl)-6-[4-(Methylsulfonyl)phenyl]spiro[2,4]hept-5-en;
4-[6-(4-Methoxyphenyl)spiro[2,4]hept-5-en-5-yl]benzensulfonamid;
4-[6-(3-Brom-4-Methoxyphenyl)spiro[2,4]hept-5-en-5-yl]benzensulfonamid;
5-(4-Trifluoromethylphenyl)-6-[4-(Methylsulfonyl)phenyl]spiro[2,4]hept-5-en;
5-(3,5-Dichlor-4-Methoxy-phenyl]-6-[4-(Methylsulfonyl)phenyl]spiro[2,4]hept-5-en;
4-[6-(4-Trifluoromethoxyphenyl)spiro[2,4]hept-5-en-5-yl]benzensulfonamid;
5-(3-Chlor-4-Fluorophenyl)-6-[4-(Methylsulfonyl)phenyl]spiro[2,4]hept-5-en;
5-(2,4-Difluorophenyl)-6-[4-(Methylsulfonyl)phenyl]spiro[2,4]hept-5-en;
5-(2,4-Dichlorphenyl)-6-[4-(Methylsulfonyl)phenyl]spiro[2,4]hept-5-en;
4-[6-(4-Trifluoromethylphenyl)spiro[2,4]hept-5-en-5-yl]benzensulfonamid;
4-[6-(3-Chlor-4-Fluorophenyl)spiro[2,4]hept-5-en-5-yl]benzensulfonamid;
5-(3,4-Dichlorphenyl)-6-[4-(Methylsulfonyl)phenyl]spiro[2,4]hept-5-en;
5-(4-Chlorphenyl)-6-[4-(Methylsulfonyl)phenyl]spiro[2,4]hept-5-en;
4-[6-(3,4-Dichlorphenyl)spiro[2,4]hept-5-en-5-yl]benzensulfonamid;
4-[6-(4-Chlorphenyl)spiro[2,4]hept-5-en-5-yl]benzensulfonamid;
5-(3-Chlor-4-Methylphenyl)-6-[4-(Methylsulfonyl)phenyl]spiro[2,4]hept-5-en;
5-(3,4-Dimethylphenyl)-6-[4-(Methylsulfonyl)phenyl]spiro[2,4]hept-5-en;
5-(4-Methylphenyl)-6-[4-(Methylsulfonyl)phenyl]spiro[2,4]hept-5-en;
5-(3-Methyl-4-Trifluoromethoxyphenyl)-6-[4-(Methylsulfonyl)phenyl]spiro[2,4]hept-5-en;
5-(3-Chlor-4-Trifluoromethoxyphenyl)-6-[4-(Methylsulfonyl)phenyl]spiro[2,4]hept-5-en;
4-[6-(3,5-Dichlor-4-Methoxyphenyl)spiro[2,4]hept-5-en-5-yl]benzensulfonamid;
4-[6-(3-Methyl-4-Trifluoromethoxyphenyl)spiro[2,4]hept-5-en-5-yl]benzensulfonamid;
4-[6-(3-Chlor-4-Trifluoromethoxyphenyl)spiro[2,4]hept-5-en-5-yl]benzensulfonamid;
5-(4-Fluorophenyl)-6-[4-(Methylsulfonyl)phenyl]spiro[2.4]hept-5-en;
4-[6-(4-Fluorophenyl)spiro[2.4]hept-5-en-5-yl]benzensulfonamid;
6-(4-Fluorophenyl)-7-[4-(Methylsulfonyl)phenyl]spiro[3.4]oct-5-en;
4-[7-(4-Fluorophenyl)spiro[3.4]oct-6-en-6-yl]benzensulfonamid; und
2-(4-Fluorophenyl)-3-[4-(Methylsulfonyl)phenyl]spiro[4.4]non-5-en.

13. Verbindung gemäß Anspruch 1, wobei A ist 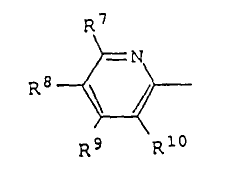
wobei n eine Zahl ist, ausgewählt aus 0, 1, 2 und 3; und
wobei R¹ bis R⁵ und R⁷ bis R¹⁰ je unabhängig ausgewählt sind aus Hydrido, Halo, Alkyl, Alkoxy, Alkylthio, Alkylamino, Cyan, Haloalkyl, Haloalkoxy, Hydroxyalkyl, Alkoxyalkyl, Hydroxyl, Mercapto, Alkylsulfonyl, Haloalkylsulfonyl und Aminosulfonyl; oder ein pharmazeutisch akzeptables Salz davon.

14. Verbindung gemäß Anspruch 13, wobei n eine Zahl ist, ausgewählt aus 0, 1, und 2; wobei R¹, R², R⁴, R⁵, R⁷, R⁹ und R¹⁰ je unabhängig voneinander ausgewählt sind aus Hydrido, Halo, Niedrigalkyl, Niedrigalkoxy, Niedrigalkylthio, Cyan, Niedrighaloalkyl, Niedrighaloalkoxy, Niedrighydroxyalkyl, Niedrigalkoxyalkyl, Hydroxyl und Mercapto; und wobei R³ ausgewählt ist aus Niedrigalkylsulfonyl und Aminosulfonyl, und R⁸ ausgewählt ist aus Hydrido, Halo, Niedrigalkyl, Niedrigalkoxy, Niedrigalkylthio, Niedrigalkylamino, Niedrighaloalkoxy, Niedrighydroxyalkyl, Mercapto, Hydroxyl, Niedrigalkoxyalkyl, Cyan und Niedrighaloalkyl; oder wobei des weiteren R⁸ und R⁹ gemeinsam Methylendioxy bilden; oder wobei des weiteren R³ ausgewählt ist aus Hydrido, Halo, Niedrigalkyl, Niedrigalkoxy, Niedrigalkylthio, Niedrigalkylamino, Niedrighaloalkoxy, Niedrighydroxyalkyl, Hydroxyl, Mercapto, Niedrigalkoxyalkyl, Cyan und Niedrighaloalkyl, und R⁸ ausgewählt ist aus Niedrigalkylsulfonyl und Aminosulfonyl; oder wobei des weiteren R³ und R⁴ gemeinsam Methylendioxy bilden; oder ein pharmazeutisch akzeptables Salz davon.

15. Verbindung gemäß Anspruch 14, wobei R¹, R², R⁴, R⁵, R⁷, R⁹ und R¹⁰ je unabhängig voneinander ausgewählt sind aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Hydroxyl, Mercapto, Methylthio, Ethylthio, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Methylamino, N,N-Dimethylamino, Trifluoromethoxy, Hydroxymethyl, Methoxymethyl und Ethoxymethyl; und wobei R³ Methylsulfonyl oder Aminosulfonyl ist, und R⁸ ist ausgewählt aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Trifluoromethoxy, Methylthio, Ethylthio, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl; oder wobei des weiteren R⁸ und R⁹ gemeinsam Methylendioxy bilden; oder wobei des weiteren R³ ausgewählt ist aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Trifluoromethoxy, Methylthio, Ethylthio, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl und Dichlorpropyl, und R⁸ Methylsulfonyl oder Aminosulfonyl ist; oder wobei R³ und R⁴ gemeinsam Methylendioxy bilden; oder ein pharmazeutisch akzeptables Salz davon.

16. Verbindung gemäß Anspruch 15, ausgewählt aus Verbindungen und deren pharmazeutisch akzeptablen Salzen der Gruppe bestehend aus
2-[6-[4-(Methylsulfonyl)phenyl]spiro[2.4]hept-5-en-5-yl]pyridin;
5-Fluoro-2-[6-[4-(Methylsulfonyl)phenyl]spiro[2.4]hept-5-en-5-yl]pyridin;
5-Chlor-2-[6-[4-(Methylsulfonyl)phenyl]spiro[2.4]hept-5-en-5-yl]pyridin;
5-Methyl-2-[6-[4-(Methylsulfonyl)phenyl]spiro[2.4]hept-5-en-5-yl]pyridin;
4-[6-(Pyridin-2-yl)spiro[2.4]hept-5-en-5-yl]benzensulfonamid;
4-[6-(5-Fluoropyridin-2-yl)spiro[2.4]hept-5-en-5-yl]benzensulfonamid;
4-[6-(5-Chlorpyridin-2-yl)spiro[2.4]hept-5-en-5-yl]benzensulfonamid;
4-[6-(5-Methylpyridin-2-yl)spiro[2.4]hept-5-en-5-yl]benzensulfonamid;
2-[7-[4-(Methylsulfonyl)phenyl]spiro[3.4]oct-6-en-6-yl]pyridin;
5-Fluoro-2-[7-[4-(Methylsulfonyl)phenyl]spiro[3.4]oct-6-en-6-yl]pyridin;
5-Chlor-2-[7-[4-(Methylsulfonyl)phenyl]spiro[3.4]oct-6-en-6-yl]pyridin;
5-Methyl-2-[7-[4-(Methylsulfonyl)phenyl]spiro[3.4]oct-6-en-6-yl]pyridin;
4-[7-(Pyridin-2-yl)spiro[3.4]oct4-en-6-yl]benzensulfonamid;
4-[7-(5-Fluoropyridin-2-yl)spiro[3.4]oct-6-en-6-yl]benzensulfonamid;
4-[7-(5-Chlorpyridin-2-yl)spiro[3.4]oct-6-en-6-yl]benzensulfonamid;
4-[7-(5-Methylpyridin-2-yl)spiro[3.4]oct-6-en-6-yl]benzensulfonamid;
2-[3-[4-(Methylsulfonyl)phenyl]spiro[4.4]non-2-en-2-yl]pyridin;
5-Fluoro-2-[3-[4-(Methylsulfonyl)phenyl]spiro[4.4]non-2-en-2-yl]pyridin;
5-Chlor-2-[3-[4-(Methylsulfonyl)phenyl]spiro[4.4]non-2-en-2-yl]pyridin;
5-Methyl-2-[3-[4-(Methylsulfonyl)phenyl]spiro[4.4]non-2-en-2-yl]pyridin;
4-[3-(Pyridin-2-yl)spiro[4.4]non-2-en-2-yl]benzensulfonamid;
4-[3-(5-Fluoropyridin-2-yl)spiro[4.4]non-2-en-2-yl]benzensulfonamid;
4-[3-(5-Chlorpyridin-2-yl)spiro[4.4]non-2-en-2-yl]benzensulfonamid;
4-[3-(5-Methylpyridin-2-yl)spiro[4.4]non-2-en-2-yl]benzensulfonamid;
2-(6-Phenylspiro[2 .4]hept-5-en-5-yl]-5-(Methylsulfonyl)pyridin;
2-[6-(4-(Fluorophenyl)spiro[2.4]hept-5-en-yl]-5-(Methylsulfonyl)pyridin;
2-[6-(4-(Chlorphenyl)spiro[2.4]hept-5-en-yl]-5-(Methylsulfonyl)pyridin;
2-[6-(4-(Methylphenyl)spiro[2.4]hept-en-yl]-5-(Methylsulfonyl)pyridin;
2-(6-Phenylspiro[2.4]hept-5-en-yl)-5-Pyridinsulfonamid;
2-[6-(4-(Fluorophenyl)spiro[2.4]hept-5-en-5-yl)-5-Pyridinsulfonamid;
2-[6-(4-(Chlorphenyl)spiro[2.4]hept-5-en-5-yl)-5-Pyridinsulfonamid;
2-[6-(4-(Methylphenyl)spiro[2.4]hept-5-en-5-yl)-5-Pyridinsulfonamid;
2-(7-Phenylspiro[3.4]oct-6-en-6-yl]-5-(Methylsulfonyl)pyridin;
2-[7-(4-Fluorophenyl)spiro[3.4]oct-6-en-6-yl]-5-(Methylsulfonyl)pyridin;
2-[7-(4-Chlorphenyl)spiro[3.4]oct-6-en-6-yl]-5-(Methylsulfonyl)pyridin;
2-[7-(4-Methylphenyl)spiro[3.4]oct-6-en-6-yl]-5-(Methylsulfonyl)pyridin;
2-(7-Phenylspiro[3.4]oct-6-en-6-yl)-5-Pyridinsulfonamid;
2-[7-(4-Fluorophenyl)spiro[3.4]oct-6-en-6-yl]-5-Pyridinsulfonamid;
2-[7-(4-Chlorphenyl)spiro[3.4]oct-6-en-6-yl]-5-Pyridinsulfonamid;
2-[7-(4-Methylphenyl)spiro[3.4]oct-6-en-6-yl]-5-Pyridinsulfonamid;
2-(3-Phenylspiro[4.4]non-2-en-2-yl)-5-(Methylsulfonyl)pyridin;
2-[3-(4-Fluorophenyl)spiro[4.4]non-2-en-2-yl]-5-(Methylsulfonyl)pyridin;
2-[3-(4-Chlorphenyl)spiro[4.4]non-2-en-2-yl]-5-(Methylsulfonyl)pyridin;
2-[3-(4-Methylphenyl)spiro[4.4]non-2-en-2-yl]-5-(Methylsulfonyl)pyridin;
2-(3-Phenylspiro[4.4]non-2-en-2-yl)-5-Pyridinsulfonamid;
2-[3-(4-Fluorophenyl)spiro[4.4]non-2-en-2-yl]-5-Pyridinsulfonamid;
2-[3-(4-Chlorphenyl)spiro[4.4]non-2-en-2-yl]-5-Pyridinsulfonamid;
2-[3-(4-Methylphenyl)spiro[4.4]non-2-en-2-yl]-5-Pyridinsulfonamid; und

17. Verbindung gemäß Anspruch 1, wobei A ist 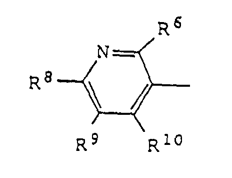
wobei n eine Zahl ist, ausgewählt aus 0, 1, 2, und 3; und
wobei R¹ bis R⁶ und R⁸ bis R¹⁰ jeweils unabhängig voneinander ausgewählt sind aus Hydrido, Halo, Alkyl, Alkoxy, Alkylthio, Alkylamino, Cyan, Haloalkyl, Haloalkoxy, Hydroxyalkyl, Alkoxyalkyl, Hydroxyl, Mercapto, Alkylsulfonyl und Aminosulfonyl; oder ein pharmazeutisch akzeptables Salz davon.

18. Verbindung gemäß Anspruch 17, wobei n eine Zahl ist, ausgewählt aus 0, 1 und 2. wobei R¹, R², R⁴, R⁵, R⁶, R⁹ und R¹⁰ unabhängig voneinander ausgewählt sind aus Hydrido, Halo. Niedrigalkyl, Niedrigalkoxy, Niedrigalkylthio, Cyan, Niedrighaloalkyl, Niedrighaloalkoxy, Niedrighydroxyalkyl, Niedrigalkoxyalkyl, Hydroxyl und Mercapto; und wobei R³ ausgewählt ist aus Niedrigalkylsulfonyl und Aminosulfonyl, und wobei R⁸ ausgewählt ist aus Hydrido, Halo, Niedrigalkyl, Niedrigalkoxy, Niedrigalkylthio, Niedrigalkylamino, Niedrighaloalkoxy, Niedrighydroxyalkyl, Mercapto. Hydroxyl, Niedrigalkoxyalkyl, Cyan und Niedrighaloalkyl; oder wobei des weiteren R⁸ und R⁹ gemeinsam Methylendioxy bilden; oder wobei des weiteren R³ ausgewählt ist aus Hydrido, Halo, Niedrigalkyl, Niedrigalkoxy, Niedrigalkylthio, Niedrigalkylamino, Niedrighaloalkoxy, Niedrighydroxyalkyl, Hydroxyl, Niedrigalkoxyalkyl, Cyan und Niedrighaloalkyl; und R⁸ ausgewählt ist aus Niedrigalkylsulfonyl und Aminosulfonyl; oder wobei des weiteren R³ und R⁴ gemeinsam Methylendioxy bilden; oder ein pharmazeutisch akzeptables Salz davon.

19. Verbindung gemäß Anspruch 18, wobei R¹, R², R⁴, R⁵, R⁶, R⁹ und R¹⁰ jeweils ausgewählt sind aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Hydroxyl, Mercapto, Methylthio, Ethylthio, Cyan, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Trifluoromethoxy, Hydroxymethyl, Methoxymethyl und Ethoxymethyl; und wobei R³ ausgewählt ist aus Methylsulfonyl oder Aminosulfonyl, und R⁸ ausgewählt ist aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Trifluoromethoxy, Hydroxyl, Methylthio, Ethylthio, Methylamino, N,N-Dimethylamino, Cyan, Mercapto, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl und Dichlorpropyl; oder wobei des weiteren R⁸ und R⁹ gemeinsam Methylendioxy bilden; oder wobei des weiteren R³ ausgewählt ist aus Hydrido, Fluoro, Chloro, Bromo, lodo, Methyl, Ethyl, n-Propyl, Isopropyl, Butyl, tert-Butyl, Isobutyl, Methoxy, Ethoxy, Propoxy, Butoxy, Trifluoromethoxy, Hydroxyl, Methylthio, Ethylthio, Cyan, Mercapto, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl und Dichlorpropyl; und R⁸ ist ausgewählt aus Methylsulfonyl oder Aminosulfonyl; oder wobei des weiteren R³ und R⁴ gemeinsam Methylendioxy bilden; oder ein pharmazeutisch akzeptables Salz davon.

20. Verbindung gemäß Anspruch 19, ausgewählt aus Verbindungen und deren pharmazeutisch akzeptablen Salzen der Gruppe bestehend aus
5-[6-[4-(Methylsulfonyl)phenyl]spiro[2.4]hept-5-en-5-yl]pyridin;
2-Fluoro-5-[6-[4-(Methylsulfonyl)phenyl]spiro[2.4]hept-5-en-5-yl]pyridin;
2-Chlor-5-[6-[4-(Methylsulfonyl)phenyl]spiro[2.4]hept-5-en-5-yl]pyridin;
2-Methyl-5-[6-[4-(Methylsulfonyl)phenyl]spiro[2.4]hept-5-en-5-yl]pyridin;
4-[6-(Pyridin-5-yl)spiro[2.4]hept-5-en-5-yl]benzensulfonamid;
4-[6-(2-Fluoropyridin-5-yl)spiro[2.4]hept-5-en-5-yl]benzensulfonamid;
4-[6-(2-Chlorpyridin-5-yl)spiro[2.4]hept-5-en-5-yl]benzensulfonamid;
4-[6-(2-Methylpyridin-5-yl)spiro[2.4]hept-5-en-5-yl]benzensulfonamid;
5-[7-[4-(Methylsulfonyl)phenyl]spiro[3.4]oct-6-en-6-yl]pyridin;
2-Fluoro-5-[7-[4-(Methylsulfonyl)phenyl]spiro[3.4]oct-6-en-6-yl]pyridin;
2-Chlor-5-[7-[4-(Methylsulfonyl)phenyl]spiro[3.4]oct-6-en-6-yl]pyridin;
2-Methyl-5-[7-[4-(Methylsulfonyl)phenyl]spiro[3.4]oct-6-en-6-yl]pyridin;
4-[7-(Pyridin-5-yl)spiro[3.4]oct-6-en-6-yl]benzensulfonamid;
4-[7-(2-Fluoropyridin-5-yl)spiro[3.4]oct-6-en-6-yl]benzensulfonamid;
4-[7-(2-Chlorpyridin-5-yl)spiro[3.4]oct-6-en-6-yl]benzensulfonamid;
4-[7-(2-Methylpyridin-5-yl)spiro[3.4]oct-6-en-6-yl]benzensulfonamid;
5-[3-[4-(Methylsulfonyl)phenyl]spiro[4.4]non-2-en-2-yl]pyridin;
2-Fluoro-5-[3-[4-(Methylsulfonyl)phenyl]spiro[4.4]non-2-en-2-yl]pyridin;
2-Chlor-5-[3-[4-(Methylsulfonyl)phenyl]spiro[4.4]non-2-en-2-yl]pyridin;
2-Methyl-5-[3-[4-(Methylsulfonyl)phenyl]spiro[4.4]non-2-en-2-yl]pyridin;
4-[3-(Pyridin-5-yl)spiro[4.4]non-2-en-2-yl]benzensulfonamid;
4-[3-(2-Fluoropyridin-5-yl)spiro[4.4]non-2-en-2-yl]benzensulfonamid;
4-[3-(2-Chlorpyridin-5-yl)spiro[4.4]non-2-en-2-yl]benzensulfonamid;
4-[3-(2-Methylpyridin-5-yl)spiro[4.4]non-2-en-2-yl]benzensulfonamid;
5-(6-Phenylspiro[2.4]hept-5-en-5-yl)-2-(Methylsulfonyl)pyridin;
5-[6-(4-(Fluorophenyl)spiro[2.4]hept-5-en-5-yl]-2-(Methylsulfonyl)pyridin;
5-[6-(4-(Chlorphenyl)spiro[2.4]hept-5-en-5-yl]-2-(Methylsulfonyl)pyridin;
5-[6-(4-(Methylphenyl)spiro[2.4]hept-5-en-5-yl]-2-(Methylsulfonyl)pyridin;
5-(6-Phenylspiro[2.4]hept-5-en-5-yl)-2-Pyridinsulfonamid;
5-[6-(4-(Fluorophenyl)spiro[2.4]hept-5-en-5-yl]-2-Pyridinsulfonamid;
5-[6-(4-(Chlorphenyl)spiro[2.4]hept-5-en-5-yl]-2-Pyridinsulfonamid;
5-[6-(4-(Methylphenyl)spiro[2.4]hept-5-en-5-yl]-2-Pyridinsulfonamid;
5-(7-Phenylspiro[3.4]oct-6-en-6-yl)-2-(Methylsulfonyl)pyridin;
5-[7-(4-Fluorophenyl)spiro[3.4]oct-6-en-6-yl]-2-(Methylsulfonyl)pyridin;
5-[7-(4-Chlorphenyl)spiro[3.4]oct-6-en-6-yl]-2-(Methylsulfonyl)pyridin;
5-[7-(4-Methylphenyl)spiro[3.4]oct-6-en-6-yl]-2-(Methylsulfonyl)pyridin;
5-(7-Phenylspiro[3.4]oct-6-en-6-yl)-2-Pyridinsulfonamid;
5-[7-(4-Fluorophenyl)spiro[3.4]oct-6-en-6-yl]-2-Pyridinsulfonamid;
5-[7-(4-Chlorphenyl)spiro[3.4]oct-6-en-6-yl]-2-Pyridinsulfonamid;
5-[7-(4-Methylphenyl)spiro[3.4]oct-6-en-6-yl]-2-Pyridinsulfonamid;
5-(3-Phenylspiro[4.4]non-2-en-2-yl)-2-(Methylsulfonyl)pyridin;
5-[3-(4-Fluorophenyl)spiro[4.4]non-2-en-2-yl]-2-(Methylsulfonyl)pyridin;
5-[3-(4-Chlorphenyl)spiro[4.4]non-2-en-2-yl]-2-(Methylsulfonyl)pyridin;
5-[3-(4-Methylphenyl)spiro[4.4]non-2-en-2-yl]-2-(Methylsulfonyl)pyridin;
5-(3-Phenylspiro[4.4]non-2-en-2-yl)-2-Pyridinsulfonamid;
5-[3-(4-Fluorophenyl)spiro[4.4]non-2-en-2-yl]-2-Pyridinsulfonamid;
5-[3-(4-Chlorphenyl)spiro[4.4]non-2-en-2-yl]-2-Pyridinsulfonamid; und
5-[3-(4-Methylphenyl)spiro[4.4]non-2-en-2-yl]-2-Pyridinsulfonamid;

21. Verbindung gemäß Anspruch 1, wobei A ist 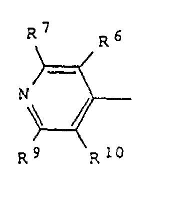
wobei n eine Zahl ist, ausgewählt aus 0, 1, 2, und 3; und
wobei R¹ bis R⁷, R⁹ und R¹⁰ jeweils unabhängig voneinander ausgewählt sind aus Hydrido, Halo Alkyl, Alkoxy, Alkylthio, Cyan, Haloalkyl, Haloalkoxy, Hydroxyalkyl, Alkoxyalkyl, Hydroxyl, Mercapto, Alkylsulfonyl und Aminosulfonyl; oder ein pharmazeutisch akzeptables Salz davon.

22. Verbindung gemäß Anspruch 21, wobei n eine Zahl ist, ausgewählt aus 0, 1 und 2; wobei R¹, R², R⁴ bis R⁷, R⁹ und R¹⁰ unabhängig voneinander ausgewählt sind aus Hydrido, Halo, Niedrigalkyl, Niedrigalkoxy, Niedrigalkylthio, Niedrighaloalkoxy, Niedrighydroxyalkyl, Hydroxyl, Niedrigalkoxyalkyl, Mercapto, Cyan und Niedrighaloalkyl; und wobei R³ ausgewählt ist aus Niedrigalkylsulfonyl und Aminosulfonyl, oder ein pharmazeutisch akzeptables Salz davon.

23. Verbindung gemäß Anspruch 22, wobei R¹, R², R⁴ bis R⁷, R⁹ und R¹⁰ jeweils unabhängig voneinander ausgewählt sind aus Hydrido, Fluoro, Chloro, Bromo, Iodo, Methyl, Ethyl n-Propyl, Isopropyl, Butyl, tert-Butyl, Isobutyl, Methoxy, Ethoxy, Propoxy, Butoxy, Trifluoromethoxy, Methylthio, Hydroxyl, Ethylthio, Cyan, Hydroxyl, Mercapto, Fluoromethyl, Difluoromethyl, Trifluoromethyl, Chlormethyl, Dichlormethyl, Trichlormethyl, Pentafluoroethyl, Heptafluoropropyl, Difluorochlormethyl, Dichlorfluoromethyl, Difluoroethyl, Difluoropropyl, Dichlorethyl, Dichlorpropyl, Hydroxymethyl, Methoxymethyl und Ethoxymethyl; und wobei R³ Methylsulfonyl oder Aminosulfonyl ist; oder ein pharmazeutisch akzeptables Salz davon.

24. Verbindung gemäß Anspruch 23, ausgewählt aus Verbindungen und deren pharmazeutisch akzeptablen Salzen der Gruppe bestehend aus
4-[6-[4-(Methylsulfonyl)phenyl]spiro[2.4]hept-5-en-5-yl]pyridin;
4-[6-(4-Pyridinyl)spiro[2.4]hept-5-en-5-yl]benzensulfonamid:
4-[7-[4-(Methylsulfonyl)phenyl]spiro[3.4]oct-6-en-6-yl]pyridin;
4-[7-(4-Pyridinyl)spiro[3.4]oct-6-en-6-yl]benzensulfonamid;
4-[3-[4-(Methylsulfonyl)phenyl]spiro[4.4]non-2-en-2-yl]pyridin; und
4-[3-(4-Pyridinyl)spiro[4.4]non-2-en-2-yl]benzensulfonamid;

25. Pharmazeutische Zusammensetzung, umfassend eine therapeutisch wirksame Menge einer Verbindung, wobei die Verbindung ausgewählt ist aus einer Verbindungsklasse einer der Ansprüche 1 oder 4 bis 10, 13, 17 oder 21 oder ein pharmazeutisch akzeptables Salz davon.

26. Verwendung einer Verbindung gemäß einem der Ansprüche 1, 4 bis 10, 13, 17 oder 21 zur Herstellung eines Medikaments zur Behandlung von Entzündungen oder einer mit einer Entzündung verbundenen Erkrankung an einem Patienten.

27. Verwendung von Anspruch 26 zum Einsatz in der Behandlung von Entzündungen.

28. Verwendung von Anspruch 26 zum Einsatz in der Behandlung einer mit einer Entzündung verbundenen Erkrankung.

29. Verwendung von Anspruch 28, wobei die mit einer Entzündung verbundenen Erkrankung Arthritis ist.

30. Verwendung von Anspruch 28, wobei die mit einer Entzündung verbundenen Erkrankung ein Schmerz ist.

31. Verwendung von Anspruch 28, wobei die mit einer Entzündung verbundenen Erkrankung Fieber ist.

## Revendications

1. Composé de formule I 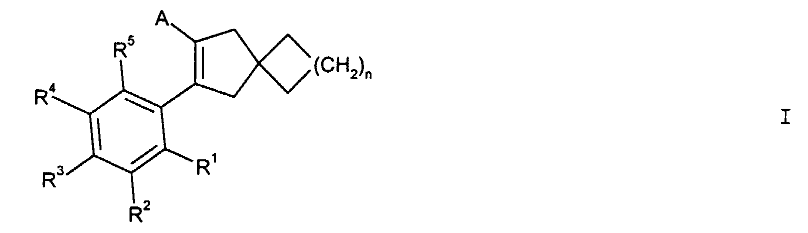
dans laquelle A est choisi parmi 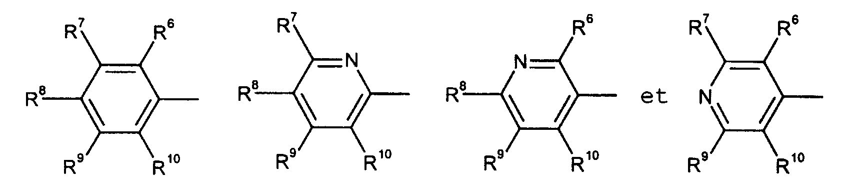 Où chacun de R¹ à R¹⁰, s'il est présent, est indépendamment choisi parmi les radicaux hydrido, halogéno, alkyle, alcoxy, alkylthio, alkylamino, cyano, halogénoalkyle, halogénoalcoxy, hydroxyalkyle, alcoxyalkyle, hydroxyle, mercapto, alkylsulfonyle, halogénoalkylsulfonyle et aminosulfonyle ;
et dans laquelle n est un nombre choisi parmi 0, 1, 2 et 3 ;
ou un sel acceptable en pharmacie d'un tel composé.

2. Composé selon la revendication 1, dans lequel chacun de R¹, R², R⁴ à R⁷, R⁹ et R¹⁰, s'il est présent, est indépendamment choisi parmi les radicaux hydrido, halogéno, alkyle inférieur, alcoxy inférieur, alkylthio inférieur, cyano, halogénoalkyle inférieur, halogénoalcoxy inférieur, hydroxyalkyle inférieur, alcoxyalkyle inférieur, hydroxyle et mercapto ; et dans lequel R³ est choisi parmi les radicaux alkylsulfonyle inférieur, halogénoalkylsulfonyle inférieur et aminosulfonyle, et R⁸, s'il est présent, est choisi parmi les radicaux hydrido, halogéno, alkyle inférieur, alcoxy inférieur, alkylthio inférieur, alkylamino inférieur, cyano, halogénoalkyle inférieur, halogénoalcoxy inférieur, hydroxyalkyle inférieur, alcoxyalkyle inférieur, hydroxyle ou mercapto ; ou dans lequel encore R⁸ et R⁹, s'ils sont présents, forment ensemble méthylènedioxy ; ou dans lequel encore R³ est choisi parmi les radicaux hydrido, halogéno, alkyle inférieur, alcoxy inférieur, alkylthio inférieur, alkylamino inférieur, cyano, halogénoalkyle inférieur, halogénoalcoxy inférieur, hydroxyalkyle inférieur, alcoxyalkyle inférieur, hydroxyle et mercapto, et R⁸ est choisi parmi les radicaux alkylsulfonyle inférieur, halogénoalkylsulfonyle inférieur et aminosulfonyle ; ou dans lequel encore R³ et R⁴, s'ils sont présents, forment ensemble méthylènedioxy ; ou un sel acceptable en pharmacie d'un tel composé.

3. Composé selon la revendication 2 dans lequel chacun de R¹, R², R⁴ à R⁷, R⁹ et R¹⁰, s'il est présent, est indépendamment choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, hydroxyle, mercapto, méthylthio, éthylthio, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, trifluorométhoxy, hydroxyméthyle, méthoxyméthyle et éthoxyméthyle ; et dans lequel R³ est choisi parmi les radicaux méthylsulfonyle, fluorométhylsulfonyle, difluorométhylsulfonyle, trifluorométhylsulfonyle et aminosulfonyle, et R⁸, s'il est présent, est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, hydroxyle, mercapto, méthylthio, éthylthio, méthylamino, N,N-diméthylamino, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, trifluorométhoxy, hydroxyméthyle, méthoxyméthyle et éthoxyméthyle ; ou dans lequel encore R⁸ et R⁹, s'ils sont présents, forment ensemble méthylènedioxy ; ou dans lequel encore R³ est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, hydroxyle, mercapto, méthylthio, éthylthio, méthylamino, N,N-diméthylamino, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, trifluorométhoxy, hydroxyméthyle, méthoxyméthyle et éthoxyméthyle, et R⁸ est choisi parmi les radicaux méthylsulfonyle, fluorométhylsulfonyle, difluorométhylsulfonyle, trifluorométhylsulfonyle et aminosulfonyle ; ou dans lequel encore R³ et R⁴, s'ils sont présents, forment ensemble méthylènedioxy ; ou un sel acceptable en pharmacie d'un tel composé.

4. Composé selon la revendication 1, dans lequel A est 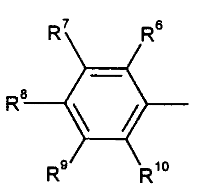
dans laquelle chacun de R¹ à R¹⁰ est indépendamment choisi parmi les radicaux hydrido, halogéno, alkyle, alcoxy, alkylthio, alkylamino, cyano, halogénoalkyle, halogénoalcoxy, hydroxyalkyle, alcoxyalkyle, hydroxyle, mercapto, alkylsulfonyle, halogénoalkylsulfonyle et aminosulfonyle ;
et dans lequel n est un nombre choisi parmi 0, 1, 2 et 3 ;
ou un sel acceptable en pharmacie d'un tel composé.

5. Composé selon la revendication 4, dans lequel n est un nombre choisi parmi 0, 1 et 2 ; dans lequel chacun de R¹, R² et R⁴ à R¹⁰ est indépendamment choisi parmi les radicaux hydrido, halogéno, alkyle inférieur, alkylthio inférieur, alkylamino inférieur, cyano, halogénoalkyle inférieur, halogénoalcoxy inférieur, alcoxy inférieur, hydroxyle, mercapto, hydroxyalkyle inférieur et alcoxyalkyle inférieur ; et dans lequel R³ est choisi parmi les radicaux alkylsulfonyle inférieur, halogénoalkylsulfonyle inférieur et aminosulfonyle ; ou dans lequel R⁸ et R⁹ forment ensemble méthylènedioxy ; ou un sel acceptable en pharmacie d'un tel composé.

6. Composé selon la revendication 5, dans lequel chacun de R¹, R² et R⁴ à R¹⁰ est indépendamment choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, hydroxyle, mercapto, méthylthio, éthylthio, méthylamino, N,N-diméthylamino, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, trifluorométhoxy, hydroxyméthyle, méthoxyméthyle et éthoxyméthyle ; et dans lequel R³ est choisi parmi les radicaux méthylsulfonyle, fluorométhylsulfonyle, difluorométhylsulfonyle, trifluorométhylsulfonyle et aminosulfonyle ; ou dans lequel R⁸ et R⁹ forment ensemble méthylènedioxy ; ou un sel acceptable en pharmacie d'un tel composé.

7. Composé selon la revendication 6, choisi parmi les composés, et leurs sels acceptables en pharmacie, de l'ensemble constitué par les suivants :
5-[6-[4-(méthylsulfonyl)phényl]spiro[2.4]hept-5-ène-5-yl]-1,3-benzodioxole ;
2,6-dichloro-4-[6-[4-(méthylsulfonyl)phényl]spiro[2.4]hept-5-ène-5-yl]phénol ;
5-(4-trifluorométhoxyphényl)-6-[4-(méthylsulfonyl)phényl]spiro[2.4]hept-5-ène ;
5-(3-bromo-4-méthoxyphényl)-6-[4-(méthylsulfonyl)phényl]spiro[2.4]hept-5-ène;
4-[6-(3-bromo-4-méthoxyphényl)spiro[2.4]hept-5-ène5-yl]benzènesulfonamide ;
5-(3,5-dichloro-4-méthoxyphényl)-6-[4-(méthylsulfonyl)phényl]spiro[2.4]hept-5-ène ;
4-[6-(4-trifluorométhoxyphényl)spiro[2.4]hept-5-ène-5-yl]benzènesulfonamide ;
5-(2,4-difluorophényl)-6-[4-(méthylsulfonyl)phényl]spiro[2.4]hept-5-ène ;
5-(2,4-dichlorophényl)-6-[4-(méthylsulfonyl)phényl]spiro[2.4]hept-5-ène ;
5-(3-chloro-4-méthylphényl)-6-[4-(méthylsulfonyl)phényl]spiro[2.4]hept-5-ène ;
5-(3,4-diméthylphényl)-6-[4-(méthylsulfonyl)phényl]spiro[2.4]hept-5-ène;
5-(3-méthyl-4-trifluorométhoxyphényl)-6-[4-(méthylsulfonyl)phényl]spiro[2.4]hept-5-ène;
5-(3-chloro-4-trifluorométhoxyphényl)-6-[4-(méthylsulfonyl)phényl]spiro[2.4]hept-5-ène ;
4-[6-(3,5-dichloro-4-méthoxyphényl)spiro[2.4]hept-5-ène-5-yl]benzènesulfonamide ;
4-[6-(3-méthyl-4-trifluorométhoxyphényl)spiro[2.4]hept-5-ène-5-yl]benzènesulfonamide ;
4-[6-(3-chloro-4-trifluorométhoxyphényl)spiro[2.4]hept-5-ène-5-yl]benzènesulfonamide ;
5-phényl-6-[4-(méthylsulfonyl)phényl]spiro[2.4]hept-5-ène ;
5-(4-fluorophényl)-6-[4-(méthylsulfonyl)phényl]spiro[2.4]hept-5-ène;
5-(4-chlorophényl)-6-[4-(méthylsulfonyl)phényl]spiro[2.4]hept-5-ène ;
5-(4-méthylphényl)-6-[4-(méthylsulfonyl)phényl]spiro[2.4]hept-5-ène ;
5-(4-méthoxyphényl)-6-[4-(méthylsulfonyl)phényl]spiro[2.4]hept-5-ène ;
5-(4-méthylthiophényl)-6-[4-(méthylsulfonyl)phényl]spiro[2.4]hept-5-ène ;
5-(4-cyanophényl)-6-[4-(méthylsulfonyl)phényl]spiro[2.4]hept-5-ène ;
5-(4-trifluorométhylphényl)-6-[4-(méthylsulfonyl)phényl]spiro[2.4]hept-5-ène ;
4-(6-phénylspiro[2.4]hept-5-ène-5-yl)benzènesulfonamide ;
4-[6-(4-fluorophényl)spiro[2.4]hept-5-ène-5-yl]benzènesulfonamide ;
4-[6-(4-chlorophényl)spiro[2.4]hept-5-ène-5-yl]benzènesulfonamide ;
4-[6-(4-méthylphényl)spiro[2.4]hept-5-ène-5-yl]benzènesulfonamide ;
4-[6-(4-méthoxyphényl)spiro[2.4]hept-5-ène-5-yl]benzènesulfonamide ;
4-[6-(4-méthylthiophényl)spiro[2.4]hept-5-ène-5-yl]benzènesulfonamide ;
4-[6-(4-cyanophényl)spiro[2.4]hept-5-ène-5-yl]benzènesulfonamide ;
4-[6-(4-trifluorométhylphényl)spiro[2.4]hept-5-ène-5-yl]benzènesulfonamide ;
6-phényl-7-[4-(méthylsulfonyl)phényl]spiro[3.4]oct-6-ène ;
6-(4-fluorophényl)-7-[4-(méthylsulfonyl)phényl]spiro[3.4]oct-6-ène ;
6-(4-chlorophényl)-7-[4-(méthylsulfonyl)phényl]spiro[3.4]oct-6-ène ;
6-(4-méthylphényl)-7-[4-(méthylsulfonyl)phényl]spiro[3.4]oct-6-ène ;
6-(4-méthoxyphényl)-7-[4-(méthylsulfonyl)phényl]spiro[3.4]oct-6-ène ;
6-(4-méthylthiophényl)-7-[4-(méthylsulfonyl)phényl]spiro[3.4]oct-6-ène;
6-(4-cyanophényl)-7-[4-(méthylsulfonyl)phényl]spiro[3.4]oct-6-ène;
6-(4-trifluorométhylphényl)-7-[4-(méthylsulfonyl)phényl]spiro[3.4]oct-6-ène ;
4-(7-phénylspiro[3.4]oct-6-ène-6-yl)benzènesulfonamide ;
4-[7-(4-fluorophényl)spiro[3.4]oct-6-ène-6-yl]benzènesulfonamide ;
4-[7-(4-chlorophényl)spiro[3.4]oct-6-ène-6-yl]benzènesulfonamide ;
4-[7-(4-méthylphényl)spiro[3.4]oct-6-ène-6-yl]benzènesulfonamide ;
4-[7-(4-méthoxyphényl)spiro[3.4]oct-6-ène-6-yl]benzènesulfonamide ;
4-[7-(4-méthylthiophényl)spiro[3.4]oct-6-ène-6-yl]benzènesulfonamide ;
4-[7-(4-cyanophényl)spiro[3.4]oct-6-ène-6-yl]benzènesulfonamide;
4-[7-(4-trifluorométhylphényl)spiro[3.4]oct-6-ène-6-yl]benzènesulfonamide ;
2-phényl-3-[4-(méthylsulfonyl)phényl]spiro[4.4]non-2-ène ;
2-(4-fluorophényl)-3-[4(méthylsulfonyl)phényl]spiro[4.4]non-2-ène ;
2-(4-chlorophényl)-3-[4-(méthylsulfonyl)phényl]spiro[4 4]non-2-ène
2-(4-méthylphényl)-3-[4-(méthylsulfonyl)phényl]spiro[4.4]non-2-ène;
2-(4-méthoxyphényl)-3-[4-(méthylsulfonyl)phényl]spiro[4.4]non-2-ène ;
2-(4-méthylthiophényl)-3-[4-(méthylsulfonyl)phényl]spiro[4.4]non-2-ène ;
2-(4-cyanophényl)-3-[4(méthylsulfonyl)phényl]spiro[4.4]non-2-ène ;
2-(4-trifluorométhylphényl)-3-[4-(méthylsulfonyl)phényl]spiro[4.4]non-2-ène;
4-(3-phénylspiro[4.4]non-2-ène-2-yl)benzènesulfonamide ;
4-[3-(4-fluorophényl)spiro[4.4]non-2-ène-2-yl)benzènesulfonamide ;
4-[3-(4-chlorophényl)spiro[4.4]non-2-ène-2-yl)benzènesulfonamide ;
4-[3-(4-méthylphényl)spiro[4.4]non-2-ène-2-yl)benzènesulfonamide ;
4-[3-(4-méthoxyphényl)spiro[4.4]non-2-ène-2-yl)benzènesulfonamide ;
4-[3-(4-méthylthiophényl)spiro[4.4]non-2-ène-2-yl)benzènesulfonamide ;
4-[3-(4-cyanophényl)spiro[4.4]non-2-ène-2-yl)benzènesulfonamide ;
4-[3-(4-trifluorométhylphényl)spiro[4.4]non-2-ène-2-yl)benzènesulfonamide ;
5-(3-méthyl-4-fluorophényl)-6-[4-(méthylsulfonyl)phényl]spiro[2.4]hept-5-ène ;
5-(3-trifluorométhyl-4-fluorophényl)-6-[4-(méthylsulfonyl)phényl]spiro[2.4]hept-5-ène ;
5-(3-méthyl-4-chlorophényl)-6-[4-(méthylsulfonyl)phényl]spiro[2.4]hept-5-ène;
5-(3-trifluorométhyl-4-chlorophényl)-6-[4-(méthylsulfonyl)phényl]spiro[2.4]hept-5-ène ;
5-(3-méthyl-4-méthoxyphényl)-6-[4-(méthylsulfonyl)phényl]spiro[2.4]hept-5-ène ;
5-(3-trifluorométhyl-4-méthoxyphényl)-6-[4-(méthylsulfonyl)phényl]spiro[2.4]hept-5-ène;
5-(3-fluoro-4-méthoxyphényl)-6-[4-(méthylsulfonyl)phényl]spiro[2.4]hept-5-ène;
5-(3-chloro-4-méthoxyphényl)-6-[4-(méthylsulfonyl)phényl]spiro[2.4]hept-5-ène;
5-(4-méthoxy-2,3,4,6-tétrafluorophényl)-6-[4-(méthylsulfonyl)phényl]spiro[2.4]hept-5-ène ;
5-(3,4-diméthoxyphényl)-6-[4-(méthylsulfonyl)phényl]spiro[2.4]hept-5-ène;
5-(3-chloro-4-fluorophényl)-6-[4-(méthylsulfonyl)phényl]spiro[2.4]hept-5-ène ;
5-(4-chloro-3-fluorophényl)-6-[4-(méthylsulfonyl)phényl]spiro[2.4]hept-5-ène;
5-(3,4-difluorophényl)-6-[4-(méthylsulfonyl)phényl]spiro[2.4]hept-5-ène;
5-(3,4-dichlorophényl)-6-[4-(méthylsulfonyl)phényl]spiro[2.4]hept-5-ène ;
4-[6-(3-trifluorométhyl-4-fluorophényl)spiro[2.4]hept-5-ène-5-yl]benzènesulfonamide ;
4-[6-(3-trifluorométhyl-4-chlorophényl)spiro[2.4]hept-5-ène-5-yl]benzènesulfonamide ;
4-[6-(3-méthyl-4-fluorophényl)spiro[2.4]hept-5-ène-5-yl]benzènesulfonamide ;
4-[6-(3-méthyl-4-chlorophényl)spiro[2.4]hept-5-ène-5-yl]benzènesulfonamide;
4-[6-(4-méthoxy-2,3,5,6-tétrafluorophényl)spiro[2.4]hept-5-ène-5-yl]benzènesulfonamide ;
4-[6-(3-fluoro-4-méthoxyphényl)spiro[2.4]hept-5-ène-5-yl]benzènesulfonamide ;
4-[6-(3-trifluorométhyl-4-méthoxyphényl)spiro[2.4]hept-5-ène-5-yl]benzènesulfonamide ;
4-[6-(3-méthyl-4-méthoxyphényl)spiro[2.4]hept-5-ène-5-yl]benzènesulfonamide;
4-[6-(3-chloro-4-méthoxyphényl)spiro[2.4]hept-5-ène-5-yl]benzènesulfonamide;
4-[6-(4-méthoxy-2,3,5,6-tétrafluorophényl)spiro[2.4]hept-5-ène-5-yl]benzènesulfonamide ;
4-[6-(3,4-diméthoxyphényl)spiro[2.4]hept-5-ène-5-yl]benzènesulfonamide;
4-[6-(3-chloro-4-fluorophényl)spiro[2.4]hept-5-ène-5-yl]benzènesulfonamide ;
4-[6-(4-chloro-3-fluorophényl)spiro[2.4]hept-5-ène-5-yl]benzènesulfonamide ;
4-[6-(3,4-difluorophényl)spiro[2.4]hept-5-ène-5-yl]benzènesulfonamide ; et
4-[6-(3,4-dichlorophényl)spiro[2.4]hept-5-ène-5-yl]benzènesulfonamide.

8. Composé selon la revendication 7, qui est le 5-(3,5-dichloro-4-méthoxyphényl)-6-[4-(méthylsulfonyl)phényl]spiro[2.4]hept-5-ène, ou un de ses sels acceptables en pharmacie.

9. Composé selon la revendication 7, qui est le 4-[6-(3-chloro-4-méthoxyphényl)spiro[2.4]hept-5-ène-5-yl]benzènesulfonamide, ou un de ses sels acceptables en pharmacie.

10. Composé selon la revendication 4,
dans lequel n est un nombre choisi parmi 0, 1 et 2 ;
dans lequel chacun de R¹, R², R⁴, R⁵ et R¹⁰ est un radical hydrido ;
dans lequel R³ est choisi parmi les radicaux alkylsulfonyle inférieur et aminosulfonyle ;
dans lequel R⁶ est choisi parmi les radicaux hydrido et halogéno ;
dans lequel R⁷ est choisi parmi les radicaux hydrido et halogéno ;
dans lequel R⁸ est choisi parmi les radicaux hydrido, halogéno, alkyle inférieur, alcoxy inférieur, halogénoalkyle inférieur, halogénoalcoxy inférieur, et hydroxyle ; et
dans lequel R⁹ est choisi parmi les radicaux hydrido, halogéno et alkyle inférieur ; ou dans lequel R⁸ et R⁹ forment ensemble méthylènedioxy ;
ou un sel acceptable en pharmacie d'un tel composé.

11. Composé selon la revendication 10, dans lequel R³ est un radical méthylsulfonyle ou aminosulfonyle ; dans lequel R⁶ est choisi parmi les radicaux hydrido, fluoro, chloro, bromo et iodo ; dans lequel R⁷ est choisi parmi les radicaux hydrido, fluoro, chloro, bromo et iodo ; dans lequel R⁸ est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, hydroxyle, fluorométhyle, difluorométhyle, trifluorométhyle, chlorométhyle, dichlorométhyle, trichlorométhyle, pentafluoroéthyle, heptafluoropropyle, difluorochlorcnéthyle, dichlorofluorométhyle, difluoroéthyle, difluoropropyle, dichloroéthyle, dichloropropyle, et trifluorométhoxy ; dans lequel R⁹ est choisi parmi les radicaux hydrido, fluoro, chloro, bromo, iodo, méthyle, éthyle, n-propyle, isopropyle, butyle, tert-butyle, et isobutyle ; ou dans lequel R⁸ et R⁹ lequel R¹¹ est un radical méthyle ou amino ; ou un sel acceptable en pharmacie d'un tel composé.

12. Composé selon la revendication 11, choisi parmi les composés, et leurs sels acceptables en pharmacie, de l'ensemble constitué par les suivants :
5-(3-chloro-4-méthoxyphényl)-6-[4-(méthylsulfonyl)phényl]spiro[2,4]hept-5-ène;
4-[6-(3-chloro-4-méthoxyphényl)spiro[2,4]hept-5-ène-5-yl]benzènesulfonamide ;
5-(3-fluoro-4-méthoxyphényl)-6-[4-(méthylsulfonyl)phényl]spiro[2,4]hept-5-ène;
4-[6-(3-fluoro-4-méthoxyphényl)spiro[2,4]hept-5-ène-5-yl]benzènesulfonamide ;
5-(3,4-difluorophényl)-6-[4-(méthylsulfonyl)phényl]spiro[2,4]hept-5-ène;
5-[6-[4-(méthylsulfonyl)phényl]spiro[2,4]hept-5-ène-5-yl]-1,3-benzodioxole ;
4-[6-(3,4-difluorophényl)spiro[2,4]hept-5-ène-5-yl]benzènesulfonamide ;
2,6-dichloro-4-[6-[4-(méthylsulfonyl)phényl]spiro[2,4]hept-5-ène-5-yl]phénol;
5-(4-trifluorométhoxyphényl)-6-[4-(méthylsulfonyl)phényl]spiro[2,4]hept-5-ène ;
5-(4-méthoxyphényl)-6-[4-(méthylsulfonyl)phényl]spiro[2,4]hept-5-ène ;
5-(3-bromo-4-méthoxyphényl)-6-[4-(méthylsulfonyl)phényl]spiro[2,4]hept-5-ène ;
4-[6-(4-méthoxyphényl)spiro[2,4]hept-5-ène-5-yl]benzènesulfonamide ;
4-[6-(3-bromo-4-méthoxyphényl)spiro[2,4]hept-5-ène-5-yl]benzènesulfonamide ;
5-(4-trifluorométhylphényl)-6-[4-(méthylsulfonyl)phényl]spiro[2,4]hept-5-ène;
5-(3,5-dichloro-4-méthoxyphényl)-6-[4-(méthylsulfonyl)phényl]spiro[2,4]hept-5-ène ;
4-[6-(4-trifluorométhoxyphényl)spiro[2,4]hept-5-ène-5-yl]benzènesulfonamide ;
5-(3-chloro-4-fluorophényl)-6-[4-(méthylsulfonyl)phényl]spiro[2,4]hept-5-ène;
5-(2,4-difluorophényl)-6-[4-(méthylsulfonyl)phényl]spiro[2,4]hept-5-ène;
5-(2,4-dichlorophényl)-6-[4-(méthylsulfonyl)phényl]spiro[2,4]hept-5-ène ;
4-[6-(4-trifluorométhylphényl)spiro[2,4]hept-5-ène-5-yl]benzènesulfonamide ;
4-[6-(3-chloro-4-fluorophényl)spiro[2,4]hept-5-ène-5-yl]benzènesulfonamide ;
5-(3,4-dichlorophényl)-6-[4-(méthylsulfonyl)phényl]spiro[2,4]hept-5-ène;
5-(4-chlorophényl)-6-[4-(méthylsulfonyl)phényl]spiro[2,4]hept-5-ène ;
4-[6-(3,4-dichlorophényl)spiro[2,4]hept-5-ène-5-yl]benzènesulfonamide ;
4-[6-(4-chlorophényl)spiro[2,4]hept-5-ène-5-yl]benzènesulfonamide ;
5-(3-chloro-4-méthylphényl)-6-[4-(méthylsulfonyl)phényl]spiro[2,4]hept-5-ène
5-(3,4-diméthylphényl)-6-[4-(méthylsulfonyl)phényl]spiro[2,4]hept-5-ène;
5-(4-méthylphényl)-6-[4-(méthylsulfonyl)phényl]spiro[2,4]hept-5-ène;
5-(3-méthyl-4-trifluorométhoxyphényl)-6-[4-(méthylsulfonyl)phényl]spiro[2,4]hept-5-ène ;
5-(3-chloro-4-trifluorométhoxyphényl)-6-[4-(méthylsulfonyl)phényl]spiro[2,4]hept-5-ène ;
4-[6-(3,5-dichloro-4-méthoxyphényl)spiro[2,4]hept-5-ène-5-yl]benzènesulfonamide ;
4-[6-(3-méthyl-4-trifluorométhoxyphényl)spiro[2,4]hept-5-ène-5-yl]benzènesulfonamide ;
4-[6-(3-chloro-4-trifluorométhoxyphényl)spiro[2,4]hept-5-ène-5-yl]benzènesulfonamide ;
5-(4-fluorophényl)-6-[4-(méthylsulfonyl)phényl]spiro[2.4]hept-5-ène;
4-[6-(4-fluorophényl)spiro[2.4]hept-5-ène-5-yl]benzènesulfonamide ;
6-(4-fluorophényl)-7-[4-(méthylsulfonyl)phényl]spiro[3.4]oct-5-ène ;
4-[7-(4-fluorophényl)spiro[3.4]oct-6-ène-6-yl]benzènesulfonamide ; et
2-(4-fluorophényl)-3-[4-(méthylsulfonyl)phényl]spiro[4.4]non-5-ène.

13. Composé selon la revendication 1, dans lequel A est 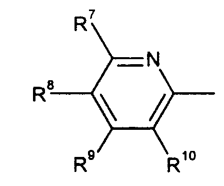
dans lequel n est un nombre choisi parmi 0, 1, 2 et 3 ; et
formule dans laquelle chacun de R¹ à R⁵ et R⁷ à R¹⁰ est indépendamment choisi parmi les radicaux hydrido, halogéno, alkyle, alcoxy, alkylthio, alkylamino, cyano, halogénoalkyle, halogénoalcoxy, hydroxyalkyle, halogénoalkyle, halogénoalcoxy, hydroxyalkyle, alcoxyalkyle, hydroxyle, mercapto, alkylsulfonyle, halogénoalkylsulfonyle et aminosulfonyle ;
ou un sel acceptable en pharmacie d'un tel composé.

14. Composé selon la revendication 13, dans lequel n est un nombre choisi parmi 0, 1 et 2 ; dans lequel chacun de R¹, R², R⁴, R⁵, R⁷, R⁹ et R¹⁰ est indépendamment choisi parmi les radicaux hydrido, halogéno, alkyle inférieur, alcoxy inférieur, alkylthio inférieur, cyano, halogénoalkyle inférieur, halogénoalcoxy inférieur, hydroxyalkyle inférieur, alcoxyalkyle inférieur, hydroxyle et mercapto ; et dans lequel R³ est choisi parmi les radicaux alkylsulfonyle inférieur et aminosulfonyle et R⁸ est choisi parmi les radicaux hydrido, halogéno, alkyle inférieur, alcoxy inférieur, alkylthio inférieur, alkylamino inférieur, halogénoalcoxy inférieur, hydroxyalkyle inférieur, mercapto, hydroxyle, alcoxyalkyle inférieur, cyano et halogénoalkyle inférieur ; ou dans lequel encore R⁸ et R⁹ forment ensemble méthylènedioxy ; ou dans lequel encore R³ est choisi parmi les radicaux hydrido, halogéno, alkyle inférieur, alcoxy inférieur, alkylthio inférieur, alkylamino inférieur, halogénoalcoxy inférieur, hydroxyalkyle inférieur, hydroxyle, mercapto, alcoxyalkyle inférieur, cyano et halogénoalkyle inférieur, et R⁸ est choisi parmi les radicaux alkylsulfonyle inférieur et aminosulfonyle ; ou dans lequel encore R³ et R⁴ forment ensemble méthylènedioxy ; ou un sèl acceptable en pharmacie d'un tel composé.

15. Composé selon la revendication 14, dans lequel chacun de R¹, R², R⁴, R⁵, R⁷, R⁹ et R¹⁰ est un radical hydrido, fluoro, chloro, bromo, iodo, méthyle, éthyle, n-méthoxy, éthoxy, propoxy, butoxy, hydroxyle, mercapto, méthylthio, éthylthio, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, méthylamino, N,N-diméthylamino, trifluorométhoxy, hydroxyméthyle, méthoxyméthyle et éthoxyméthyle ; et dans lequel R³ est un radical méthylsulfonyle ou aminosulfonyle, et R⁸ est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, trifluorométhoxy, méthylthio, éthylthio, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle et dichloropropyle ; ou dans lequel encore R⁸ et R⁹ forment ensemble méthylènedioxy ; ou dans lequel encore R³ est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, trifluorométhoxy, méthylthio, éthylthio, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle et dichloropropyle, et R⁸ est un radical méthylsulfonyle ou aminosulfonyle ; ou dans lequel encore R³ et R⁴ forment ensemble méthylènedioxy ; ou un sel acceptable en pharmacie d'un tel composé.

16. Composé selon la revendication 15, choisi parmi les composés, et leurs sels acceptables en pharmacie, de l'ensemble constitué par les suivants :
2-[6-[4-(méthylsulfonyl)phényl]spiro[2.4]hept-5-ène-5-yl]pyridine ;
5-fluoro-2-[6-[4-(méthylsulfonyl)phényl]spiro[2.4]hept-5-ène-5-yl]pyridine ;
5-chloro-2-[6-[4-(méthylsulfonyl)phényl]spiro[2.4]hept-5-ène-5-yl]pyridine ;
5-méthyl-2-[6-[4-(méthylsulfonyl)phényl]spiro[2.4]hept-5-ène-5-yl]pyridine ;
4-[6-(pyridine-2-yl)spiro[2.4]hept-5-ène-5-yl]benzènesulfonamide ;
4-[6-(5-fluoropyridine-2-yl)spiro[2.4]hept-5-ène-5-yl]benzènesulfonamide ;
4-[6-(5-chloropyridine-2-yl)spiro[2.4]hept-5-ène-5-yl]benzènesulfonamide ;
4-[6-(5-méthylpyridine-2-yl)spiro[2.4]hept-5-ène-5-yl]benzènesulfonamide ;
2-[7-[4-(méthylsulfonyl)phényl]spiro[3.4]oct-6-ène-6-yl]pyridine ;
5-fluoro-2-[7-[4-(méthylsulfonyl)phényl]spiro[3.4]oct-6-ène-6-yl]pyridine ;
5-chloro-2-[7-[4-(méthylsulfonyl)phényl]spiro[3.4]oct-6-ène-6-yl]pyridine ;
5-méthyl-2-[7-[4-(méthylsulfonyl)phényl]spiro[3.4]oct-6-ène-6-yl]pyridine ;
4-[7-(pyridine-2-yl)spiro[3.4]oct-6-ène-6-yl]benzènesulfonamide ;
4-[7-(5-fluoropyridine-2-yl)spiro[3.4]oct-6-ène-6-yl]benzènesulfonamide;
4-[7-(5-chloropyridine-2-yl)spiro[3.4]oct-6-ène-6-yl]benzènesulfonamide;
4-[7-(5-méthylpyridine-2-yl)spiro[3.4]oct-6-ène-6-yl]benzènesulfonamide ;
2-[3-[4-(méthylsulfonyl)phényl]spiro[4.4]non-2-ène-2-yl]pyridine ;
5-fluoro-2-[3-[4-(méthylsulfonyl)phényl]spiro[4.4]non-2-ène-2-yl]pyridine ;
5-chloro-2-[3-[4-(méthylsulfonyl)phényl]spiro[4.4]non-2-ène-2-yl]pyridine ;
5-méthyl-2-[3-[4-(méthylsulfonyl)phényl]spiro[4.4]non-2-ène-2-yl]pyridine ;
4-[3-(pyridine-2-yl)spiro[4.4]non-2-ène-2-yl]benzènesulfonamide ;
4-[3-(5-fluoropyridine-2-yl)spiro[4.4]non-2-ène-2-yl]benzènesulfonamide ;
4-[3-(5-chloropyridine-2-yl)spiro[4.4]non-2-ène-2-yl]benzènesulfonamide ;
4-[3-(5-méthylpyridine-2-yl)spiro[4.4]non-2-ène-2-yl]benzènesulfonamide ;
2-(6-phénylspiro[2.4]hept-5-ényl)-5-(méthylsulfonyl)pyridine ;
2-[6-(4-fluorophényl)spiro[2.4]hept-5-ényl]-5-(méthylsulfonyl)pyridine ;
2-[6-(4-chlorophényl)spiro[2.4]hept-5-ényl]-5-(méthylsulfonyl)pyridine ;
2-[6-(4-méthylphényl)spiro[2.4]hept-5-ényl]-5-(méthylsulfonyl)pyridine ;
2-(6-phénylspiro[2.4]hept-5-ène-5-yl)-5-pyridinesulfonamide ;
2-[6-(4-fluorophényl)spiro[2.4]hept-5-ène-5-yl)-5-pyridinesulfonamide ;
2-[6-(4-chlorophényl)spiro[2.4]hept-5-ène-5-yl)-5-pyridinesulfonamide ;
2-[6-(4-méthylphényl)spiro[2.4]hept-5-ène-5-yl)-5-pyridinesulfonamide ;
2-(7-phénylspiro[3.4]oct-6-ène-6-yl)-5-(méthylsulfonyl)pyridine ;
2-[7-(4-fluorophényl)spiro[3.4]oct-6-ène-6-yl]-5-(méthylsulfonyl)pyridine ;
2-[7-(4-chlorophényl)spiro[3.4]oct-6-ène-6-yl]-5-(méthylsulfonyl)pyridine ;
2-[7-(4-méthylphényl)spiro[3.4]oct-6-ène-6-yl]-5-(méthylsulfonyl)pyridine ;
2-(7-phénylspiro[3.4]oct-6-ène-6-yl)-5-pyridinesulfonamide ;
2-[7-(4-fluorophényl)spiro[3.4]oct-6-ène-6-yl]-5-pyridinesulfonamide ;
2-[7-(4-chlorophényl)spiro[3.4]oct-6-ène-6-yl]-5-pyridinesulfonamide ;
2-[7-(4-méthylphényl)spiro[3.4]oct-6-ène-6-yl]-5-pyridinesulfonamide ;
2-(3-phénylspiro[4.4]non-2-ène-2-yl)-5-(méthylsulfonyl)pyridine ;
2-[3-(4-fluorophényl)spiro[4.4]non-2-ène-2-yl]-5-(méthylsulfonyl)pyridine ;
2-[3-(4-chlorophényl)spiro[4.4]non-2-ène-2-yl]-5-(méthylsulfonyl)pyridine ;
2-[3-(4-méthylphényl)spiro[4.4]non-2-ène-2-yl]-5-(méthylsulfonyl)pyridine ;
2-(3-phénylspiro[4.4]non-2-ène-2-yl)-5-pyridinesulfonamide ;
2-[3-(4-fluorophényl)spiro[4.4]non-2-ène-2-yl]-5-pyridinesulfonamide ;
2-[3-(4-chlorophényl)spiro[4.4]non-2-ène-2-yl]-5-pyridinesulfonamide ; et
2-[3-(4-méthylphényl)spiro[4.4]non-2-ène-2-yl]-5-pyridinesulfonamide.

17. Composé selon la revendication 1, dans lequel A est 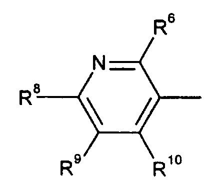
dans lequel n est un nombre choisi parmi 0, 1, 2 et 3 ; et
formule dans laquelle chacun de R¹ à R⁶ et R⁸ à R¹⁰ est indépendamment choisi parmi les radicaux hydrido, halogéno, alkyle, alcoxy, alkylthio, alkylamino, cyano, halogénoalkyle, halogénoalcoxy, hydroxyalkyle, alcoxyalkyle, hydroxyle, mercapto, alkylsulfonyle et aminosulfonyle ;
ou un sel acceptable en pharmacie d'un tel composé.

18. Composé selon la revendication 17, dans lequel n est un nombre choisi parmi 0, 1 et 2 ; dans lequel chacun de R¹, R², R⁴, R⁵, R⁶, R⁹ et R¹⁰ est indépendamment choisi parmi les radicaux hydrido, halogéno, alkyle inférieur, alcoxy inférieur, alkylthio inférieur, cyano, halogénoalkyle inférieur, halogénoalcoxy inférieur, hydroxyalkyle inférieur, alcoxyalkyle - inférieur, hydroxyle et mercapto ; et dans lequel R³ est choisi parmi les radicaux alkylsulfonyle inférieur et aminosulfonyle et R⁸ est choisi parmi les radicaux hydrido, halogéno, alkyle inférieur, alcoxy inférieur, alkylthio inférieur, alkylamino inférieur, halogénoalcoxy inférieur, hydroxyalkyle inférieur, mercapto, hydroxyle, alcoxyalkyle inférieur, cyano et halogénoalkyle inférieur ; ou dans lequel encore R⁸ et R⁹ forment ensemble méthylènedioxy ; ou dans lequel encore R³ est choisi parmi les radicaux hydrido, halogéno, alkyle inférieur, alcoxy inférieur, alkylthio inférieur, alkylamino inférieur, halogénoalcoxy inférieur, hydroxyalkyle inférieur, hydroxyle, alcoxyalkyle inférieur, cyano et halogénoalkyle inférieur, et R⁸ est choisi parmi les radicaux alkylsulfonyle inférieur et aminosulfonyle ; ou dans lequel encore R³ et R⁴ forment ensemble méthylènedioxy ; ou un sel acceptable en pharmacie d'un tel composé.

19. Composé selon la revendication 18, dans lequel chacun de R¹, R², R⁴, R⁵, R⁶, R⁹ et R¹⁰ est un radical hydrido, fluoro, chloro, bromo, iodo, méthyle, éthyle, n-propyle, isopropyle, butyle, tert-butyle, isobutyle, méthoxy, éthoxy, propoxy, butoxy, hydroxyle, mercapto, méthylthio, éthylthio, cyano, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, trifluorométhoxy, hydroxyméthyle, méthoxyméthyle et éthoxyméthyle ; et dans lequel R³ est un radical méthylsulfonyle ou aminosulfonyle, et R⁸ est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, trifluorométhoxy, hydroxyle, méthylthio, éthylthio, méthylamino, N,N-diméthylamino, cyano, mercapto, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle et dichloropropyle ; ou dans lequel encore R⁸ et R⁹ forment ensemble méthylènedioxy ; ou dans lequel encore R³ est choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, trifluorométhoxy, hydroxyle, méthylthio, éthylthio, cyano, mercapto, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle et dichloropropyle, et R⁸ est un radical méthylsulfonyle ou aminosulfonyle ; ou dans lequel encore R³ et R⁴ forment ensemble méthylènedioxy ; ou un sel acceptàble en pharmacie d'un tel composé.

20. Composé selon la revendication 19, choisi parmi les composés, et leurs sels acceptables en pharmacie, de l'ensemble constitué par les suivants :
5-[6-[4-(méthylsulfonyl)phényl]spiro[2.4]hept-5-ène-5-yl]pyridine ;
2-fluoro-5-[6-[4-(méthylsulfonyl)phényl]spiro[2.4]hept-5-ène-5-yl]pyridine ;
2-chloro-5-[6-[4-(méthylsulfonyl)phényl]spiro[2.4]hept-5-ène-5-yl]pyridine ;
2-méthyl-5-[6-[4-(méthylsulfonyl)phényl]spiro[2.4]hept-5-ène-5-yl]pyridine ;
4-[6-(pyridine-5-yl)spiro[2.4]hept-5-ène-5-yl]benzènesulfonamide ;
4-[6-(2-fluoropyridine-5-yl)spiro[2.4]hept-5-ène-5-yl]benzènesulfonamide ;
4-[6-(2-chloropyridine-5-yl)spiro[2.4]hept-5-ène-5-yl]benzènesulfonamide ;
4-[6-(2-méthylpyridine-5-yl)spiro[2.4]hept-5-ène-5-yl]benzènesulfonamide ;
5-[7-[4-(méthylsulfonyl)phényl]spiro[3.4]oct-6-ène-6-yl]pyridine ;
2-fluoro-5-[7-[4-(méthylsulfonyl)phényl]spiro[3.4]oct-6-ène-6-yl]pyridine ;
2-chloro-5-[7-[4-(méthylsulfonyl)phényl]spiro[3.4]oct-6-ène-6-yl]pyridine ;
2-méthyl-5-[7-[4-(méthylsulfonyl)phényl]spiro[3.4]oct-6-ène-6-yl]pyridine ;
4-[7-(pyridine-5-yl)spiro[3.4]oct-6-ène-6-yl]benzènesulfonamide ;
4-[7-(2-fluoropyridine-5-yl)spiro[3.4]oct-6-ène-6-yl]benzènesulfonamide ;
4-[7-(2-chloropyridine-5-yl)spiro[3.4]oct-6-ène-6-yl]benzènesulfonamide ;
4-[2-(2-méthylpyridine-5-yl)spiro[3.4]oct-6-ène-6-yl]benzènesulfonamide ;
5-[3-[4-(méthylsulfonyl)phényl]spiro[4.4]non-2-ène-2-yl]pyridine ;
2-fluoro-5-[3-[4-(méthylsulfonyl)phényl]spiro[4.4]non-2-ène-2-yl]pyridine ;
2-chloro-5-[3-[4-(méthylsulfonyl)phényl]spiro[4.4]non-2-ène-2-yl]pyridine ;
2-méthyl-5-[3-[4-(méthylsulfonyl)phényl]spiro[4.4]non-2-ène-2-yl]pyridine ;
4-[3-(pyridine-5-yl)spiro[4.4]non-2-ène-2-yl]benzènesulfonamide ;
4-[3-(2-fluoropyridine-5-yl)spiro[4.4]non-2-ène-2-yl]benzènesulfonamide ;
4-(3-(2-chloropyridine-5-yl)spiro[4.4]non-2-ène-2-yl]benzènesulfonamide ;
4-[3-(2-méthylpyridine-5-yl)spiro[4.4]non-2-ène-2-yl]benzènesulfonamide ;
5-(6-phénylspiro[2.4]hept-5-ène-5-yl)-2-(méthylsulfonyl)pyridine ;
5-[6-(4-fluorophényl)spiro[2.4]hept-5-ène-5-yl]-2-(méthylsulfonyl)pyridine ;
5-[6-(4-chlorophényl)spiro[2.4]hept-5-ène-5-yl]-2-(méthylsulfonyl)pyridine ;
5-[6-(4-méthylphényl)spiro[2.4]hept-5-ène-5-yl]-2-(méthylsulfonyl)pyridine ;
5-(6-phénylspiro[2.4]hept-5-ène-5-yl)-2-pyridinesulfonamide ;
5-[6-(4-fluorophényl)spiro[2.4]hept-5-ène-5-yl]-2-pyridinesulfonamide ;
5-[6-(4-chlorophényl)spiro[2.4]hept-5-ène-5-yl]-2-pyridinesulfonamide ;
5-[6-(4-méthylphényl)spiro[2.4]hept-5-ène-5-yl]-2-pyridinesulfonamide ;
5-(7-phénylspiro[3.4]oct-6-ène-6-yl)-2-(méthylsulfonyl)pyridine ;
5-[7-(4-fluorophényl)spiro[3.4]oct-6-ène-6-yl]-2-(méthylsulfonyl)pyridine ;
4-[7-(4-chlorophényl)spiro[3.4]oct-6-ène-6-yl]-2-(méthylsulfonyl)pyridine ;
5-[7-(4-méthylphényl)spiro[3.4]oct-6-ène-6-yl]-2-(méthylsulfonyl)pyridine ;
5-(7-phénylspiro[3.4]oct-6-ène-6-yl)-2-pyridinesulfonamide
5-[7-(4-fluorophényl)spiro[3.4]oct-6-ène-6-yl]-2-pyridinesulfonamide ;
5-[7-(4-chlorophényl)spiro[3.4]oct-6-ène-6-yl]-2-pyridinesulfonamide ;
5-[7-(4-méthylphényl)spiro[3.4]oct-6-ène-6-yl]-2-pyridinesulfonamide ;
5-(3-phénylspiro[4.4]non-2-ène-2-yl)-2-(méthylsulfonyl)pyridine ;
5-[3-(4-fluorophényl)spiro[4.4]non-2-ène-2-yl]-2-(méthylsulfonyl)pyridine ;
5-[3-(4-chlorophényl)spiro[4.4]non-2-ène-2-yl]-2-(méthylsulfonyl)pyridine ;
5-[3-(4-méthylphényl)spiro[4.4]non-2-ène-2-yl]-2-(méthylsulfonyl)pyridine ;
5-(3-phénylspiro[4.4]non-2-ène-2-yl)-2-pyridinesulfonamide ;
5-[3-(4-fluorophényl)spiro[4.4]non-2-ène-2-yl]-2-pyridinesulfonamide ;
5-[3-(4-chlorophényl)spiro[4.4]non-2-ène-2-yl]-2-pyridinesulfonamide ; et
5-[3-(4-méthylphényl)spiro[4.4]non-2-ène-2-yl]-2-pyridinesulfonamide.

21. Composé selon la revendication 1, dans lequel A est 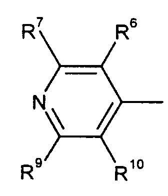
dans lequel n est un nombre choisi parmi 0, 1, 2 et 3 ; et
formule dans laquelle chacun de R¹ à R⁷, R⁹ et R¹⁰ est indépendamment choisi parmi les radicaux hydrido, halogéno, alkyle, alcoxy, alkylthio, cyano, halogénoalkyle, halogénoalcoxy, hydroxyalkyle, alcoxyalkyle, hydroxyle, mercapto, alkylsulfonyle et aminosulfonyle ;
ou un sel acceptable en pharmacie d'un tel composé.

22. Composé selon la revendication 21, dans lequel n est un nombre choisi parmi 0, 1 et 2 ; dans lequel chacun de R¹, R², R⁴ à R⁷, R⁹ et R¹⁰ est indépendamment choisi parmi les radicaux hydrido, halogéno, alkyle inférieur, alcoxy inférieur, alkylthio inférieur, halogénoalcoxy inférieur, hydroxyalkyle inférieur, hydroxyle, alcoxyalkyle inférieur, mercapto, cyano et halogénoalkyle inférieur ; et dans lequel R³ est choisi parmi les radicaux alkylsulfonyle inférieur et aminosulfonyle ; ou un sel acceptable en pharmacie d'un tel composé.

23. Composé selon la revendication 22, dans lequel chacun de R¹, R², R⁴ à R⁷, R⁹ et R¹⁰ est indépendamment choisi parmi les radicaux hydrido, fluoro, chloro, bromo, iodo, méthyle, éthyle, n-propyle, isopropyle, butyle, tert-butyle, isobutyle, méthoxy, éthoxy, propoxy, butoxy, trifluorométhoxy, méthylthio, éthylthio, cyano, hydroxyle, mercapto, fluorométhyle, difluorométhyle, trifluorométhyle, chlorométhyle, dichlorométhyle, trichlorométhyle, pentafluoroéthyle, heptafluoropropyle, difluorochlorométhyle, dichlorofluorométhyle, difluoroéthyle, difluoropropyle, dichloroéthyle, dichloropropyle, hydroxyméthyle, méthoxyméthyle et éthoxyméthyle ; et dans lequel R³ est un radical méthylsulfonyle ou aminosulfonyle ; ou un sel acceptable en pharmacie d'un tel composé.

24. Composé selon la revendication 23, choisi parmi les composés, et leurs sels acceptables en pharmacie, de l'ensemble constitué par les suivants :
4-[6-[4-(méthylsulfonyl)phényl]spiro[2.4]hept-5-ène-5-yl]pyridine ;
4-[6-(4-pyridinyl)spiro[2.4]hept-5-ène-5-yl]benzènesulfonamide ;
4-[7-[4-(méthylsulfonyl)phényl]spiro[3.4]oct-6-ène-6-yl]pyridine ;
4-[7-(4-pyridinyl)spiro[3.4]oct-6-ène-6-yl]benzènesulfonamide ;
4-[3-[4-(méthylsulfonyl)phényl]spiro[4.4]non-2-ène-2-yl]pyridine ; et
4-[3-(4-pyridinyl)spiro[4.4]non-2-ène-2-yl]benzènesulfonamide.

25. Composition pharmaceutique comprenant une quantité, efficace du point de vue pharmaceutique, d'un composé, ledit composé étant choisi parmi une famille de composés selon l'une des revendications 1 ou 4 à 10, 13, 17 ou 21 ou leurs sels acceptables en pharmacie.

26. Utilisation d'un composé selon l'une quelconque des revendications 1 ou 4 à 10, 13, 17 ou 21, pour préparer un médicament destiné au traitement d'une inflammation ou d'un trouble associé à une inflammation chez un sujet.

27. Utilisation selon la revendication 26, pour une utilisation dans le traitement d'une inflammation.

28. Utilisation selon la revendication 26, pour une utilisation dans le traitement d'un trouble associé à une inflammation.

29. Utilisation selon la revendication 28, dans laquelle le trouble associé à l'inflammation est une arthrite.

30. Utilisation selon la revendication 28, dans laquelle le trouble associé à l'inflammation est une douleur.

31. Utilisation selon la revendication 28, dans laquelle le trouble associé à l'inflammation est une fièvre.
